# EUROPEAN PATENT APPLICATION

(11) **EP 3 919 080 A1**
(43) Date of publication of application: **08.12.2021**
(21) Application number: 19912455.3
(22) Date of filing: 03.06.2019
(51) Int. Cl.: A61K 47/60, A61K 9/08, A61K 47/02, A61K 45/00, A61P 35/00

(54) **POLYETHYLENE GLYCOL CONJUGATE MEDICAMENT, PREPARATION METHOD THERFOR AND USE THEREOF**

(30) Priority: 03.02.2019 CN 201910108226
(71) Applicant: Chongqing Upgra Biotechnology Co., Ltd., Chongqing 401120 (CN)
(72) Inventor: LI, Gaoquan, Chongqing 401120 (CN); LI, Dajun, Chongqing 401120 (CN); ZHANG, Qian, Chongqing 401120 (CN); DING, Xiaoling, Chongqing 401120 (CN); LI, Diedie, Chongqing 401120 (CN); HUANG, Lei, Chongqing 401120 (CN); WAN, Jinping, Chongqing 401120 (CN); LIU, Yue, Chongqing 401120 (CN); WEI, Yusong, Chongqing 401120 (CN); YANG, Xiangwei, Chongqing 401120 (CN); PENG, Yongchen, Chongqing 401120 (CN); LIU, Jiao, Chongqing 401120 (CN); LI, Jianhuan, Chongqing 401120 (CN); LUO, Qiang, Chongqing 401120 (CN); LIU, Xi, Chongqing 401120 (CN); WANG, Bin, Chongqing 401120 (CN); GONG, Hui, Chongqing 401120 (CN); CHEN, Yu, Chongqing 401120 (CN); JIANG, Yunjie, Chongqing 401120 (CN); LIU, Zhongquan, Chongqing 401120 (CN); XU, Kun, Chongqing 401120 (CN); LOU, Jie, Chongqing 401120 (CN); CHEN, Huiyu, Chongqing 401120 (CN); YANG, Yue, Chongqing 401120 (CN); WANG, Yuanqiang, Chongqing 401120 (CN)
(74) Representative: Nony
(86) International application number: PCT/CN2019/089835
(87) International publication number: WO 2020/155497

(57) **Abstract**

The disclosure relates to the technical field of medicine and in particular to a polyethylene glycol conjugated drug of formula I or a pharmaceutically acceptable salt thereof. The disclosure further relates to a method for preparation and an intermediate of the polyethylene glycol conjugated drug or a pharmaceutically acceptable salt thereof, a pharmaceutical composition comprising the polyethylene glycol conjugated drug or a pharmaceutically acceptable salt thereof, and use of the polyethylene glycol conjugated drug or a pharmaceutically acceptable salt thereof in preparation of a medicament.

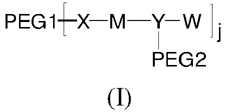

## Description

### TECHNICAL FIELD

The disclosure belongs to the technical field of medicine, and relates to a polyethylene glycol conjugated drug, method for preparation thereof and use thereof.

### BACKGROUND

Polymer-conjugated drug is an important direction in drug research. The water solubility of drug molecules can be increased by conjugation with polymer, which is very important for molecules with extremely low solubility such as paclitaxel, camptothecin or platinum. Most small-molecule drugs can only stay in the blood circulation for a few minutes, while polymer-conjugated drugs can stay for tens or hundreds of hours or even longer, which is beneficial to producing or enhancing the "enhanced penetration and retention" effect, i.e., the EPR effect, caused by capillary leak at the diseased site. Due to the increased hydrodynamic volume of polymer-conjugated drugs, the renal elimination of the drugs is weakened, the drugs are protected from enzymatic degradation, the half-life of the drugs in plasma is extended, and the bioavailability of the drugs is increased. Moreover, the drugs can be highly enriched in diseased organs, tissues or cells through EPR passive targeting or active targeting, thereby greatly reducing the toxic side effects caused by small molecule drugs spreading all over the body. In addition, polymer-conjugated drugs can limit the cell absorption of drugs to the endocytic pathway, which is conducive to drug delivery to the lysosome, thereby avoiding drug resistance caused by p-glycoprotein pumping; polymer-conjugated drugs can also stimulate or restore immune function, and this is conducive to killing cancer cells for anti-cancer conjugated drugs.

Polyethylene glycol conjugated drugs are one of the most successful directions for polymer-conjugated drugs. At present, there are 12 polyethylene glycol conjugated drugs which have been approved by the FDA to enter the market, and half of them are related to cancers. The ENZON Pharmaceuticals Company in the United States has developed polyethylene glycol conjugated SN-38 (ENZN-2208) and promoted it to phase III clinical trials. In addition, the NEKTAR THERAPEUTICS Company in the United States has developed polyethylene glycol conjugated irinotecan (NKTR-102), for which an NDA has been submitted with the EU and a phase III clinical trial has also been conducted in the United States, and it has a special effect on breast cancer that metastasizes to the brain.

### SUMMARY

The existing polyethylene glycol conjugated drugs have the following problems: with the increase of drug loading, the dissolution of polyethylene glycol conjugated drugs (especially four-arm or eight-arm polyethylene glycol conjugated drugs) in normal saline will usually be severely deteriorated, or even the polyethylene glycol conjugated drugs become completely insoluble, which limits their concentration in liquid preparations (e.g., injections with normal saline as a carrier), thus bringing inconvenience to the application of the drug.

According to the disclosure, by coupling a single-arm polyethylene glycol to a specific position on polyethylene glycol conjugated drugs (such as four-arm or eight-arm polyethylene glycol conjugated drugs), the solubility of polyethylene glycol conjugated drugs in normal saline can be greatly improved, and thus the above technical problems can be solved.

### Polyethylene glycol conjugated drug

According to one aspect, the present application relates to a polyethylene glycol conjugated drug of formula I or a pharmaceutically acceptable salt thereof;

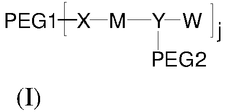

In the polyethylene glycol conjugated drug of the disclosure, multiple identical or different drug molecules are conjugated together by using an amino acid or a polypeptide as a linking chain, and a dicarboxylic acid with an amino group (for example, a natural amino acid with two carboxyl groups) as a linking bridge. The type, ratio and drug loading of the drug may be adjusted. The introduction of PEG2 can greatly improve the solubility of the polyethylene glycol conjugated drug in normal saline.

In formula (I), PEG1 is a single-arm or multi-arm (for example, four-arm or eight-arm) polyethylene glycol segment, and its number-average molecular weight may be 5k-40k, such as 5k-10k or 10k-40k, and may be, for example, about 5k, about 10k, about 20k, about 30k, or about 40k; j represents the number of arms of PEG1, such as 1, 4, or 8.

X represents a linking group which is 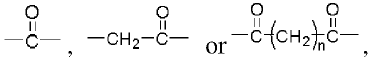 wherein n is 2 or 3. When j is greater than 1 (such as, 4 or 8), there are multiple (such as, 4 or 8) Xs at the same time, and in this case, the Xs are the same or different.

M represents an amino acid residue or polypeptide which is G, GG, GLG, GFA, GLA or GFLG. When j is greater than 1 (such as, 4 or 8), there are multiple (such as, 4 or 8) Ms at the same time, and in this case, the Ms are the same or different.

PEG2 represents a single-arm polyethylene glycol segment, which is linked to Y through an amide bond. In some embodiments, PEG2 reacts with a carboxyl group on Y through a terminal amino group to form an amide bond. Alternatively, PEG2 reacts with an amino group on Y through a terminal carboxyl group to form an amide bond. The molecular weight of PEG2 may be 2k-40k, such as 2k-3k, 3k-5k, 5k-10k or 10k-40k, and may be, for example, about 2k, about 3k about 5k, about 10k, or about 40k.

Y represents 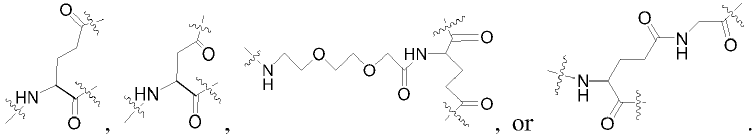 Alternatively, j may be greater than 1 (such as, 4 or 8), and in this case, there are multiple (such as, 4 or 8) Ys at the same time, and the Ys may be the same or different.

W may be Q, and its structure is 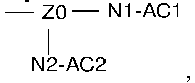 wherein Z0 may be selected from 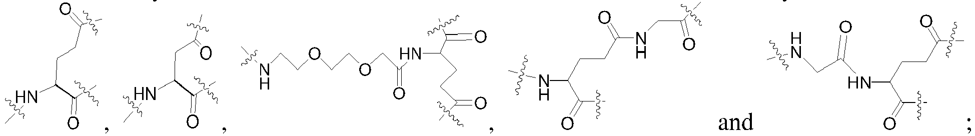 when there are multiple Z0s at the same time, the Z0s may be the same or different.

Q may comprise two identical drug molecules or two different drug molecules, that is, AC1 and AC2 may be the same or different. The two drug molecules are linked by an amino acid or a polypeptide as a linking chain (N1, N2), and dicarboxylic acid with an amino group as a linking bridge (Z0).

A natural amino acid with two carboxyl groups, such as glutamic acid, aspartic acid or a derivative thereof, may be selected as the linking bridge. The glutamic acid derivative or aspartic acid derivative may be a derivative formed by connecting a long chain with an amino group at the terminal to the amino group of glutamic acid or aspartic acid, such as Boc-NH-CH₂CH₂-O-CH₂CH₂-O-CH₂-CO-glutamic acid or Boc-NH-CH₂CH₂-O-CH₂CH₂-O-CH₂-CO-aspartic acid. The glutamic acid derivative or aspartic acid derivative may also be a dipeptide comprising glutamic acid or aspartic acid, such as glutamic acid glycine or glycine glutamic acid.

In the disclosure, when glutamic acid is used as the linking bridge Z0, the linking chain N linked to the carboxyl group at 5-position of the glutamic acid backbone is regarded as N1, and the linking chain linked to the carboxyl group at 1-position of the glutamic acid backbone is regarded as N2; accordingly, drug molecule AC linked to the linking chain N1 is regarded as AC1, and drug molecule AC linked to the linking chain N2 is regarded as AC2. Similarly, when aspartic acid is used as the linking bridge Z0, the linking chain N linked to the carboxyl group at 4-position of the aspartic acid backbone is regarded as N1, and the linking chain linked to the carboxyl group at 1-position of the aspartic acid backbone is regarded as N2; accordingly, drug molecule AC linked to the linking chain N1 is regarded as AC1, and drug molecule AC linked to the linking chain N2 is regarded as AC2. This pattern may also be applied to the case where a glutamic acid derivative or an aspartic acid derivative is used as the linking bridge Z0. Thus, Q may be selected from

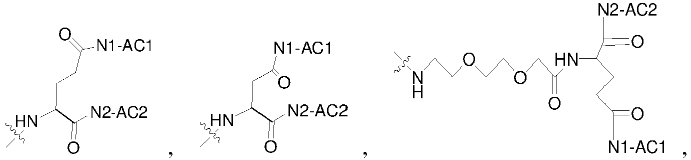

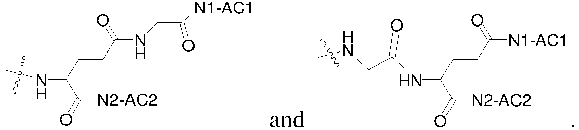

Furthermore, a higher-level intermediate comprising two or more drug molecules may be constructed with Q as the basic building unit and a dicarboxylic acid with an amino group as the linking bridge (Z1, Z2, Z3 or Z4). Therefore, W may also be selected from

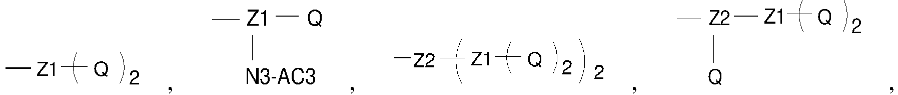

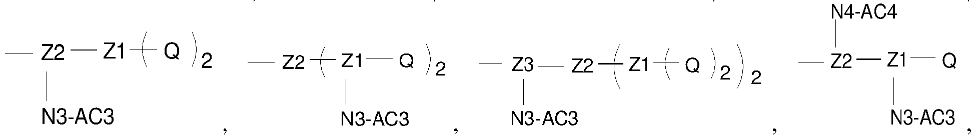

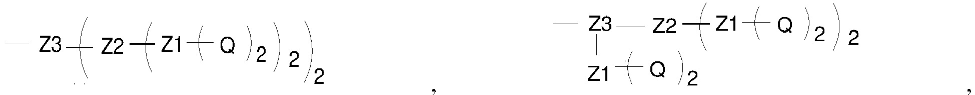

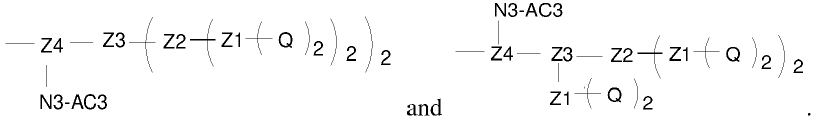

When j is greater than 1 (such as, 4 or 8), there are multiple (such as, 4 or 8) Ws at the same time, and in this case, the Ws may be the same or different.

Similar to Z0, a natural amino acid with two carboxyl groups, such as glutamic acid, aspartic acid or a derivative thereof, may be selected as the linking bridge Z1, Z2, Z3 or Z4. Therefore each of Z1, Z2, Z3 and Z4 independently is selected from 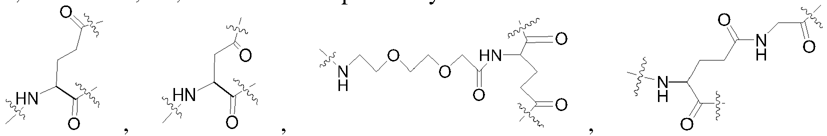 and

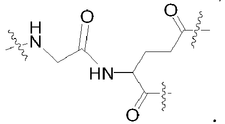

Z1, Z2, Z3, and Z4 may be the same or different, and when there are multiple Z1s, multiple Z2s, multiple Z3s, or multiple Z4s at the same time, the Z1s are the same or different, the Z2s are the same or different, the Z3s are the same or different or the Z4s are the same or different.

In some embodiments, Z1, Z2, Z3, or Z4 is glutamic acid. When the two intermediates linked by Z1, Z2, Z3, or Z4 have different structures, considering the steric hindrance, the bulkier intermediate may be selected to be linked to the carboxyl group at 5-position of the glutamic acid backbone. In some embodiments, Z1, Z2, Z3, or Z4 is aspartic acid. When the two intermediates linked by Z1, Z2, Z3, or Z4 have different structures, considering the steric hindrance, the bulkier intermediate may be selected to be linked to the carboxyl group at 4-position of the aspartic acid backbone. This pattern may also be applied to the case where a glutamic acid derivative or an aspartic acid derivative is used as the linking bridge Z1, Z2, Z3 or Z4.

N1, N2, N3, and N4 which are the linking chains between the drug molecule and the linking bridge may be amino acid residues or polypeptides. Each of N1, N2, N3 and N4 independently may be G, GG, GLG, GFA, GLA, or GFLG. N1, N2, N3, and N4 may be the same or different, and when there are multiple N1s, multiple N2s, multiple N3s, or multiple N4s at the same time, the N1s are the same or different, the N2s are the same or different, the N3s are the same or different or the N4s are the same or different.

AC1, AC2, AC3, and AC4 are drug molecules (for example, drug molecules with anti-tumor activity). AC1, AC2, AC3 and AC4 may be the same or different, and when there are multiple AC1s, multiple AC2s, multiple AC3s, or multiple AC4s at the same time, the AC1s are the same or different, the AC2s are the same or different, the AC3s are the same or different or the AC4s are the same or different.

In the disclosure, the active ingredient suitable for being conjugated with polyethylene glycol may be a drug molecule with at least one amino group, hydroxyl group, carboxyl group or acyl group, for example, a drug molecule having anti-tumor activity with at least one amino group, hydroxyl group, carboxyl group or acyl group, such as MK2, LPT, PCB, SB7, PKI, and NPB, which represent the following meanings:

| Abbreviation | Name | CAS number or structural formula |
|---|---|---|
| MK2 | MK-2206·2HCl | 1032350-13-2 |
| LPT | Lapatinib | 231277-92-2 |
| PCB | Palbociclib | 571190-30-2 |
| SB7 | SB-743921 | 940929-33-9 |
| PKI | Allosteric PKI-587 | 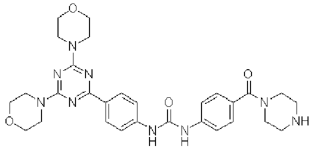 |
| NPB | Niraparib (MK-4827) | 1038915-60-4 |

In some embodiments, PEG1 is a single-arm polyethylene glycol segment with a number-average molecular weight of 5k-10k, or a four-arm polyethylene glycol segment with a number-average molecular weight of 10k-40k; X represents 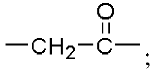 M represents G, GLG, or GFLG; the number-average molecular weight of PEG2 is 2k-3k or 3k-5k; Y represents 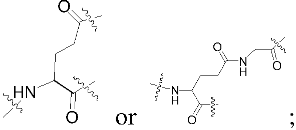
W is Q which is 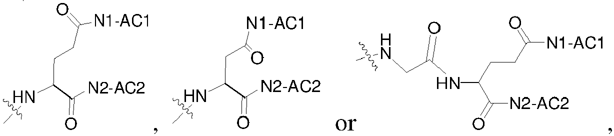
wherein N1 and N2 are both GFLG or GLG, and AC1 and AC2 are selected from:
a combination in which AC1 is MK2 and AC2 is LPT,
a combination in which AC1 is LPT and AC2 is SB7,
a combination in which AC1 is PCB and AC2 is SB7, and
a combination in which AC2 is SB7 and AC1 is PCB;
preferably, the polyethylene glycol conjugated drug is: 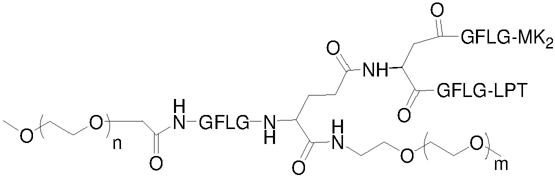
preferably, the number-average molecular weight of 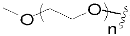 is 5k; preferably, the number-average molecular weight of 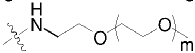 is 2k;

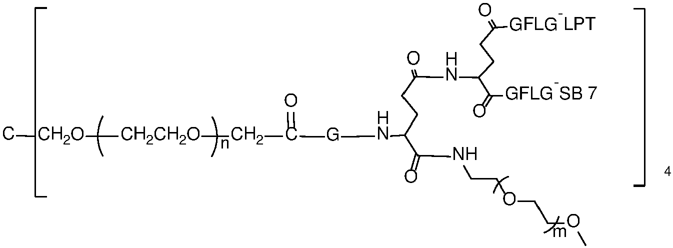

preferably, the number-average molecular weight of 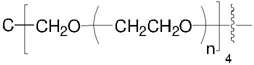 is 40k;
preferably, the number-average molecular weight of 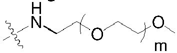 is 2k;

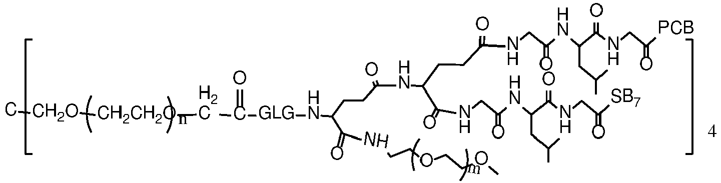

preferably, the number-average molecular weight of 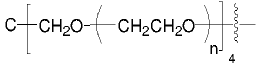 is 40k;
preferably, the number-average molecular weight of 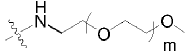 is 2k;

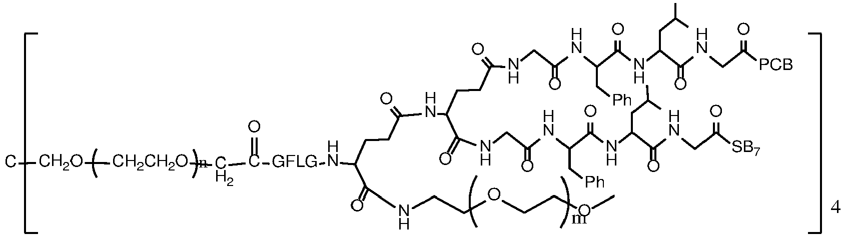

preferably, the number-average molecular weight of 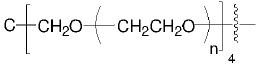 is 40k;
preferably, the number-average molecular weight of 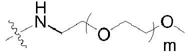 is 2k; or

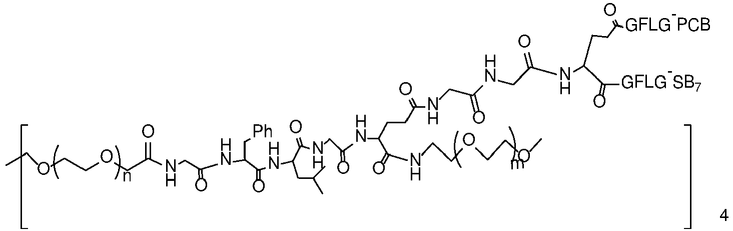

preferably, the number-average molecular weight of 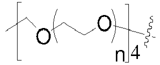 is 40k; preferably, the number-average molecular weight of 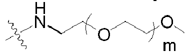 is 2k.

In some embodiments, PEG1 is a four-arm polyethylene glycol segment with a number-average molecular weight of 10k-40k; X represents 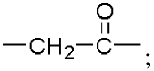 M represents GFLG; the number-average molecular weight of PEG2 is 2k-3k; Y represents 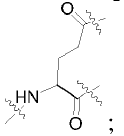
W represents 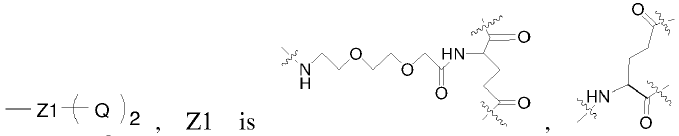 or 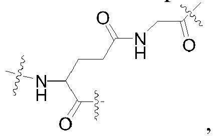 and Q is 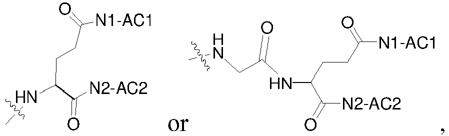
N1 and N2 are both GFLG, and AC1 and AC2 are selected from:
a combination in which AC1 and AC2 are both SB7,
a combination in which AC1 and AC2 are both LPT,
a combination in which AC1 is PCB and AC2 is SB7, and
a combination in which AC1 and AC2 are both PCB;
preferably, the polyethylene glycol conjugated drug is: 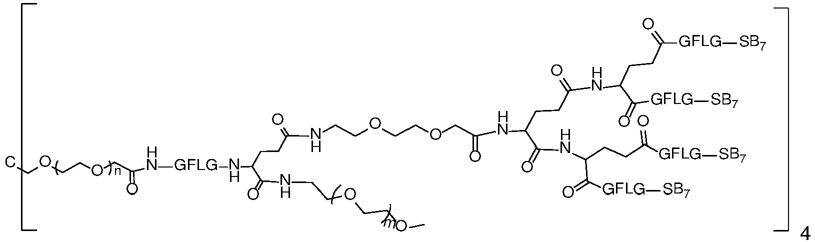
preferably, the number-average molecular weight of 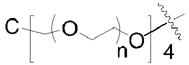 is 40k;
preferably, the number-average molecular weight of 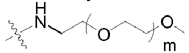 is 2k;

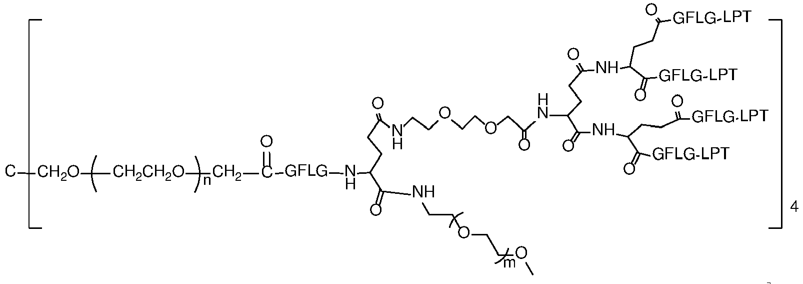

preferably, the number-average molecular weight of 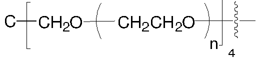 is 40k;
preferably, the number-average molecular weight of 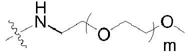 is 2k;

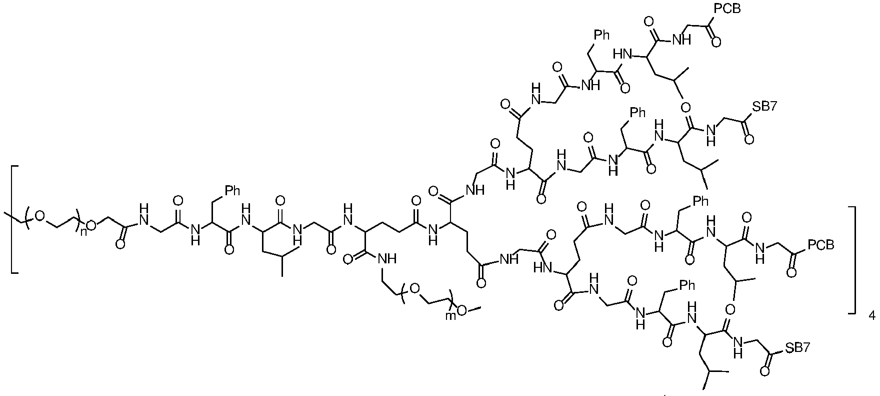

preferably, the number-average molecular weight of 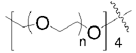 is 40k; preferably, the number-average molecular weight of 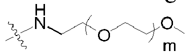 is 2k; or

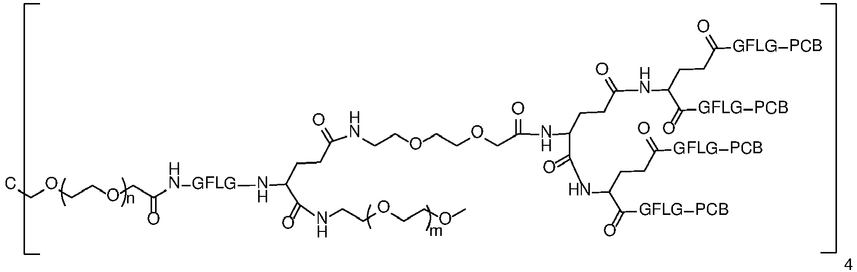

preferably, the number-average molecular weight of 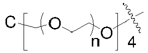 is 40k; preferably, the number-average molecular weight of 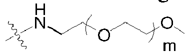 is 2k.

In some embodiments, PEG1 is a four-arm polyethylene glycol segment with a number-average molecular weight of 10k-40k; X represents 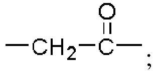 M represents GFLG; the number-average molecular weight of PEG2 is 2k-3k; Y represents 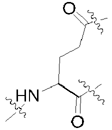 or 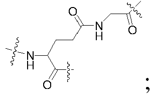
W represents 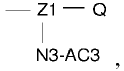 wherein Z1 is 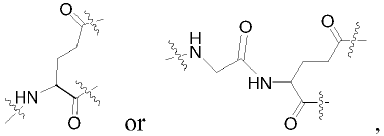 Q represents 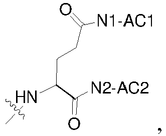 N1, N2, and N3 are all GFLG, and the combination of AC1, AC2, and AC3 is:
a combination in which AC1 is PCB, AC2 is SB7, and AC3 is PKI,
a combination in which AC1 and AC2 are PCB and AC3 is SB7,
a combination in which AC1 and AC2 are NPB and AC3 is SB7, or
a combination in which AC1 and AC2 are SB7 and AC3 is PCB;
preferably, the polyethylene glycol conjugated drug is: 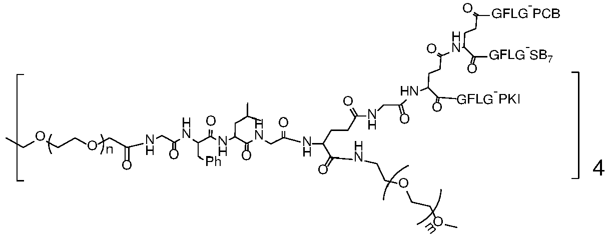
preferably, the number-average molecular weight of 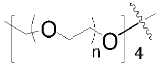 is 40k; preferably, the number-average molecular weight of 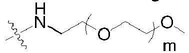 is 2k;

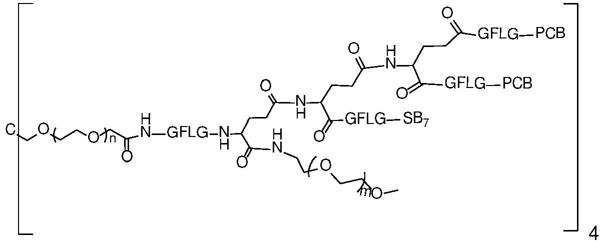

preferably, the number-average molecular weight of 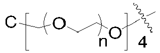 is 40k; preferably, the number-average molecular weight of 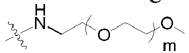 is 2k;

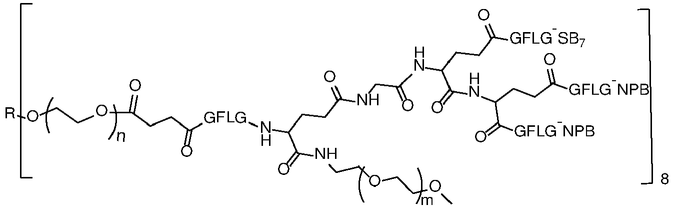

R is a core structure of eight-arm polyethylene glycol; preferably, the number-average molecular weight of 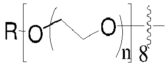 is 40k; preferably, the number-average molecular weight 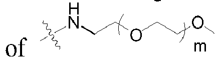 is 2k; or

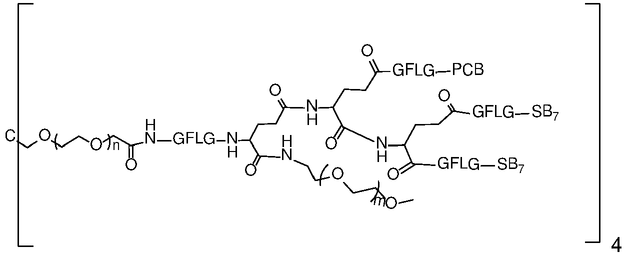

preferably, the number-average molecular weight of 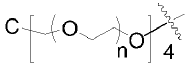 is 40k; preferably, the number-average molecular weight of 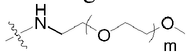 is 2k.

In some embodiments, PEG1 is a four-arm polyethylene glycol segment with a number-average molecular weight of 10k-40k; X represents 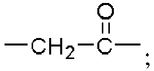 M represents GFLG; the number-average molecular weight of PEG2 is 2k-3k or 3k-5k; Y represents 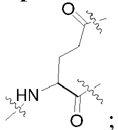
W represents 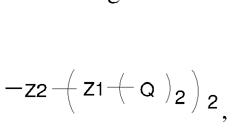 wherein Z2 is 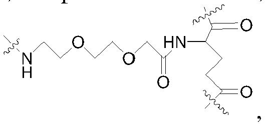 Z1 is 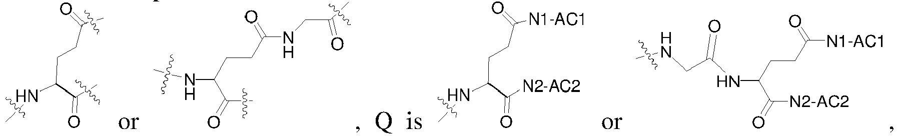 N1 and N2 are both GFLG, and AC1 and AC2 are selected from:
a combination in which AC1 is LPT and AC2 is SB7, and
a combination in which AC1 is PCB and AC2 is SB7;
preferably, the polyethylene glycol conjugated drug is:

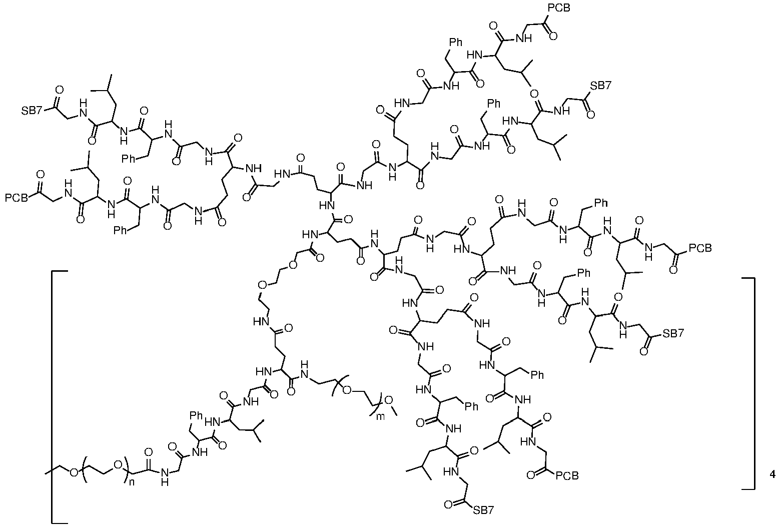

preferably, the number-average molecular weight of 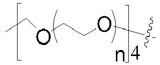 is 40k; preferably, the number-average molecular weight of 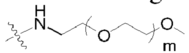 is 3k or 5k; or

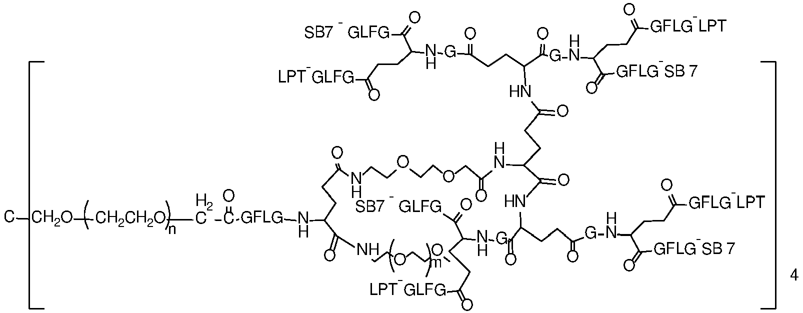

preferably, the number-average molecular weight of 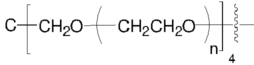 is 40k;
preferably, the number-average molecular weight of 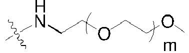 is 2k.

In some embodiments, PEG1 is a four-arm polyethylene glycol segment with a number-average molecular weight of 10k-40k; X represents 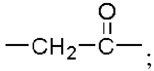 M represents GFLG; the number-average molecular weight of PEG2 is 2k-3k or 3k-5k; Y represents 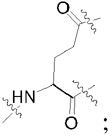
W represents 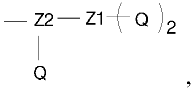 wherein Z2 is 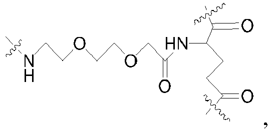 Z1 is 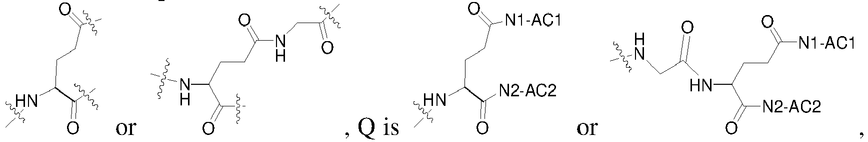 N1 and N2 are both GFLG, AC1 is PCB, and AC2 is SB7;
preferably, the polyethylene glycol conjugated drug is:

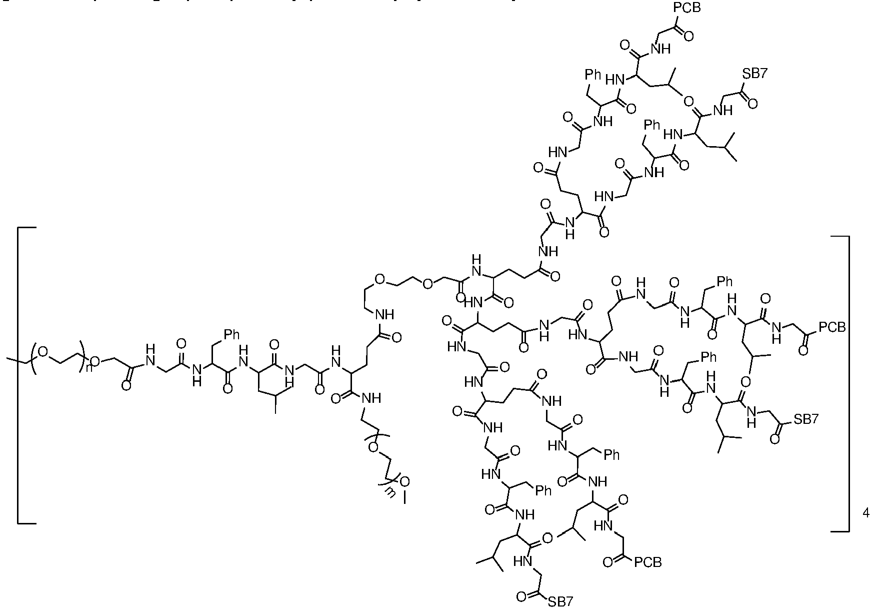

preferably, the number-average molecular weight of 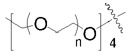 is 40k; preferably, the number-average molecular weight of 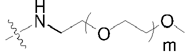 is 3k.

In some embodiments, PEG1 is a four-arm polyethylene glycol segment with a number-average molecular weight of 10k-40k; X represents 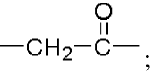 M represents GFLG; the number-average molecular weight of PEG2 is 2k-3k or 3k-5k; Y represents 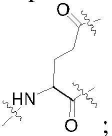
W represents 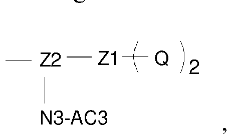 wherein Z2 is 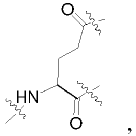 Z1 is 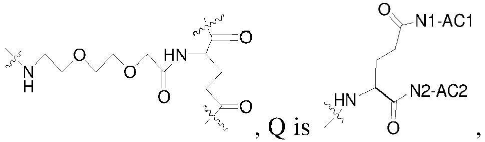 N1, N2 and N3 are all GFLG, AC1 and AC2 are both PCB, and AC3 is SB7;
preferably, the polyethylene glycol conjugated drug is: 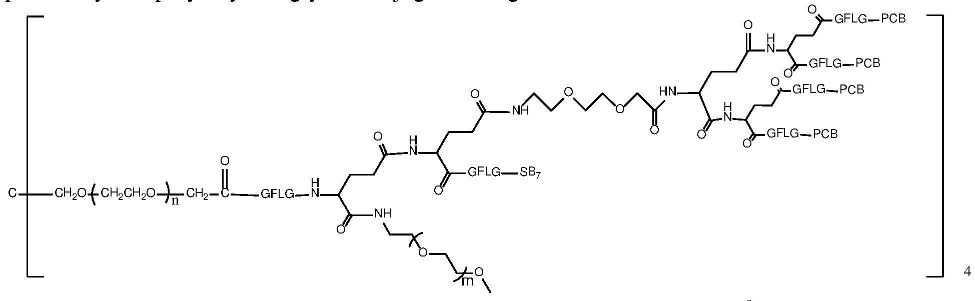
preferably, the number-average molecular weight of 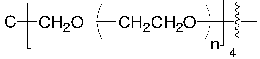 is 40k;
preferably, the number-average molecular weight of 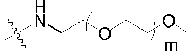 is 3k.

In some embodiments, PEG1 is a four-arm polyethylene glycol segment with a number-average molecular weight of 10k-40k; X represents 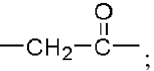 M represents GFLG; the number-average molecular weight of PEG2 is 2k-3k or 3k-5k; Y represents 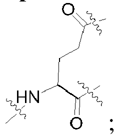
W represents 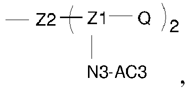 wherein Z2 and Z1 are 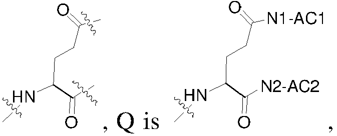 AC1 and AC2 are LPT, and AC3 is PKI;
preferably, the polyethylene glycol conjugated drug is:

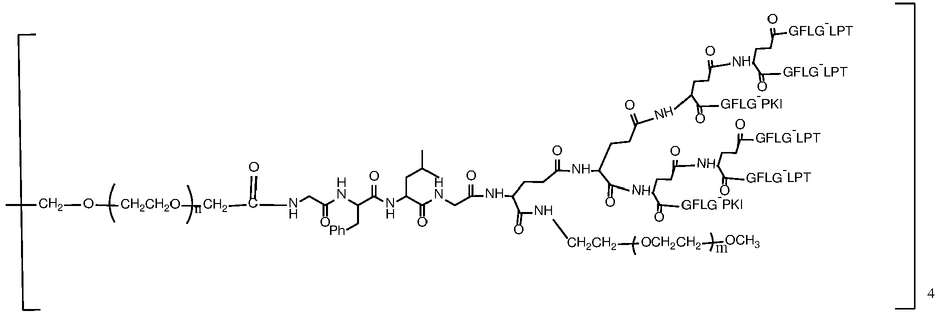

preferably, the number-average molecular weight of 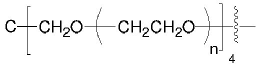 is 40k; preferably, the number-average molecular weight of 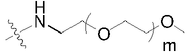 is 3k.

In some embodiments, PEG1 is a four-arm polyethylene glycol segment with a number-average molecular weight of 10k-40k; X represents 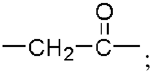 M represents GFLG; the number-average molecular weight of PEG2 is 2k-3k or 3k-5k; Y represents 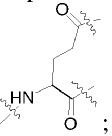
W represents 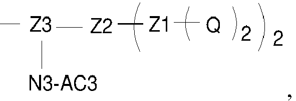 wherein Z3 is 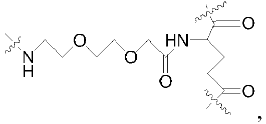 Z2 and Z1 are 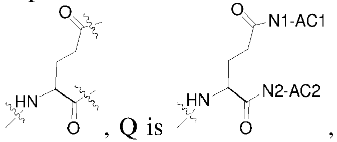 N1, N2 and N3 are GFLG, AC1 and AC2 are LPT, and AC3 is PCB;
preferably, the polyethylene glycol conjugated drug is:

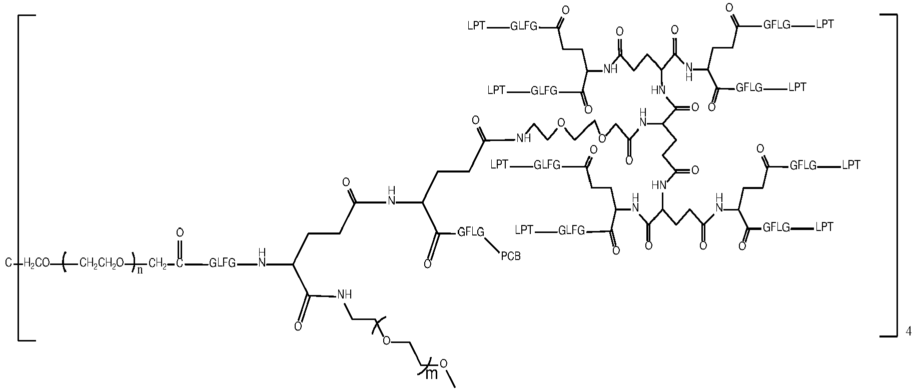

preferably, the number-average molecular weight of 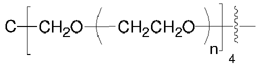 is 40k; preferably, the number-average molecular weight of 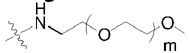 is 3k.

In some embodiments, PEG1 is a four-arm polyethylene glycol segment with a number-average molecular weight of 10k-40k; X represents 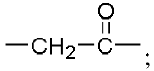 M represents GFLG; the number-average molecular weight of PEG2 is 2k-3k or 3k-5k; Y represents 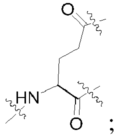 W represents 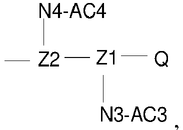 wherein Z2 is 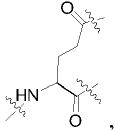 Z1 is 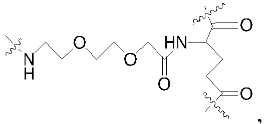 Q is 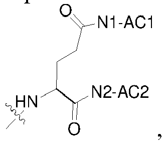 N1, N2, N3 and N4 are GFLG, AC1, AC2 and AC3 are PCB, and AC4 is SB7;
preferably, the polyethylene glycol conjugated drug is:

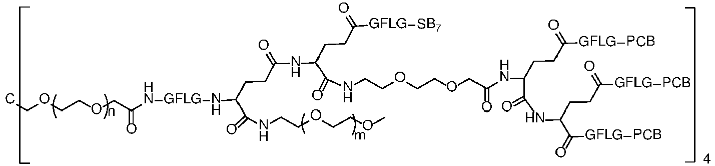

preferably, the number-average molecular weight of 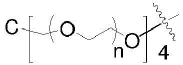 is 40k;
preferably, the number-average molecular weight of 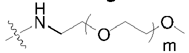 is 2k.

The polyethylene glycol conjugated drug of the disclosure may be selected from the following compounds:

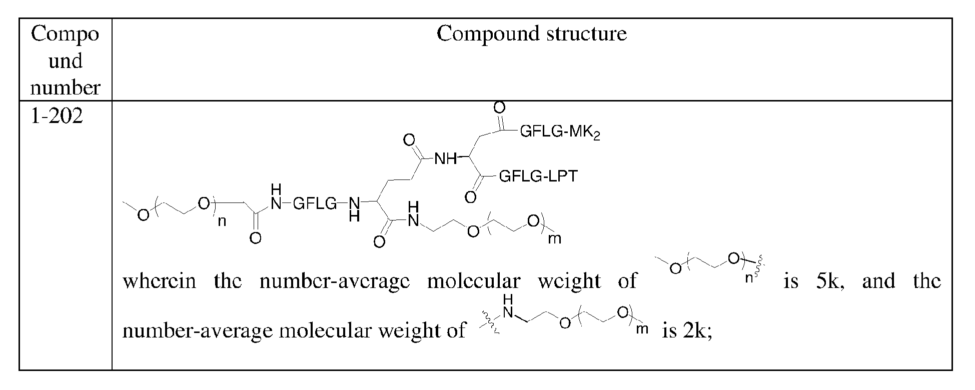

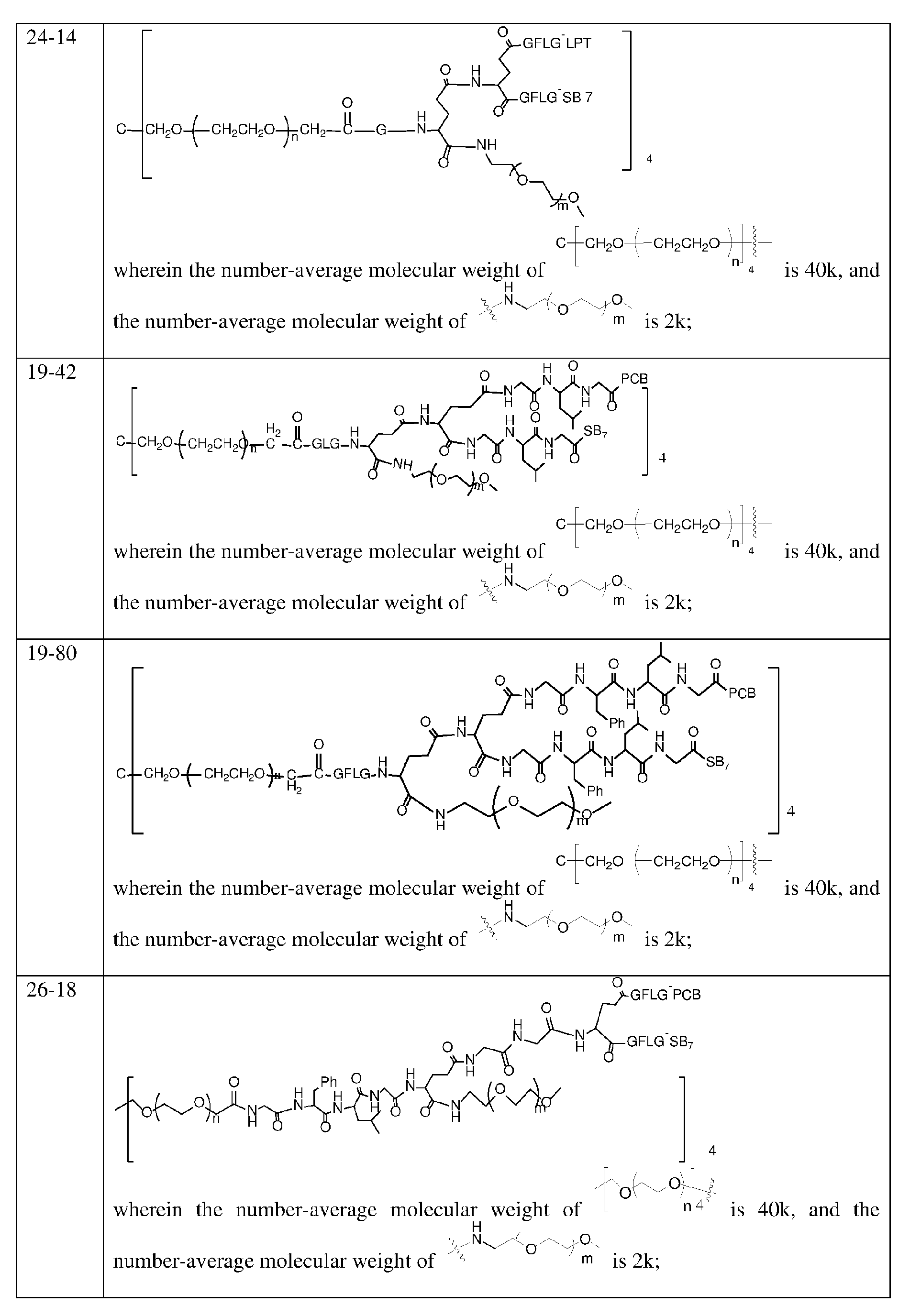

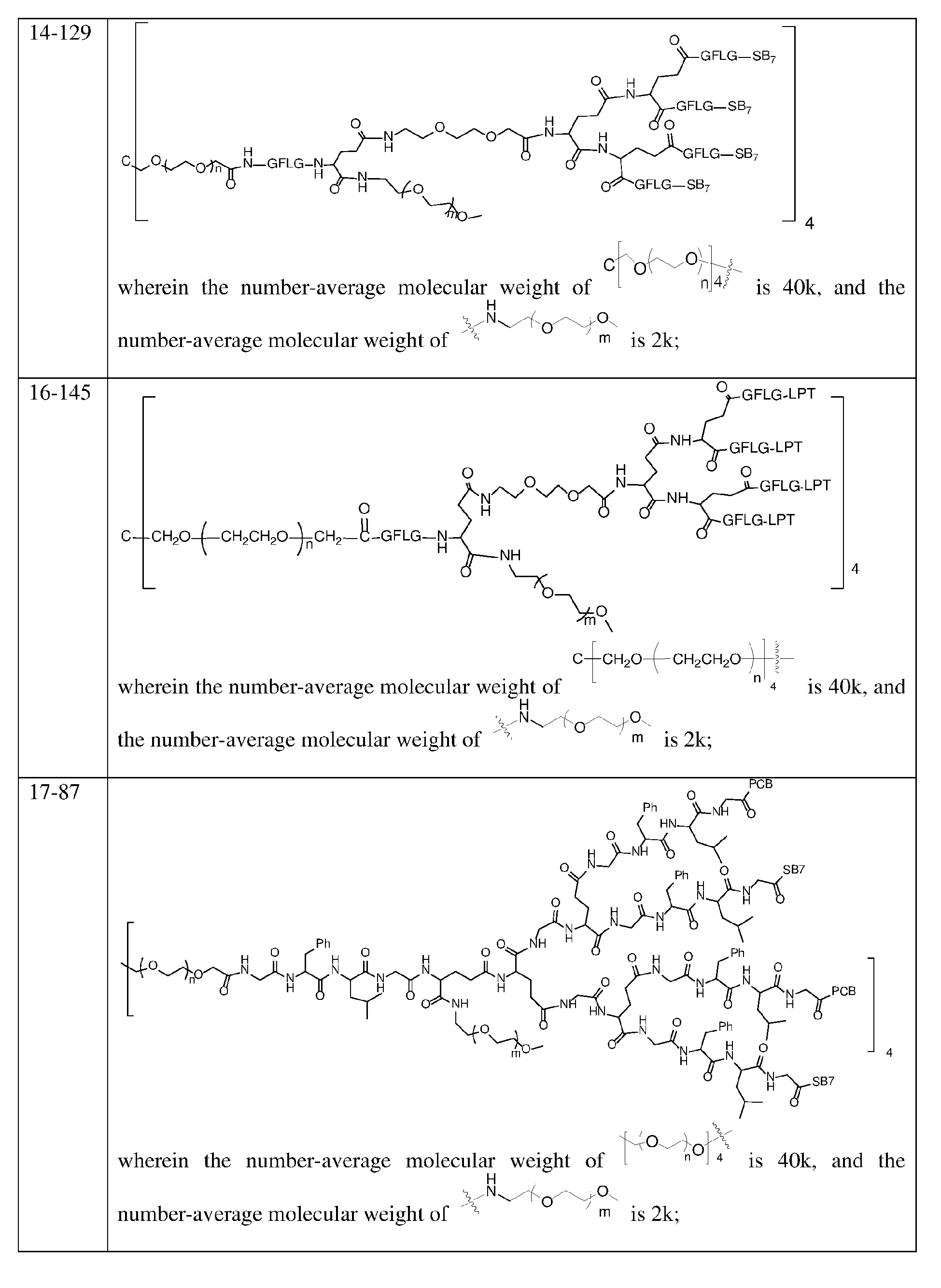

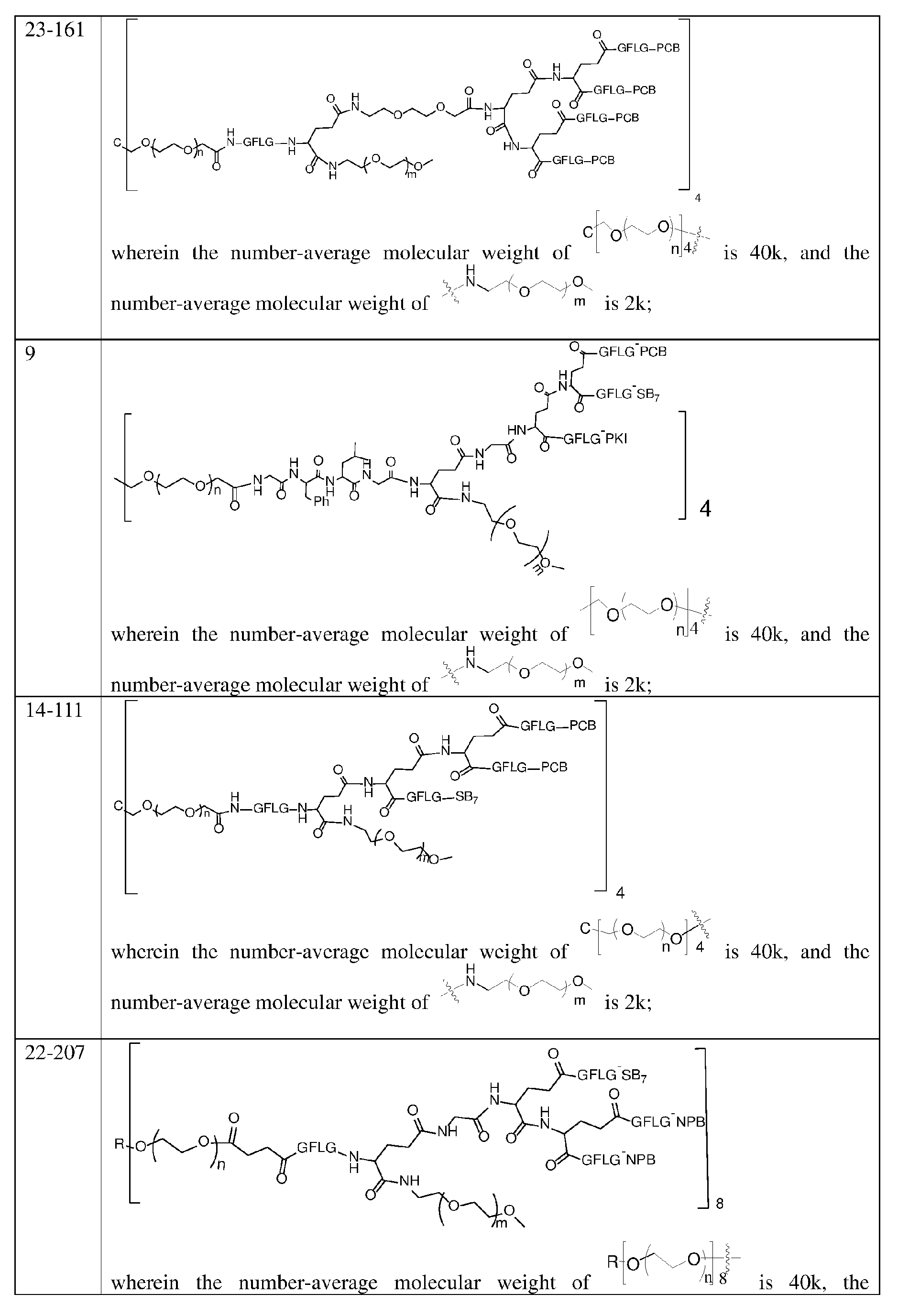

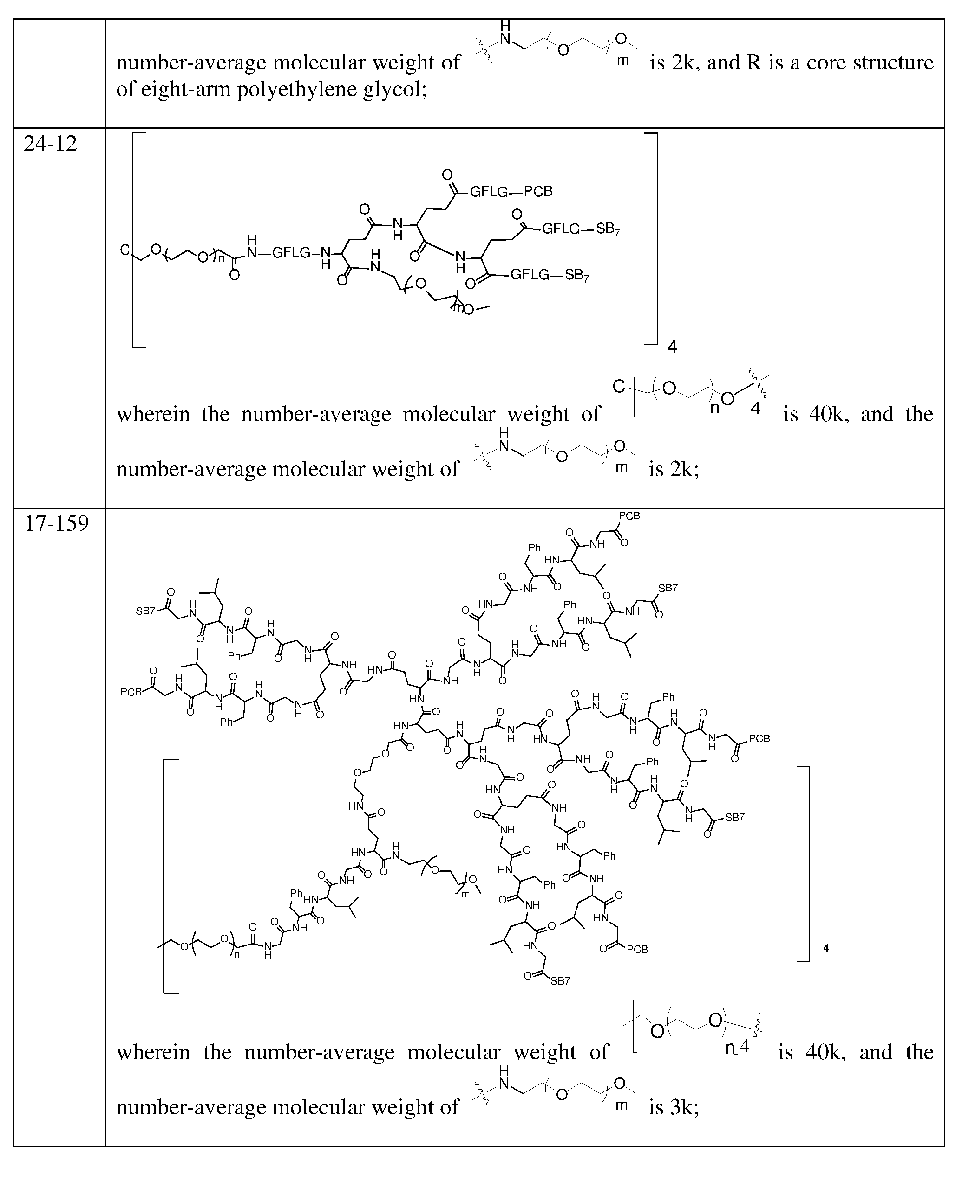

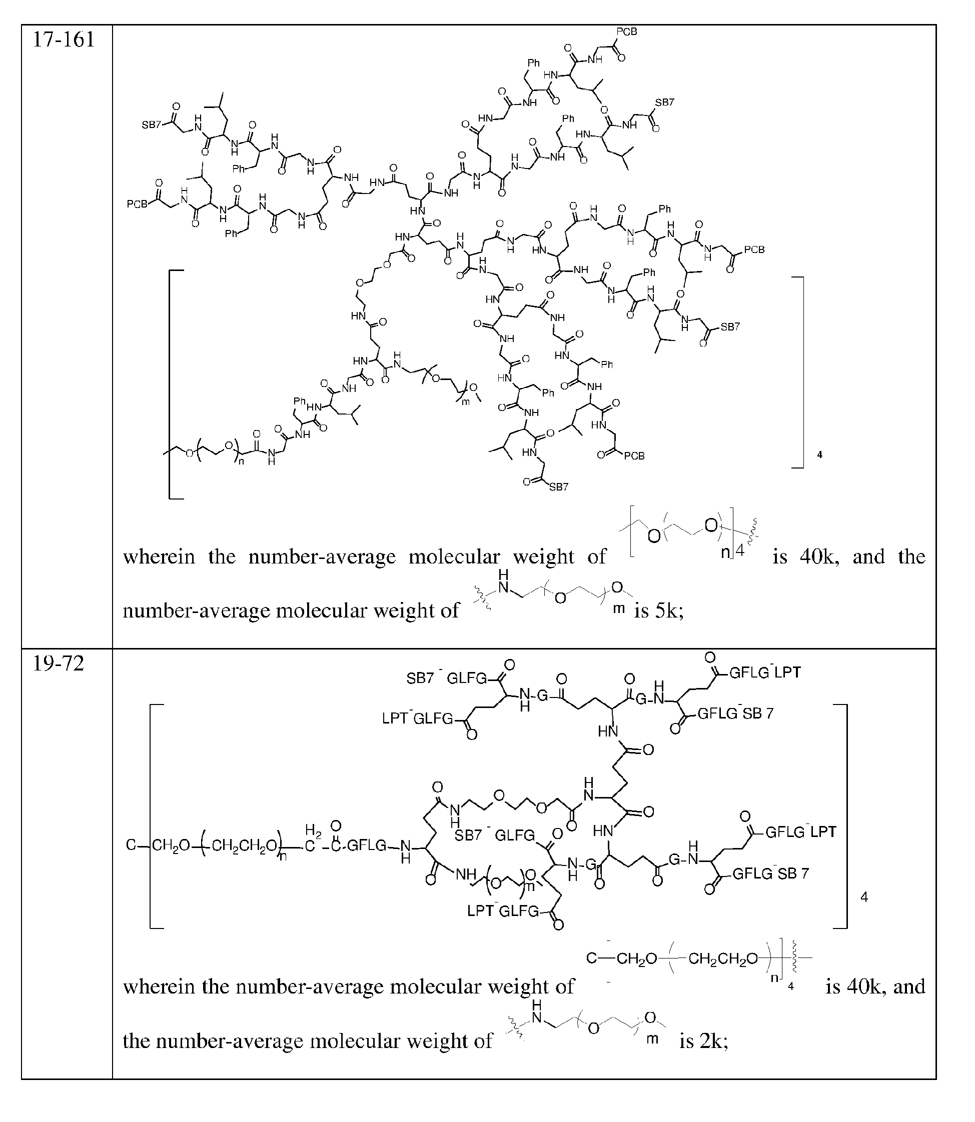

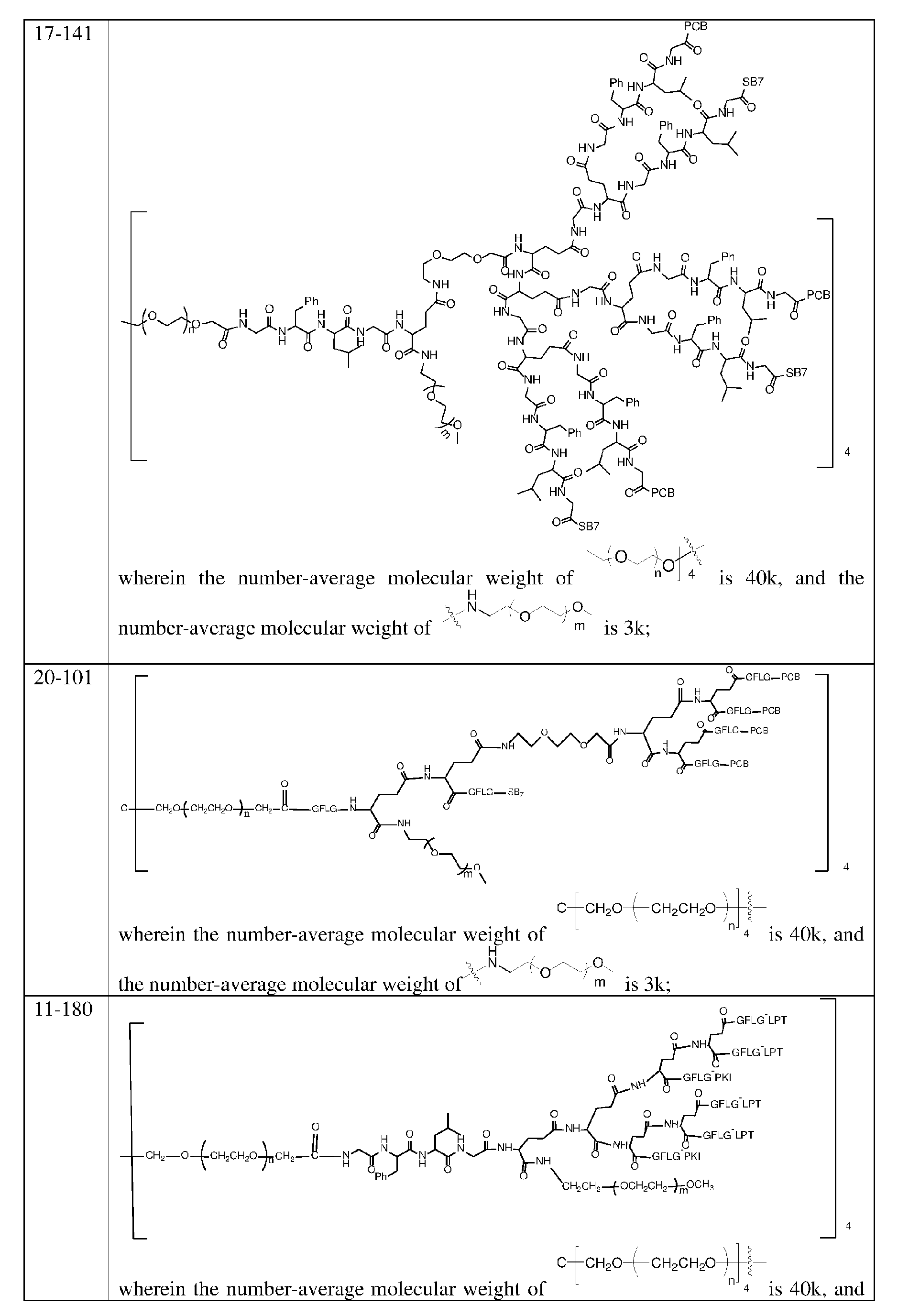

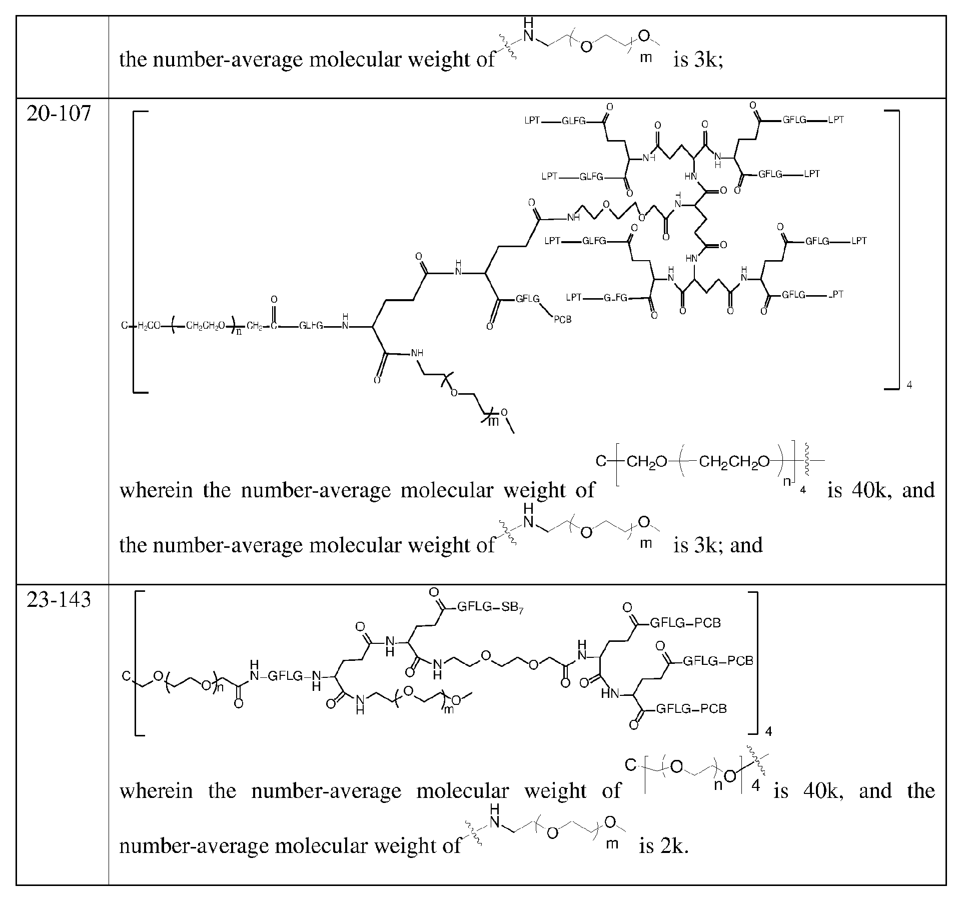

### Preparation method and intermediates

This application also relates to a method for preparing the polyethylene glycol conjugated drug of the disclosure or a pharmaceutically acceptable salt thereof, including the following steps:
S1: preparing an intermediate with amino group(s) 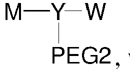 wherein at least one amino group is located on M, and
S2: carrying out an amidation reaction of PEG1 with a carboxyl group or an activated carboxyl groupwith the intermediate 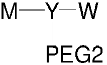 to obtain the polyethylene glycol conjugated drug according to any one of claims 1-12;
preferably, S1 comprises the following steps:
   step (1): preparing W;
   step (2): carrying out an amidation reaction with raw materials W and a dicarboxylic acid with an amino group to obtain an intermediate W-Y-COOH;
   step (3): linking the intermediate W-Y-COOH to PEG2 with an amino group through an amidation reaction to obtain an intermediate W-Y-PEG2; and
   step (4): carrying out an amidation reaction with raw materials, i.e., the intermediate W-Y-PEG2, an amino acid or a polypeptide, and a dicarboxylic acid with an amino group, to prepare an intermediate

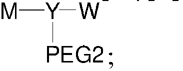

   wherein PEG1, PEG2, Y, W, and M are as defined in any one of claims 1-12.

This application also relates to another method for preparing the polyethylene glycol conjugated drug of the disclosure or a pharmaceutically acceptable salt thereof, including the following steps:
S1: preparing an intermediate 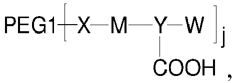 and
S2: linking PEG2 with an amino group to Y through an amidation reaction to obtain the polyethylene glycol conjugated drug according to any one of claims 1-12;
preferably, S1 comprises the following steps:
   step (1): preparing W;
   step (2): making W to react with a carboxyl group in a dicarboxylic acid with an amino group to obtain an intermediate W-Y-COOH;
   step (3): carrying out an amidation reaction with raw materials, i.e., the intermediate W-Y-COOH and an amino acid or a polypeptide to prepare an intermediate with amino group(s) 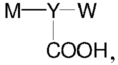 wherein at least one amino group is located on M; and
   step (4): carrying out an amidation reaction with raw materials, i.e., the intermediate 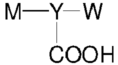 and PEG1 with a carboxyl group or an activated carboxyl group to prepare an intermediate 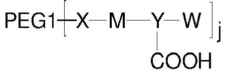
   wherein, PEG1, PEG2, X, Y, W, M, and j are as defined above.

For the above two methods, W in step (1) may be prepared by the following method:
wherein PEG1, PEG2, X, Y, W, M, and j are as defined in any one of claims 1-12.
(1) when W is Q, the method includes: carrying out an amidation reaction with raw materials, i.e., a drug, an amino acid or a polypeptide, and a dicarboxylic acid with an amino group, to prepare Q;
(2) when W is 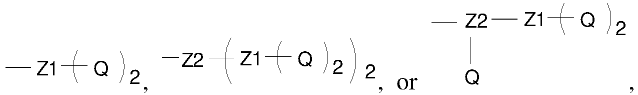 the method comprises: carrying out an amidation reaction with raw materials, i.e., a drug, an amino acid or a polypeptide, and a dicarboxylic acid with an amino group,to prepare Q; carrying out an amidation reaction with raw materials Q and a dicarboxylic acid with an amino group to prepare W;
(3) when W is 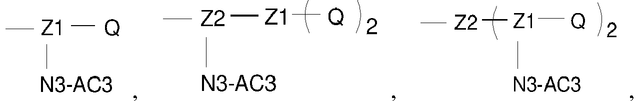 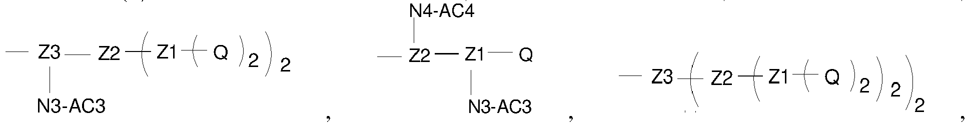 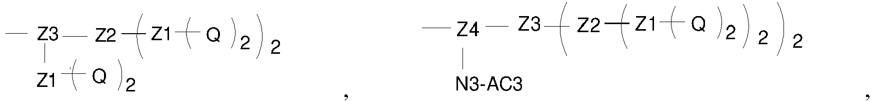 or 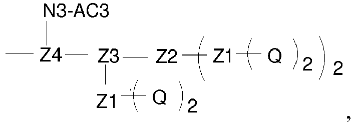 the method comprises: carrying out an amidation reaction with raw materials, i.e., a drug, an amino acid or a polypeptide, and a dicarboxylic acid with an amino group, to prepare Q; preparing N3-AC3 and optionally N4-AC4 by using a drug, and an amino acid or a polypeptide as raw materials; carrying out an amidation reaction with raw materials, i.e., Q, N3-AC3, optionally N4-AC4, and a dicarboxylic acid with an amino group, to prepare W.

In the disclosure, each intermediate may be prepared by an amidation reaction. The amidation reaction has high selectivity, high reaction rate, and few isomeric by-products, so the product obtained by this synthesis method has high yield and high reaction speed.

In order to enhance the specificity of the reaction, the amino group on the amino acid or polypeptide may be protected with a protecting group before the amidation reaction. The protecting group may be alkoxycarbonyl protecting group, such as tert-butoxycarbonyl (Boc), fluorenyl methoxycarbonyl (Fmoc), benzyloxycarbonyl (Cbz), and trimethylsilyl ethoxycarbonyl (Teoc). After the reaction, the amino group may be deprotected.

The amidation reaction of an amino acid or polypeptide with a drug may be carried out in the presence of a condensation reagent for polypeptide. The condensation reagent may be HOBT or HBTU. The alkaloid DIEA (N,N-diisopropylethylamine) may also be added during the reaction. The reaction temperature is preferably -10 °C to 10 °C, more preferably -4 °C to 4 °C.

Before the amidation reaction of the dicarboxylic acid with an amino group, the amino group and the carboxy group may be protected with protecting groups. The protecting group for amino group may be selected from the above-mentioned alkoxycarbonyl amino protecting groups. The protection for the carboxyl group may be achieved by esterification of the carboxyl group. The amidation reaction may be carried out in the presence of PyAOP. 2,4,6-trimethylpyridine may also be added during the reaction. The reaction temperature is preferably -10 °C to 10 °C.

This application also relates to a compound having any one of the following structures

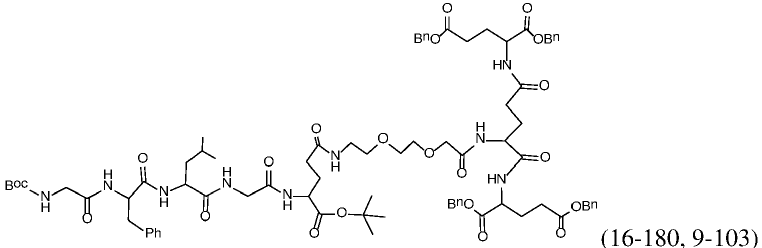

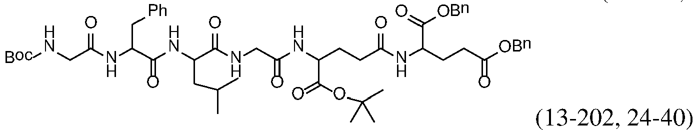

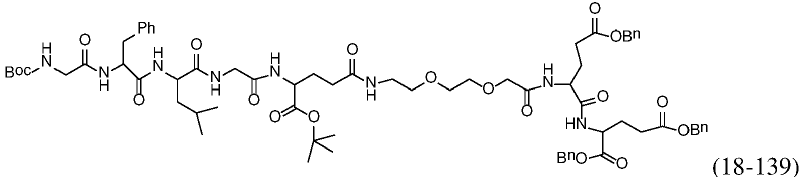

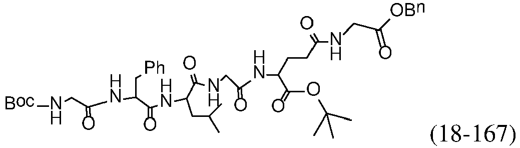

These compounds may be used as intermediates for the preparation of the polyethylene glycol conjugated drug of the disclosure. Therefore, this application also relates to the use of these compounds in preparation of the polyethylene glycol conjugated drug or a pharmaceutically acceptable salt thereof according to the disclosure.

### Pharmaceutical composition and pharmaceutical application

In one aspect, this application provides a pharmaceutical composition, including a therapeutically and/or prophylactically effective amount of the polyethylene glycol conjugated drug or a pharmaceutically acceptable salt thereof according to the disclosure; preferably, the composition also includes one or more pharmaceutically acceptable excipients, such as carriers and/or vehicles. The carriers and/or vehicles include, but are not limited to: ion exchangers, alumina, aluminum stearate, lecithin, serum proteins such as human serum protein, buffer substances such as phosphate, glycerin, sorbic acid, potassium sorbate, a mixture of partial glycerides of saturated plant fatty acids, water, salt or electrolyte, such as protamine sulfate, disodium hydrogen phosphate, potassium hydrogen phosphate, sodium chloride, zinc salt, colloidal silica, magnesium trisilicate, polyvinylpyrrolidone, cellulose material, polyethylene glycol, sodium carboxymethyl cellulose, polyacrylate, beeswax, polyethylene-polyoxypropylene block polymer, and lanolin.

The pharmaceutical composition may be prepared into any pharmaceutically acceptable dosage form. The pharmaceutical composition may also be applied to individuals in need of such treatment in any suitable way of administration, such as oral, parenteral, rectal or pulmonary administration. In the case of oral administration, the pharmaceutical composition may be made into conventional solid preparations, such as tablets, capsules, pills, granules, etc.; it may also be made into oral liquid preparations, such as oral solutions and oral suspensions, and syrup. When the pharmaceutical composition is made into oral preparations, suitable fillers, binders, disintegrants, lubricants, etc. may be added. In the case of parenteral administration, the pharmaceutical composition may be made into injection preparations, including injection solutions, sterile powders for injection, and concentrated solutions for injection. When the pharmaceutical composition is made into injection preparations, they may be produced by a conventional method in the current pharmaceutical field. In the case of preparation of injection preparations, it is not required to add additives, or appropriate additives may be added according to the nature of the drug. In the case of rectal administration, the pharmaceutical composition may be made into suppositories and the like. In the case of pulmonary administration, the pharmaceutical composition may be made into an inhalant or a spray. Preferably, the pharmaceutical composition of the disclosure may be made into an injection preparation, such as an injection solution. Alternatively, normal saline is used as the carrier of the injection solutions.

This application also provides an injection solution, comprising the polyethylene glycol conjugated drug or a pharmaceutically acceptable salt thereof according to the disclosure, or the pharmaceutical composition of the disclosure. Preferably, normal saline is used as a carrier for the injection solution.

In one aspect, this application provides use of the polyethylene glycol conjugated drug of the disclosure or a pharmaceutically acceptable salt thereof in the preparation of a medicament for treating and/or preventing a disease (such as a cancer). The disease refers to a disease treated by an active ingredient in the polyethylene glycol conjugated drug.

In the disclosure, cancer refers to a disease state characterized by cell proliferative, including but not limited to: colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, sarcoma, etc., including metastasis of the aforementioned cancers.

In one aspect, this application provides a method for treating and/or preventing a disease (such as a cancer), including administering an effective amount of the polyethylene glycol conjugated drug of the disclosure or a pharmaceutically acceptable salt thereof to an individual in need thereof. The dosage regimen may be adjusted to provide the optimum desired response. For example, a single amount of drug may be administered, several divided doses may be administered over time, or the dose may be proportionally reduced or increased as indicated by the urgent need for the treatment. It should be noted that the dose value may vary with the type and severity of the condition to be alleviated, and may include single or multiple doses. It should be further understood that for any particular individual, the specific dosage regimen should be adjusted over time according to the individual's needs and the professional judgment of the person administering the composition or supervising the administration of the composition.

In the disclosure, "individual" includes a human or a non-human animal. Exemplary human individuals include human individuals (referred to as patients) or normal individuals suffering from diseases such as those described herein. In the disclosure, "non-human animals" include all vertebrates, such as non-mammals (such as birds, amphibians, and reptiles) and mammals, such as non-human primates, livestock and/or domesticated animals (such as sheep, dogs, cats, cows, and pigs).

### Definition of terms

Unless otherwise defined below, the meanings of all technical and scientific terms used herein are intended to be the same as those commonly understood by those skilled in the art. The reference to the technology used herein is intended to refer to the technology generally understood in the art, including those technical changes or equivalent technology substitutions that are obvious to those skilled in the art. Although it is believed that the following terms are well understood by those skilled in the art, the following definitions are still set forth to better explain the disclosure.

As used herein, "PEG" is an abbreviation for polyethylene glycol, which refers to a homopolymer with a repeating unit of -CH₂CH₂O-, including single-arm polyethylene glycol, multi-arm polyethylene glycol and their derivatives, such as a derivative with a reactive functional group such as amino or carboxyl group at the terminal. In the disclosure, the arms of the multi-arm polyethylene glycol preferably have the same degree of polymerization. When referring to the molecular weight of a multi-arm polyethylene glycol, the molecular weight means the total molecular weight of each arm. In the structural formula of the disclosure, the letter "m" or "n" in the subscript of the repeating unit of polyethylene glycol represents the degree of polymerization of polyethylene glycol. When the polyethylene glycol is a multi-arm polyethylene glycol, the letter "m" or "n" represents the degree of polymerization of each arm.

As used herein, the "pharmaceutically acceptable salt" of the compound of the disclosure includes an acid addition salt and base addition salt of the compound, such as hydrochloride, hexafluorophosphate, and meglumine salt.

As used herein, the wavy line " 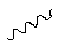 " in the structural formula means the position where another group is bonded to the structure represented by the structural formula.

As used herein, the term "effective amount" refers to the amount of a compound that will relieve one or more symptoms of the disease being treated to a certain extent after being administered.

As used herein, the term "treating" means reversing, alleviating, or inhibiting the disease or condition to which such term is applied or the progression of one or more symptoms of such a disease or condition, or preventing such a disease or condition or one or more symptoms of such a disease or condition.

### Beneficial Effect

The polyethylene glycol conjugated drug of the disclosure may achieve high drug loading capacity and good solubility. Through the method of preparation of the disclosure, the polyethylene glycol conjugated drug of the disclosure may be prepared efficiently and conveniently.

The embodiments of the disclosure will be described in detail below in conjunction with the accompanying drawings and examples. However, those skilled in the art will understand that the following drawings and examples are only used to illustrate the disclosure, not to limit the scope of the disclosure. Various objectives and advantageous aspects of the disclosure will become apparent to those skilled in the art based on the accompanying drawings and the following detailed description of the preferred embodiments.

### BRIEF DESCRIPTION OF THE DRAWINGS

Fig. 1 shows the experimental results of Example 25. As shown in the figure, Compound 24-6 in bottle A is still yellow lumps, and Compound 24-12 in bottle B is completely dissolved and is in a state of a yellow transparent solution.
Fig. 2 shows the experimental results of Example 26. As shown in the figure, Compound 18-158 in bottle A is still small yellow lumps, and Compound 17-141 in bottle B is completely dissolved and is in a state of a slightly viscous transparent yellow solution.
Fig. 3 shows the experimental results of Example 27. As shown in the figure, Compound 13-141 in bottle A is still small yellow lumps, and Compound 26-18 in bottle B is completely dissolved and is in a state of a transparent yellow solution.
Fig. 4 shows the weight growth curve of the laboratory animals in Experimental Example 1.
Fig. 5 shows the tumor growth curve (volume) in Experimental Example 1.
Figs. 6A-6C show the photos of the tumor tissues of each group in Experimental Example 1, and from top to bottom, they are the 19-80 group, the SB7 + PCB group, and the normal saline group.
Fig. 7 is a histogram of tumor weight in Experimental Example 1.
Fig. 8 shows the tumor inhibition rate of the drug in Experimental Example 1.
Fig. 9 shows the weight growth curve of the laboratory animals in Experimental Example 2.
Fig. 10 shows the tumor growth curve (volume) in Experimental Example 2.
Figs. 11A-11C show the photos of the tumor tissues of each group in Experimental Example 2, and from top to bottom, they are the 23-161 group, the PCB group, and the normal saline group.
Fig. 12 is a histogram of tumor weight in Experimental Example 2.
Fig. 13 shows the tumor inhibition rate of the drug in Experimental Example 2.
Fig. 14 shows the weight growth curve of the laboratory animals in Experimental Example 3.
Fig. 15 shows the tumor growth curve (volume) in Experimental Example 3.
Figs. 16A-16C show the photos of the tumor tissues of each group in Experimental Example 3, and from top to bottom, they are the normal saline group, the 14-111 group, and the SB7 + PCB group.
Fig. 17 is a histogram of tumor weight in Experimental Example 3.
Fig. 18 shows the tumor inhibition rate of the drug in Experimental Example 3.
Fig. 19 shows the weight growth curve of each group of animals in Experimental Example 4.
Fig. 20 shows the tumor growth curve (volume) of each group of animals in Experimental Example 4.
Fig. 21 shows the tumor weight of each group of animals in Experimental Example 4 on Day 17 after administration.

### DETAILED DESCRIPTION

The embodiments of the disclosure will be described in detail below in conjunction with examples. However, those skilled in the art will understand that the following examples are only used to illustrate the disclosure, not to limit the scope of the disclosure. Those without specific conditions among the examples are generally implemented under conventional conditions or under conditions recommended by the manufacturers. The reagents or instruments used without specifying the manufacturers are all conventional products that may be purchased commercially.

The meanings of abbreviations in the examples are as follows:

| | | | |
|---|---|---|---|
| G | Glycine residue | L | Leucine residue |
| F | Phenylalanine residue | Asp | Aspartic acid residue |
| E | Glutamate residue | Glu | Glutamate residue |
| DMF | N,N-dimethylformamide | TFA | Trifluoroacetic acid |
| t-Bu | Tert-butvl | Bn | Benzvl |
| Boc | Tert-butoxycarbonyl | Fmoc | Fluorenyl methoxycarbonyl |
| HOBT | 1-hvdroxvbenzotriazole | Ts | p-toluenesulfonyl |
| HBTU | O-benzotriazole-tetramethylurea hexafluorophosphate | LPT | Lapatinib |
| DIEA | N,N-diisopropylethylamine | SB7 | SB-743921 |
| EA | Ethyl acetate | PCB | Palbociclib |
| TMP | 2,4,6-trimethylpvridine | NPB | Niraparib |
| PyAOP | (3H-1,2,3-triazolo[4,5-b]pyridin-3-oxy)tris-1-pyrrolidinylphosphonium hexafluorophosphate | | |
| LC | NH₂-CH₂CH₂O-CH₂CH₂O-CH₂-COOH or -NH-CH₂CH₂O-CH₂CH₂O-CH₂-CO- | | |

The source and structure of some raw materials are as follows:
M-NH₂-2K.HCl
JenKem, **mPEG―**CH₂CH₂―NH₂HCl
M-NH₂-3K.HCl
JenKem, **mPEG―**CH₂CH₂-NH₂HCl
M-NH₂-5K.HCl
JenKem, **mPEG―**CH₂CH₂―NH₂HCl
M-SCM-10K
JenKem, 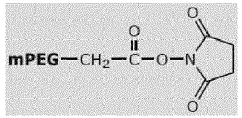
8ARM-SS-40K
JenKem, 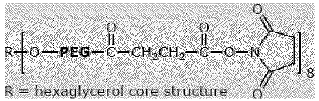
4ARM-SCM-40K
JenKem,

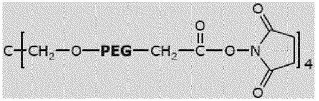

### Example 1: Synthesis of Compound 1-202

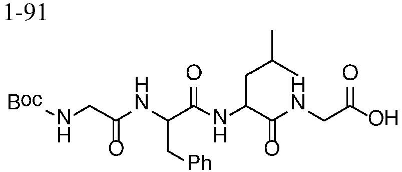

The raw material Boc-GFLG-OBn (8.0 g, 13.73 mmol, purchased from Nanjing Yaoshi) and 10% Pd/C (Palladium/Carbon) catalyst (150 mg) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (20 mL×3) and DMF was transferred dropwise into the diatomaceous earth. The diatomaceous earth was washed with DMF until it did not contain any product, and then the reaction product was obtained.

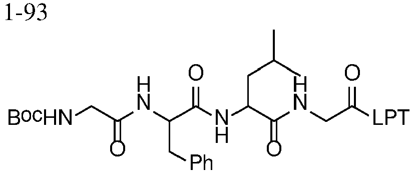

Product 1-91 Boc-GFLG-OH (13.73 mmol), Lapatinib (6.65 g, 11.441 mmol, referred to as LPT), HBTU (6.51 g, 17.16 mmol) and HOBT (2.32 g, 17.16 mmol) were added in a 250 mL round-bottomed flask and then dissolved with DMF (100 mL), and the mixed solution was stirred at -5 °C for 30 minutes. Then DIEA (8.51 mL, 51.48 mmol) was slowly added dropwise, and then the mixed solution reacted at a low temperature for 2 h; after that, the reaction device was placed at room temperature and the reaction solution was stirred overnight to react. At the end of the reaction, the reaction solution was transferred to a 1000 mL separatory funnel, saturated sodium bicarbonate solution (200 mL) was then added, the obtained solution was extracted three times with ethyl acetate (200 mL×3), and the obtained organic phases were combined and then washing with saturated sodium chloride was carried out three times (100 mL×3); the organic phase was dried with anhydrous sodium sulfate and filtered by suction. The filtrate was concentrated and evaporated to dryness and then dried in a vacuum oven for the next reaction.

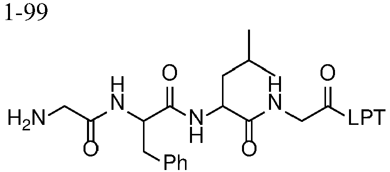

Product 1-93 (12.07 g, 11.441 mmol) was added in a 250 mL round-bottomed flask and then dissolved with dichloromethane (42 mL), and then TFA (8.49 mL, 114.41 mmol) was added and the obtained solution was stirred at room temperature to react overnight. At the end of the reaction, the reaction solution was concentrated and evaporated to dryness under reduced pressure, and the obtained dry product was then dissolved with an appropriate amount of ethyl acetate and transferred to a 1000 mL separatory funnel; saturated sodium bicarbonate solution (250 mL) was added to neutralize the remaining TFA, the organic phase was then separated, the product in the aqueous phase was extracted three times with ethyl acetate (150 mL×3), and the obtained organic phases were combined, then dried with anhydrous sodium sulfate, filtered by suction, and concentrated to 100 mL; silica gel powder (12 g) was added to the concentrated solution; the operations of dry sample loading, column chromatography, and elution with 4% methanol/1% ammonia water/dichloromethane were carried out, and then the elution product was then collected, concentrated, evaporated to dryness, dried in a vacuum oven, and provided 11.25 g of the product.

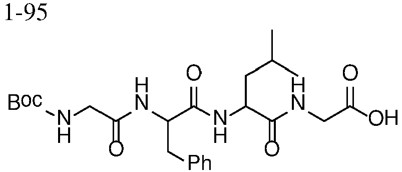

The raw material Boc-GFLG-OBn (4.50 g, 7.72 mmol) and 10% Pd/C catalyst (80 mg) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (20 mL×3) until the reaction device did not contain any product, and then the reaction product was obtained.

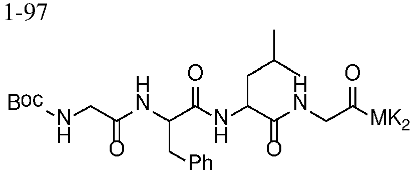

Product 1-95 Boc-GFLG-OH (7.72 mmol), MK-2206·2HCl (2.97 g, 6.18 mmol, referred to as MK2), HBTU (3.52 g, 9.27 mmol) and HOBT (1.25 g, 9.27 mmol) were added in a 250 mL round-bottomed flask and then dissolved with DMF (90 mL), and the mixed solution was stirred at -5 °C for 30 minutes. Then DIEA (6.6 mL, 27.81 mmol) was added dropwise, and then the mixed solution reacted at a low temperature for 2h; after that, the reaction device was placed at room temperature and the reaction solution was stirred overnight to react. At the end of the reaction, the reaction solution was transferred to a 1000 mL separatory funnel, saturated sodium bicarbonate solution (200 mL) was then added, the obtained solution was extracted three times with ethyl acetate (200 mL×3), and the obtained organic phases were combined and then washed with saturated sodium chloride solution three times (100 mL×3); the organic phase was dewatered, evaporated to dryness under reduced pressure, and then dried in a vacuum box, thus obtaining 8.1 g of the product, with a yield of 148.6%, for next reaction.

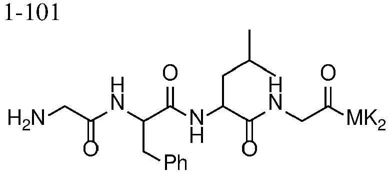

Product 1-97 (5.45 g, 6.18 mmol) was added in a 250 mL round-bottomed flask and then dissolved with dichloromethane (45 mL), and then TFA (4.59 mL, 61.80 mmol) was added and the obtained solution was stirred at room temperature to react overnight. At the end of the reaction, the reaction solution was concentrated and evaporated to dryness under reduced pressure, and the obtained dry product was then dissolved with ethyl acetate (300 mL) and transferred to a 1000 mL separatory funnel; saturated sodium bicarbonate solution (200 mL) was added to neutralize the remaining TFA, the organic phase was then separated, the product in the aqueous phase was extracted three times with ethyl acetate (150 mL×3), the obtained organic phases were collected, and the product sticked to the flask wall was washed with dichloromethane (20 mL) and methanol (5 mL); the collected organic phases were combined and evaporated to dryness under reduced pressure; the operations of dry sample loading, column chromatography, and elution with 5% methanol/2% ammonia water/dichloromethane were carried out, and then the elution product was then collected, concentrated, evaporated to dryness, and dried in a vacuum oven, thus obtaining 4.297 g of the product with a yield of 89.0%.

MALDI-TOF MS: [M+H⁺] 782.30, [M+Na⁺] 804.25.

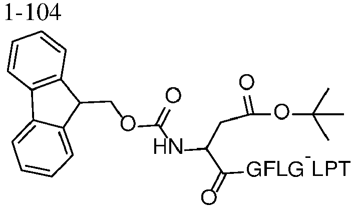

Fmoc-Asp-OtBu (3.92 g, 9.524 mmol) was added in a 250 mL round-bottomed flask and dissolved with DMF (103 mL), and then Product 1-99 GFLG-LPT (6.5 g, 6.803 mmol) and PyAOP (4.97 g, 9.524 mmol) were added; the obtained solution was stirred for 30 min at 0 °C, and then 2,4,6-trimethylpyridine (0.90 mL, 6.803 mmol) was slowly added dropwise to react for 2 days at this low temperature. At the end of the reaction, the reaction solution was transferred to a 1000 mL separatory funnel and deionized water (200 mL) was added for washing; the reaction flask was washed with ethyl acetate; the mixed phase in the separatory funnel was shaken vigorously, the organic phase was separated, the aqueous phase was extracted with ethyl acetate three times (200 mL×3), and the obtained organic phases were combined and then washing with saturated sodium chloride solution (100 mL×3) was carried out three times; the organic phase was dried with anhydrous sodium sulfate and filtered by suction. The filtrate was concentrated, the operations of dry sample loading, column chromatography, and elution with 4% methanol/dichloromethane were carried out, and then the elution product was then collected, concentrated, evaporated to dryness, and dried in a vacuum oven, thus obtaining 8.2 g of the product with a yield of 89.4%.

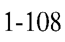

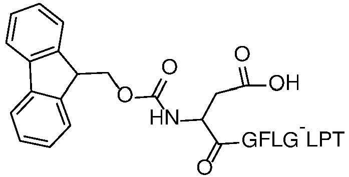

Product 1-104 (8.2 g, 6.079 mmol) was added in a 250 mL round-bottomed flask and then dissolved with dichloromethane (30 mL), and then TFA (4.52 mL, 60.79 mmol) was added and the obtained solution was stirred at room temperature to react overnight. At the end of the reaction, the reaction solution was concentrated and evaporated to dryness under reduced pressure, then the obtained dry product was dissolved with dichloromethane (5 mL) and then precipitated with n-hexane (100 mL), the supernatant was discarded, and this operation was repeated twice; the precipitate was evaporated to dryness and weighed, and because the product was overweight, the operations of column purification treatment, dry sample loading, column chromatography and elution with 5% methanol/dichloromethane were carried out; the elution product was then collected, concentrated, evaporated to dryness, and dried in a vacuum oven, thus obtaining 5.21 g of the product with a yield of 66.37 %.

MALDI-TOF MS: [M+H⁺] 1292.30, [M+Na⁺] 1314.35.

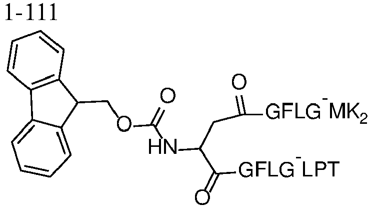

Product 1-108 (4.6 g, 3.56 mmol) was added in a 250 mL round-bottomed flask and dissolved with DMF (90 mL), and then Product 1-101 (2.53 g, 3.23 mmol) and PyAOP (2.36 g, 4.53 mmol) were added; the obtained solution was stirred for 30 min at 0 °C, and then 2,4,6-trimethylpyridine (0.43 mL, 3.23 mmol) was slowly added dropwise to react for 2 days at this low temperature. At the end of the reaction, the reaction solution was transferred to a 1 L separatory funnel and deionized water (200 mL) was added for washing; the reaction flask was washed with ethyl acetate; the mixed phase in the separatory funnel was shaken vigorously, the organic phase was separated, the aqueous phase was extracted with ethyl acetate three times (200 mL×3), the product attached to the wall was then washed away with a mixed solvent of dichloromethane (30 mL) and methanol (6 mL), and the obtained organic phases were combined. The filtrate was concentrated, the operations of dry sample loading, column chromatography, and elution with 6% methanol/dichloromethane were carried out, and then the elution product was then collected, concentrated, evaporated to dryness, and dried in a vacuum oven, thus obtaining 4.6 g of the product with a yield of 70.0%.

¹H-NMR (400 MHz, DMSO-d₆) δ 12.68 (s, 1H), 10.00 (s, 1H), 8.78-8.53 (m, 2H), 8.32-7.70 (m, 15H), 7.54-7.48 (m, 6H), 7.34-7.20 (m, 27H), 7.00 (d, *J*=8.0Hz, 1H), 6.69-6.54 (m, 1H), 5.26 (s, 2H), 4.73 (d, *J*=20.0Hz, 1H), 4.54 (d, *J*=6.0Hz, 1H), 4.35-4.16 (m, 7H), 3.70-3.64 (m, 7H), 3.25-2.77 (m, 13H), 2.40-1.79 (m, 4H), 1.48-1.23 (m, 7H), 0.86-0.76 (m, 14H).

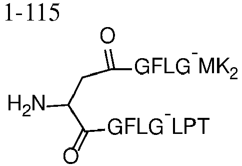

Product 1-111 (4.6 g, 2.24 mmol) was added in a 250 mL round-bottomed flask and then dissolved with DMF (53 mL), and then morpholine (5.85 mL, 67.1 mmol) was added and the obtained solution was stirred at room temperature to react for 2h. At the end of the reaction, the reaction solution was transferred to a 2 L separatory funnel and saturated sodium chloride solution (100 mL) was added; the reaction flask was washed with ethyl acetate; the mixed phase in the separatory funnel was shaken vigorously, the organic phase was separated, the aqueous phase was extracted with ethyl acetate three times (200 mL×3), and the obtained organic phases were combined and then washing with saturated sodium chloride solution (100 mL) was carried out three times. Methanol (5 mL) and dichloromethane (20 mL) were added to dissolve the reaction product attached to the wall of the flask, and the obtained organic phase products were combined and filtered by suction. The filtrate was concentrated, the operations of dry sample loading, column chromatography, and elution with 7% methanol/1% ammonia water/dichloromethane were carried out, and then the elution product was collected, concentrated, evaporated to dryness, and dried in a vacuum oven, thus obtaining 3.1 g of the product with a yield of 75%.

¹H-NMR (400 MHz, DMSO-d₆) δ 9.86 (d, *J*=8.0Hz, 1H), 8.75 (d, *J*=4.0Hz, 1H), 8.55 (s, 1H), 8.33-8.08 (m, 11H), 7.99-7.70 (m, 4H), 7.33-7.21 (m, 17H), 7.15-6.92 (m, 12H), 6.69-6.54 (m, 1H), 5.26 (s, 2H), 4.75-4.09 (m, 10H), 3.68-3.53 (m, 10H), 3.06-2.67 (m, 9H), 2.41-2.25 (m, 2H), 2.00-1.81 (m, 2H), 1.54-1.47 (m, 6H), 0.86-0.78 (m, 14H).

MALDI-TOF MS: [M+H⁺] 1833.90, [M+Na⁺] 1856.90.

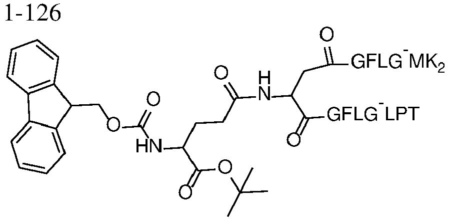

Fmoc-Glu-OtBu (0.96 g, 2.25 mmol) was added in a 500 mL round-bottomed flask and dissolved with DMF (70 mL), and then Product 1-115 (2.95 g, 1.61 mmol) and PyAOP (1.17 g, 2.25 mmol) were added; the obtained solution was stirred for 30 min at 0 °C, and then 2,4,6-trimethylpyridine (0.22 mL, 1.61 mmol) was added dropwise to react for 3 days at this low temperature. At the end of the reaction, the reaction solution was transferred to a 2000 mL separatory funnel and deionized water (200 mL) was added for washing; the reaction flask was then washed with ethyl acetate; the mixed phase in the separatory funnel was shaken vigorously, the organic phase was separated and extracted with ethyl acetate three times (200 mL×3), and the obtained organic phases were combined and washing with saturated sodium chloride (100 mL) was carried out three times; the organic phase was concentrated, the operations of dry sample loading, column chromatography, and elution with 8% methanol/dichloromethane were carried out, and then the elution product was then collected, concentrated, evaporated to dryness, and dried in a vacuum oven, thus obtaining 3.8 g (theoretical weight: 3.61 g) of the product.

¹H-NMR (400 MHz, DMSO-d₆) δ 12.62 (s, 1H), 9.80 (s, 2H), 8.70-8.45 (m, 5H), 8.26-8.15 (m, 8H), 7.86-7.63 (m, 15H), 7.45-7.26 (m, 8H), 7.13-6.85 (m, 17H), 5.19 (s, 2H), 4.68-4.45 (m, 6H), 4.21-4.08 (m, 8H), 3.69-3.52 (m, 8H), 2.82-2.72 (m, 11H), 2.43-2.13 (m, 3H), 1.68-1.46 (m, 4H), 1.31-1.16 (m, 14H), 0.78-0.69 (m, 15H).

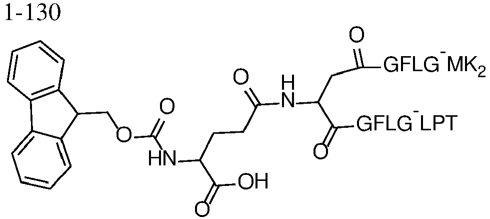

Product 1-126 (3.6 g, 1.61 mmol) was added in a 500 mL round-bottomed flask and dissolved with dichloromethane (30 mL), TFA (1.20 mL, 16.1 mmol) was then added, the obtained solution was stirred for 2 h at room temperature, and TFA (12.3 mL) was added again; the obtained solution further reacted for 4 h and then the reaction ended; the reaction solution was concentrated and evaporated to dryness under reduced pressure and weighed, and because the product was overweight, the operations of column purification treatment, including dry sample loading, column chromatography and elution with 8% methanol/dichloromethane were carried out; the elution product was then collected, concentrated, evaporated to dryness, and dried in a vacuum oven, thus obtaining 2.4 g of the product with a yield of 68.20%.

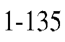

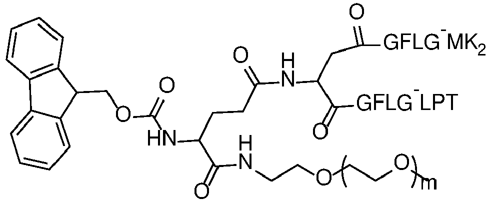

Product 1-130 (2.25 g, 1.03 mmol) was added in a 500 mL round-bottomed flask and dissolved with DMF (42 mL), and then M-NH2-2K. HCl (1.96 g, 0.98 mmol, purchased form JenKem) and PyAOP (0.72 g, 1.37 mmol) were added; the obtained solution was stirred for 30 min at 0 °C, and then 2,4,6-trimethylpyridine (0.26 mL, 1.96 mmol) was slowly added dropwise to react for 3 days at this low temperature. At the end of the reaction, the reaction solution was precipitated with ether, the supernatant was poured out, and then the reaction solution was precipitated again with n-hexane three times (180 mL×3); the precipitate was dried with suction, and then dried in a vacuum oven, thus obtaining 4.2 g of product (overweight).

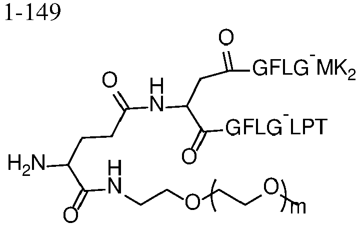

Product 1-135 (4.08 g, 0.98 mmol) was added in a 500 mL round-bottomed flask and then dissolved with DMF (40 mL), morpholine (2.56 mL, 29.4 mmol) was then added and the obtained solution was stirred at room temperature to react for 2.5h. At the end of the reaction, the reaction solution was precipitated with methyl tert-butyl ether (170 mL) and then precipitated again with n-hexane three times (150 mL×3); the precipitate was dried with suction and dried; silica gel powder (8 g) was added, and the operations of dry sample loading, column chromatography and elution with 9% methanol/dichloromethane were carried out; the elution product was then collected, concentrated, evaporated to dryness, and dried in a vacuum oven, thus obtaining 2.3 g of the product with a yield of 60%.

¹H-NMR (400 MHz, DMSO-d₆) δ 9.88 (s, 2H), 8.76 (d, *J*=8.0Hz, 1H), 8.55 (s, 2H), 8.34-8.02 (m, 15H), 7.96-7.78 (m, 3H), 7.46 (dd, *J*=4.0, 8.0Hz, 3H), 7.13-7.07 (m, 24H), 6.92 (d, *J*=4.0Hz, 1H), 6.69-6.54 (m, 1H), 5.26 (s, 2H), 4.73 (d, *J*=20.0Hz, 2H), 4.52-4.48 (m, 4H), 4.35-4.11 (m, 7H), 3.68-3.50 (m, 166H), 3.24-2.73 (m, 36H), 2.33-1.81 (m, 6H), 1.49-1.23 (m, 10H), 1.04 (t, *J*=8.0Hz, 2H), 0.86-0.78 (m, 8H).

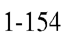

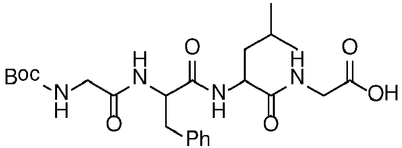

The raw material Boc-GFLG-OBn (0.46 g, 0.781 mmol) and 10% Pd/C catalyst (50 mg) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (15 mL×3) until the reaction device did not contain any product, and then the reaction solution 1-154 was obtained.

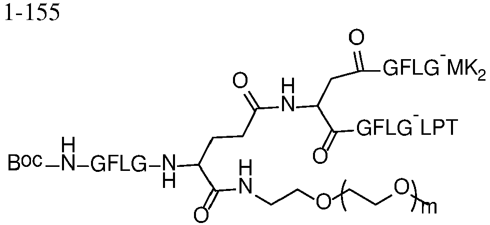

Product 1-154 Boc-GFLG-OH (0.781 mmol), Product 1-149 (2.2 g, 0.558 mmol), HBTU (0.32 g, 0.837 mmol) and HOBT (0.11 g, 0.837 mmol) were added in a 250 mL round-bottomed flask and then dissolved with DMF (80 mL), and the mixed solution was stirred at -5 °C for 30min. Then DIEA (0.42 mL, 2.511 mmol) was slowly added dropwise, and then the mixed solution reacted at a low temperature for 2h; after that, the reaction device was placed at room temperature and the reaction solution was stirred overnight to react. At the end of the reaction, the reaction solution was taken out, precipitated with methyl tert-butyl ether (350 mL) and then precipitated again with n-hexane three times (150 mL×3); the precipitate was dried with suction and dried; silica gel powder (5 g) was added, and the operations of dry sample loading, column chromatography and elution with 8% methanol/dichloromethane were carried out; the elution product was then collected, concentrated, evaporated to dryness, and dried in a vacuum oven, thus obtaining 2.4 g of the product with a yield of 97.2%.

¹H-NMR (400 MHz, DMSO-d₆) δ 9.72 (s, 1H), 8.75 (d, *J*=8.0Hz, 1H), 8.55 (s, 2H), 8.34-8.05 (m, 10H), 7.99-7.71 (m, 12H), 7.48-7.19 (m, 30H), 6.93 (d, *J*=4.0Hz, 3H), 6.68-6.54 (m, 1H), 5.26 (s, 2H), 4.73 (d, *J*=20.0Hz, 2H), 4.52 (s, 5H), 4.35-4.11 (m, 7H), 3.68-3.49 (m, 172H), 3.02-2.89 (m, 21H), 2.75-2.67 (m, 16H), 2.33-1.81 (m, 5H), 1.53-1.47 (m, 13H), 1.19-1.12 (m, 8H), 0.81-076 (m, 18H).

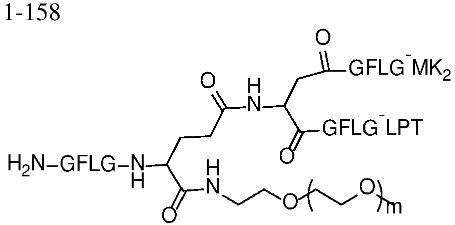

Product 1-155 (2.35 g, 0.532 mmol) was added in a 250 mL round-bottomed flask and dissolved with dichloromethane (35 mL), TFA (0.4 mL, 5.32 mmol) was then added, the obtained solution was stirred for 3 h at room temperature, and TFA (1.8 mL) was added again; the obtain solution further reacted for 4 h and then the reaction ended; the reaction solution was concentrated and evaporated to dryness under reduced pressure and weighed, and because the product was overweight, the operations of column purification treatment, including dry sample loading, column chromatography and elution with 10% methanol/dichloromethane were carried out; the elution product was then collected, concentrated, evaporated to dryness, and dried in a vacuum oven, thus obtaining 1.1 g of the product with a yield of 47.9%.

¹H-NMR (400 MHz, DMSO-d₆) δ 9.85 (s, 1H), 8.76-8.74 (m, 1H), 8.42-8.22 (m, 3H), 8.13-7.88 (m, 10H), 7.78-7.64 (m, 2H), 7.57-7.40 (m, 1H), 7.40-7.16 (m, 30H), 7.10-7.03 (m, 10H), 4.75-4.52 (m, 4H), 4.32-4.11 (m, 5H), 3.74-3.57 (m, 14H), 3.55-3.42 (m, 173H), 3.24-3.17 (m, 15H), 3.06-2.99 (m, 9H), 2.75-2.67 (m, 3H), 2.46-2.33 (m, 5H), 2.12-2.02 (m, 3H), 1.82-1.72 (m, 3H), 1.58-1.48 (m, 5H), 1.27-1.18 (m, 1H), 0.87-076 (m, 18H).

MALDI-TOF MS: from 4315.73 to 4338.68

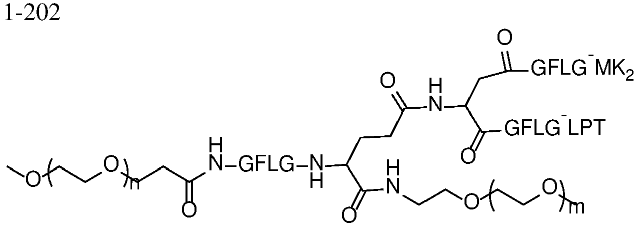

Product 1-158 (0.55 g, 0.127 mmol) was added in a 100 mL round-bottomed flask and then dissolved with dichloromethane (15 mL) and DMF (2 mL), and then high-molecular M-SCM-5K (Lot Number: YF278P110) (0.557 g, 0.107 mmol, purchased from Jenkem) was added, and the mixed solution was stirred in the dark at the lowest speed at room temperature for 5 days. The reaction was under continuous monitoring and the reaction was stopped when the reaction no longer changed. The reaction solution was concentrated to 10 mL under reduced pressure, and then precipitated with methyl tert-butyl ether (150 mL) for 2 h and dried with suction; the operations of dry sample loading, column chromatography, and elution with 6% methanol/dichloromethane were carried out; the elution product was then collected, concentrated, evaporated to dryness, and dried in a vacuum oven, thus obtaining 0.3419 g of the product.

¹H-NMR (400 MHz, DMSO-d₆) δ 12.68 (s, 1H), 9.86-9.85 (m, 1H), 8.75-8.70 (m, 1H), 8.56 (s, 1H), 8.34 (s, 2H), 8.27-7.88 (m, 17H), 7.82-7.74 (m, 2H), 7.49-7.47 (m, 2H), 7.34-7.11 (m, 30H), 5.27 (s, 2H), 4.75-4.70 (m, 2H), 4.56-4.54 (m, 5H), 4.37-4.22 (m, 3H), 4.11-4.01 (m, 10H), 3.89-3.81 (m, 12H), 3.79-3.68 (m, 33H), 3.57-3.51 (m, 594H), 3.24-3.17 (m, 20H), 3.06-3.01 (m, 5H), 2.76-2.68 (m, 3H), 2.66-2.60 (m,3H), 2.43-2.34 (m, 3H), 2.13-2.00 (m, 2H), 1.82-1.74 (m, 2H), 1.57-1.48 (m, 3H), 1.30-1.24 (m, 4H), 0.86-0.77 (m,18H).

MALDI-TOF MS: from 9424.33 to 9499.87

### Example 2: Synthesis of Compound 17-159

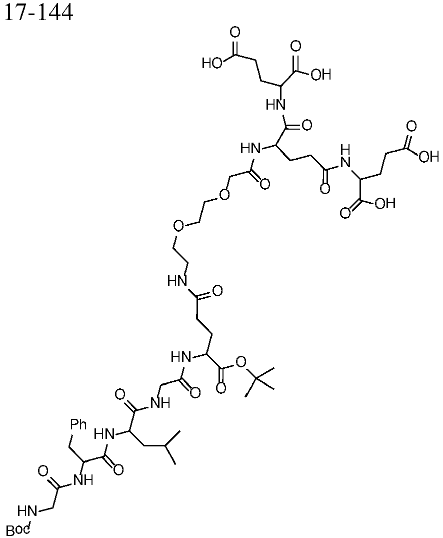

BOC-GFLG-E(OtBu){LC(EE)(OBn)₄} (0.2540 g, 0.1617 mmol, also numbered as 16-180 in this example, see 9-103 of Example 9 for its synthesis process) and 10% Pd/C (0.1000 g) were added in a reactor and then dissolved with DMF (30 mL); the air in the reactor was then pumped out to reach a vacuum state by a water pump; hydrogen was introduced to a pressure of 0.14 MPa in the reactor, hydrogen was then discharged, the reactor was pumped to reach a vacuum state by the water pump, hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth, the filter cake was washed with DMF (20 mL×3), and the filtrate was put into a 250 mL round-bottomed flask as the raw material for the next step.

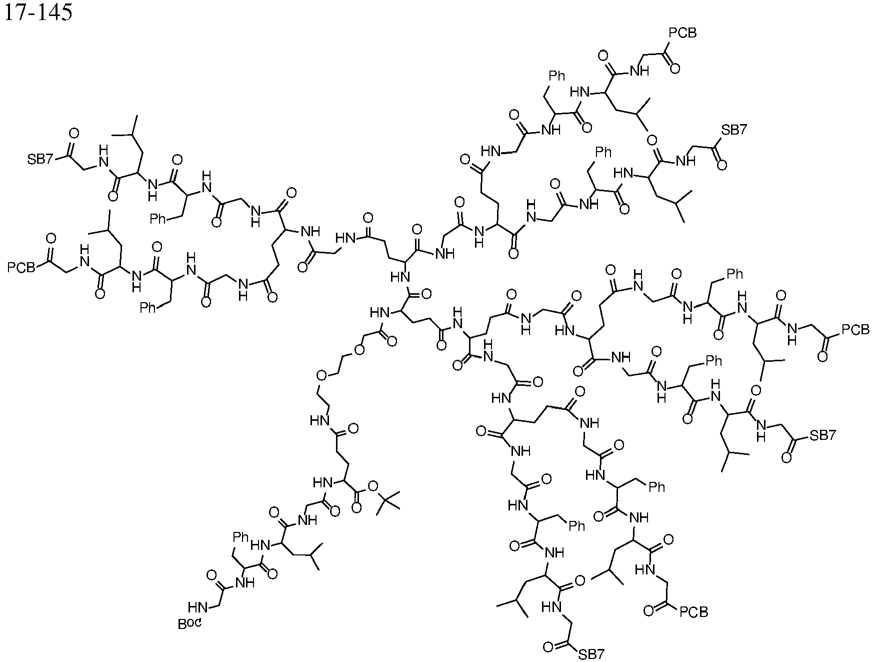

Product 17-102 (1.4000 g, 0.7440 mmol, for its structure and synthesis process, refer to the synthesis of Compound L-10 in Example M-10 of PCT International Patent Application PCT/CN2018/073662, with a difference of replacing Boc-GLG-OBn with Boc-GFLG-OBn), HBTU (0.3679 g, 0.9702 mmol), and HOBT (0.1311 g, 0.9702 mmol) were added to a DMF (90 mL) solution containing Product 17-144 (0.1957 g, 0.1617 mmol), the obtained solution was stirred for about 10 min at -5 °C; then, DIEA (0.4811 mL, 2.9106 mmol) was dropwise added to the reaction solution slowly over 1 min to further react at -5 °C for 1h, and then the reaction solution was stirred overnight at room temperature to further react. At the end of the reaction, n-hexane (150 mL) was added to layer the reaction solution, the supernatant was discarded, and n-hexane (150 mL × 4) was added to the lower liquid to obtain an oily final product; methyl tert-butyl ether (100 mL) was added to the oily product to separate out a solid; suction filtering was carried out, the resulting filter cake was dissolved with a methanol (5 mL)-dichloromethane (20 mL) solution, and ethyl acetate (100 mL) was then added to separate out a solid; then, suction filtering was carried out and the resulting filter cake was dried in a vacuum oven, thus obtaining 21.4 g of Product 17-145 with a yield of 100%.

¹H-NMR (400 MHz, DMSO-d₆) δ 10.14 (s, 4H), 9.12 - 8.93 (m, 5H), 8.51 - 7.76 (m, 66H), 7.53 (d, *J* = 8.9 Hz, 14H), 7.36 - 7.12 (m, 74H), 5.89 - 5.67 (m, 7H), 4.55 (s, 15H), 4.41 - 4.23 (m, 23H), 4.13 - 3.99 (m, 29H), 3.93 - 3.62 (m, 59H), 3.24 - 2.89 (m, 25H), 2.80 (s, 20H), 2.42 (s, 11H), 2.35 - 2.12 (m, 40H), 1.99 (s, 18H), 1.92 - 1.68 (m, 27H), 1.61 - 1.43 (m, 33H), 1.36 (d, *J* = 7.2 Hz, 15H), 1.20 (s, 6H), 1.18 (s, 9H), 1.16 (s, 5H), 0.99 - 0.75 (m, 61H), 0.51 (s, 8H).

MALDI-TOF MS: [M+H⁺] 8658.13

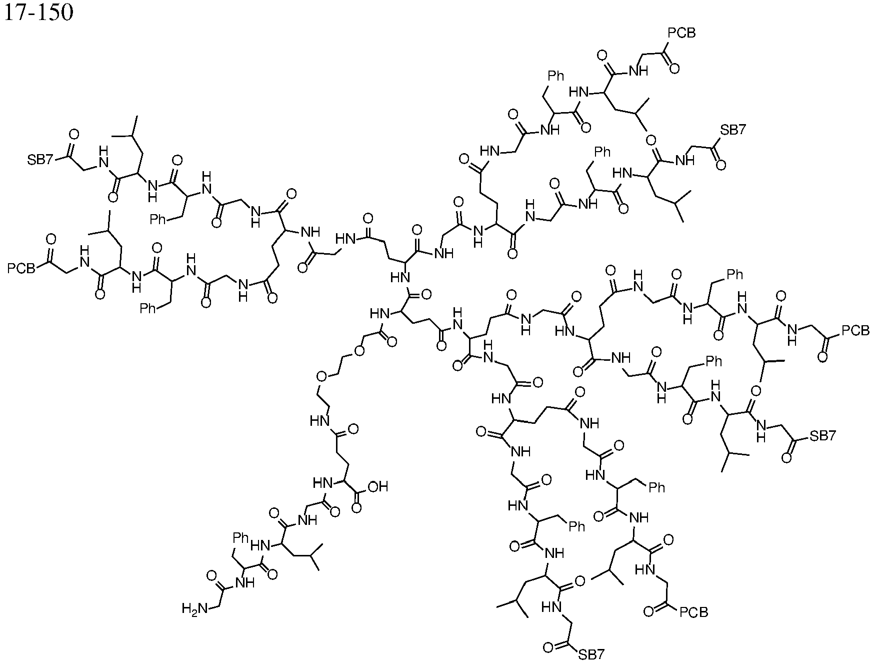

Product 17-145 (1.4010 g, 0.1617 mmol) was added in a 250 mL flask and then dissolved with dichloromethane (7 mL), TFA (2.0000 mL, 26.9315 mmol) was then added in the flask, and the mixed solution was stirred overnight at room temperature to react. At the end of the reaction, the reaction solution was concentrated and precipitated with methyl tert-butyl ether (100 mL) to obtain a solid; suction filtering was then carried out, the filter cake was washed with methyl tert-butyl ether (50 mL×3) and then dissolved with a mixed solvent of methanol (10 mL)-dichloromethane (40 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 6% of methanol were carried out; the elution product was then collected, concentrated, evaporated to dryness, and dried in a vacuum oven, thus obtaining 0.5088 g of Product 17-150 with a yield of 37%.

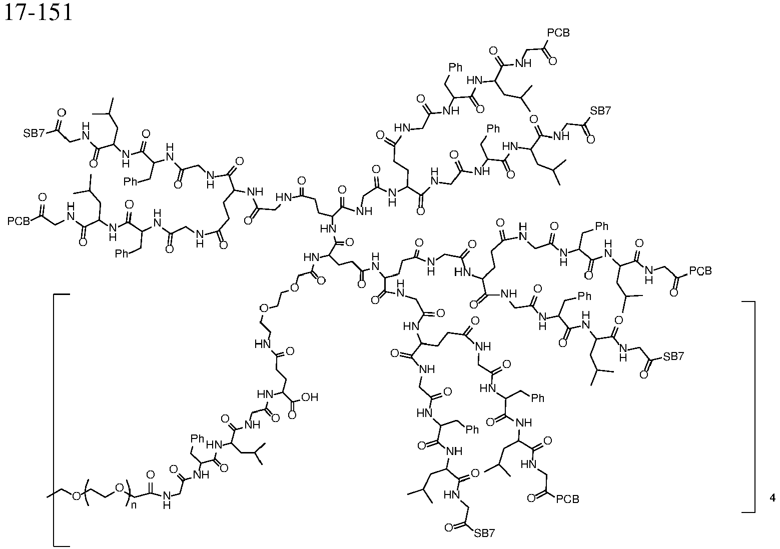

Product 17-150 (0.5088 g, 0.0598 mmol) was added to a 250 mL flask and then dissolved with DMF (20 mL); the mixed solution was stirred at -5 °C at a low speed for about 10 min to react, DIEA (0.1000 mL, 0.6050 mmol) was slowly added dropwise, 4ARM-SCM-40K (0.5702 g, 0.0136 mmol, purchased from JenKem) was then added, and the obtained solution was stirred for a week in the dark at room temperature at a low speed to react. At the end of the reaction, n-hexane (150 mL) and methyl tert-butyl ether (50 mL) were added to layer the reaction solution, the supernatant was discarded, n-hexane (150 mL) and methyl tert-butyl ether (50 mL) were further added to the lower oily solution, and such operations were repeated three times to separate out a solid; the obtained solution was filtered, the resulting filter cake was dissolved with a mixed solvent of methanol (10 mL) and dichloromethane (40 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 5%-7% of methanol were carried out; the elution product was then collected, concentrated, evaporated to dryness, and dried in a vacuum oven, thus obtaining 0.8420 g of Product 17-151 with a yield of 82%.

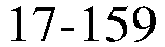

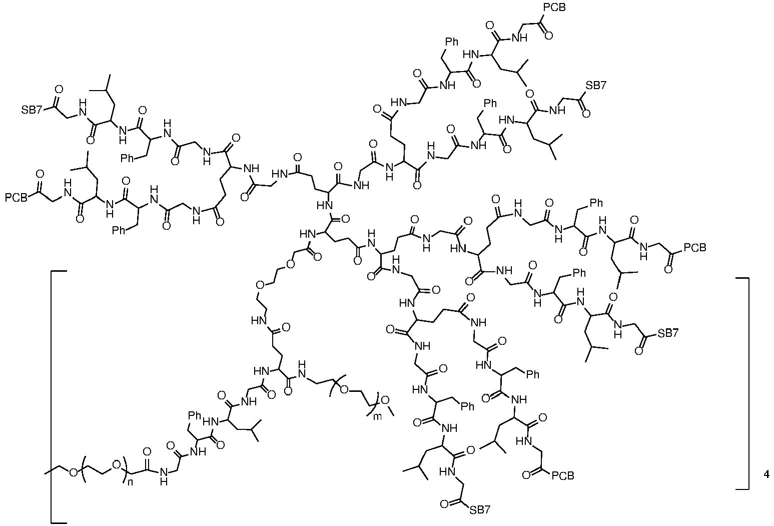

Product 17-151 (0.4100 g,0.0054 mmol) was added to a 250 mL flask and then dissolved with DMF (20 mL), and M-NH₂-3K.HCl (0.1012 g, 0.0326 mmol), HBTU (0.1235 g, 0.3257 mmol), and HOBT (0.0408 g, 0.3257 mmol) were then added to the obtained solution; the mixed solution was stirred at -5 °C at a low speed for about 20 min to react, DIEA (0.1970 mL, 1.1940 mmol) was then slowly added dropwise, and the obtained solution further reacted at -5 °C for 1h; and the solution was stirred for 8 days in the dark at room temperature at a low speed to react. At the end of the reaction, n-hexane (100 mL) and methyl tert-butyl ether (50 mL) were added to the solution, the supernatant was discarded, and such operations were repeated twice to separate out a solid; the obtained solution was filtered by suction, the resulting filter cake was dissolved with a mixed solvent of methanol (10 mL) and dichloromethane (40 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 6%-7% of methanol were carried out; the elution product was then collected, concentrated, evaporated to dryness, and dried for 1 h in a vacuum oven; the product was dissolved with absolute ethanol (10 mL) and dichloromethane (3 mL), and methyl tert-butyl ether (100 mL) was added to the obtained solution to separate out a solid; the solution was filtered and the filter cake was washed with methyl tert-butyl ether (50 mL×3) and dried with the vacuum oven, thus obtaining 0.4132 g of Product 17-159 with a yield of 86%.

### Example 3: Synthesis of Compound 17-161

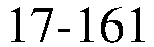

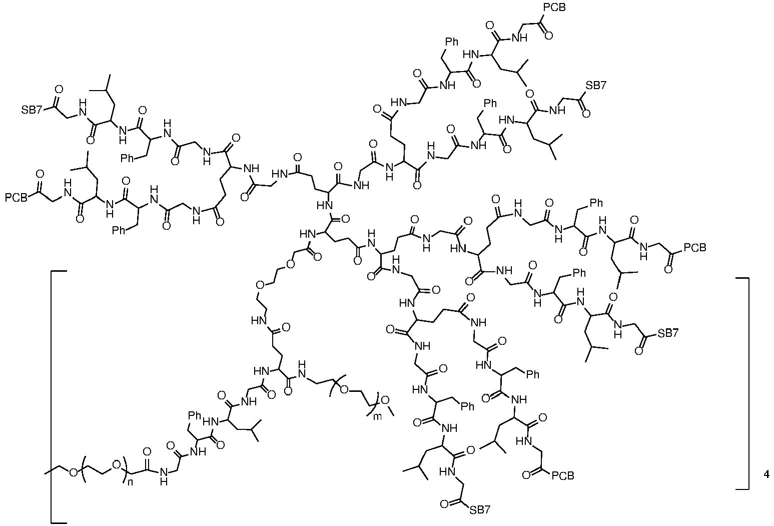

Product 17-151 (0.4100 g,0.0054 mmol) was added to a 250 mL flask and then dissolved with DMF (25 mL), and M-NH₂-5K.HCl (0.1929 g, 0.0367 mmol), HBTU (0.0930 g, 0.2460 mmol), and HOBT (0.0330 g, 0.2460 mmol) were then added to the obtained solution; the mixed solution was stirred at -5 °C for about 10 min to react, DIEA (0.1490 mL, 0.9030 mmol) was then slowly added dropwise, and the obtained solution further reacted at -5 °C for 30min; and the solution was stirred in the dark at a low speed at room temperature for 5 days. At the end of the reaction, n-hexane (100 mL) and methyl tert-butyl ether (50 mL) were added to the solution to separate out a solid; the obtained solution was filtered by suction, the resulting filter cake was washed twice with methyl tert-butyl ether (50 mL × 2) and dissolved with a mixed solvent of methanol (10 mL) and dichloromethane (40 mL), silica gel powder (15 g) was added to the obtained solution, and the solution was then evaporated to dryness to obtain a powdery solid; the operations of dry sample loading, column chromatography, and gradient elution with a dichloromethane mixed solution containing 1% of ammonia water and 4%-7% of methanol were carried out; the elution product was then collected, concentrated, evaporated into a solid, and dried for 2 h in a vacuum oven; the product was dissolved with absolute ethanol (10 mL) and dichloromethane (5 mL), and methyl tert-butyl ether (100 mL) was added to the obtained solution to separate out a solid; the solution was filtered and the filter cake was washed with methyl tert-butyl ether (50 mL×3) and dried, thus obtaining 0.5198 g of Product 17-161 with a yield of 84%.

### Example 4: Synthesis of Compound 20-107

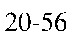

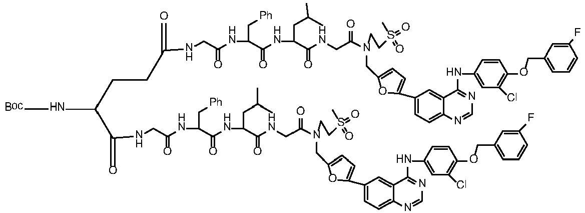

Boc-Glu-OH (0.739 g, 2.9902 mmol), GFLG-LPT (6.0 g, 6.2794 mmol), HOBT (1.212 g, 8.9706 mmol), and HBTU (3.402 g, 8.9706 mmol) were weighed and then dissolved in DMF solution, the obtained solution was placed in a low-temperature constant temperature bath (-5 °C) and stirred for 30min, DIEA (4.44 mL, 26.9118 mmol) was then added dropwise, and the obtained solution first reacted at a low temperature for 2 h and then stirred at room temperature to react. At the end of the reaction, the reaction solution was extracted three times with saturated sodium bicarbonate solution and ethyl acetate (150 mL×3), and the obtained organic phases were combined and washing with a saturated NaCl solution was carried out three times (150 mL×3). The obtained solution was concentrated and then subjected to dry sample loading and column chromatography. Gradient elution with 4% methanol/dichloromethane-10% methanol/dichloromethane was carried out. The elution product was evaporated to dryness, thus obtaining 5.3 g of the product with a yield of 84%.

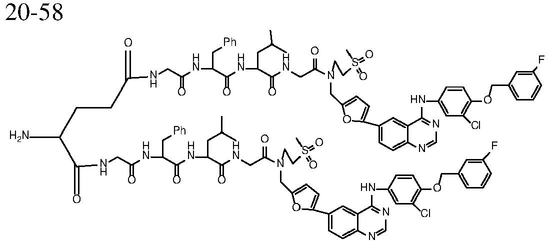

Product 20-56 (5.3 g, 2.4974 mmol) was added in a 250 mL flask and then dissolved with dichloromethane (20 mL), TFA (5.56 mL, 74.92 mmol) was then added, and the solution was stirred at room temperature to react. At the end of the reaction, the reaction solution was precipitated with methyl tert-butyl ether (100 mL) and n-hexane (200 mL) and then evaporated to dryness. The operations of dry sample loading and column chromatography were carried out. With the column height of 10cm, gradient elution with 1% ammonia water: 4% methanol/dichloromethane-1% ammonia water: 6% methanol/dichloromethane was carried out, and the elution product was evaporated to dryness, thus obtaining 4.5 g of the product with a yield of 90%.

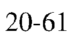

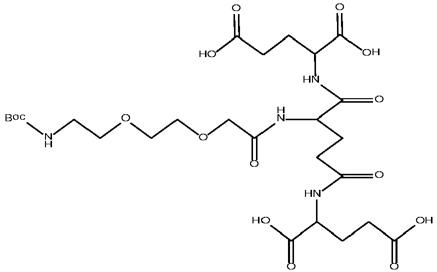

The reactant Boc-LC-E[E(OBn)₂]₂ (0.469 g, 0.464 mmol, see the synthesis of 16-148 for its synthesis process) was added in a hydrogenation reactor, DMF was added dropwise along the inner wall to dissolve the reactant, 10% Pd/C (0.1 g) was then added, the device was set ready and the air was pumped out to reach a vacuum state, hydrogen (16 psi) was introduced, and such operations were repeated three times. The reaction solution was stirred overnight. At the end of the reaction, diatomaceous earth was added to a sand core funnel to carry out suction filtering of the reaction solution, and the diatomaceous earth was then washed three times with DMF (25 mL×3), thus obtaining the product solution for next reaction.

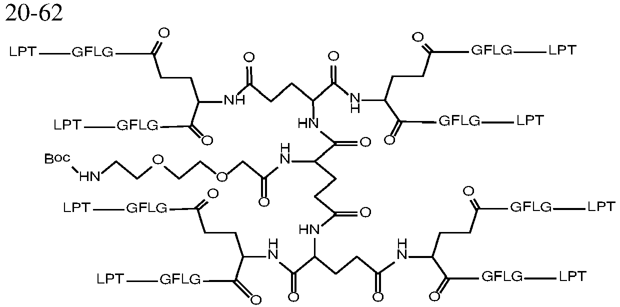

Product 20-61 (0.464 mmol), Product 20-58 (4.5 g, 2.228 mmol), HOBT (0.376 g, 2.785 mmol), and HBTU (1.05 g, 2.785 mmol) were weighed and added to the DMF solution (100 mL) of Product 20-61; the obtained solution was placed in a low-temperature constant temperature bath (-5 °C) and stirred for 30min, DIEA (1.38 mL, 8.355 mmol) was added dropwise, and the mixed solution first reacted at a low temperature for 2 h and then was stirred at room temperature to react. At the end of the reaction, the reaction solution was precipitated with methyl tert-butyl ether (100 mL) and n-hexane (200 mL) and then evaporated to dryness. The operations of dry sample loading and column chromatography were carried out. With the column height of 15cm, gradient elution with 6% methanol/dichloromethane-10% methanol/dichloromethane was carried out, and the elution product was evaporated to dryness and purified again.

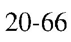

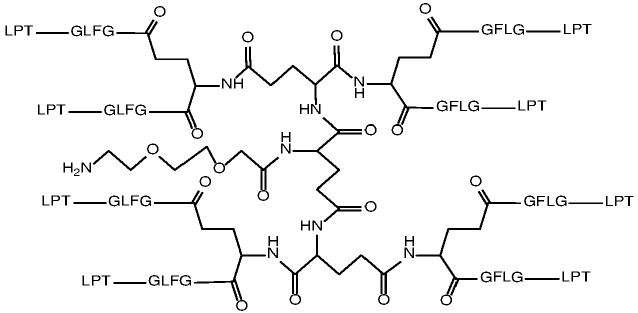

The reactant 20-62 (3.8 g) was added to dichloromethane (20 mL), TFA (0.96 mL, 12.9 mmol) was added, and the mixed solution was stirred at room temperature to react. At the end of the reaction, the reaction solution was concentrated, and methyl tert-butyl ether (50 mL) and n-hexane (50 mL) were added to precipitate the concentrated solution, thus obtaining a powder. The column chromatography was carried out; with the column height of 10cm, gradient elution with 1% ammonia water: 4% methanol/dichloromethane - 1% ammonia water: 6% methanol/dichloromethane was carried out. The elution product was evaporated to dryness and dried in vacuum, thus obtaining 1.4 g of the product with a yield of 37.8%.

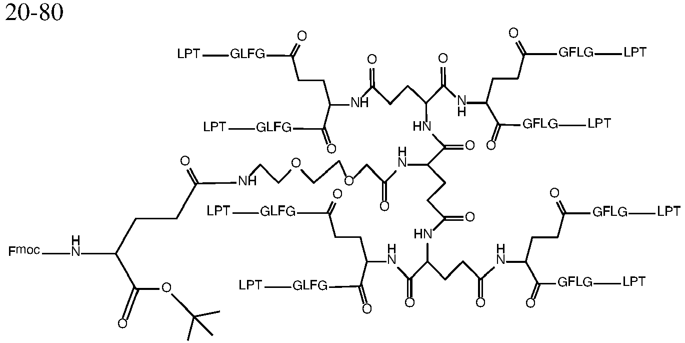

Fmoc-Glu-OtBu (0.08 g, 0.193 mmol), Product 20-66 (1.4 g, 0.1614 mmol), HOBT (0.0326 g, 0.242 mmol), and HBTU (0.091 g, 0.242 mmol) were weighed and dissolved with the DMF solution (40 mL); the obtained solution was placed in a low-temperature constant temperature bath (-5 °C) and stirred for 30min, DIEA (0.119 mL, 0.726 mmol) was added dropwise, and the mixed solution first reacted at a low temperature for 2 h and then was stirred at room temperature to react. At the end of the reaction, the reaction solution was concentrated, and methyl tert-butyl ether (100 mL) and n-hexane (200 mL) were added to precipitate the concentrated solution, thus obtaining a powder.

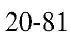

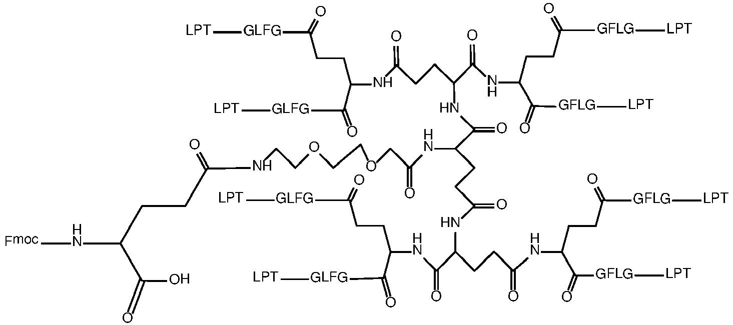

Product 20-80 was added in a 250 mL flask and then dissolved with dichloromethane (10 mL) and TFA (0.35 mL, 4.842 mmol), and the obtained solution was stirred at room temperature to react. At the end of the reaction, the reaction solution was concentrated, and methyl tert-butyl ether (100 mL) and n-hexane (200 mL) were added to precipitate the concentrated solution, thus obtaining a powder. The powder was dried in vacuum for next step.

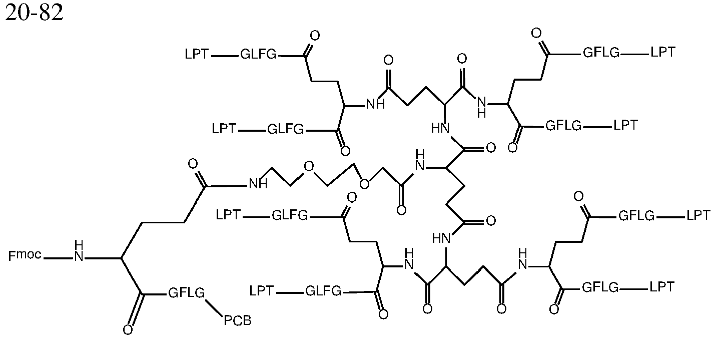

Product 20-81 (0.1614 mmol), GFLG-PCB (0.17 g, 0.2098 mmol), HOBT (0.0326 g, 0.242 mmol), and HBTU (0.091 g, 0.242 mmol) were weighed and then dissolved with the DMF solution (40 mL); the obtained solution was placed in a low-temperature constant temperature bath (-5 °C) and stirred for 30min, DIEA (0.119 mL, 0.726 mmol) was added dropwise, and the mixed solution first reacted at a low temperature for 2 h and then was stirred at room temperature to react. At the end of the reaction, the reaction solution was concentrated, and methyl tert-butyl ether (100 mL) and n-hexane (200 mL) were added to precipitate the concentrated solution, thus obtaining a powder for next step.

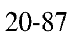

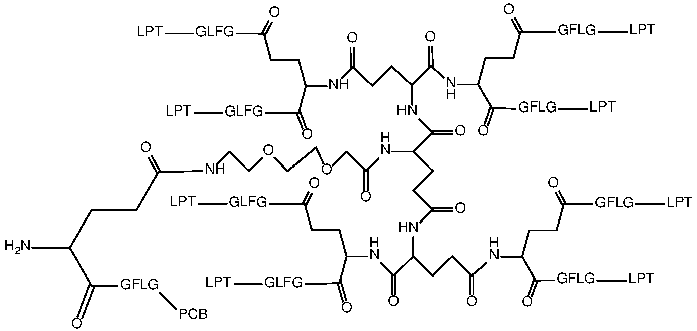

Product 20-82 (0.1614 mmol) was added in a 500 mL flask and then dissolved with the DMF solution (100 mL), morpholine (0.42 mL) was added in the flask, the mixed solution was stirred at room temperature to react, and TLC was carried out every 0.5h. Three hours later, the reaction ended. Methyl tert-butyl ether (100 mL) and n-hexane (200 mL) were added to precipitate the solution, thus obtaining a powder. The operations of column chromatography, including dry sample loading and elution with 1% ammonia water: 6% methanol/dichloromethane were carried out, thus obtaining 1.1 g of the product with a yield of 71%.

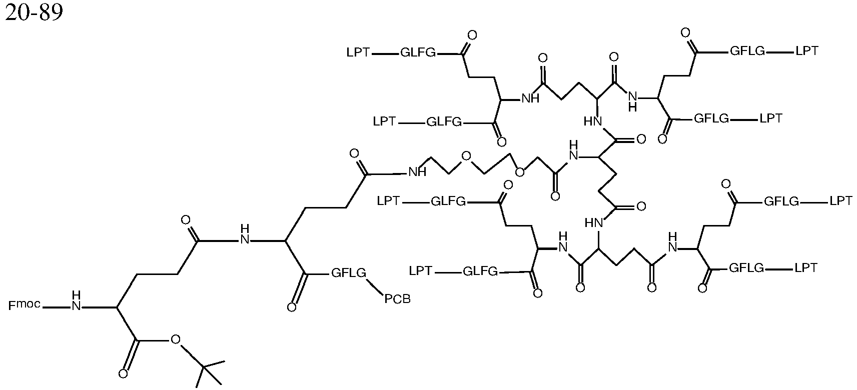

Fmoc-Glu-OtBu (0.059 g, 0.1388 mmol), Product 20-87 (1.1 g, 0.1157 mmol), HOBT (0.023 g, 0.1735 mmol), and HBTU (0.065 g, 0.1735 mmol) were weighed; the obtained solution was placed in a low-temperature constant temperature bath (-5 °C) and stirred for 30min, DIEA (0.086 mL, 0.5206 mmol) was added dropwise, and the mixed solution first reacted at a low temperature for 2 h and then was stirred at room temperature to react. At the end of the reaction, methyl tert-butyl ether (100 mL×2) and n-hexane (200 mL×2) were added to precipitate the reaction solution, thus obtaining a powder. The powder was dried in vacuum for next step.

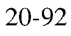

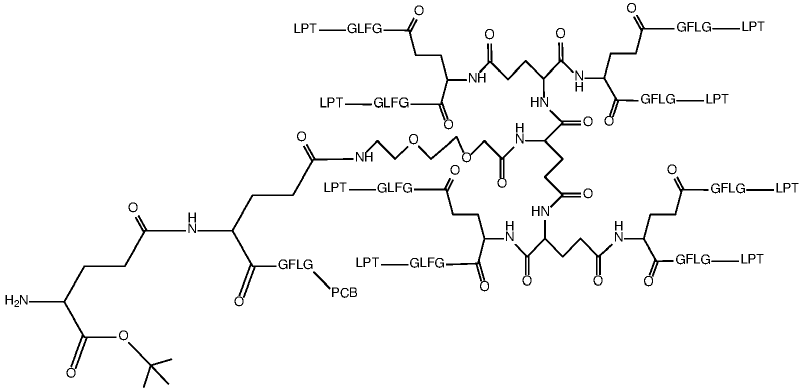

Product 20-89 (0.1157 mmol) was weighed and added in a 250 mL flask and then dissolved with the DMF solution (30 mL), morpholine (0.3 mL, 3.3471 mmol) was added in the flask, the mixed solution was stirred at room temperature to react, and TLC was carried out every 0.5h. Three hours later, the reaction ended. Methyl tert-butyl ether (100 mL) and n-hexane (200 mL) were added to precipitate the solution, thus obtaining a powder. The product was dried in vacuum, thus obtaining 1.3 g of the product.

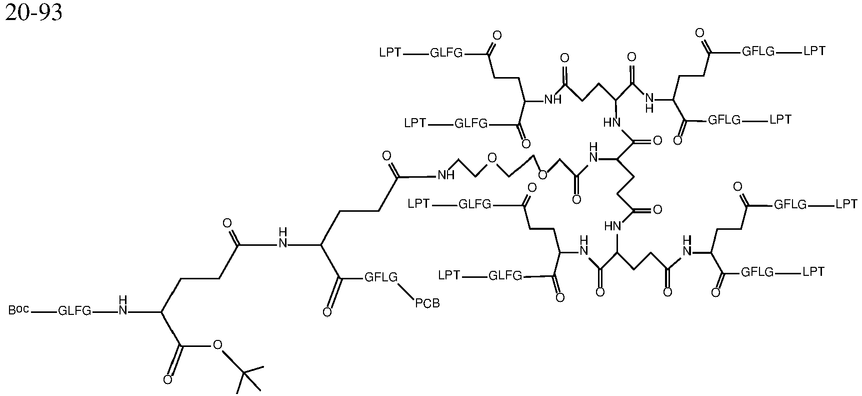

Boc-GFLG-OH (0.1389 mmol), Product 20-92 (0.1157 mmol), HOBT (0.023 g, 0.1735 mmol), and HBTU (0.065 g, 0.1735 mmol) were weighed; the obtained solution was placed in a low-temperature constant temperature bath (-5 °C) and stirred for 30min, DIEA (0.086 mL, 0.5206 mmol) was added dropwise, and the mixed solution first reacted at a low temperature for 2 h and then was stirred at room temperature to react. At the end of the reaction, methyl tert-butyl ether (100 mL×2) and n-hexane (200 mL×2) were added to precipitate the reaction solution, thus obtaining a powder.

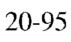

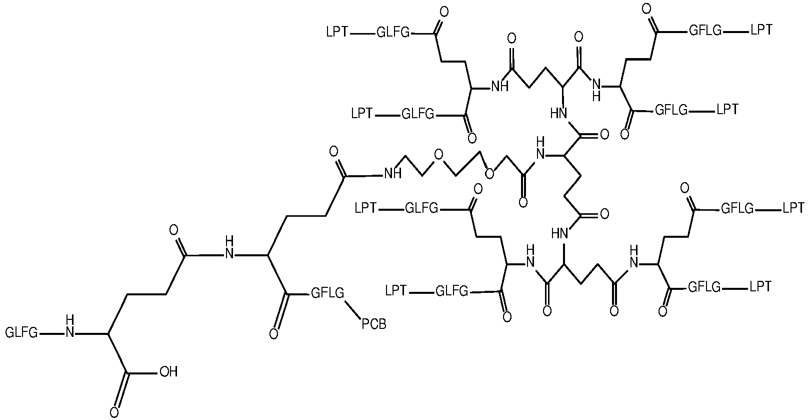

Product 20-93 (0.1157 mmol) was weighed and added in a 250 mL flask and then dissolved with dichloromethane (5 mL) and TFA (10 mL) in a condition of ultrasonic, and the obtained solution was stirred at room temperature to react. At the end of the reaction, the reaction solution was concentrated by evaporating to dryness. When a little concentrated product was obtained, methyl tert-butyl ether (100 mL) was added to it and ultrasonic treatment was then carried out; n-hexane (200 mL) was added to precipitate the solution to obtain a powder, and the powder was eluted with 1% ammonia: 8% methanol/dichloromethane, thus obtaining 0.6 g of the product.

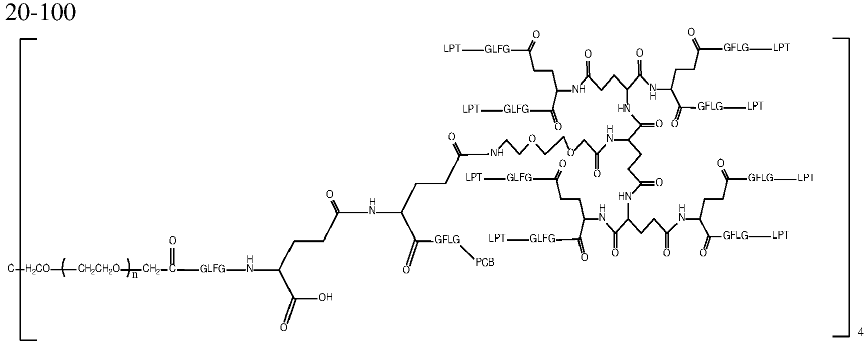

Product 20-95 (0.6 g) was weighed and added in a 250 mL flask and then dissolved with DMF (90 mL), DIEA (0.3 mL) was added dropwise, and 4ARM-SCM-40K (0.5 g) and dichloromethane (5 mL) were then added in sequence. The obtained solution reacted in the dark at room temperature and stirred at low speed. At the end of the reaction, methyl tert-butyl ether (150 mL) was added in a conical flask, the reaction solution was poured into the conical flask, and then n-hexane (200 mL) was added. Column chromatography was carried out with the column height of 7cm. Elution with an eluent (1% ammonia water: 8% methanol/dichloromethane) was carried out, thus obtaining 0.9 g of the product.

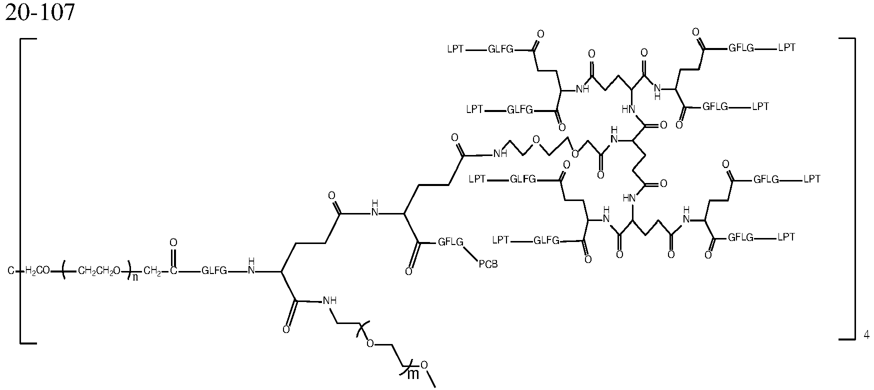

M-NH₂-3K·HCl (0.22 g, 0.1246 mmol), 20-98 (0.9 g,), HOBT (0.009 g, 0.073 mmol), and HBTU (0.027 g, 0.073 mmol) were weighed and added in a 500 mL flask and then dissolved with the DMF solution (50 mL); the obtained solution was placed in a low-temperature constant temperature bath, and 30 min later, DIEA (2 mL) was added dropwise, and one hour later, the solution was taken out and stirred in the dark at room temperature at a low speed to react. At the end of the reaction, TLC was carried out and no obvious point was observed. Methyl tert-butyl ether (150 mL) was added in a conical flask, the reaction solution was poured into the conical flask, and then n-hexane (200 mL) was added to generate precipitate, and then the solution was filtered by suction. The operations of dry sample loading and column chromatography were carried out on the solid. With the column height of 5 cm, elution with 1% ammonia water: 5% methanol/dichloromethane was carried out, the elution product was evaporated to dryness and then dissolved with absolute ethanol (5 mL) and dichloromethane (2 mL); the obtained solution was treated by ultrasonic to obtain homogeneous phase; n-hexane (100 mL) was added and then suction filtering was carried out. The process of dissolution and precipitation was repeated three times. The precipitate was dried in vacuum, thus obtaining 0.3 g of the product.

### Example 5: Synthesis of Compound 24-14

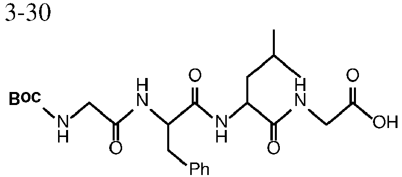

Boc-GFLG-OBn (home-made 4.0047 g, 6.8646 mmol) and 10% Pd/C (0.1006 g) were added in a hydrogenation reactor, DMF (25 mL) was then added, the device was set ready, the air was pumped out to reach a vacuum state, H2 (16psi) was introduced, and after the reaction continued for 2 min, the air was pumped and H2 was introduced again; such operations were repeated three times and air inlet and pump valves were closed (it should be noted that the vacuum pump was not closed during the whole operation) to seal H₂ in the reactor, and the reaction solution was placed at room temperature overnight. At the end of the reaction, diatomaceous earth was used as a filter cake to carry out suction filtering, and then the reactor and the funnel were washed with DMF three times (25 mL×3), and the solution was then transferred to a 250 mL flask as the raw material for Product 3-31.

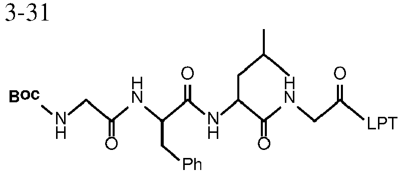

LPT (Lapatinib, 3.7999 g, 6.5377 mmol), HBTU (3.7195 g, 9.8066 mmol) and HOBT (1.3253 g, 9.8066 mmol) were weighed and added to the DMF solution (100 mL) of Product 3-30, and the obtained solution was placed in a low-temperature constant temperature reaction bath (-5 °C) to react for 20min, DIEA (5 mL, 12.8714 mmol) was added dropwise, and the mixed solution was first stirred at a low temperature for 2 h and then taken out and placed at room temperature overnight on a magnetic stirrer. At the end of the reaction, the reaction solution was transferred to a 2 L separatory funnel, saturated sodium bicarbonate solution (300 mL) and ethyl acetate (400 mL) were added to the separatory funnel, the obtained solution was shaken thoroughly to separate the organic phase, and the aqueous phase was extracted three times with ethyl acetate (150 mL× 3); the obtained organic phases were combined, washing with a saturated sodium bicarbonate solution (150 mL) was carried out once, and washing with a saturated sodium chloride solution was carried out twice (150 mL×2); the obtained organic phase was poured into a 2 L conical flask, dried with anhydrous magnesium sulfate, and then filtered by suction and concentrated to 100 mL, silica gel powder (15 g) was added to the concentrated solution and the obtained solution was subjected to rotary evaporation to obtain a solid solution; the operations of dry sample loading and column chromatography were then carried out. Elution with 0.5% ammonia water: 2% methanol/dichloromethane was carried out; the product was collected and rotary evaporated to dryness to obtain an oily product; the oily product was dissolved with ethyl acetate (50 mL); n-hexane (100 mL) was added to the obtained solution, and the solution was homogenized by ultrasonic and then filtered by suction; and the obtained solution was dried in vacuum, thus obtaining 5.9098 g of the product with a yield of 85.65%.

¹H-NMR (400 MHz, DMSO-d₆) δ 9.87 (s, 1H), 8.75 (s, 1H), 8.56 (s, 1H), 8.12 (s, 4H), 7.91 - 7.64 (m, 3H), 7.56 - 7.41 (m, 1H), 7.40 - 6.84 (m, 10H), 6.62 (m, 2H), 5.27 (s, 2H), 4.73 (s, 2H), 4.55 (s, 1H), 4.48 - 4.11 (m, 3H), 4.03 (s, 1H), 3.89 - 3.39 (m, 6H), 3.03 (s, 4H), 2.69 (s, 3H), 1.99 (s, 1H), 1.70 - 1.41 (m, 4H), 1.40 - 1.09 (m, 9H), 0.84 (s, 6H).

MALDI-TOF MS: [M+H⁺] 1055.30, [M+Na⁺] 1077.25

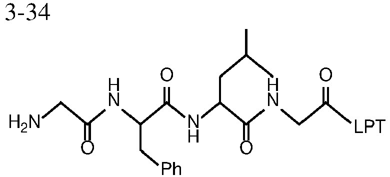

Product 3-31 (5.9 g, 5.5891 mmol) was added in a 250 mL flask and dissolved with dichloromethane (15 mL), TFA (4.2 mL, 55.8910 mmol) was then added to the solution, and the obtained solution was stirred overnight at room temperature. At the end of the reaction, the reaction solution was evaporated to remove the solvent; the obtained product was dissolved with ethyl acetate and the obtained solution was then transferred to a 2 L separatory funnel; saturated sodium bicarbonate solution (300 mL, being in aqueous phase and alkaline) and ethyl acetate (400 mL) were added to the separatory funnel, the obtained solution was shaken thoroughly to separate the organic phase, and the aqueous phase was extracted three times with ethyl acetate (150 mL× 3); the obtained organic phases were combined, washing with a saturated sodium bicarbonate solution (150 mL) was carried out once, and washing with a saturated sodium chloride solution was carried out twice (150 mL×2); the obtained organic phase was poured into a 2 L conical flask, dried with anhydrous magnesium sulfate, and then filtered by suction and concentrated to 100 mL; n-hexane (100 mL) was added to the concentrated solution, and the mixed solution was treated by ultrasonic into a homogeneous phase and then filtered by suction to obtain a product; silica gel powder (15 g) was added to the filtrate and the obtained solution was subjected to rotary evaporation to obtain a solid solution; the operations of dry sample loading, column chromatography, elution with 1% ammonia water: 5% methanol/chloromethane, and rotary evaporation were carried out, thus obtaining 4.0926 g of the product with a yield of 76.64%.

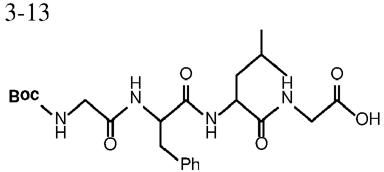

Boc-GFLG-OBn (2.0036 g, 3.4323 mmol) and 10% Pd/C (0.05502 g) were added in a hydrogenation reactor, DMF (30 mL) was then added to the hydrogenation reactor, the device was set ready, the air was pumped out to reach a vacuum state, H2 (16psi) was introduced, and after the reaction continued for 2 min, the air was pumped and H2 was introduced again; such operations were repeated three times and air inlet and pump valves were closed (it should be noted that the vacuum pump was not closed during the whole operation) to seal H2 in the reactor, and the reaction solution was placed at room temperature overnight. At the end of the reaction, diatomaceous earth was used as a filter cake to carry out suction filtering, and then the reactor and the funnel were washed with DMF three times (25 mL×3), and the solution was then transferred to a 250 mL flask as the raw material for Product 3-14.

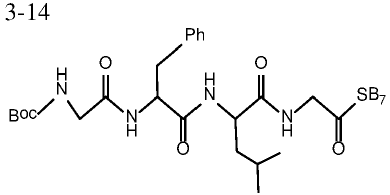

SB7 (1.4790 g, 2.8603 mmol, also referred to as SB-743921), HBTU (1.6270 g, 4.2905 mmol) and HOBT (0.5797 g, 4.2905 mmol) were weighed and added to the flask with Product 3-13, and the reaction solution was placed in a low-temperature constant temperature reaction bath (-5 °C) to react for 20min, DIEA (2.2 mL, 12.8714 mmol) was added dropwise, and the mixed solution first reacted for 2 h and then taken out and placed at room temperature overnight on a magnetic stirrer. At the end of the reaction, the reaction solution was transferred to a 2 L separatory funnel, saturated sodium bicarbonate solution (300 mL) and ethyl acetate (400 mL) were added to the separatory funnel, the obtained solution was shaken thoroughly to separate the organic phase, and the aqueous phase was extracted three times with ethyl acetate (150 mL× 3); the obtained organic phases were combined and washing with a saturated sodium chloride solution (300 mL) was carried out three times; the obtained organic phase was poured into a 2 L conical flask, dried with anhydrous magnesium sulfate, and then filtered and concentrated, silica gel powder (12 g) was added to the concentrated solution and the obtained solution was evaporated to obtain a solid powder; the operations of dry sample loading, column chromatography, and gradient elution with a dichloromethane solution containing 2% of methanol and 1% of ammonia water were carried out, thus obtaining 3.7 g of the product with a yield of 100%.

¹H-NMR (400 MHz, DMSO-d₆) δ = 8.17 - 8.06 (m, 3H),7.92-7.89 (m, 2H), 7.58 - 7.52 (m, 2H), 7.12-7.10(m, 13H), 6.93-6.92 (m, 1H), 6.53-6.53 (m, 1H), 4.51-4.50 (m, 1H), 4.25-4.24 (m, 1H), 4.14-4.13 (m, 1H), 3.89-3.88 (m, 1H), 3.60 - 3.41 (m, 4H), 3.10 - 2.94 (m,2H), 2.81-2.80 (m,2H), 2.60-2.59 (m,2H), 2.32-2.32 (m, 4H), 1.51-1.49 (m, 3H), 1.35 -1.33(m, 9H), 1.12-1.10 (m,1H), 0.95 -0.87(m, 1H),0.87- 0.78 (m, 9H), 0.51-0.50(m, 3H). MALDI-TOF MS: [M+H⁺]991.45, [M+Na⁺] 1013.40

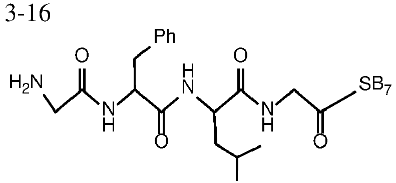

Product 3-14 (3.6 g, 3.6304 mmol) was added in a 250 mL flask and dissolved with dichloromethane (15 mL), TFA (2.7 mL, 36.304 mmol) was then added to the solution, and the obtained solution was stirred overnight at room temperature. At the end of the reaction, the reaction solution was evaporated to remove the solvent; the obtained product was dissolved with ethyl acetate and the obtained solution was then transferred to a 2 L separatory funnel; saturated sodium bicarbonate solution (300 mL, the aqueous phase was alkaline) and ethyl acetate (400 mL) were added to the separatory funnel, the obtained solution was shaken thoroughly to separate the organic phase, and the aqueous phase was extracted three times with ethyl acetate (100 mL× 3); the obtained organic phases were combined, washing with a saturated sodium bicarbonate solution (100 mL) was carried out once, and washing with a saturated sodium chloride solution was carried out twice (100 mL×2); the obtained organic phase was poured into a 2 L conical flask, dried with anhydrous magnesium sulfate, and then filtered and concentrated; silica gel powder (10 g) was added to the concentrated solution and the obtained solution was evaporated to dryness to obtain a solid powder; the operations of dry sample loading, column chromatography and elution with 5% methanol/1% ammonia water/dichloromethane; the elution product was collected and evaporated, thus obtaining 1.7587 g of the product with a yield of 54.33%.

¹H-NMR (400 MHz, DMSO-d₆)δ 8.17 (s, 1H), 8.03-8.02 (m, 3H), 7.87 -7.86(m, 1H), 7.53 ― 7.42 (m, 2H), 7.14 -7.12(m, 16H), 4.49 -4.48(m, 1H), 4.20 -4.19(m, 1H), 4.08-4.07 (m, 1H), 3.83-3.82 (m, 1H), 3.47 -3.46(m, 4H), 2.92-2.90 (m,4H), 2.76-2.75(m, 1H), 2.65 -2.64(m, 1H), 2.25-2.22 (m,4H), 1.79-1.77(m, 2H), 1.51 - 1.36 (m, 3H), 1.25 -1.24(m, 1H), 1.12 - 0.99 (m, 1H), 0.88 - 0.72 (m, 12H), 0.45 -0.44(m, 3H).

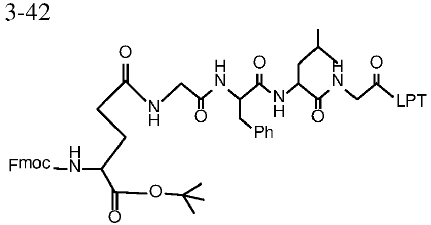

Fmoc-GLu-OtBu (1.8740 g, 4.3956 mmol) was weighed and dissolved with DMF (70 mL), the obtained solution was placed in a low-temperature constant temperature reaction bath (0 °C), and Product 3-34 (3.0007 g, 3.1397 mmol) and PyAop (2.3008 g, 4.3956 mmol) were then added; the obtained solution was stirred for 30min, TMP (0.42 mL, 3.1397 mmol) was dropwise added to the solution, and the obtained solution was stirred overnight at a low temperature. At the end of the reaction, the reaction solution was poured into a 2 L separatory funnel, deionized water (300 mL) and ethyl acetate (400 mL) were then added in the separatory funnel, the obtained solution was shaken to separate the organic phase, the aqueous phase was extracted three times with ethyl acetate (150 mL×3), the obtained organic phases were combined and then washing with saturated saline solution was carried out three times (150 mL×3). The operations of dry sample loading and column chromatography were carried out. Elution with 5% methanol/dichloromethane was carried out, thus obtaining 3.5 g of the product with a yield of 82.06%.

¹H-NMR (400 MHz, DMSO-d₆) δ 10.06 (s, 1H), 8.77 (s, 1H), 8.65 - 8.42 (m, 1H), 8.31 - 7.99 (m, 5H), 7.94 ― 7.78 (m, 2H), 7.71 (s, 5H), 7.53 ― 7.02 (m, 15H), 6.62 (s, 1H), 5.27 (s, 2H), 4.73 (s, 2H), 4.54 (s, 1H), 4.38 - 4.12 (m, 4H), 3.93 - 3.53 (m, 3H), 3.08 - 2.90 (m, 5H), 2.75-2.74 (m, 1H), 2.20 (s, 2H), 1.92 (s, 2H), 1.80 - 1.63 (m, 6H), 1.55-1.53 (m, 3H), 1.38-1.36 (m, 9H), 0.93 - 0.77 (m, 6H). MALDI-TOF MS: [M+Na⁺] 1385.35.

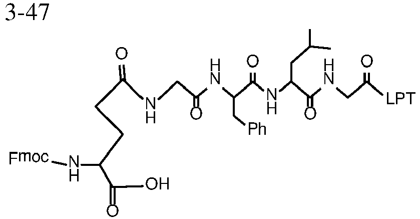

The raw material Product 3-42 (3.5 g, 2.5680 mmol) was weighed and added in a 250 mL flask and then dissolved with dichloromethane (30 mL), TFA (2 mL) was then added to the solution, and the obtained solution was stirred at room temperature overnight on a magnetic stirrer. At the end of the reaction, the reaction solution was evaporated to remove the dichloromethane, the obtained solid was dissolved with dichloromethane (10 mL), the obtained solution was precipitated with n-hexane (100 mL), treated by ultrasonic, and cooled in a refrigerator, and the supernatant was discarded; dichloromethane (10 mL) was added to the lower liquid, the obtained solution was precipitated with n-hexane (100 mL) and cooled, and the supernatant was discarded; such precipitation operations were repeated three times, and evaporation was carried out, thus obtaining 3.32 g of the product with a yield of 100%.

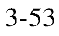

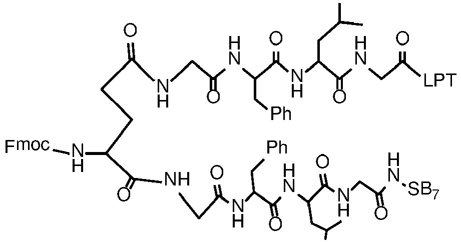

Product 3-47 (1.9 g, 1.284 mmol) was weighed and added to a reactor and then dissolved with DMF (50 mL), the obtained solution was placed in a 0 °C low-temperature constant temperature reaction bath, and Product 3-16 (1.1440 g, 1.284 mmol) and PyAop (0.6705 g, 1.284 mmol) were then added; 30 min later, TMP (0.46 mL, 1.284 mmol) was dropwise added to the solution, and the obtained solution was stirred overnight at a low temperature. At the end of the reaction, the reaction solution was poured into a 1 L separatory funnel, ethyl acetate (200 mL) was then added in the separatory funnel, the obtained solution was shaken to separate the organic phase, the aqueous phase was extracted four times with ethyl acetate (100 mL×4), the obtained organic phases were combined and then washing with a saturated sodium chloride solution was carried out twice (150 mL×2). The obtained solution was concentrated and then subjected to dry sample loading and column chromatography. Gradient elution with 5%-10% methanol/dichloromethane was carried out, thus obtaining 1.9265 g of the product with a yield of 68.82%.

¹H-NMR (400 MHz, DMSO-d₆)δ 9.87 (s, 1H), 8.74 (s, 1H), 8.56 (s, 1H), 8.27 - 7.96 (m, 9H), 7.93 - 7.46 (m, 10H), 7.42 - 7.03 (m, 30H), 6.62 (s, 1H), 5.26 (s, 2H), 4.73 (s, 2H), 4.54 (s, 2H), 4.38 - 4.06 (m,6H), 3.94 - 3.55 (m, 4H), 3.39 (s, 2H), 3.17 (s, 2H), 3.02 (s, 9H), 2.85 - 2.62 (m, 3H), 2.41 - 2.05 (m, 9H), 1.88 (s, 2H), 1.75 (s, 2H), 1.48 (s, 8H), 1.24-1.23(m, 1H), 1.10-1.09 (m, 1H), 0.97 - 0.74 (m, 15H),0.49-0.48(m, 3H)

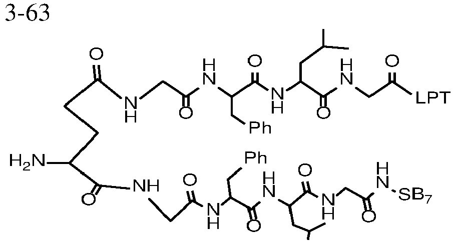

Product 3-53 (1.9 g, 0.8771 mmol) was added in a 250 mL flask and then dissolved with DMF (20 mL), the obtained solution was placed on a magnetic stirrer, morpholine (1.9 mL, 21.9275 mmol) was added to the solution, the obtained solution reacted at room temperature, and TLC was carried out every 1 h. 1.5 h later, the reaction ended, diethyl ether (200 mL) was added to the reaction solution with stirring, the obtained solution was cooled in a refrigerator, and the supernatant was discarded when precipitate came out of the solution; the precipitate was dissolved with dichloromethane (10 mL), the obtained solution was precipitated with n-hexane and then cooled, and the supernatant was discarded; such precipitation operation was repeated three times, rotary evaporation was then carried out to remove the solvent, thus obtaining 1.5 g of the product with a yield of 88.24%.

¹H-NMR (400 MHz, DMSO-d₆) δ 9.87 (s, 1H), 8.75 (s, 1H), 8.56 (s, 1H), 8.28 - 7.97 (m, 11H), 7.81 - 7.71 (m,2H), 7.51-7.49 (m, 3H), 7.39 - 7.05 (m, 25H), 6.76 - 6.51 (m, 1H), 5.27 (s, 2H), 4.73 (s, 2H), 4.54 (s, 2H), 4.38 - 4.09 (m,5H), 3.70 -3.65(m, 8H), 3.11-3.06 (m, 8H), 2.89 (s, 1H), 2.82 - 2.66 (m,5H), 2.31 (s, 4H), 2.12 (s, 2H), 1.84 (s, 2H), 1.54 (s, 8H), 1.18 (s, 3H), 0.85-0.84 (m, 15H), 0.50 (s, 3H).

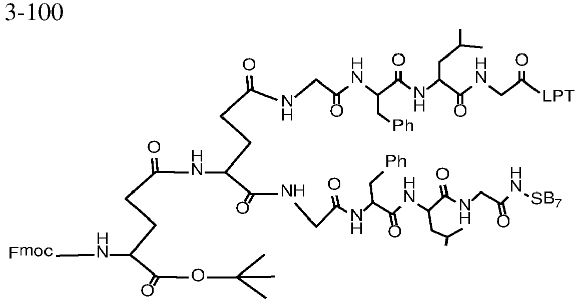

Fmoc-GLu-otBu (0.7301 g, 1.7160 mmol) was weighed and dissolved with DMF (40 mL), the obtained solution was placed in a low-temperature constant temperature reaction bath (0 °C), and Product 3-63 (2.4 g, 1.2257 mmol) and PyAop (0.8947 g, 1.7160 mmol) were then added; the obtained solution was stirred for 30min, TMP (0.16 mL, 1.2257 mmol) was dropwise added to the solution, and the obtained solution was stirred overnight at a low temperature. At the end of the reaction, methyl tert-butyl ether (200 mL) was added to the reaction solution, the mixed solution was cooled, and the supernatant was then discarded; ethyl acetate (10 mL) was added to the solution and the obtained solution was treated by ultrasonic to obtain a homogeneous phase; n-hexane (150 mL) was added for precipitation; the process of dissolution with ethyl acetate and precipitation with n-hexane was repeated twice, the supernatant was discarded and the lower precipitate was dissolved with a mixed solvent (20% methanol: 80% dichloromethane 40 mL); silica gel powder (9 g) was added to the obtained solution, and then the operations of evaporation, dry sample loading and column chromatography were carried out. Gradient elution with 4%-5% methanol/dichloromethane was carried out, thus obtaining 2.8 g of the product with a yield of 96.6%. MALDI-TOF MS: [M+H⁺] 2366.43.

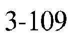

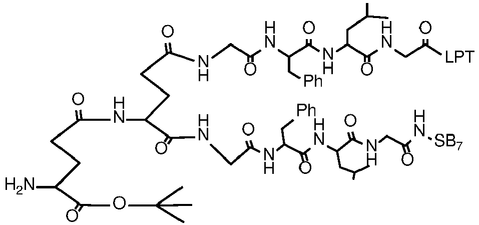

The reactant Product 3-100 (2.8 g, 1.1846 mmol) was added to the reaction flask and dissolved with DMF (20 mL), morpholine (2.58 mL, 29.615 mmol) was added to the obtained solution, and TLC was carried out every 0.5h. Three hours later, and at the end of the reaction, methyl tert-butyl ether (200 mL) was added to the reaction solution with stirring, the mixed solution was cooled in a refrigerator until the precipitate came out, and the supernatant was then discarded; dichloromethane (10 mL) was added to dissolve the precipitate; the obtained solution was precipitated with n-hexane (150 mL) and cooled, the supernatant was then discarded, and ethyl acetate (10 mL) was added to dissolve the precipitate; and the obtained solution was treated by ultrasonic to obtain a homogeneous phase; n-hexane (150 mL) was added for precipitation; the process of dissolution with ethyl acetate and precipitation with n-hexane was repeated twice, the supernatant was discarded and the lower precipitate was dissolved with a mixed solvent (20% methanol: 80% dichloromethane 40 mL); silica gel powder was added to the obtained solution, and then the operations of evaporation, dry sample loading and column chromatography were carried out. Gradient elution with 3%-10% methanol/dichloromethane was carried out, thus obtaining 2.2 g of the product with a yield of 86.96%.

MALDI-TOF MS: [M+H⁺] 2143.92, [M+Na⁺] 2166.11

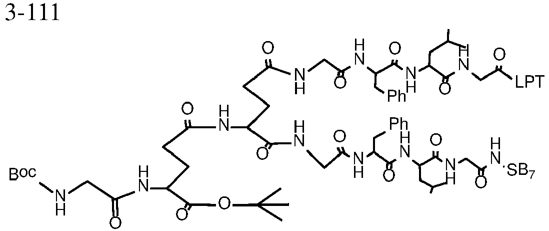

Product 3-109 (2.2 g, 1.0265 mmol), Boc-Gly-OH (0.2158 g, 1.2318 mmol), HBTU (0.5840 g, 1.5340 mmol), and HOBT (0.2081 g, 1.5398 mmol) were weighed and added to the flask, and the reaction solution was placed in a low-temperature constant temperature reaction bath (-5 °C) to react for 20min, DIEA (0.7635 mL, 4.6193 mmol) was added dropwise, and the mixed solution first reacted for 2 h and then taken out and placed at room temperature overnight on a magnetic stirrer. At the end of the reaction, the reaction solution was transferred to a 1 L separatory funnel, saturated sodium bicarbonate solution (200 mL) and ethyl acetate (300 mL) were added to the separatory funnel, the obtained solution was shaken thoroughly to separate the organic phase, and the aqueous phase was extracted twice with ethyl acetate (150 mL×2); the obtained organic phases were combined, washing with saturated sodium chloride solution was carried out three times (200 mL×3); the obtained solution was concentrated, and silica gel powder was added to obtain a solid solution; the operations of dry sample loading, column chromatography, and gradient elution with 0.5%-4% methanol/dichloromethane were carried out, thus obtaining 1.9 g of the product with a yield of 80.50%.

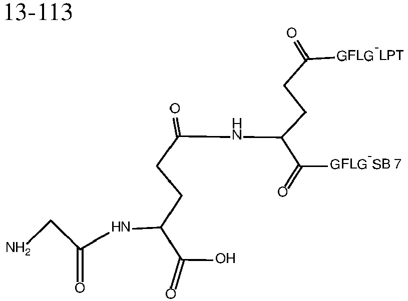

The raw material Product 3-111 (1.9 g, 0.8259 mmol) was weighed and added in a 250 mL flask and then dissolved with dichloromethane (20 mL), TFA (1.84 mL, 24.777 mmol) was then added to the solution, and the obtained solution was stirred at room temperature overnight on a magnetic stirrer. At the end of the reaction, the reaction solution was evaporated to remove dichloromethane; ethyl acetate (10 mL) was added to the solid and the obtained solution was treated by ultrasonic to obtain a homogeneous phase; n-hexane (150 mL) was added for precipitation; the process of dissolution with ethyl acetate (10 mL) and precipitation with n-hexane (150 mL) was repeated twice, the supernatant was discarded and the lower precipitate was dissolved with a mixed solvent (20% methanol: 80% dichloromethane 50 mL); silica gel powder was added to the obtained solution, and then the operations of evaporation, dry sample loading and column chromatography were carried out. Gradient elution with 2% methanol-20% methanol/dichloromethane was carried out, thus obtaining 1.7 g of the product with a yield of 100%.

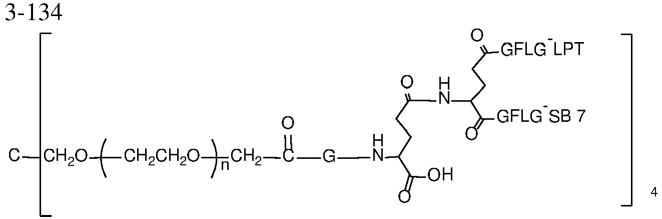

Product 3-113 (1.6 g, 0.7462 mmol) was added in a 250 mL reaction flask and then dissolved with DMF (30 mL), and pyridine (0.1282 mL, 1.5857 mmol) was added to the obtained solution; 4ARM-SCM-40K (5.7715 g, 0.1321 mmol) was weighed and dissolved with dichloromethane (45 mL); the DMF pyridine solution of Product 3-113 was added to the dichloromethane solution of 4ARM-SCM-40K to have a reaction in the dark at a low speed stirring. At the end of the reaction, methyl tert-butyl ether (500 mL) was added to the reaction solution, the mixed solution was filtered by suction, and the obtained solid was dissolved with a mixed solvent (20% methanol: 80% dichloromethane 30 mL); silica gel powder (10 g) was added to the obtained solution, and then the operations of evaporation, dry sample loading and column chromatography were carried out. Gradient elution with 6%-20% methanol: 1% ammonia water/dichloromethane was carried out, thus obtaining 5.2 g of the product with a yield of 78.95%.

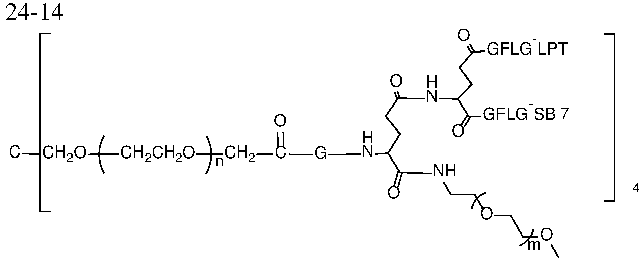

The reactants, Product 3-134 (2.6 g, 0.0503 mmol), M-NH2HCL-2000 (0.8199 g, 0.4024 mmol), HBTU (0.1145 g, 0.3018 mmol), and HOBT (0.0408 g, 0.3018 mmol), were weighed and added in a 250 mL reaction flask and dissolved with DMF (150 mL); the obtained solution was stirred at -5 °C for 30min; DIEA (0.2 mL, 1.3078 mmol) was slowly added dropwise and the obtained solution was stirred for 1 h and cooled to room temperature to react in the dark at a low speed stirring. At the end of the reaction, methyl tert-butyl ether was added to precipitate the reaction solution, suction filtering was carried out to obtain a powder product, the powder product was dissolved with a mixed solvent of methanol and dichloromethane, silica gel was added to the obtained solution, and the operations of evaporation, dry sample loading and column chromatography were carried out. Gradient elution with 1% ammonia water: 3%-10% methanol/dichloromethane was carried out, the pure product was collected and evaporated to dryness, the dry product was dissolved by ultrasonic with absolute ethanol (5 mL) and dichloromethane (5 mL), methyl tert-butyl ether (100 mL) was added to the obtained solution until a solid came out; suction filtering was carried out, and the obtained solid was further dissolved with absolute ethanol (5 mL) and dichloromethane (5 mL); the obtained solution was precipitated with methyl tert-butyl ether (100 mL); the process of dissolution and precipitation was repeated three times, and vacuum drying was carried out, thus obtaining 1.5 g of the product with a yield of 50%.

### Example 6: Synthesis of Compound 9

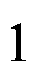

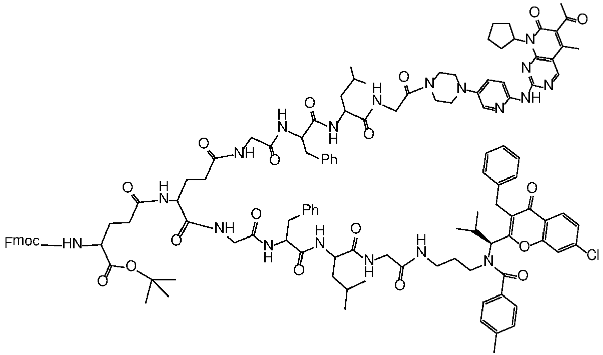

Fmoc-Glu-(OtBu)(OH) (1.6339 g, 3.8402 mmol), Product 19-68 (5 g, 2.7430 mmol, see Example 14 for its structure and synthesis process), HBTU (1.5604 g, 4.1145 mmol), and HOBT (0.5560 g, 4.1145 mmol) were added to a 500 mL flask and then dissolved with DMF (30 mL), and the obtained solution was stirred at -5 °C for 30min; DIEA (1.2240 mL, 7.4062 mmol) was slowly added dropwise over 10min. The reaction solution was first stirred at -5 °C for 1 h to react, and then stirred at room temperature overnight to further react. At the end of the reaction, n-hexane (100 mL × 3) was added to the reaction solution and the obtained solution was shaken; the supernatant was discarded and the lower oily precipitate was dissolved with dichloromethane (20 mL); the obtained solution was precipitated with methyl tert-butyl ether (30 mL) to obtain a solid; suction filtering was then carried out, the filter cake was washed with methyl tert-butyl ether (40 mL×3) and then dissolved with a methanol/dichloromethane (1:5) mixed solvent (100 mL), silica gel powder (15 g) was added to the obtained solution, and the solution was then evaporated to dryness to obtain a powdery solid; the operations of dry sample loading, column chromatography, and gradient elution with a dichloromethane mixed solution containing 1% of ammonia water and 4%-6% of methanol were carried out; the elution product was then collected, concentrated, evaporated to dryness, and dried in a vacuum oven, thus obtaining 6.3821 g of Product 1 with a yield of 79%.

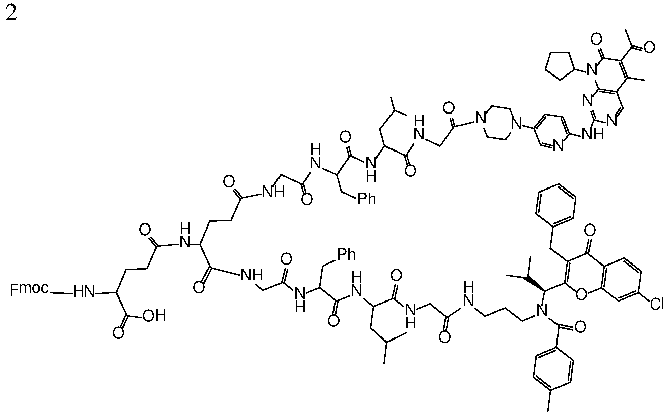

Product 1 (6.3726 g, 2.1670 mmol) was added in a 500 mL flask and then dissolved with dichloromethane (10 mL), TFA (2.4750 mL, 29.287 mmol) was then added to the obtained solution, and the mixed solution was stirred overnight at room temperature to react. At the end of the reaction, the dichloromethane in the reaction solution was evaporated in vacuum, and methyl tert-butyl ether (20 mL) was then added for precipitation until a solid came out; the filtering suction was carried out and the filter cake was washed with methyl tert-butyl ether (40 mL×3), collected and dried in a vacuum oven, thus obtaining 4.7149 g of Product 2 with a yield of 100%.

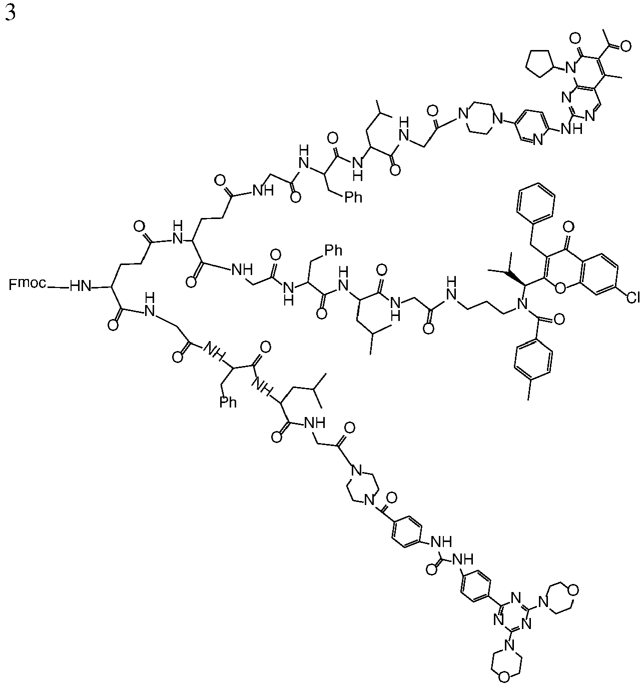

Product 2 (4.7 g, 2.1670 mmol), Product 11-83 (2.4654 g, 2.6004 mmol), HBTU (1.2327 g, 3.2504 mmol), and HOBT (0.4392 g, 3.2504 mmol) were added to a 500 mL flask and then dissolved with DMF (50 mL), and the obtained solution was stirred at -5 °C to react for 30min; DIEA (2.4175 mL, 14.6268 mmol) was slowly added dropwise over 10min. The reaction solution was first stirred at -5 °C to react for 1h, and then stirred at room temperature overnight to further react. At the end of the reaction, n-hexane (100 mL×3) was added to precipitate the reaction solution, and the lower oily precipitate decreased in volume; methyl tert-butyl ether (30 mL) was then added to separate out a solid; suction filtering was then carried out, the filter cake was washed with methyl tert-butyl ether (40 mL×3) and then dissolved with a methanol/dichloromethane (1:5) mixed solvent (100 mL), silica gel powder (15 g) was added to the obtained solution, and the solution was then evaporated to dryness to obtain a powdery solid; the operations of dry sample loading, column chromatography, and gradient elution with a dichloromethane mixed solution containing 1% of ammonia water and 4%-8% of methanol were carried out; the elution product was then collected, concentrated, evaporated to dryness, and dried in a vacuum oven, thus obtaining 5.1826 g of Product 3 with a yield of 77%.

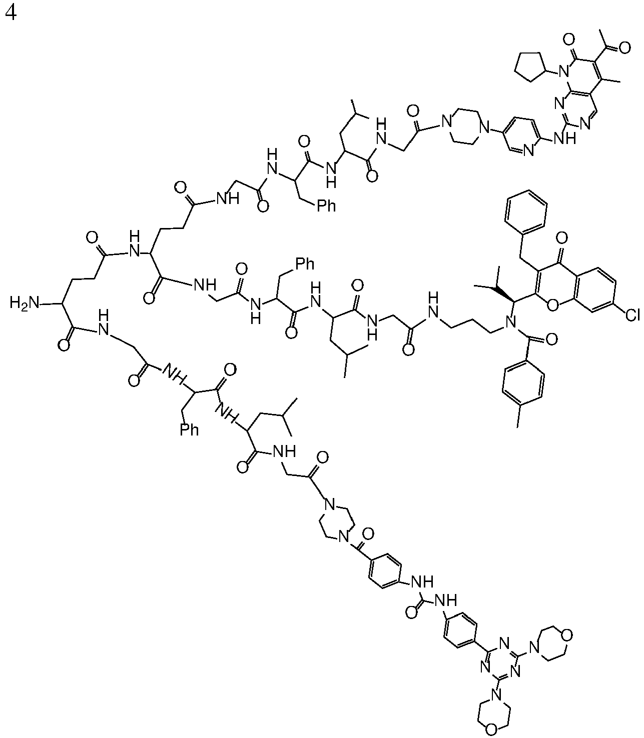

Product 3 (5.1360 g, 1.6686 mmol) was added in a 250 mL flask and then dissolved with DMF (30 mL), morpholine (1.4537 g, 16.6859 mmol) was added to the obtained solution, and the mixed solution was stirred at room temperature to react for 1h. At the end of the reaction, n-hexane (60 mL×3) was added to precipitate the reaction solution, and the lower oily precipitate decreased in volume; methyl tert-butyl ether (30 mL) was then added to separate out a solid; the filtering suction was carried out and the filter cake was washed with methyl tert-butyl ether (40 mL×3), collected and dried in a vacuum oven, thus obtaining 4.8096 g of Product 4 with a yield of 100%.

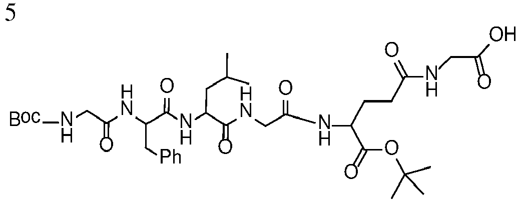

Product 18-167 (0.6902 g, 0.8266 mmol) and 10% Pd/C catalyst (0.1 g) were added into a hydrogenation reactor and then dissolved with DMF (30 mL), hydrogen was introduced, and the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 0.18 MPa At the end of the reaction, the reaction solution was filter with suction using diatomaceous earth, the filter cake was washed with DMF (20 mLx3), thus obtaining Product 5.

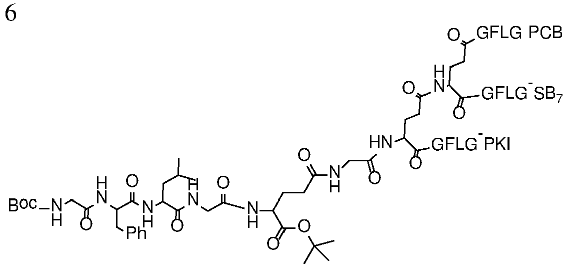

Product 5 (0.8366 mmol), Product 4 (2 g, 0.6972 mmol), HBTU (0.3966 g, 1.0458 mmol), and HOBT (0.1414 g, 1.0458 mmol) were added to a 250 mL flask and then dissolved with DMF (20 mL), and the obtained solution was stirred at -5 °C for 30 min to react; DIEA (0.5185 mL, 3.1373 mmol) was slowly added dropwise over 5min. The reaction solution was first stirred at -5 °C to react for 1h, and then stirred at room temperature overnight to further react. At the end of the reaction, n-hexane (100 mL×3) was added to precipitate the reaction solution, and the lower oily precipitate decreased in volume; methyl tert-butyl ether (30 mL) was then added to separate out a solid; suction filtering was then carried out, the filter cake was washed with methyl tert-butyl ether (40 mL×3) and then dissolved with a methanol/dichloromethane (1:5) mixed solvent (100 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 4%-8% of methanol were carried out; the elution product was then collected, concentrated, evaporated to dryness, and dried in a vacuum oven, thus obtaining 1.9643 g of Product 6 with a yield of 78%.

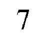

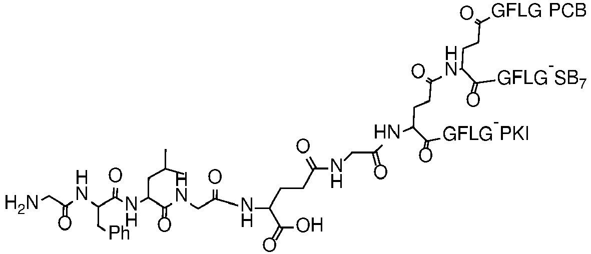

Product 6 (1.9580 g, 0.6825 mmol) was added in a 250 mL flask and then dissolved with dichloromethane (10 mL), TFA (0.7603 mL, 10.2380 mmol) was then added to the obtained solution, and the mixed solution was stirred overnight at room temperature to react. At the end of the reaction, the dichloromethane in the reaction solution was evaporated in vacuum, and methyl tert-butyl ether (20 mL) was then added for precipitation until a solid came out; suction filtering was then carried out, the filter cake was washed with methyl tert-butyl ether (40 mL×3) and then dissolved with a methanol/dichloromethane (1:5) mixed solvent (100 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 3%-9% of methanol were carried out; the elution product was then collected, concentrated, evaporated to dryness, and dried in a vacuum oven, thus obtaining 1.7862 g of Product 7 with a yield of 76%.

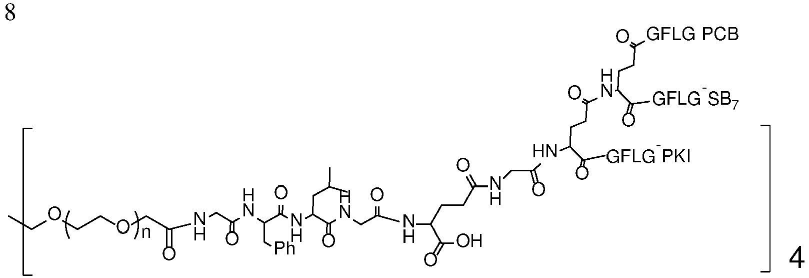

Product 7 (0.3939 g, 0.1144 mmol) was added to a 250 mL flask and then dissolved with DMF (50 mL), DIEA (0.25 mL, 1.7160 mmol) was added in the flask, and then 4ARM-SCM-40K (1.0 g, 0.0238 mmol) was added and dissolved with dichloromethane (10 mL), and the mixed solution was stirred in the dark at room temperature at a low speed to react for one week. At the end of the reaction, n-hexane (100 mL × 3) was added to precipitate the reaction solution, and the lower oily precipitate decreased in volume; methyl tert-butyl ether (30 mL) was then added to separate out a solid; suction filtering was then carried out, the filter cake was washed with methyl tert-butyl ether (40 mL×3) and then dissolved with a methanol/dichloromethane (1:5) mixed solvent (100 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 3%-12% of methanol were carried out; the elution product was then collected, concentrated, evaporated to dryness, and dried in a vacuum oven, thus obtaining 1.1103 g of Product 8 with a yield of 81%.

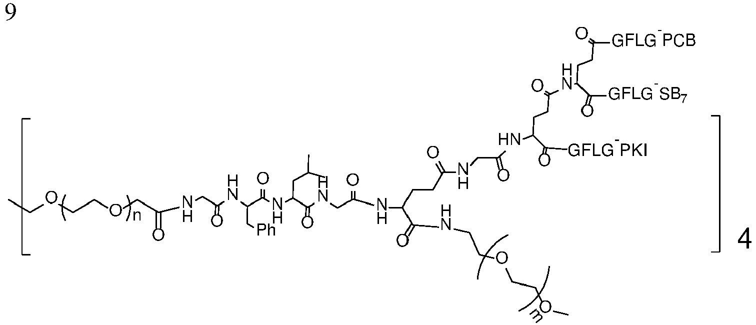

Product 8 (1.0606 g, 0.0236 mmol), M-NH2-2K.HCl (0.2884 g, 0.1416 mmol), HBTU (0.0537 g, 0.1416 mmol), and HOBT ( 0.0191 g, 1.1416 mmol) were added to a 250 mL flask and then dissolved with DMF (30 mL), and the obtained solution was stirred at -5 °C for 30 min; DIEA (0.0936 mL, 0.5664 mmol) was slowly added dropwise over 3min. The reaction solution was first stirred at -5 °C to react for 1h, and then stirred at room temperature overnight to further react. At the end of the reaction, n-hexane (100 mL × 3) was added to precipitate the reaction solution, and the lower oily precipitate decreased in volume; methyl tert-butyl ether (30 mL) was then added to separate out a solid; suction filtering was then carried out, the filter cake was washed with methyl tert-butyl ether (40 mL×3) and then dissolved with a methanol/dichloromethane (1:5) mixed solvent (100 mL), silica gel powder (8 g) was added to the obtained solution, and the solution was then evaporated to dryness to obtain a powdery solid; the operations of dry sample loading, column chromatography, and gradient elution with a dichloromethane mixed solution containing 1% of ammonia water and 2%-10% of methanol were carried out; the elution product was then collected, concentrated, evaporated to dryness, and dried in a vacuum oven, thus obtaining 1.0189 g of Product 9 with a yield of 92%.

### Example 7: Synthesis of Compound 14-111

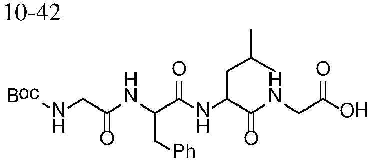

The raw material BocNH-GFLG-OBn (13.54 g, 23.244 mmol) and 10% Pd/C catalyst (400 mg) were added into a hydrogenation reactor and then dissolved with DMF (45 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (90 mL) until the reaction device did not contain any product, and then the reaction product 10-42 was obtained.

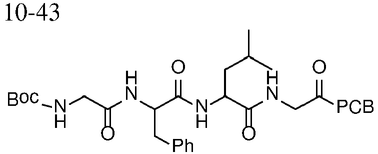

Product 10-42 BocNH-GFLG-OH (23.24 mmol), PCB (Palbociclib) (8 g, 17.88 mmol), HBTU (10.17 g, 26.82 mmol) and HOBT (3.6 g, 26.82 mmol) were added in a 500 mL round-bottomed flask and then dissolved with DMF (90 mL), and the mixed solution was stirred at -5 °C for 30min. Then DIEA (13.3 mL, 80.46 mmol) was slowly added dropwise, and then the mixed solution reacted at a low temperature for 2h; after that, the reaction device was placed at room temperature and the reaction solution was stirred overnight to react. At the end of the reaction, the reaction solution was transferred to a 1000 mL separatory funnel, saturated sodium bicarbonate solution (200 mL) was then added, the obtained solution was extracted three times with ethyl acetate (200 mL×3), and the obtained organic phases were combined and washed with saturated sodium bicarbonate (100 mL) once, and saturated sodium chloride (100 mL) was added to remove water; the organic phase was dried with anhydrous sodium sulfate and filtered by suction. The filtrate was concentrated and evaporated to dryness and then dried in a vacuum oven, thus obtaining 16.48 g of the product for the next reaction.

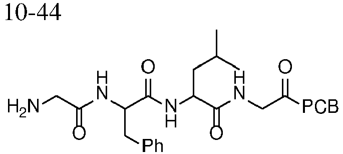

Product 10-43 (16.48 g, 17.88 mmol) was added in a 500 mL round-bottomed flask and then dissolved with dichloromethane (100 mL), and then TFA (18.67 mL, 251.37 mmol) was added and the obtained solution was stirred at room temperature to react overnight. At the end of the reaction, the reaction solution was concentrated and evaporated to dryness under reduced pressure, and the obtained dry product was then dissolved with an appropriate amount of ethyl acetate and transferred to a 500 mL separatory funnel; saturated sodium bicarbonate (100 mL) was added to neutralize the remaining TFA, the organic phase was then separated, the product in the aqueous phase was extracted three times with ethyl acetate (150 mL×3), and the obtained organic phases were combined, then dried with anhydrous sodium sulfate, filtered by suction, and concentrated; the operations of dry sample loading, column chromatography, and elution with 4% methanol/1% ammonia water/dichloromethane were carried out, and then the elution product was then collected, concentrated, evaporated to dryness, and dried in a vacuum oven, thus obtaining 15.4 g (overweight) of the product.

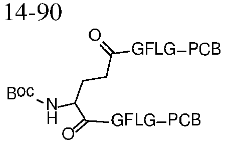

Boc-Glu-OH (0.85 g, 3.44 mmol), GFLG-PCB (6.01 g, 7.31 mmol, i.e., Product 10-44), HOBT (1.61 g, 11.91 mmol) and HBTU (4.14 g, 10.92 mmol) were added in a 500 mL round-bottomed flask and then dissolved with DMF (60 mL), and the mixed solution was stirred at -5 °C for 30min. Then DIEA (5.2 mL, 31.46 mmol) was slowly added dropwise, and then the mixed solution reacted at a low temperature for 2h; after that, the reaction device was placed at room temperature and the reaction solution was stirred overnight to react. At the end of the reaction, the reaction solution was precipitated with n-hexane (200 mL) and methyl tert-butyl ether (400 mL) and then filtered by suction; the solid was taken, and then dissolved with a small amount of ethyl acetate (3 mL); the obtained solution was precipitated with methyl tert-butyl ether (300 mL) and filtered with suction; the solid was taken and dried in a vacuum oven, thus obtaining Product 14-90 with a theoretical weight of 6.41 g.

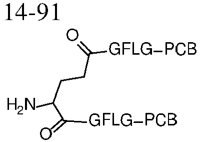

Product 14-90 (6.41 g, 3.44 mmol) was added in a 500 mL round-bottomed flask and then dissolved with CH₂Cl₂ (20 mL), and the mixed solution was stirred at room temperature. TFA (5.00 mL, 67.32 mmol) was then added dropwise, and the mixed solution reacted at room temperature for 2 h. At the end of the reaction, the reaction solution was evaporated to dryness and then precipitated with n-hexane (50 mL) and methyl tert-butyl ether (200 mL); the precipitate was evaporated to dryness; the operations of dry sample loading, column chromatography, and elution with a mixed solvent (3% methanol/0.5% ammonia water/dichloromethane) were carried out; the elution product was then collected, concentrated, evaporated to dryness, and dried in a vacuum oven, thus obtaining 4.3 g of Product 14-91 and 0.39 g of a mixed product.

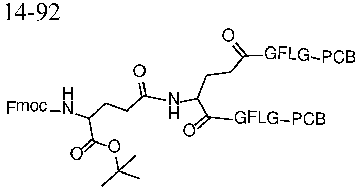

Product 14-91 (4.3 g, 2.45 mmol), Fmoc-Glu-OtBu (1.46 g, 3.43 mmol), HOBT (0.54 g, 11.91 mmol) and HBTU (1.49 g, 10.92 mmol) were added in a 500 mL round-bottomed flask and then dissolved with DMF (100 mL), and the mixed solution was stirred at 0 °C for 30min; DIEA (1.20 mL, 2.28 mmol) was slowly added dropwise and the obtained solution reacted at a low temperature overnight. At the end of the reaction, the reaction solution was taken out, precipitated with n-hexane (200 mL) and methyl tert-butyl ether (400 mL) and then filtered by suction; the solid was taken, and then dried in a vacuum oven, thus obtaining solid Product 14-92 with a theoretical weight of 5.30 g.

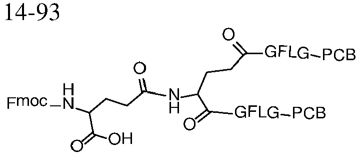

Product 14-92 (5.30 g, 2.45 mmol) was added in a 500 mL round-bottomed flask and then dissolved with CH₂Cl₂ (20 mL), and the mixed solution was stirred at room temperature.. TFA (3.00 mL, 40.39 mmol) was then added dropwise, and the mixed solution reacted at room temperature for 2h. At the end of the reaction, the reaction solution was evaporated to dryness and then precipitated with n-hexane (50 mL) and methyl tert-butyl ether (400 mL); the solid was taken and subjected to the operations of dry sample loading, column chromatography, and gradient elution with a mixed solvent (4%-8% methanol/dichloromethane); the elution product was then collected, concentrated, evaporated to dryness, and dried in a vacuum oven, thus obtaining 4.50 g of Product 14-93 with a yield of 87.2%.

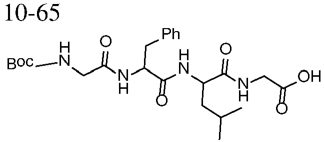

The raw material Boc-GFLG-OBn (6.30 g, 10.80 mmol) and 10% Pd/C catalyst (100 mg) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (20 mL×3) three times until the reaction device did not contain any product, and then the reaction product 10-65 was obtained.

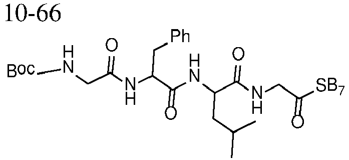

Product 10-65 (5.33 g, 10.80 mmol), SB-743921 (4.00 g, 7.74 mmol, referred to as SB7), HOBT (1.70 g, 12.58 mmol) and HBTU (4.66 g, 12.29 mmol) were added in a 500 mL round-bottomed flask and then dissolved with DMF (170 mL), and the mixed solution was stirred at -5 °C for 30min. Then DIEA (5.80 mL, 35.09 mmol) was slowly added dropwise, and then the mixed solution reacted at a low temperature for 2h; after that, the reaction device was placed at room temperature and the reaction solution was stirred overnight to react. At the end of the reaction, the reaction solution was washed with saturated sodium chloride (300 mL) and extracted with EA (200 mL) three times; the organic phases were combined and concentrated by rotary evaporation to 100 mL, and the water was removed by anhydrous sodium sulfate; the suction filtering was carried out, and the filtrate was subjected to rotary evaporation and dried in a vacuum oven, thus obtaining Product 10-66 with a theoretical weight of 7.68 g.

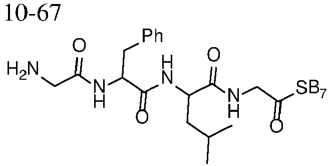

Product 10-66 (7.68 g, 7.74 mmol) was added in a 500 mL round-bottomed flask and then dissolved with CH₂Cl₂ (40 mL), and the mixed solution was stirred at room temperature. TFA (9.00 mL, 67.32 mmol) was then added dropwise, and the mixed solution reacted at room temperature for 2h. At the end of the reaction, the reaction solution was evaporated to dryness and washed with saturated sodium chloride (200 mL) and extracted with EA (200 mL) three times; the organic phases were combined and concentrated by rotary evaporation to 100 mL, and the water was removed by anhydrous sodium sulfate; the suction filtering was carried out, and the filtrate was subjected to rotary evaporation, dry sample loading, column chromatography, and gradient elution with a mixed solvent (3%-5% methanol/1% ammonia water/dichloromethane); the elution product was then collected, concentrated, evaporated to dryness, and dried in a vacuum oven, thus obtaining Product 10-67 with a theoretical weight of 6.20 g and a yield of 89.9%.

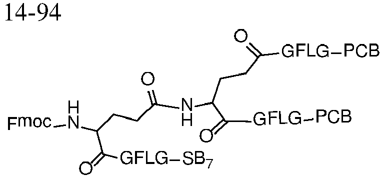

Product 14-93 (4.50 g, 2.14 mmol), GFLG-SB7 (1.80 g, 7.31 mmol, i.e., Product 10-67), HOBT (0.54 g, 11.91 mmol) and HBTU (1.32 g, 10.92 mmol) were added in a 500 mL round-bottomed flask and then dissolved with DMF (150 mL), and the mixed solution was stirred at -5 °C for 30min. Then DIEA (1.0 mL, 31.46 mmol) was slowly added dropwise, and then the mixed solution reacted at a low temperature for 2h; after that, the reaction device was placed at room temperature and the reaction solution was stirred overnight to react. At the end of the reaction, the reaction solution was precipitated with n-hexane (200 mL) and methyl tert-butyl ether (400 mL) and then filtered by suction; the solid was taken, and then dissolved with a small amount of ethyl acetate (3 mL); the obtained solution was precipitated with methyl tert-butyl ether (300 mL) and filtered with suction; the solid was taken and dried in a vacuum oven, thus obtaining Product 14-90 with a theoretical weight of 6.41 g.

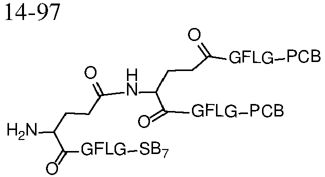

Product 14-94 (6.00 g, 2.01 mmol) was added in a 500 mL round-bottomed flask and then dissolved with DMF (230 mL), and then morpholine (5.30 mL, 60.84 mmol) was added and the obtained solution was stirred at room temperature to react for 2 h. At the end of the reaction, the reaction solution was precipitated with n-hexane and methyl tert-butyl ether and filtered; the solid was taken and subjected to dry sample loading, column chromatography, and elution with a mixed solvent (5% methanol/0.5% ammonia water/dichloromethane); the elution product was then collected, concentrated, evaporated to dryness, and dried in a vacuum oven, thus obtaining 2.7 g of the product and 0.32 g of the raw material.

¹H-NMR (400 MHz, DMSO-d₆) δ 10.13 (s, 2H), 8.95 (s, 2H), 8.31-8.18 (m, 6H), 8.12-8.00 (m, 9H), 7.88 (d, *J*=8.0Hz, 5H), 7.55-7.49 (m, 4H), 7.22-7.16 (m, 25H), 5.82 (t, *J*=16.0Hz, 2H), 4.55 (s, 3H), 4.35 (dd, *J*=16.0Hz, 8.0Hz, 2H), 4.14 (t, *J*=16.0Hz, 2H), 4.06-4.00 (m, 5H), 3.71-3.61 (m, 17H), 3.19-3.13 (m, 10H), 3.07-3.03 (m, 3H), 2.89 (s, 1H), 2.77-2.73 (m, 3H), 2.42 (s, 6H), 2.31 (s, 8H), 2.24-2.20 (m, 6H), 2.14-2.13 (m, 2H), 1.88-1.77 (m, 15H), 1.62-1.57 (m, 8H), 1.50 (t, *J*=12.0Hz, 6H), 1.35-1.34 (m, 1H), 1.24 (d, *J*=8.0Hz, 2H), 0.93-0.80 (m, 24H) .

MALDI-TOF MS: [M+H⁺] 2756.49, [M+Na⁺] 2778.45.

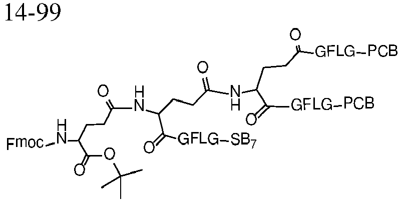

Product 14-97 (2.7 g, 1.37 mmol), Fmoc-Glu-OtBu (0.61 g,1.35 mmol), HOBT (0.24 g, 1.78 mmol) and HBTU (0.62 g, 1.63 mmol) were added in a 500 mL round-bottomed flask and then dissolved with DMF (80 mL), and the mixed solution was stirred at 0 °C for 30 min; DIEA (0.74 mL, 4.48 mmol) was slowly added dropwise and the obtained solution reacted at a low temperature overnight. At the end of the reaction, the reaction solution was taken out, precipitated with n-hexane (200 mL) and methyl tert-butyl ether (400 mL) and then filtered by suction; the solid was taken, and then dried in a vacuum oven, thus obtaining solid Product 14-99 with a theoretical weight of 3.10 g.

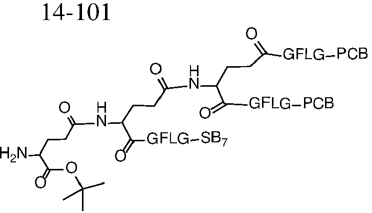

Product 14-99 (3.10 g, 0.98 mmol) was added in a 500 mL round-bottomed flask and then dissolved with DMF (200 mL), and the mixed solution was stirred at room temperature. Morpholine (2.56 mL, 29.38 mmol) was then added dropwise, and the mixed solution reacted at room temperature for 2h. At the end of the reaction, the reaction solution was evaporated to dryness, precipitated with n-hexane (300 mL) and methyl tert-butyl ether (600 mL) and then filtered by suction; the solid was taken, and then dried in a vacuum oven, thus obtaining solid Product 14-101 with a theoretical weight of 2.88 g.

¹H-NMR (400 MHz, DMSO-d₆) δ 10.13 (s, 2H), 8.96 (s, 2H), 8.27-8.16 (m, 6H), 8.08-8.03 (m, 10H), 7.95 (s, 3H), 7.89-7.87 (m, 5H), 7.55-7.49 (m, 4H), 7.22-7.16 (m, 25H), 5.84-5.80 (t, *J*=16.0Hz, 2H), 4.56 (s, 3H), 4.37-4.35 (d, *J*=8.0Hz, 2H), 4.23-4.12 (m, 4H), 4.06-3.95 (m, 3H), 3.61-3.51 (m, 17H), 3.19-3.14 (m, 10H), 3.06-3.03 (m, 3H), 2.89 (s, 6H), 2.73 (m, 8H), 2.42 (s, 3H), 2.31 (s, 8H), 2.24-2.20 (m, 6H), 1.88-1.78 (m, 16H), 1.58-1.50 (m, 17H), 1.50-1.39 (m, 9H), 0.89-0.84 (m, 24H) .

MALDI-TOF MS: [M+H⁺] 2941.93, [M+Na⁺] 2963.63.

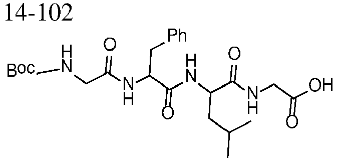

The raw material Boc-GFLG-OBn (0.80 g, 1.37 mmol) and 10% Pd/C catalyst (100 mg) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (20 mL×3) three times until the reaction device did not contain any product, and then the reaction product 14-102 was obtained.

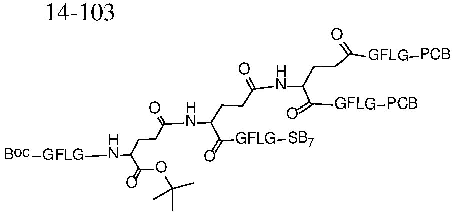

Product 14-101 (2.88 g, 0.98 mmol), Product 14-102 (0.68 g, 1.37 mmol), HOBT (0.29 g, 2.15 mmol) and HBTU (0.70 g, 1.85 mmol) were added in a 500 mL round-bottomed flask and then dissolved with DMF (200 mL), and the mixed solution was stirred at -5 °C for 30min. Then DIEA (0.74 mL, 4.48 mmol) was slowly added dropwise, and then the mixed solution reacted at a low temperature for 2h; after that, the reaction device was placed at room temperature and the reaction solution was stirred overnight to react. At the end of the reaction, the reaction solution was precipitated with n-hexane (200 mL) and methyl tert-butyl ether (500 mL) and then filtered by suction; the solid was taken, and then dissolved with a small amount of ethyl acetate (3 mL); the obtained solution was precipitated with methyl tert-butyl ether (300 mL) and filtered with suction; the solid was taken and dried in a vacuum oven, thus obtaining Product 14-103 with a theoretical weight of 3.35g.

¹H-NMR (400 MHz, DMSO-d₆) δ 10.12 (s, 2H), 8.95 (s, 2H), 8.16-8.05 (m, 15H), 7.95-7.87 (m, 11H), 7.55-7.49 (m, 4H), 7.22-7.15 (m, 30H), 5.84-5.80 (t, *J*=16.0Hz, 2H), 4.55 (s, 2H), 4.37-4.35 (d, *J*=8.0Hz, 2H), 4.17-4.01 (m, 4H), 3.72-3.46 (m, 14H), 3.18-3.13 (m, 8H), 3.06-2.98 (m, 6H), 2.89 (s, 10H), 2.80-2.73 (m, 14H), 2.42 (s, 6H), 2.31-2.20 (m, 16H), 1.88-1.85 (m, 6H), 179-1.72 (m, 8H), 1.58-1.49 (m, 17H), 1.40-1.26 (m, 22H), 0.91-0.82 (m, 30H).

MALDI-TOF MS: [M+Na⁺] 3437.10.

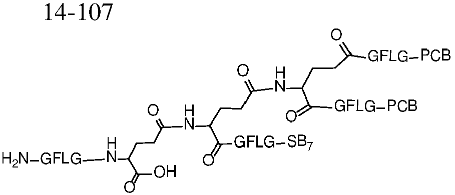

Product 14-103 (3.35 g, 0.98 mmol) was added in a 500 mL round-bottomed flask and then dissolved with CH₂Cl₂ (40 mL), and the mixed solution was stirred at room temperature. TFA (3.00 mL, 40.39 mmol) was then added dropwise, and the mixed solution reacted at room temperature for 2h. At the end of the reaction, the reaction solution was evaporated to dryness and then precipitated with n-hexane and methyl tert-butyl ether and evaporated to dryness; the solid was taken and subjected to the operations of dry sample loading, column chromatography, and gradient elution with a mixed solvent (5%-10% methanol/dichloromethane); the elution product was then collected, concentrated, evaporated to dryness, and dried in a vacuum oven, thus obtaining 1.5 g of Product 14-107.

¹H-NMR (400 MHz, DMSO-d₆) δ 10.12 (s, 2H), 8.95 (s, 2H), 8.33-8.17 (m, 5H), 8.11-8.05 (m, 14H), 7.89-7.87 (m, 5H), 7.55-7.48 (m, 4H), 7.24-7.14 (m, 30H), 5.84-5.80 (t, *J*=16.0Hz, 2H), 4.55-4.53 (m, 3H), 4.39-4.31 (m, 3H), 4.17-4.13 (m, 4H), 4.03-3.99 (m, 3H), 3.76-3.58 (m, 16H), 3.51 (s, 1H), 3.19-3.13 (m, 11H), 3.07-3.00 (m, 5H), 2.89 (s, 1H), 2.80-2.69 (m, 5H), 2.58-2.54 (m, 4H), 2.42 (s, 6H), 2.31 (s, 8H), 2.24-2.14 (m, 8H), 1.62-1.57 (m, 9H), 152-1.48 (m, 9H), 1.24-1.23 (m, 1H), 0.92-0.79 (m, 30H).

MALDI-TOF MS: [M+H⁺] 3259.66, [M+Na⁺] 3257.58.

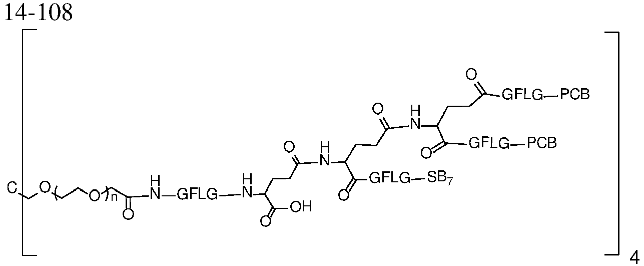

Product 14-107 (1.30 g, 0.40 mmol) was added in a 250 mL round-bottomed flask and then dissolved with CH₂Cl₂ (80 mL) and DMF (100 mL), and the mixed solution was stirred at -5 °C for 30min. Then DIEA (0.26 mL, 1.59 mmol) was slowly added dropwise, and 4ARM-SCM-40K (3.67 g, 0.089 mmol, purchased from JenKem) was added to the obtained solution to react at a low temperature for 2h, and then the reaction device was placed at room temperature and the reaction solution was stirred for 4 days, and applied to the TLC plate every day for monitoring. At the end of the reaction, CH₂Cl₂ was removed by rotary evaporation and then the reaction solution was precipitated with n-hexane (200 mL) and methyl tert-butyl ether (400 mL) and filtered by suction; the solid was taken and subjected to the operations of dry sample loading, column chromatography (if the column was blocked, take out the silica gel powder and wash it with a mixed solvent, and then filter with suction, and evaporate the filtrate to dryness), and gradient elution with a mixed solvent (6%-10% methanol/dichloromethane); the elution product was then collected, concentrated, and combined with the product obtained from the column; the obtained product was evaporated to dryness, and dried in a vacuum oven, thus obtaining 3.84 g of Product 14-108.

¹H-NMR (400 MHz, DMSO-d₆) δ 10.11 (s, 6H), 8.95 (s, 6H), 8.20-8.16 (m, 9H), 8.15-8.04 (m, 42H), 8.02-8.00 (m, 12H), 7.95-7.94 (m, 21H), 7.89-7.87 (m, 20H), 7.78-7.77 (m, 2H), 7.54-7.48 (m, 7H), 7.21-7.14 (m, 127H), 4.56-4.55 (m, 10H), 4.36-4.33 (m, 100H), 4.01-4.00 (m, 23H), 3.88-3.87 (m, 29H), 3.75-3.51 (m, 3488H), 3.17-3.16 (m, 41H), 3.05-3.00 (m, 40H), 2.89 (s, 78H), 2.73 (s, 64H), 2.68-2.67 (m, 18H), 2.60-2.59 (m, 22H), 2.42 (s, 38H), 2.31-2.30 (m, 50H), 2.25-2.17 (m, 95H), 1.98-1.86 (m, 66H), 1.58-1.51 (m, 71H), 1.19-1.17 (m, 12H), 0.91-0.82 (m, 120H).

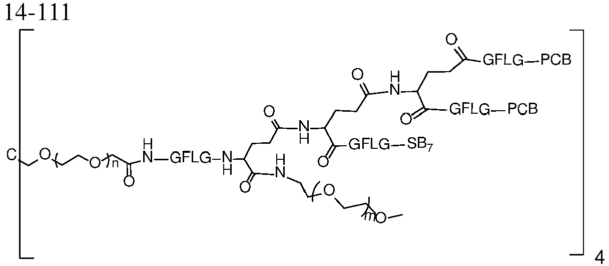

Product 14-108 (3.00 g, 0.055 mmol), HOBT (0.12 g, 0.89 mmol) and HBTU (0.25 g, 0.66 mmol) were added in a 500 mL round-bottomed flask and then dissolved with DMF (170 mL), and the mixed solution was stirred at -5 °C for 30min. Then DIEA (0.20 mL,1.22 mmol) was slowly added dropwise, and M-NH2 -2K·HCl (0.67 g,0.33 mmol, purchased from JenKem) was added to the obtained solution and dissolved completely to react at a low temperature for 2h, and then the reaction device was placed at room temperature and the reaction solution was stirred overnight to react. At the end of the reaction, the reaction solution was precipitated with n-hexane (200 mL) and methyl tert-butyl ether (500 mL); the operations of dry sample loading, column chromatography, and gradient elution with a mixed solvent (4%-14% methanol/dichloromethane) were carried out; the elution product was then collected, concentrated, evaporated to dryness, and dried in a vacuum oven, thus obtaining crude Product 14-111. The obtained crude product was then dissolved with a small amount of CH₂Cl₂ (2 mL) and DMF (2 mL), and then absolute ethanol (30 mL), n-hexane (50 mL), and methyl tert-butyl ether (200 mL) were added to remove water and methanol, the filtering function was carried out, and the obtained product was dried in a vacuum oven, thus obtaining 2.60 g of the final product 14-111.

¹H-NMR (400 MHz, DMSO-d₆) δ 10.12 (s, 8H), δ 8.95 (s, 11H), 8.20-8.16 (m, 31H), 8.08-8.05 (m, 42H), 7.95-7.94 (m, 8H), 7.89-7.87 (m, 19H), 7.77-7.76 (m, 5H), 7.55-7.49 (m, 15H), 7.22-7.21 (m, 88H), 7.17-7.16 (m, 22H), 4.90-4.55 (m, 91H), 4.36-4.35 (m, 50H), 4.17-4.13 (m, 92H), 4.01-3.51 (m, 4382H), 3.25-3.24 (m, 12H), 3.19-3.13 (m, 19H), 3.08-3.00 (m, 31H), 2.89 (s, 4H), 2.76-2.73 (m, 20H), 2.67-2.58 (m, 19H), 2.42 (s, 29H), 2.31 (s, 39H), 2.24-2.14 (m, 36H), 1.88-1.76 (m, 58H), 1.58-1.49 (m, 68H), 1.20-1.12 (m, 22H), 0.88-0.83 (m, 120H).

### Example 8: Synthesis of Compound 14-129

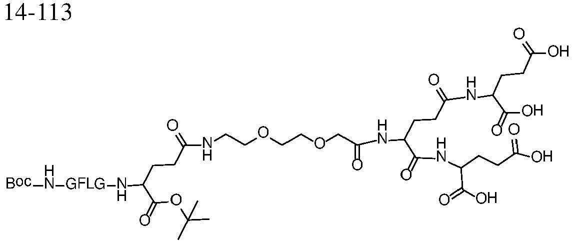

The raw material Product 16-180 (0.41 g, 0.26 mmol) and 10% Pd/C catalyst (300 mg) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (20 mL×3) three times until the reaction device did not contain any product, and then the reaction product 14-113 was obtained.

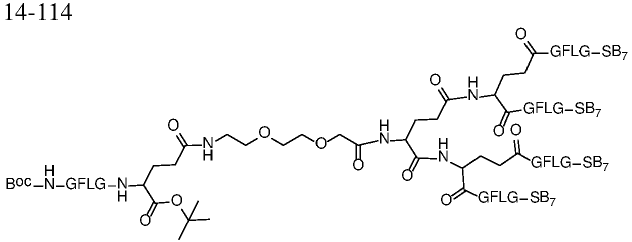

Product 14-113 (0.31 g, 0.26 mmol), Product 10-67 (1.03 g, 1.37 mmol), HOBT (0.24 g, 1.781 mmol) and HBTU (1.07 g, 2.82 L mmol) were added in a 500 mL round-bottomed flask and then dissolved with DMF (80 mL), and the mixed solution was stirred at -5 °C for 30min. Then DIEA (0.78 mL, 4.72 mmol) was slowly added dropwise, and then the mixed solution reacted at a low temperature for 2h; after that, the reaction device was placed at room temperature and the reaction solution was stirred overnight to react. At the end of the reaction, the reaction solution was transferred to a 1000 mL separatory funnel and saturated sodium bicarbonate solution (200 mL) was added to the reaction solution; the reaction flask was washed with ethyl acetate; the mixed phase in the separatory funnel was shaken vigorously, the organic phase was separated, the aqueous phase was extracted with ethyl acetate three times (200 mL×3), and the obtained organic phases were combined and then washing with saturated sodium chloride (100 mL) was carried out three times. The obtained organic phase products were combined and concentrated by rotary evaporation, and then the operations of water removal with anhydrous sodium sulfate, washing with ethyl acetate and suction filtering were carried out. The filtrate was evaporated to dryness, and the solid was dried in a vacuum oven, thus obtaining 0.95 g of a solid product.

¹H-NMR (400 MHz, DMSO-d₆) δ 8.16-8.14 (m, 3H), 8.08-8.01 (m, 16H), 7.96-7.92 (m, 2H), 7.88-7.80 (m, 5H), 7.68-7.67 (m, 1H), 7.55-7.45 (m, 7H), 7.28-7.16 (m, 50H), 7.14-7.10 (m, 18H), 5.78-5.72 (m, 3H), 4.60-4.50 (m, 4H), 4.30-4.14 (m, 7H), 3.95-3.85 (m, 3H), 3.73-3.39 (m, 17H), 3.09-3.02 (m, 7H), 2.99-2.84 (m, 9H), 2.81-2.74 (m, 10H), 2.70-2.64 (m, 18H), 2.31 (s, 17H), 2.15-2.13 (m, 9H), 1.92-1.76 (m, 3H), 1.60-1.55 (m, 5H), 1.53-1.48 (m, 13H), 1.38-1.31 (m, 18H), 1.27-1.23 (m, 12H), 1.08-1.04 (m, 2H), 0.92-0.79 (m, 54H).

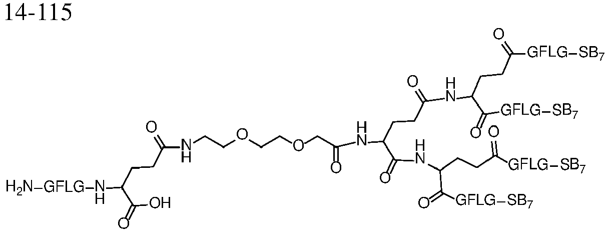

Product 14-114 (0.95 g, 0.20 mmol) was added in a 250 mL round-bottomed flask and then dissolved with CH₂Cl₂ (10 mL), and the mixed solution was stirred at room temperature. TFA (2.00 mL, 26.93 mmol) was then added dropwise, and the mixed solution reacted at room temperature for 2h. At the end of the reaction, the reaction solution was evaporated to dryness and then precipitated with n-hexane (50 mL) and methyl tert-butyl ether (200 mL) and evaporated to dryness; the solid was taken and subjected to the operations of dry sample loading, column chromatography, and gradient elution with a mixed solvent (5%-8% methanol/dichloromethane); the elution product was then collected, concentrated, evaporated to dryness, and dried in a vacuum oven, thus obtaining 0.54 g of Product 14-115.

¹H-NMR (400 MHz, DMSO-d₆) δ 8.49 (d, J=8.0Hz, 4H), 8.35 (d, J=8.0Hz, 4H), 8.09-8.10 (m, 6H), 8.06 (s, 2H), 7.66 (s, 4H), 7.78-7.64 (m, 5H), 7.59-7.53 (m, 9H), 7.27-7.26 (m, 40H), 7.21-7.16 (m, 19H), 7.13-7.13 (m, 10H), 4.62-4.56 (m, 3H), 4.34-4.29 (m, 4H), 4.18-4.08 (m, 5H), 3.91-3.83 (m, 6H),3.80- 3.79 (m, 3H), 3.73-3.72 (m, 3H), 3.69-3.68 (m, 2H), 3.50-3.51 (m, 4H), 3.17 (d, J=8.0Hz, 4H), 3.09-3.08 (m, 2H), 3.05-3.06 (m, 2H),2.92-2.88 (m, 16H), 2.70-2.76 (m,12H), 2.64 (s, 20H), 2.32 (s, 16H), 1.56-1.61 (m, 5H), 1.47-1.53 (m, 10H), 1.35-1.34 (m, 9H), 1.16-1.25 (m, 20H), 0.82-0.94 (m, 54H) .

MALDI-TOF MS: [M+H⁺] 4631.67

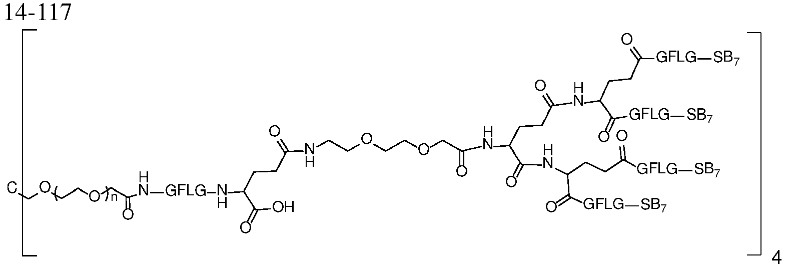

Product 14-115 (0.54 g, 0.117 mmol) was added in a 250 mL round-bottomed flask and then dissolved with CH₂Cl₂ (10 mL) and DMF (20 mL), and the mixed solution was stirred at -5 °C for 30min. Then DIEA (0.10 mL, 0.61 mmol) was slowly added dropwise, and 4ARM-SCM-40K (1.20 g, 0.089 mmol) was added to and dissolved in the obtained solution to react at a low temperature for 2h, and then the reaction device was placed at room temperature and the reaction solution was stirred for 4 days, and the reaction solution was applied to the TLC plate every day for monitoring. At the end of the reaction, CH₂Cl₂ was removed by rotary evaporation and then the reaction solution was precipitated with n-hexane (50 mL) and methyl tert-butyl ether (200 mL) and filtered by suction; the solid was taken and subjected to the operations of dry sample loading, column chromatography (after loading on the column, the silica gel powder was taken out and washed with a mixed solvent, and then filtered with suction, and the filtrate was applied to the TLC plate and then it was found that the product was obtained, and then the filtrate was evaporated to dryness, thus obtaining 0.60 g of impure product), and gradient elution with a mixed solvent (6%-10% methanol/dichloromethane); the elution product was then collected, concentrated, evaporated to dryness, and dried in a vacuum oven, thus obtaining 0.90 g of Product 14-117.

¹H-NMR (400 MHz, DMSO-d₆) δ 10.54 (s, 4H), 8.70-8.69 (m, 174H), 8.48-8.46 (m, 1H), 8.34-8.33 (m, 1H), 8.13-8.05 (m, 15H), 7.84-7.83 (m, 1H), 7.72-7.71 (m, 1H), 7.38-7.13 (m, 214H), 6.70-6.64 (m, 2H), 5.99-5.97 (m, 11H), 5.51 (s, 2H), 5.33-5.30 (m, 6H), 4.02-4.00 (m, 34H), 3.95-3.90 (m, 89H), 3.81-3.80 (m, 142H), 3.70-3.53 (m, 3590H), 3.17-3.14 (m, 32H), 2.89-2.88 (m, 65H), 2.74-2.72 (m, 68H), 2.66-2.58 (m, 93H), 2.33-2.32 (m, 43H), 2.02-1.96 (m, 80H), 1.37-1.33 (m, 95H), 0.91-0.81 (m, 216H).

MALDI-TOF MS: from 58824.42 to 60796.75

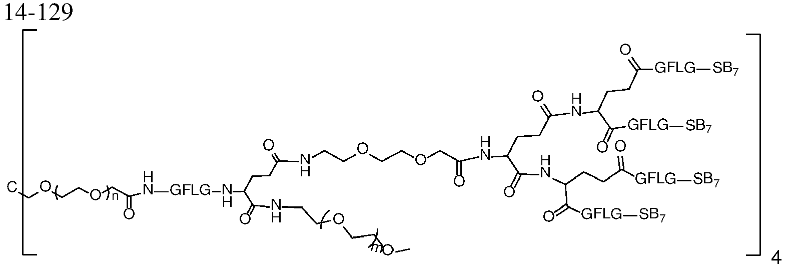

Product 14-108 (0.90 g, 0.015 mmol), HOBT (0.04 g, 0.30 mmol) and HBTU (0.06 g, 0.16 mmol) were added in a 500 mL round-bottomed flask and then dissolved with DMF (80 mL), and the mixed solution was stirred at -5 °C for 30min. Then DIEA (0.10 mL, 0.61 mmol) was slowly added dropwise, and then M-NH₂-2K.HCl (0.24 g, 0.118 mmol) was added to react at a low temperature for 2h; after that, the reaction device was placed at room temperature and the reaction solution was stirred overnight to react. At the end of the reaction, the reaction solution was precipitated with n-hexane (150 mL) and methyl tert-butyl ether (300 mL) and then placed in a refrigerator with a temperature between 2 °C and 8 °C for about 0.5h; the solution was then taken out and filtered by suction; the solid was taken and subjected to the operations of dry sample loading, column chromatography, and gradient elution with a mixed solvent (5%-10% methanol/dichloromethane) were carried out; the elution product was then collected, concentrated, evaporated to dryness, and dried in a vacuum oven, thus obtaining crude Product 14-129. The obtained crude product was then dissolved with a small amount of dichloromethane (2 mL) and DMF (2 mL), and then absolute ethanol (30 mL), n-hexane (50 mL), and methyl tert-butyl ether (200 mL) were added to remove water and methanol, the filtering function was carried out, and the obtained product was dried in a vacuum oven, thus obtaining 0.60 g of the final product 14-129.

¹H-NMR (400 MHz, DMSO-d₆) δ 8.15-7.95 (m, 132H), 7.85-7.80 (m, 20H), 7.68-7.64 (m, 9H), 7.55-7.49 (m, 45H), 7.24-7.19 (m, 102H), 7.15-7.13 (m, 104H), 4.55-4.50 (m, 48H), 4.23-4.14 (m, 120H), 2.88-3.51 (m, 4337H), 4.24-4.17 (m, 32H), 3.08 (s, 25H), 3.01-2.97 (m, 29H), 2.89-2.85 (m, 36H), 2.75-2.73 (m, 69H), 2.67-2.59 (m, 93H), 2.33-2.30 (m, 76H), 2.15-2.11 (m, 42H), 1.82-1.73 (m, 42H), 1.62-1.46 (m, 82H), 1.35-1.32 (m, 22H), 1.24-1.23 (m, 12H), 1.15-1.14 (m, 18H) , 0.92-0.79 (m, 216H). MALDI-TOF MS: from 67208.19 to 67746.29

### Example 9: Synthesis of Compound 16-145

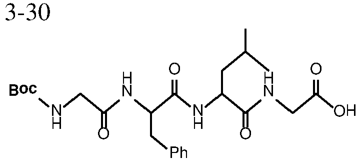

Boc-GFLG-OBn (home-made 4.0047 g, 6.8646 mmol) and 10% Pd/C (0.1006 g) were added in a micro-reactor, DMF (25 mL) was then added to the micro-reactor, the device was set ready, air inlet and pump valves were opened to pump the internal environment to be vacuum, and then the pump valve was closed; H2 (16psi) was introduced, and after the reaction continued for 2min, the air was pumped and H2 was introduced again; pumping was repeated three times and air inlet and pump valves were closed (it should be noted that the vacuum pump was not closed during the whole operation) to seal H2 in the reactor, and the reaction solution was placed at room temperature overnight. At the end of the reaction, the funnel was filled with diatomaceous earth (compacted) to a half, the product was transferred drop by drop into the funnel with a plastic dropper and filtered by suction, and then the reactor and the funnel were washed with DMF three times (25 mL×3), and the solution was transferred in a 250 mL flask as the raw material for Product 3-31.

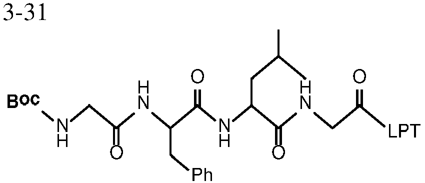

LPT (Lapatinib, 3.7999 g, 6.5377 mmol), HBTU (3.7195 g, 9.8066 mmol) and HOBT (1.3253 g, 9.8066 mmol) were weighed and added to the DMF solution of Product 3-30, and the obtained solution was placed in a low-temperature constant temperature reaction bath (-5 °C) to react for 20 min, DIEA (5 mL, 12.8714 mmol) was added dropwise, and the mixed solution was first stirred at a low temperature for 2 h and then taken out and placed at room temperature overnight on a magnetic stirrer. At the end of the reaction, the reaction solution was transferred to a 2 L separatory funnel, saturated sodium bicarbonate solution (300 mL) and ethyl acetate (400 mL) were added to the separatory funnel, the obtained solution was shaken thoroughly to separate the organic phase, and the aqueous phase was extracted three times with ethyl acetate (150 mL× 3); the obtained organic phases were combined, washing with a saturated sodium bicarbonate solution (150 mL) was carried out once, and washing with a saturated sodium chloride solution was carried out twice (150 mL×2); the obtained organic phase was poured into a 2 L conical flask, dried with anhydrous magnesium sulfate, and then filtered by suction and concentrated to 100 mL, silica gel powder (15 g) was added to the concentrated solution and the obtained solution was subjected to rotary evaporation to obtain a solid solution; the operations of dry sample loading and column chromatography were then carried out. Elution with 0.5% ammonia water: 2% methanol/dichloromethane was carried out; the product was collected and rotary evaporated to dryness to obtain an oily product; the oily product was dissolved with ethyl acetate (50 mL); n-hexane (100 mL) was added to the obtained solution, and the solution was homogenized by ultrasonic and then filtered by suction; and the obtained solution was dried, thus obtaining 5.9098 g of the product with a yield of 85.65%.

¹H-NMR (400 MHz, DMSO-d₆) δ 9.87 (s, 1H), 8.75 (s, 1H), 8.56 (s, 1H), 8.12 (s, 4H), 7.91 - 7.64 (m, 3H), 7.56 - 7.41 (m, 1H), 7.40 - 6.84 (m, 10H), 6.62 (s, 2H), 5.27 (s, 2H), 4.73 (s, 2H), 4.55 (s, 1H), 4.48 - 4.11 (m, 3H), 4.03 (s, 1H), 3.89 - 3.39 (m, 6H), 3.03 (s, 4H), 2.69 (s, 3H), 1.99 (s, 1H), 1.70 - 1.41 (m, 4H), 1.40 - 1.09 (m, 9H), 0.84 (s, 6H).

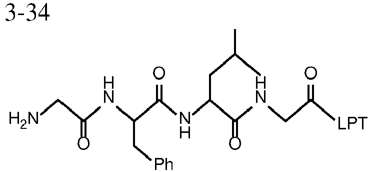

Product 3-31 (5.9 g, 5.5891 mmol) was added in a 250 mL flask and dissolved with dichloromethane (15 mL), TFA (4.2 mL, 55.8910 mmol) was then added to the solution, and the obtained solution was stirred overnight at room temperature. At the end of the reaction, the reaction solution was evaporated to remove the solvent; the obtained product was dissolved with ethyl acetate and the obtained solution was then transferred to a 2 L separatory funnel; saturated sodium bicarbonate solution (300 mL, being in aqueous phase and alkaline) and ethyl acetate (400 mL) were added to the separatory funnel, the obtained solution was shaken thoroughly to separate the organic phase, and the aqueous phase was extracted three times with ethyl acetate (150 mL× 3); the obtained organic phases were combined, washed once with saturated sodium bicarbonate solution (150 mL), and washed twice with saturated sodium chloride solution (150 mL×2); the obtained organic phase was poured into a 2 L conical flask, dried with anhydrous magnesium sulfate, and then filtered by suction and concentrated to 100 mL; n-hexane (100 mL) was added to the concentrated solution, and the mixed solution was treated by ultrasonic into a homogeneous phase and then filtered by suction to obtain a product; silica gel powder (5 g) was added to the filtrate and the obtained solution was subjected to rotary evaporation to obtain a solid solution; the operations of dry sample loading, column chromatography, elution with 1% ammonia water: 5% methanol/chloromethane, rotary evaporation, and vacuum drying were carried out, thus obtaining 4.0926 g of the product with a yield of 76.64%.

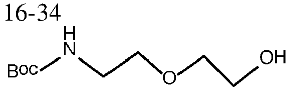

2-(-2aminoethoxy) ethanol (18.8680 g, 190.2226 mmol) was weighed and poured into a 500 mL round-bottomed flask and then diluted with dichloromethane (100 mL), triethylamine (38.4972 mL, 380.4452 mmol) was then added, and (Boc)₂O (49.8261 g, 228.2671 mmol) was then added slowly with stirring, and the obtained solution was stirred to react at room temperature. At the end of the reaction, the reaction solution was evaporated to dryness, then sodium bicarbonate powder was added, the obtained mixture was diluted with dichloromethane, silica gel powder was added, and the operations of evaporating, dry sample loading, and column chromatography were carried out. Elution with 50% ethyl acetate/petroleum ether was carried out, thus obtaining 27.3 g of the product with a yield of 70%.

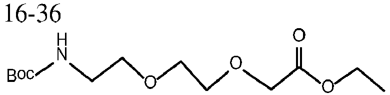

Product 16-24 (27.3 g, 132.8144 mmol) was added in a 500 mL reaction flask, nitrogen was introduced in the flask for protective purpose, the THF solution of potassium tert-butoxide was added, the mixed solution was placed at 0 °C to react, ethyl bromoacetate (17.6265 mL, 159.3773 mmol) was then added, and the obtained solution was first stirred for 3h, and then moved to room temperature for reaction. At the end of the reaction, the reaction solution was first evaporated to dryness, then deionized water and ethyl acetate were added, the organic phase was separated, the aqueous phase was extracted with ethyl acetate until there was no product, the organic phases were combined, dried with anhydrous sodium sulfate powder, and filtered by suction. The filtrate was subjected to dry sample loading and column chromatography. Elution with 30%-100% ethyl acetate/petroleum ether was carried out, thus obtaining 20 g of the product with a yield of 52%.

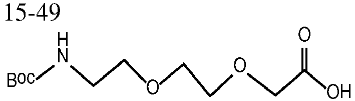

Product 16-36 (8.8 g, 30.2053 mmol) was added in a 250 mL reaction flask, 1,4-dioxane was added, and lithium hydroxide (1.5922 g, 66.4516 mmol) was further added with stirring, and 30 min later, deionized water was added until the solution became clear. At the end of the reaction, the reaction solution was extracted three times (100 mL×3) with a mixed solvent of methyl tert-butyl ether and n-hexane (1:1). The aqueous phase was adjusted to pH=1 with concentrated hydrochloric acid, and then extracted three times with ethyl acetate (300 mL×3), the ethyl acetate phases were combined, the dissolution and washing with saturated sodium chloride was carried out three times (100 mL×3), the obtained solution was concentrated, and the operations of dry sample loading, column chromatography, and elution with 40% ethyl acetate/petroleum ether were carried out, thus obtaining 6 g of the product with a yield of 75%.

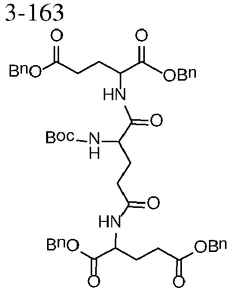

Boc-Glu-(OH)2 (6 g, 24.2669 mmol), Glu(OBn)2 (25.4588 g, 50.9605 mmol), HBUT (27.6089 g, 72.8007 mmol), and HOBT (9.8368 g, 72.8007 mmol) were weighed and added in a 1 L reaction flask and dissolved with DMF (100 mL); the obtained solution was stirred for 30 min at -5 °C, DIEA (40 mL, 242.669 mmol) was added dropwise, and the mixed solution was first stirred at a low temperature for 2 h and then reacted at room temperature. At the end of the reaction, the reaction solution was poured into a 1 L separatory funnel, a saturated sodium bicarbonate solution (400 mL) and ethyl acetate (300 mL) were then added to separate the organic phase, the aqueous phase was extracted three times with ethyl acetate (150 mL×3), the obtained organic phases were combined, and washing with deionized water was carried out three times (200 mL×3); the obtained solution was then concentrated and the operations of dry sample loading and column chromatography were carried out. Elution with 60%-70% ethyl acetate/petroleum ether was carried out, thus obtaining 25 g of the product, 4 g being extra-quota product.

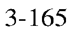

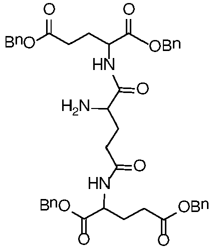

Product 3-163 (47.4 g, 54.7369 mmol) was weighed and dissolved by ultrasonic with 50 mL of dichloromethane and TFA (60 mL, 821.0541 mmol), a ground glass stopper was used, and the obtained solution was stirred to react at room temperature. At the end of the reaction, the reaction solution was transferred in a 2 L separatory funnel, a saturated sodium bicarbonate solution (600 mL) and ethyl acetate (500 mL) were then added to separate the organic phase, the aqueous phase was extracted three times with EA (150 mL×3), the obtained organic phases were combined, and washing with deionized water was carried out once (300 mL); the obtained solution was then concentrated and evaporated to dryness, and the operations of dry sample loading and column chromatography were carried out. Gradient elution with 5% methanol: 1% ammonia water/dichloromethane was carried out, thus obtaining 34 g of the product with a yield of 83%.

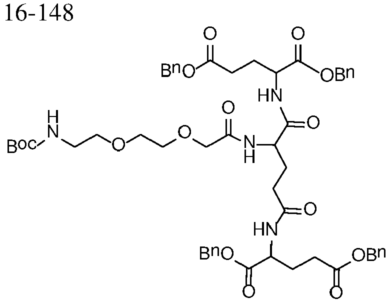

Product 15-49 (9 g, 24.7736 mmol), Product 3-165 (17.2481 g, 22.5215 mmol), HBUT (11.9575 g, 31.5301 mmol), and HOBT (4.2603 g, 31.5301 mmol) were weighed and added in a 500 mL reaction flask and dissolved with DMF (200 mL); the obtained solution was stirred for 30 min at -5 °C, DIEA (17 mL, 101.3468 mmol) was added dropwise, and the mixed solution was first stirred at a low temperature for 2 h and then reacted at room temperature. At the end of the reaction, the reaction solution was transferred in a 2 L separatory funnel, a saturated sodium bicarbonate solution (600 mL) and ethyl acetate (500 mL) were then added to separate the organic phase, the aqueous phase was extracted three times with ethyl acetate (150 mL×3), the obtained organic phases were combined, and washing with deionized water was carried out once (300 mL); the obtained solution was then concentrated and evaporated to dryness, and the operations of dry sample loading and column chromatography were carried out. Elution with 60% ethyl acetate/petroleum ether was carried out, thus obtaining 12.5 g of the product with a yield of 56%.

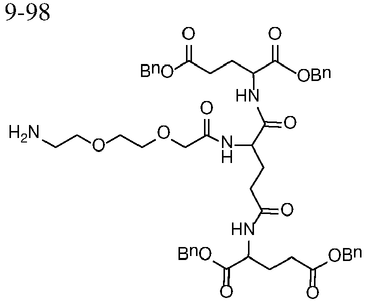

Product 16-98 (2 g, 1.978 mmol) was weighed and partially dissolved with dichloromethane (10 mL), and then TFA (4.4075 mL, 59.340 mmol) was added, the obtained solution was treated by ultrasonic until the Product 16-98 was dissolved completely; a ground glass stopper was used, and the mixed solution was stirred at room temperature. After TLC detection and color development with phosphomolybdic acid, the reaction was completed; the reaction solution was evaporated to dryness, the dichloromethane was removed, and then the obtained solid was dissolved with ethyl acetate (200 mL), and a saturated sodium bicarbonate solution was added until the aqueous phase became alkaline; then, the organic phase was separated, and the aqueous phase was extracted with ethyl acetate until there was no product in the aqueous phase; the organic phases were combined, and washing with saturated saline solution was carried out three times (100 mL×3); the obtained solution was concentrated and evaporated to dryness, and then subjected to column chromatography. With the column height of 5cm, elution with 1% ammonia water: 3% methanol/dichloromethane was carried out, and the elution product was collected, concentrated and evaporated to dryness, thus obtaining the product with a theoretical weight of 1.802 g, an actual weight of 1.8 g, and a yield of 99.9%.

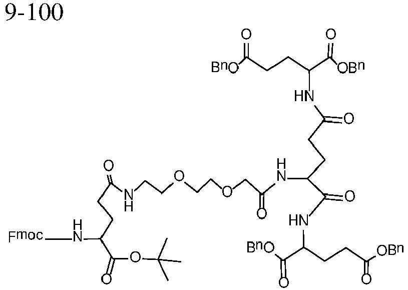

The reactant Fmoc-Glu-OtBu (1.1769 g, 2.7682 mmol) was added in a 250 mL reaction flask and then dissolved with DMF (20 mL), and the obtained solution was then placed at 0 °C; then, Product 9-99 (1.8 g, 1.9758 mmol) and PyAOP (1.4433 g, 2.7682 mmol) were added to the solution and the obtained solution was stirred for 0.5h; TMP (0.2602 mL, 1.9758 mmol) was slowly added dropwise and the obtained solution was stirred at a low temperature until the reaction ended. At the end of the reaction, a saturated sodium bicarbonate solution and ethyl acetate were then added to separate the organic phase, the aqueous phase was extracted three times with ethyl acetate (150 mL×3) until there was no product in the aqueous phase, the obtained organic phases were combined, and washing with a saturated saline solution was carried out three times (200 mL×3); the obtained solution was then concentrated, and the operations of dry sample loading and column chromatography were carried out. Elution with 2% methanol/dichloromethane was carried out, the elution product was concentrated, evaporated to dryness, and dried at room temperature, thus actually obtaining 2.8 g of the product, 0.2 g being extra-quota product.

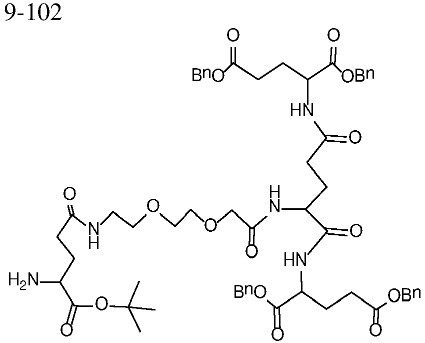

The reactant Product 9-100 (1.9758 mmol) was added in a 250 mL round-bottomed flask and dissolved with DMF (40 mL), morpholine (5.1639 mL, 59.274 mmol) was then added, and the obtained solution was stirred at room temperature and applied to the TLC plate every 0.5h. One hour later, the reaction ended. A saturated sodium bicarbonate solution and ethyl acetate were then added to separate the organic phase, the aqueous phase was extracted with ethyl acetate until there was no product in the aqueous phase, the obtained organic phases were combined, and washing with a saturated saline solution was carried out; the obtained solution was then concentrated and evaporated to dryness, and the operations of dry sample loading, column chromatography, and gradient elution with 1%-5% methanol/dichloromethane were carried out, thus obtaining 1.8 g of the product with a yield of 83.106%.

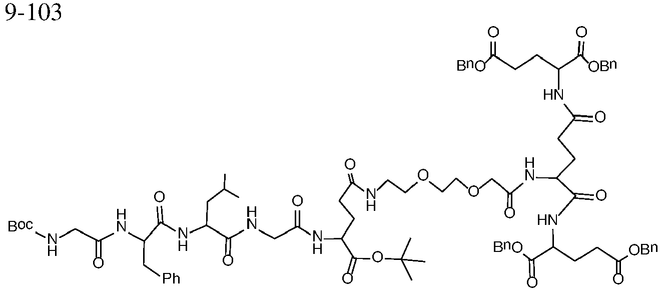

Product 9-102 (1.8 g, 1.6420 mmol), Product 3-30 (theoretical value, 2.2988 mmol), HBTU (84.3030 g, 222.2946 mmol), and HOBT (30.0387 g, 222.2946 mmol) were added in a round-bottomed flask and dissolved with DMF (50 mL); the obtained solution was stirred at -5 °C for 0.5 h and DIEA (132.2681 mL, 800.2606 mmol) was then slowly added dropwise; the obtained solution first reacted for 2 h and then stirred at room temperature overnight to react. At the end of the reaction, a saturated sodium bicarbonate solution and ethyl acetate were then added to separate the organic phase, the aqueous phase was extracted with ethyl acetate until there was no organic product, the obtained organic phases were combined, and washing with a saturated saline solution was carried out; the obtained solution was then concentrated and evaporated to dryness, and the operations of dry sample loading, column chromatography, and gradient elution with 3% methanol/dichloromethane were carried out, thus obtaining 1.8 g of the product with a yield of 72 %.

¹H-NMR (400 MHz, DMSO-d₆) δ 8.52 (s, 1H), 8.30 (s, 1H), 8.16 (s, 1H), 8.11 - 8.00 (m, 2H), 7.93 - 7.83 (m, 2H), 7.69 (s, 1H), 7.33 (s, 16H), 7.25 (s, 2H), 7.22 - 7.21 (m, 1H), 7.20 (s, 3H), 7.18 (s, 1H), 7.15 (s, 2H), 6.90 (s, 1H), 4.54 (s, 1H), 4.41 - 4.26 (m, 4H), 4.07 (s, 1H), 3.90 (s, 2H), 3.72 (s, 2H), 3.61 - 3.49 (m, 6H), 3.39 (s, 4H), 3.19 (s, 3H), 3.00 (s, 1H), 2.77 (s, 1H), 2.43 (s, 4H), 2.30 (s, 2H), 2.14 (s, 4H), 2.02 (s, 2H), 1.89 (s, 4H), 1.81 - 1.72 (m, 2H), 1.62 - 1.56 (m, 1H), 1.48 (s, 3H), 1.37 (s, 18H), 1.25 (s, 6H), 0.88 (s, 3H), 0.83 (s, 3H).

MALDI-TOF MS: [M+H⁺] 1570.36, [M+Na⁺] 1592.52.

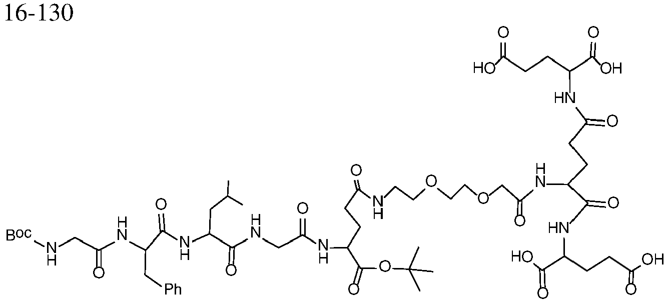

Product 9-103 (0.6846 g, 0.4361 mmol) and 10% Pd/C (50mg) were added in a hydrogenation reactor and then dissolved with DMF (40 mL); the hydrogenation reactor was pumped to reach a vacuum state and H2 was then introduced; and suction operations were repeated three times until the hydrogen pressure reached 16 psi, and the solution was stirred overnight. At the end of the reaction, diatomaceous earth was added to a sand core funnel to carry out suction filtering of the reaction solution, and the diatomaceous earth was then washed three times with DMF (25 mL×3), and the solution was directly used for next reaction.

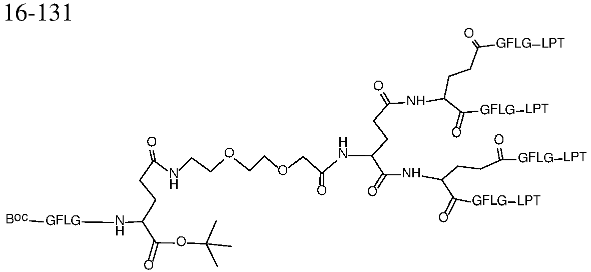

GFLG-LPT (2 g, 2.0931 mmol), HBTU (0.9923 g, 2.6166 mmol), HOBT (0.3536 g, 2.6166 mmol) were weighed and dissolved in the DMF (110 mL) solution of Product 16-130, the obtained solution was stirred at -5 °C for 30min, DIEA (0.2962 mL, 7.8498 mmol) was then slowly added dropwise, and the obtained solution first reacted 2 h and then was placed at room temperature until the reaction ended. At the end of the reaction, the reaction solution was transferred to a 1 L separatory funnel, and a saturated sodium bicarbonate solution (400 mL) was added to separate out a solid; suction filtering was then carried out and filtrate was extracted twice with ethyl acetate (150 mLx2). The organic phases were combined, washing with saturated sodium chloride was washed once (150 mL), and the obtained solution was concentrated. The filter cake was dissolved and extracted with a mixed solvent of methanol and dichloromethane to obtain the final organic phase, silica gel powder was added, and the obtained solution was evaporated to dryness. The operations of dry sample loading and column chromatography were carried out. Elution with 1% ammonia water: 7% methanol/dichloromethane was carried out, thus obtaining 1.5 g of the product with a yield of 71%.

¹H -NMR (400 MHz, DMSO-d₆) δ 10.52 (s, 4H), 8.80 (s, 4H), 8.70 (m, 4H), 8.29 (s, 4H), 8.19 (s, 12H), 8.06 (s, 9H), 8.00 - 7.94 (m, 10H), 7.82 (s, 4H), 7.69 - 7.65 (m, 4H), 7.47 (s, 5H), 7.32 (s, 13H), 7.19 (s, 26H), 7.14 - 7.11 (m, 4H), 7.09 (s, 2H), 6.69 (s, 3H), 6.55 (s, 1H), 5.27 (s, 8H), 4.72 (s, 8H), 4.57 (s, 6H), 4.33 (s, 7H), 4.27 (s, 2H), 4.25 - 4.18 (m, 7H), 4.15 (s, 2H), 4.09 - 4.05 (m, 2H), 3.86 (s, 7H), 3.74 (s, 8H), 3.62 (s, 8H), 3.06 (s, 6H), 3.01 (s, 8H), 2.74 (s, 6H), 2.68 - 2.66 (m, 1H), 2.33 (m, 1H), 2.16 - 2.09 (m, 8H), 1.59 (s, 5H), 1.49 - 1.46 (m, 10H), 1.35 (s, 18H), 1.24 (s, 18H), 0.84 (s, 28H), 0.79 (s, 7H).

MALDI-TOF MS: [M+H⁺] 4956.03, [M+Na⁺] 4977.62.

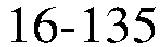

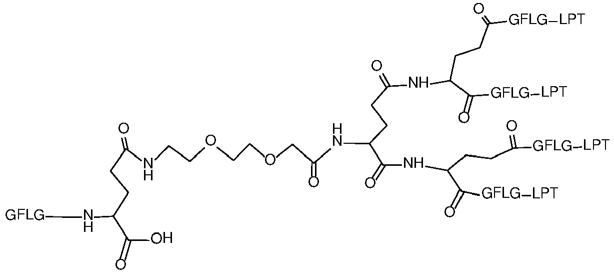

Product 16-131 (1.4 g, 0.2822 mmol) was added in a 250 mL reaction flask and then dissolved by ultrasonic with dichloromethane (20 mL) and TFA (1.0480 mL, 14.1100 mmol), a ground glass stopper was used, and the obtained solution was stirred to react. At the end of the reaction, the reaction solution was first evaporated to dryness and then dissolved with ethyl acetate (30 mL), the obtained solution was precipitated with n-hexane, and the process of dissolution and precipitation was repeated until a powdery product was obtained; the suction filtering was carried out, and the obtained solid was dissolved with a mixed solvent (20% methanol: 80% dichloromethane 40 mL); silica gel powder (8 g) was added, the obtained solution was evaporated to dryness into a solid powder, and the operations of sample loading and column chromatography were carried out. Gradient elution with 12%-40% methanol/dichloromethane was carried out, thus obtaining 1.3 g of the product with a yield of 95%.

¹H- NMR (400 MHz, DMSO-d₆) δ 9.86 (s, 3H), 8.74 (s, 3H), 8.55 (s, 3H), 8.26-7.94 (m, 25H), 7.75 (s, 8H), 7.47 (s, 4H), 7.36 - 7.03 (m, 41H), 6.60 (s, 2H), 5.25 (s, 5H), 4.72 (s, 6H), 4.57 (s, 5H), 4.34 (s, 7H), 4.23 (s, 7H), 3.76 (s, 18H), 3.60 (s, 15H), 3.03 (s, 21H), 2.89 (s, 7H), 2.73 (s, 11H), 2.69 (s, 7H), 2.36 - 2.31 (m, 3H), 2.12 (s, 9H), 1.49 (s, 13H), 1.12 (s, 15H), 0.87 - 0.75 (m, 30H).

MALDI-TOF MS: [M+H⁺] 4799.95, [M+Na⁺] 4821.63.

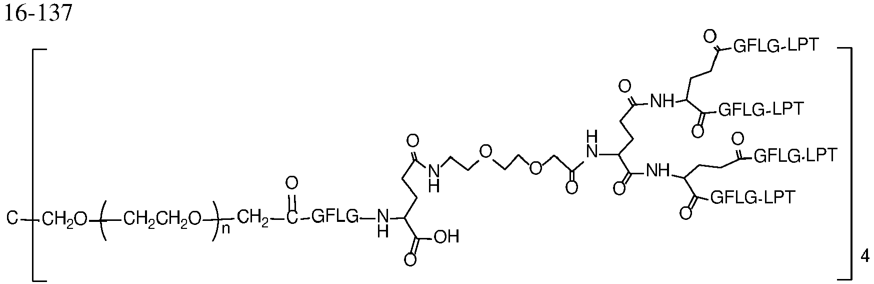

Product 16-135 (1.3 g, 0.2706 mmol) was weighed and dissolved with DMF, DIEA (0.18 mL, 1.080 mmol) and 4ARM-SCM-40K (2.3598 g, 0.0541 mmol) were added to the obtained solution in sequence, DMF (10 mL) was added again, and the obtained solution reacted in the dark at a low speed stirring. Eight days later, the reaction ended; methyl tert-butyl ether (300 mL) was poured into the reaction solution, the obtained solution was then filtered by suction; the filter cake was subjected to dry sample loading and column chromatography. Elution with 10%-15% methanol/dichloromethane was carried out, thus obtaining 1.6 g of the product with a yield of 50%.

¹H- NMR (400 MHz, DMSO-d₆) δ 10.49 (s, 2H), 8.86 (s, 6H), 8.67 (s, 7H), 8.33 - 7.64 (m, 125H), 7.50 - 6.85 (m, 144H), 6.72 (s, 13H), 6.55 (s, 4H), 5.27 (s, 17H), 4.72 (s, 20H), 4.56 (s, 14H), 4.37 - 4.13 (s, 54H), 4.01 (s, 9H), 3.45 - 3.29 (s, 728H), 3.01 (s, 63H), 2.89 (s, 74H), 2.73 (s, 74H), 2.15 (s, 19H), 1.50 - 1.12 (m, 312H), 0.93 - 0.76 (m, 120H).

MALDI-TOF MS: from 60740.1 to 61026.38.

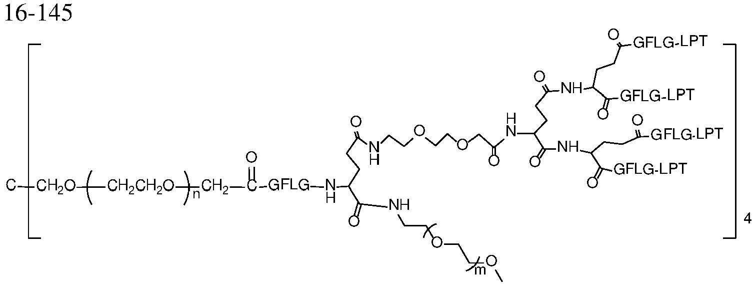

The reactants, Product 16-137 (1.6 g, 0.0264 mmol), M-NH2-2K.HCl (0.4293 g, 0.2108 mmol), HBTU (0.0600 g, 0.1581 mmol), and HOBT (0.0214 g, 0.1581 mmol), were weighed and added in a 250 mL reaction flask and dissolved with DMF (100 mL); the obtained solution was stirred at -5 °C for 30min; DIEA (87.1uL, 0.5270 mmol) was slowly added dropwise and the obtained solution was stirred for 1 h and cooled to room temperature to react in the dark at a low speed stirring. At the end of the reaction, methyl tert-butyl ether was added to precipitate the reaction solution, suction filtering was carried out to obtain a powder product, the powder product was dissolved with a mixed solvent of methanol and dichloromethane, silica gel was added to the obtained solution, and the operations of evaporation, dry sample loading and column chromatography were carried out. Elution with 1% ammonia water: 8% methanol/dichloromethane was carried out, the pure product was collected and evaporated to dryness, absolute ethanol (10 mL) and dichloromethane (10 mL) were added for ultrasonic treatment, methyl tert-butyl ether was added to the obtained solution until a solid came out; suction filtering was carried out, and the obtained solid was further dissolved with absolute ethanol (10 mL) and dichloromethane (10 mL); the obtained solution was precipitated with methyl tert-butyl ether; the process of dissolution and precipitation was repeated three times, thus obtaining 1.3 g of the product with a yield of 72%.

¹H-NMR (400 MHz, DMSO-d₆) δ 9.85 (s, 11H), 8.75 (s, 13H), 8.55 (s, 12H), 8.10 (s, 118H), 7.88 - 7.68 (m, 37H), 7.46 (s, 23H), 7.38 - 7.02 (m, 175H), 6.60 (s, 10H), 5.25 (s, 31H), 4.72 (s, 37H), 4.57 (s, 28H), 4.28 (s, 88H), 3.88 (s, 129H), 3.33 (s, 2725H), 3.09 - 2.86 (m, 108H), 2.80 ― 2.67 (m, 27H), 2.11 (s, 23H), 1.54 (s, 55H), 0.97 - 0.72 (m, 120H).

### Example 10: Synthesis of Compound 17-87

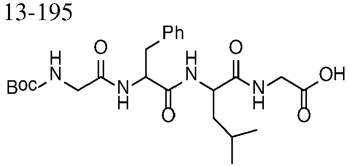

Boc-GFLG-OBn (15.6242 g, 26.8138 mmol) and 10% Pd/C (0.2500 g) were added in a hydrogenation reactor and then dissolved with DMF (50 mL); the air in the reactor was then pumped out to reach a vacuum state by a water pump; hydrogen was introduced to a pressure of 14 Psi in the reactor, hydrogen was then discharged, the reactor was pumped to reach a vacuum state by the water pump, hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth, the diatomaceous earth was washed with DMF (20 mL×3), and the filtrate was put into a 500 mL round-bottomed flask as the raw material for the next reaction.

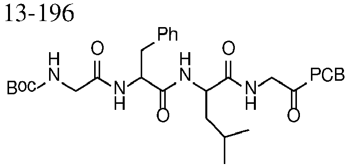

Palbociclib (10.0000 g, 22.3448 mmol, referred to as PCB), HBTU (12.7111 g, 33.5172 mmol), and HOBT (7.8809 g, 33.5172 mmol) were added to the DMF (200 mL) solution of Product 13-195 (13.2076 g, 26.8138 mmol); the obtained solution was stirred at -5 °C for about 20 min to react, and then DIEA (16.6200 mL, 100.5516 mmol) was slowly added dropwise over 20min; the obtained solution was further stirred at -5 °C for 2h, and then stirred overnight at room temperature to react. At the end of the reaction, the reaction solution was transferred to a 2 L beaker, a saturated sodium bicarbonate solution (150 mL) was added to separate out a solid, the suction filtering was carried out, and the filter cake was washed with deionized water (100 mL × 4) and then dried in a vacuum oven, thus obtaining 20.6 g of Product 13-196 with a yield of 100%. H -NMR (400 MHz, DMSO-d₆) δ 10.17 (s, 1H), 8.96 (s, 1H), 8.10 (dd, J = 12.0, 4.2 Hz, 2H), 7.88 (t, J = 7.8 Hz, 2H), 7.52 (m,7H), 6.97 (s, 1H), 5.94 - 5.61 (m, 1H), 4.39 (s, 1H), 4.00 (d, J = 5.4 Hz, 2H), 3.58 (dd, J = 16.1, 5.4 Hz, 4H), 3.16 (d, J = 19.0 Hz, 4H), 2.43 (s, 3H), 2.31 (s, 3H), 2.25 (s, 2H), 1.89 (s, 2H), 1.82 - 1.70 (m, 5H), 1.66 - 1.56 (m, 3H), 1.52 - 1.43 (m, 2H), 1.38 (s, 9H), 1.24 (d, J = 6.4 Hz, 2H), 0.86 (dd, J = 15.4, 6.5 Hz, 6H)

MALDI-TOF MS: [M+H⁺] 922.50

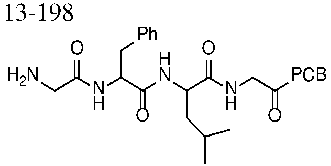

Product 13-196 (20.6000 g, 22.3448 mmol) was added in a 500 mL flask and then dissolved with dichloromethane (30 mL), TFA (24.8800 mL, 335.172 mmol) was then added in the flask, and the mixed solution was stirred overnight at room temperature to react. At the end of the reaction, the reaction solution was concentrated and precipitated with methyl tert-butyl ether (200 mL) to obtain a solid; suction filtering was then carried out, the filter cake was dissolved with a mixed solvent of ethanol (10 mL)-dichloromethane (40 mL), silica gel powder (50 g) was added to the obtained solution, and the solution was then evaporated to dryness to obtain a powdery solid; the operations of dry sample loading, column chromatography, and gradient elution with a dichloromethane mixed solution containing 1% of ammonia water and 3%-8% of methanol were carried out; the elution product was then collected, concentrated, evaporated into a solid, and dried in a vacuum oven, thus obtaining 17.6012 g of Product 13-198 with a yield of 96%. ¹H-NMR (400 MHz, DMSO-d₆) δ 10.17 (s, 1H), 8.96 (s, 1H), 8.16 (t, J = 5.5 Hz, 1H), 8.08 (d, J = 2.9 Hz, 1H), 7.98 (d, J = 8.9 Hz, 1H), 7.89 (d, J = 9.0 Hz, 1H), 7.52 (m, 7H), 5.83 (p, J = 8.6 Hz, 1H), 4.43 (d, J = 5.9 Hz, 1H), 4.00 (d, J = 5.5 Hz, 2H), 3.60 (s, 4H), 3.23 - 3.07 (m, 6H), 2.42 (s, 3H), 2.31 (s, 3H), 2.24 (s, 2H), 2.05 (s, 1H), 1.89 (s, 2H), 1.81 - 1.71 (m, 5H), 1.60 (dd, J = 12.3, 6.2 Hz, 3H), 1.47 (ddd, J = 19.4, 13.3, 6.1 Hz, 2H), 1.23 (s, 1H), 0.88 (dd, J = 11.5, 6.5 Hz, 6H); MALDI-TOF MS: [M+H⁺] 822.64, [M+Na⁺] 844.42

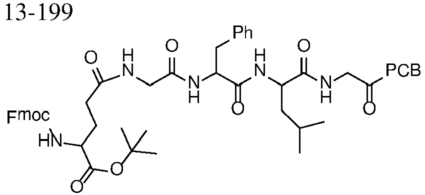

Fmoc-Glu-OtBu (7.2467 g, 17.0323 mmol) was added in a 500 mL flask and dissolved with DMF (150 mL); the obtained solution reacted at 0 °C, and the Product 13-198 (10.0000 g, 12.1659 mmol) and PyAOP (8.8800 g, 17.0323 mmol) were added to the reaction solution with stirring; the obtained solution was stirred for 20min, and then TMP (1.6 000 mL, 12.1659 mmol) was slowly added dropwise over 5min; the obtained solution was stirred overnight at 0 °C to react. At the end of the reaction, n-hexane (200 mL) was added to layer the reaction solution, the supernatant was discarded, n-hexane (150 mL × 6) was further added to the lower solution, and the oily product was finally obtained and then dissolved with methanol (20 mL) and dichloromethane (80 mL), silica gel powder (45 g) was added, and the obtained solution was then evaporated to dryness to obtain a powdery solid; the operations of dry sample loading, column chromatography, and elution with a dichloromethane mixed solution containing 5% of methanol were carried out; the elution product was then collected, concentrated, evaporated into a solid, and dried in a vacuum oven, thus obtaining 13.4636 g of Product 13-199 with a yield of 100%. ¹H-NMR (400 MHz, DMSO-d₆) δ 10.17 (s, 1H), 8.96 (s, 1H), 8.14 - 7.99 (m, 4H), 7.89 (d, J = 7.7 Hz, 3H), 7.73 (d, J = 7.5 Hz, 3H), 7.51 (dd, J = 9.1, 2.8 Hz, 1H), 7.42 (t, J = 7.4 Hz, 2H), 7.33 (m,8H), 5.84 (dd, J = 17.8, 8.9 Hz, 1H), 4.49 - 4.19 (m, 4H), 4.05 - 3.83 (m, 3H), 3.79 - 3.54 (m, 6H), 3.16 (d, J = 19.6 Hz, 5H), 2.42 (s, 3H), 2.31 (s, 3H), 2.27 - 2.20 (m, 3H), 1.93 (m, 7H), 1.77 (s, 2H), 1.66 - 1.47 (m, 5H), 1.39 (s, 9H), 0.86 (dd, J = 16.0, 6.5 Hz, 6H); MALDI-TOF MS: [M+H⁺] 1229.61

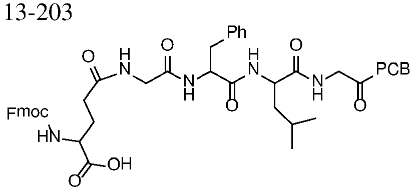

Product 13-199 (13.4636 g, 10.9511 mmol) was added in a 500 mL flask and then dissolved with dichloromethane (30 mL), TFA (13.5500 mL, 182.4885 mmol) was then added with stirring, and the mixed solution was stirred overnight at room temperature to react. At the end of the reaction, the reaction solution was concentrated and precipitated with methyl tert-butyl ether (250 mL) to obtain a solid; suction filtering was then carried out, the filter cake was washed with methyl tert-butyl ether (100 mL×3) and then dissolved with a mixed solvent of methanol (20 mL)-dichloromethane (80 mL), silica gel powder (35 g) was added to the obtained solution, and the solution was then evaporated to dryness to obtain a powdery solid; the operations of dry sample loading, column chromatography, and gradient elution with a dichloromethane mixed solution containing 1% of ammonia water and 3%-8% of methanol were carried out; the elution product was then collected, concentrated, evaporated into a solid, and dried in a vacuum oven, thus obtaining 11.7000 g of Product 13-203 with a yield of 82%. ¹H-NMR (400 MHz, DMSO-d₆) δ 10.91 (s, 1H), 8.99 (s, 1H), 8.15 - 8.00 (m, 4H), 7.87 (t, J = 11.9 Hz, 3H), 7.72 (t, J = 7.2 Hz, 3H), 7.55 (dt, J = 8.4, 7.8 Hz, 1H), 7.42 (d, J = 7.1 Hz, 2H), 7.33 (m,8H), 5.93 - 5.80 (m, 1H), 4.27 (dd, J = 6.9, 2.8 Hz, 4H), 3.99 (d, J = 5.2 Hz, 3H), 3.85 - 3.68 (m, 2H), 3.61 (s, 4H), 3.39 - 3.14 (m, 4H), 3.02 (td, J = 6.6, 3.9 Hz, 1H), 2.44 (s, 3H), 2.34 (s, 3H), 2.25 (d, J = 7.6 Hz, 3H), 1.93 (s, 2H), 1.83 (m, 7H), 1.61 (dd, J = 12.7, 6.4 Hz, 3H), 1.50 (d, J = 6.9 Hz, 2H), 0.86 (dd, J = 16.3, 6.5 Hz, 6H);MALDI-TOF MS: [M+H⁺] 1173.82, [M+Na⁺] 1195.54

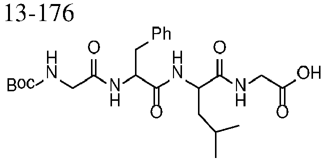

Boc-GFLG-OBn (7.1784 g, 12.3194 mmol) was added in a hydrogenation reactor, 10% Pd/C (0.1500 g) was then added, and the obtained mixture was dissolved with DMF (30 mL); the air in the reactor was then pumped out to reach a vacuum state by a water pump; hydrogen was introduced to a pressure of 14 Psi in the reactor, hydrogen was then discharged, the reactor was pumped to reach a vacuum state by the water pump, hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth, the diatomaceous earth was washed three times with DMF (20 mL×3), and the filtrate was put into a 500 mL round-bottomed flask as the raw material for the next reaction.

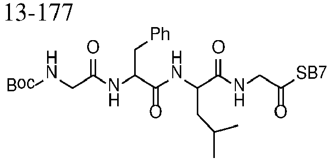

SB-743921 (4.9000 g, 9.4765 mmol, referred to as SB7), HBTU (5.3908 g, 14.2148 mmol) and HOBT (1.9208 g, 14.2148 mmol) were added to the DMF (100 mL) solution of Product 13-176 (6.0682 g, 12.3194 mmol); the obtained solution was stirred at -5 °C for 20 min to react, and then DIEA (7.0483 mL, 42.6443 mmol) was slowly added dropwise over 10min; the obtained solution was further stirred at -5 °C for 1h, and then stirred overnight at room temperature to react. At the end of the reaction, the reaction solution was transferred to a 1 L separatory funnel and extracted with saturated sodium bicarbonate solution (250 mL) and ethyl acetate (200 mL) to obtain the organic phase; the aqueous phase was washed three times with ethyl acetate (100 mL×3), and the obtained organic phases were combined, washed with saturated saline solution (100 mL×2), then dried with anhydrous sodium sulfate (30 g) for 20min, and filtered by suction to obtain the filtrate; the filtrate was concentrated, and evaporated to obtain a solid; the solid was dried in a vacuum oven for 3h, thus obtaining 9.4000 g of Product 13-177 with a yield of 100%. ¹H-NMR (400 MHz, DMSO) δ 8.09 (dd, J = 9.3, 5.2 Hz, 3H), 7.89 (d, J = 7.5 Hz, 1H), 7.65 - 7.42 (m, 2H), 7.42 - 7.06 (m, 14H), 6.98 (s, 1H), 5.75 (d, J = 6.8 Hz, 1H), 4.39 - 4.11 (m, 2H), 3.90 (d, J = 14.5 Hz, 1H), 3.58 (dd, J = 25.3, 6.0 Hz, 4H), 3.15 (dd, J = 11.9, 7.5 Hz, 1H), 2.89 (s, 1H), 2.64 (s, 2H), 2.33 (s, 3H), 1.57 (m,4H), 1.47 (d, J = 7.0 Hz, 2H), 1.36 (s, 9H), 1.23 (dd, J = 21.9, 9.2 Hz, 5H), 0.96 (d, J = 6.3 Hz, 3H), 0.84 (dd, J = 14.8, 6.4 Hz, 6H), 0.53 (d, J = 4.8 Hz, 2H)

MALDI-TOF MS: [M+H⁺] 991.47

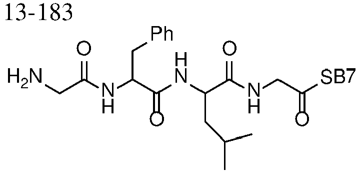

Product 13-177 (9.4000 g, 9.4765 mmol) was added in a 500 mL flask and then dissolved with dichloromethane (30 mL), TFA (10.5000 mL, 142.1475 mmol) was then added in the flask, and the mixed solution was stirred overnight at room temperature to react. At the end of the reaction, the reaction solution was concentrated and precipitated with methyl tert-butyl ether (150 mL) to obtain a powdery solid; suction filtering was then carried out, the filter cake was washed with methyl tert-butyl ether (100 mL×3) and then dissolved with a mixed solvent of methanol (20 mL)-dichloromethane (80 mL), silica gel powder (30 g) was added to the obtained solution, and the solution was then evaporated to dryness to obtain a powdery solid; the operations of dry sample loading, column chromatography, and elution with a dichloromethane mixed solution containing 1% of ammonia water and 4% of methanol were carried out; the elution product was then collected, concentrated, and evaporated to obtain a solid, and the solid was dried in a vacuum oven, thus obtaining 7.3 g of Product 13-183 with a yield of 86.4%. ¹H-NMR (400 MHz, DMSO-d6) δ 8.17 (dt, J = 11.4, 8.0 Hz, 4H), 7.57 (dd, J = 8.6, 2.0 Hz, 2H), 7.43 - 6.96 (m, 15H), 6.24 (s, 2H), 5.76 (s, 1H), 4.34 (d, J = 6.7 Hz, 1H), 4.17 (d, J = 14.4 Hz, 1H), 3.90 (d, J = 14.8 Hz, 1H), 3.53 (d, J = 11.5 Hz, 2H), 2.85 - 2.53 (m, 3H), 2.33 (s, 3H), 1.52 (m, 7H), 1.32 (s, 1H), 1.15 (dd, J = 38.5, 6.6 Hz, 5H), 0.96 (d, J = 6.4 Hz, 3H), 0.86 (dd, J = 11.2, 6.5 Hz, 6H), 0.53 (d, J = 5.2 Hz, 3H); MALDI-TOF MS: [M+H⁺] 891.23, [M+Na⁺] 913.41

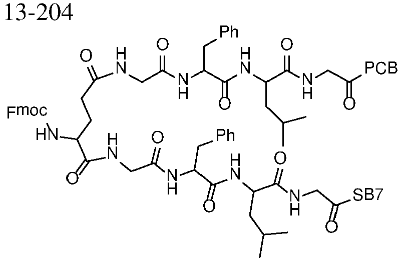

Product 13-203 (9.1503 g, 7.7986 mmol) was added in a 500 mL flask and dissolved with DMF (40 mL); the obtained solution reacted at 0 °C, and the Product 13-183 (7.3 g, 8.1885 mmol) and PyAOP (5.6924 g, 10.9180 mmol) were added to the reaction solution; the obtained solution was stirred for 10min, and then TMP (1.0270 mL, 7.7986 mmol) was slowly added dropwise over 5min; the obtained solution was stirred overnight at 0 °C to react. At the end of the reaction, n-hexane (200 mL) was added to layer the reaction solution, the supernatant was discarded, n-hexane (150 mL) was further added to the lower solution, the solution was layered again, the supernatant was discarded, n-hexane (150 mL) was further added to the lower solution, and such operations were repeated six times to finally obtain an oily product; methyl tert-butyl ether (200 mL) was added to the oily product to obtain a powder solid; suction filtering was then carried out, the filter cake was washed with methyl tert-butyl ether (100 mL×3) and then dissolved with a mixed solvent of methanol (10 mL)-dichloromethane (40 mL), silica gel powder (20 g) was added to the obtained solution, and the solution was then evaporated to dryness to obtain a powdery solid; the operations of dry sample loading, column chromatography, and gradient elution with a dichloromethane mixed solution containing 1% of ammonia water and 3%-6% of methanol were carried out; the elution product was then collected, concentrated, evaporated to obtain a solid, and the solid was then dried in a vacuum oven, thus obtaining 15.962 g of Product 13-204 with a yield of 100%. ¹H-NMR (400 MHz, DMSO-d₆) δ 10.16 (s, 1H), 8.96 (s, 1H), 8.24 (d, J = 5.7 Hz, 1H), 8.17 - 7.98 (m, 7H), 7.89 (d, J = 9.5 Hz, 4H), 7.79 - 7.64 (m, 3H), 7.61 - 7.10 (m, 28H), 5.94 - 5.68 (m, 2H), 4.46 - 4.12 (m, 6H), 3.98 (dd, J = 12.4, 7.2 Hz, 3H), 3.77 - 3.56 (m, 9H), 3.24 - 3.13 (m, 4H), 3.01 (td, J = 6.6, 3.9 Hz, 7H), 2.42 (s, 3H), 2.36 - 2.21 (m, 10H), 1.90 (d, J = 11.0 Hz, 3H), 1.83 - 1.72 (m, 13H), 1.67 - 1.43 (m, 8H), 1.19 (dd, J = 18.9, 11.8 Hz, 1H), 0.88 (ddt, J = 13.5, 9.7, 7.8 Hz, 13H), 0.52 (s, 2H); MALDI-TOF MS: [M+Na⁺] 2068.53

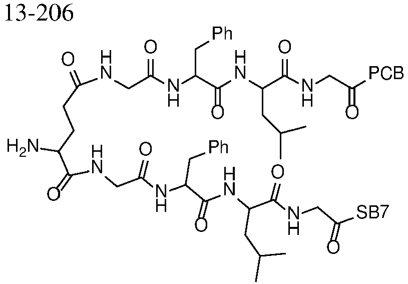

Product 13-204 (15.9620 g, 7.7986 mmol) was added in a 500 mL flask and then dissolved with DMF (30 mL) and morpholine (10.2000 mL, 116.979 mmol) was then added, and the mixed solution was stirred for 1 h at room temperature to react. At the end of the reaction, n-hexane (200 mL) was added to layer the reaction solution, the supernatant was discarded, n-hexane (150 mL x 6) was further added to the lower solution, and the oily product was finally obtained and then dissolved with methanol (20 mL) and dichloromethane (80 mL), silica gel powder (40 g) was added, and the obtained solution was then evaporated to dryness to obtain a powdery solid; the operations of dry sample loading, column chromatography, and elution with a dichloromethane mixed solution containing 1% of ammonia water and 3%-7% of methanol were carried out; the elution product was then collected, concentrated, evaporated to obtain a solid, and the solid was then dried in a vacuum oven, thus obtaining 11.9000 g of Product 13-206 with a yield of 84%. ¹H-NMR (400 MHz, DMSO-d₆) δ 10.16 (s, 1H), 8.96 (s, 1H), 8.36 - 8.02 (m, 10H), 7.89 (d, J = 9.0 Hz, 1H), 7.53 (ddd, J = 12.1, 8.8, 2.4 Hz, 3H), 7.20 (dd, J = 28.1, 13.3 Hz, 22H), 5.82 (dt, J = 24.3, 12.1 Hz, 2H), 4.48 - 4.22 (m, 2H), 4.13 (dd, J = 22.6, 10.2 Hz, 1H), 4.08 - 3.89 (m, 3H), 3.81 - 3.50 (m, 11H), 3.23 - 3.09 (m, 6H), 2.81 - 2.67 (m, 3H), 2.42 (s, 3H), 2.39 - 2.18 (m, 10H), 1.91 - 1.75 (m, 11H), 1.67 - 1.43 (m, 10H), 1.19 (dd, J = 18.9, 11.8 Hz, 1H), 0.88 (ddd, J = 19.8, 16.3, 4.3 Hz, 16H), 0.52 (s, 2H)

MALDI-TOF MS: [M+H⁺] 1822.87, [M+Na⁺] 1845.64

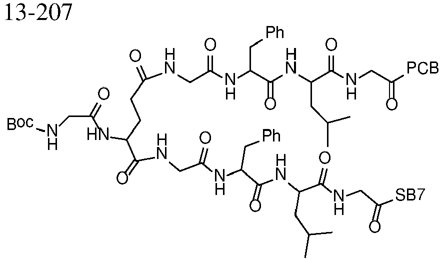

Product 13-206 (11.9000 g, 6.5221 mmol), Boc-Gly-0H (1.4853 g, 8.4788 mmol), HBTU (3.7102 g, 9.7832 mmol), and HOBT (1.3220 g, 9.7832 mmol) were added in a 500 mL flask and dissolved with a DMF (40 mL), the obtained solution was stirred for 10 min at -5 °C; then, DIEA (4.8509 mL, 29.3495 mmol) was slowly added dropwise over about 15 min to further react at -5 °C for 30min, and then the reaction solution was stirred overnight at room temperature to further react. At the end of the reaction, n-hexane (150 mL) was added to layer the reaction solution, the supernatant was discarded, n-hexane (150 mL x 5) was further added to the lower solution, and finally an oily product was obtained; methyl tert-butyl ether (200 mL) was added to the oily product to obtain a powder solid; suction filtering was then carried out, the filter cake was washed with methyl tert-butyl ether (100 mL×3) and then dissolved with a mixed solvent of methanol (20 mL)-dichloromethane (80 mL), silica gel powder (35 g) was added to the obtained solution, and the solution was then evaporated to dryness to obtain a powdery solid; the operations of dry sample loading, column chromatography, and gradient elution with a dichloromethane mixed solution containing 1% of ammonia water and 4%-6% of methanol were carried out; the elution product was then collected, concentrated, evaporated to obtain a solid, and the solid was then dried in a vacuum oven, thus obtaining 12.9250 g of Product 13-207 with a yield of 100%. ¹H-NMR (400 MHz, DMSO-d₆) δ 10.16 (s, 1H), 8.96 (s, 1H), 8.27 (s, 1H), 8.15 - 7.86 (m, 10H), 7.53 (ddd, J = 12.0, 8.9, 2.4 Hz, 3H), 7.36 - 7.09 (m, 21H), 6.92 (t, J = 5.7 Hz, 1H), 5.90 - 5.74 (m, 2H), 4.46 - 4.16 (m, 4H), 3.96 (dd, J = 23.4, 9.8 Hz, 3H), 3.81 - 3.50 (m, 12H), 3.24 - 3.06 (m, 6H), 2.86 - 2.61 (m, 3H), 2.42 (s, 3H), 2.35 - 2.15 (m, 10H), 1.83 (m,11H), 1.69 - 1.40 (m, 10H), 1.36 (s, 9H), 1.18 (d, J = 18.9 Hz, 1H), 1.01 - 0.79 (m, 16H), 0.52 (d, J = 4.8 Hz, 2H); MALDI-TOF MS: [M+Na⁺] 2003.81

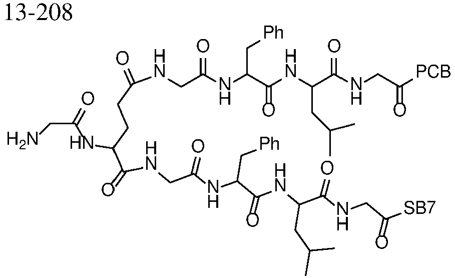

Product 13-207 (12.9250 g, 6.5221 mmol) was added in a 500 mL flask, dichloromethane (30 mL) and TFA (7.2652 mL, 97.8315 mmol) were then added in sequence, and the mixed solution was stirred overnight at room temperature to react. At the end of the reaction, methyl tert-butyl ether (200 mL) was added to the reaction solution to obtain a solid; suction filtering was then carried out, the filter cake was washed with methyl tert-butyl ether (100 mL×3) and then dissolved with a mixed solvent of methanol (20 mL)-dichloromethane (80 mL), silica gel powder (30 g) was added to the obtained solution, and the solution was then evaporated to dryness to obtain a powdery solid; the operations of dry sample loading, column chromatography, and gradient elution with a dichloromethane mixed solution containing 1% of ammonia water and 4%-6% of methanol were carried out; the elution product was then collected, concentrated, evaporated to dryness, and dried in a vacuum oven, thus obtaining 10.9 g of Product 13-208 with a yield of 87%. ¹H-NMR (400 MHz, DMSO-d₆) δ 10.16 (s, 1H), 8.96 (s, 1H), 8.33 (t, J = 5.5 Hz, 1H), 8.21 - 7.78 (m, 10H), 7.65 - 7.41 (m, 3H), 7.41 - 7.06 (m, 21H), 5.92 - 5.71 (m, 2H), 4.48 - 4.12 (m, 4H), 4.01 - 3.83 (m, 3H), 3.80 - 3.56 (m, 11H), 3.15 (dd, J = 18.0, 5.7 Hz, 7H), 2.81 - 2.60 (m, 3H), 2.42 (s, 3H), 2.37 - 2.16 (m, 10H), 1.95 - 1.45 (m, 21H), 1.36 - 1.09 (m, 3H), 0.87 (ddd, J = 21.2, 16.1, 6.4 Hz, 16H), 0.53 (d, J = 5.1 Hz, 2H); MALDI-TOF MS: [M+H⁺] 1880.84, [M+Na⁺] 1903.66

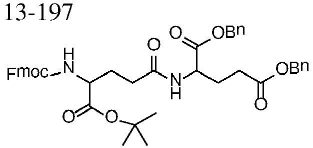

Fmoc-Glu-OtBu (4.0900 g, 9.6151 mmol) was added in a 500 mL flask and dissolved with DMF (25 mL); the obtained solution reacted at 0 °C, and H-Glu-(OBn)2.TsOH (5.3372 g, 10.6834 mmol) and PyAOP (7.8000 g, 14.9572 mmol) were added to the reaction solution with stirring; the obtained solution was stirred for about 20min, and then TMP (3.3770 mL, 25.6402 mmol) was slowly added dropwise over 4min; the obtained solution was stirred overnight at 0 °C. At the end of the reaction, the reaction solution was transferred to a 1 L separatory funnel and extracted with deionized water (150 mL) and ethyl acetate (250 mL) to obtain the organic phase; the aqueous phase was washed with ethyl acetate (150 mL×2), and the obtained organic phases were combined, washed with saturated saline solution (150 mL×2), then concentrated and evaporated to dryness; the operations of dry sample loading, column chromatography, and gradient elution with a petroleum ether mixed solution containing 20%-50% ethyl acetate were carried out; the elution product was then collected, concentrated, evaporated to dryness, and dried in a vacuum oven for 4h, thus obtaining 6.1 g of Product 13-197 with a yield of 77%. MALDI-TOF MS: [M+Na⁺] 756.99.

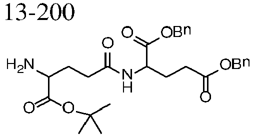

Product 13-197 (6.1000 g, 8.3012 mmol) was added in a 500 mL flask and then dissolved with DMF (25 mL) and morpholine (7.2000 mL, 83.0120 mmol) was then added, and the mixed solution was stirred for 1 h at room temperature to react. At the end of the reaction, the reaction solution was transferred to a 1 L separatory funnel and extracted with deionized water (200 mL) and ethyl acetate (200 mL) to obtain the organic phase; the aqueous phase was washed with ethyl acetate (100 mL×2), and the obtained organic phases were combined and washed with saturated saline solution (150 mL×2); the obtained solution was then collected, concentrated, evaporated to dryness, and dried in a vacuum oven, thus obtaining 4.25 g of Product 13-200 with a yield of 100%. MALDI-TOF MS: [M+H⁺]513.19, [M+Na⁺]534.12.

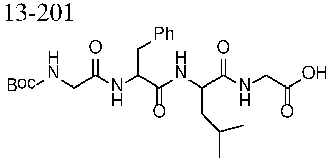

Boc-GFLG-OBn (6.2881 g, 10.7916 mmol) and Pd/C (0.2000 g) were added in a hydrogenation reactor and then dissolved with DMF (30 mL); the air in the reactor was then pumped out to reach a vacuum state by a water pump; hydrogen was introduced to a pressure of 0.14 MPa in the reactor, hydrogen was then discharged, the reactor was pumped to reach a vacuum state by the water pump, hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth, the diatomaceous earth was washed with DMF (20 mL×3), and the filtrate was put into a 500 mL round-bottomed flask, thus obtaining the DMF solution of Product 13-201.

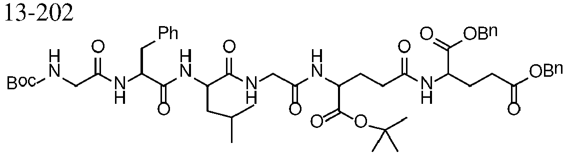

Product 13-200 (4.2000 g, 8.3012 mmol), HBTU (4.7222 g, 12.4518 mmol), and HOBT (1.6826 g, 12.4518 mmol) were added to a DMF (500 mL) solution containing Product 13-201 (5.3156 g, 10.7916 mmol); the obtained solution was stirred to react at -5 °C for 20min, and then DIEA (6.1760 mL, 37.3554 mmol) was slowly added dropwise over 15min; the obtained solution was further stirred at -5 °C for 2h, and then stirred overnight at room temperature to react. At the end of the reaction, the reaction solution was transferred to a 1 L separatory funnel and extracted with saturated sodium bicarbonate solution (200 mL) and ethyl acetate (250 mL) to obtain the organic phase; the aqueous phase was washed with ethyl acetate (150 mL×2), and the obtained organic phases were combined, washed with saturated saline solution (150 mL), then concentrated and evaporated to dryness; the operations of dry sample loading, column chromatography, and gradient elution with a petroleum ether mixed solution containing 30%-50% ethyl acetate were carried out; the elution product was then collected, concentrated, and evaporated to dryness to obtain a solid, and the solid was then dried in a vacuum oven, thus obtaining 6.4 g of Product 13-202 with a yield of 78%. ¹H -NMR (400 MHz, DMSO-d₆) δ 8.31 (d, *J* = 7.4 Hz, 1H), 8.16 (d, *J* = 7.7 Hz, 1H), 8.06 (d, *J* = 6.3 Hz, 2H), 7.85 (d, *J* = 8.0 Hz, 1H), 7.42 - 7.29 (m, 10H), 7.24 - 7.12 (m, 5H), 6.90 (t, *J* = 5.9 Hz, 1H), 4.54 (d, *J* = 3.9 Hz, 1H), 4.31 (ddd, *J* = 19.2, 14.5, 7.9 Hz, 1H), 4.06 (ddd, *J* = 25.5, 13.3, 6.9 Hz, 1H), 3.81 - 3.67 (m, 2H), 3.60 - 3.46 (m, 2H), 3.00 (dd, *J* = 13.8, 4.3 Hz, 1H), 2.84 - 2.75 (m, 1H), 2.45 - 2.37 (m, 2H), 2.27 - 2.13 (m, 2H), 2.08 - 1.70 (m, 5H), 1.66 - 1.45 (m, 4H), 1.37 (d, *J* = 10.8 Hz, 18H), 1.27 - 1.11 (m, 2H), 0.86 (dd, *J* = 19.4, 6.4 Hz, 7H).

MALDI-TOF MS: [M+H⁺] 987.50, [M+Na⁺] 1009.40.

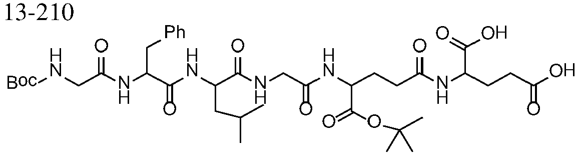

Product 13-202 (0.6557 g, 0.6643 mmol) and 10% Pd/C (0.1000 g) were added in a hydrogenation reactor and then dissolved with DMF (30 mL); the air in the reactor was then pumped out to reach a vacuum state by a water pump; hydrogen was introduced to a pressure of 14 Psi in the reactor, hydrogen was then discharged, the reactor was pumped to reach a vacuum state by the water pump, hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth, the diatomaceous earth was washed with DMF (20 mL×3), and the filtrate was put into a 500 mL round-bottomed flask as the raw material for the next reaction.

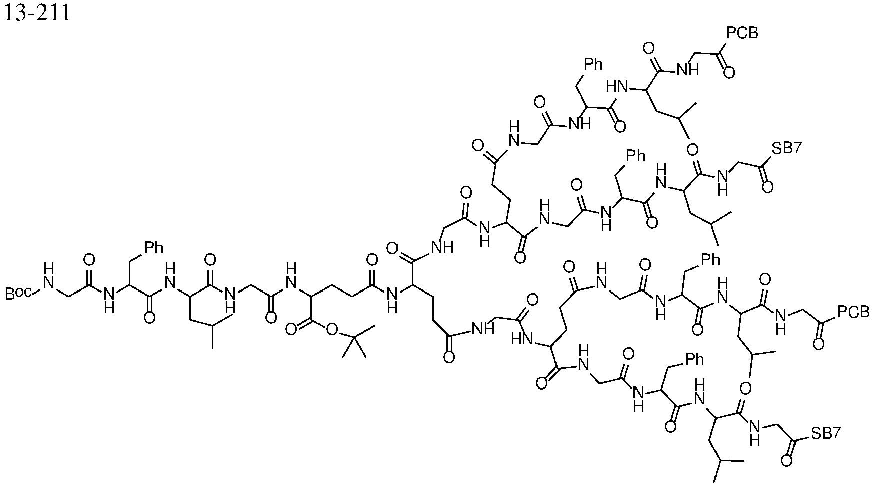

Product 13-208 (3.000 g, 1.5944 mmol), HBTU (0.7558 g, 1.9929 mmol), and HOBT (0.2693 g, 1.9929 mmol) were added to a DMF (100 mL) solution containing Product 13-210 (0.5360 g,0.6643 mmol); the obtained solution was stirred to react at -5 °C for about 20min, and then DIEA (0.9881 mL, 5.9787 mmol) was slowly added dropwise over 3min; the obtained solution was further stirred at -5 °C for 1h, and then stirred overnight at room temperature to react. At the end of the reaction, n-hexane (150 mL) was added to layer the reaction solution, the supernatant was discarded, and n-hexane (150 mL × 5) was added to the lower liquid to obtain an oily final product; methyl tert-butyl ether (200 mL) was added to the oily product to separate out a powdery solid; suction filtering was carried out, the resulting filter cake was dissolved with methyl tert-butyl ether (100 mL × 3) and then washed with a mixed solvent (20% methanol: 80% dichloromethane) (50 mL × 2), and dried in a vacuum oven, thus obtaining 2.6 g of Product 13-211 with a yield of 86%. ¹H -NMR (400 MHz, DMSO-d₆) δ 10.13 (s, 2H), 9.00 (d, *J* = 32.7 Hz, 2H), 8.38 - 7.75 (m, 40H), 7.52 (ddd, *J =* 11.9, 8.8, 2.4 Hz, 7H), 7.35 - 7.03 (m, 38H), 5.94 - 5.67 (m, 2H), 4.50 (d, *J* = 44.7 Hz, 3H), 4.39 - 4.15 (m, 6H), 4.11 - 3.88 (m, 5H), 3.82 - 3.50 (m, 21H), 3.37 (d, *J* = 31.5 Hz, 12H), 3.24 - 3.00 (m, 15H), 2.75 (d, *J* = 12.3 Hz, 5H), 2.32 (t, *J* = 34.5 Hz, 33H), 2.04 - 1.66 (m, 18H), 1.64 - 1.45 (m, 18H), 1.33 (dd, *J* = 21.5, 17.0 Hz, 20H), 1.26 - 1.16 (m, 7H), 1.04 - 0.74 (m, 38H), 0.50 (s, 7H). MALDI-TOF MS: [M+Na⁺]4553.27

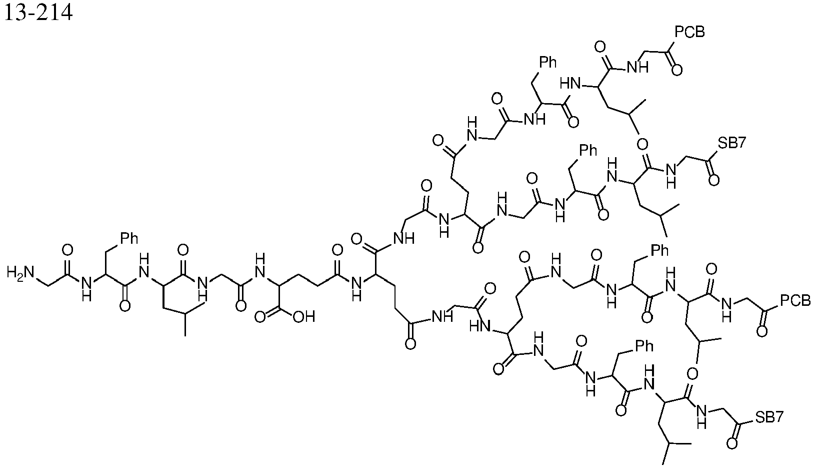

Product 13-211 (2.6000 g, 0.5734 mmol) was added in a 500 mL flask and then dissolved with dichloromethane (10 mL). TFA (1.7000 mL, 22.4378 mmol) was then added in the flask, and the mixed solution was stirred overnight at room temperature to react. At the end of the reaction, the reaction solution was concentrated and precipitated with methyl tert-butyl ether (200 mL) to obtain a powdery solid; suction filtering was then carried out, the filter cake was washed with methyl tert-butyl ether (100 mL×2) and then dissolved with a mixed solvent of methanol (10 mL)-dichloromethane (40 mL), silica gel powder (15 g) was added to the obtained solution, and the solution was then evaporated to dryness to obtain a powdery solid; the operations of dry sample loading, column chromatography, and gradient elution with a dichloromethane mixed solution containing 1% of ammonia water and 8%-12% of methanol were carried out; the elution product was then collected, concentrated, evaporated to dryness, and dried in a vacuum oven, thus obtaining 1.8 g of Product 13-214 with a yield of 72%. ¹H-NMR (400 MHz, DMSO-d₆) δ 10.13 (s, 2H), 8.95 (s, 2H), 8.50 - 7.74 (m, 33H), 7.52 (ddd, *J* = 12.0, 8.9, 2.3 Hz, 7H), 7.33 - 7.01 (m, 45H), 5.82 (p, *J* = 8.9 Hz, 2H), 4.54 (d, *J* = 8.8 Hz, 4H), 4.38 - 4.15 (m, 7H), 3.98 (dd, *J* = 40.7, 16.5 Hz, 5H), 3.63 (d, *J* = 19.3 Hz, 21H), 3.00 (dd, *J* = 26.6, 13.6 Hz, 12H), 2.77 (d, *J* = 5.9 Hz, 7H), 2.58 (s, 4H), 2.42 (s, 6H), 2.35 - 2.05 (m, 28H), 1.87 (t, *J* = 46.8 Hz, 20H), 1.65 - 1.39 (m, 22H), 1.32 - 1.10 (m, 9H), 1.00 - 0.67 (m, 40H), 0.50 (s, 6H);MALDI-TOF MS: [M+H⁺]4375.92, [M+Na⁺]4397.67.

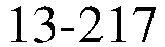

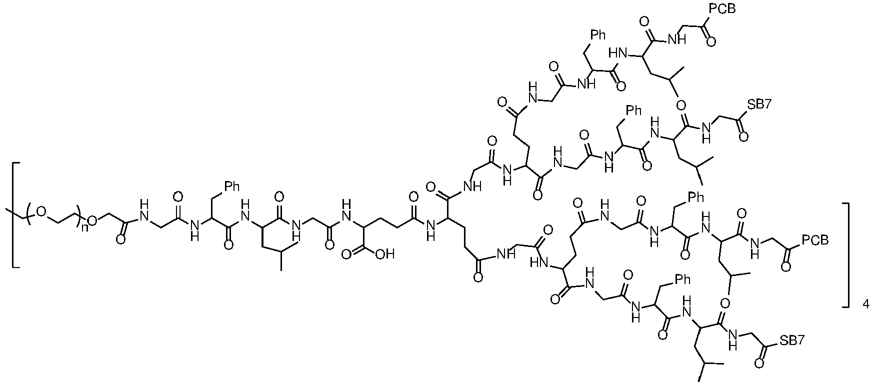

Product 13-214 (1.8000 g, 0.4112 mmol) was added to a 500 mL flask and then dissolved with dichloromethane (10 mL) and DMF (70 mL); DIEA (0.28 mL, 1.7140 mmol) was slowly added at -5 °C, 4ARM-SCM-40K (3.5940 g, 0.0857 mmol, purchased from JenKem) was then added and dissolved through stirring, and the obtained solution was stirred for a week in the dark at room temperature at a low speed to react. At the end of the reaction, n-hexane (150 mL) and methyl tert-butyl ether (50 mL) were added to layer the reaction solution, the supernatant was discarded, n-hexane (150 mL) and methyl tert-butyl ether (50 mL) were further added to the lower oily solution, and such operations were repeated three times to separate out a solid; the obtained solution was filtered, the resulting filter cake was dissolved with a mixed solvent of methanol (20 mL)-dichloromethane (80 mL), silica gel powder (15 g) was added to the obtained solution, and the solution was then evaporated to dryness to obtain a powdery solid; the operations of dry sample loading, column chromatography, and gradient elution with a dichloromethane mixed solution containing 1% of ammonia water and 10% of methanol were carried out; the elution product was then collected, concentrated, evaporated to dryness, and dried in a vacuum oven, thus obtaining 2.7 g of Product 13-21 with a yield of 53%. ¹H-NMR (400 MHz, DMSO-d6) δ 10.12 (s, 5H), 8.99 (d, *J* = 27.8 Hz, 7H), 8.48-7.60 (m, 136H), 7.50 (d, *J* = 15.8 Hz, 24H), 7.34 ― 7.11 (m, 182H), 5.98 - 5.78 (m, 8H), 5.32 (s, 6H), 5.13 (d, *J* = 6.0 Hz, 10H), 4.63 - 4.02 (m, 133H), 3.51 (s, 3048H), 3.26 ― 2.94 (m, 35H), 2.70 (d, *J* = 24.5 Hz, 58H), 2.42 (s, 24H), 2.27 (d, *J* = 27.3 Hz, 85H), 2.01 ― 1.65 (m, 80H), 1.64 - 1.49 (m, 94H), 1.34 (d, *J* = 6.2 Hz, 38H), 1.17 (s, 26H), 0.91 - 0.78 (m, 182H), 0.49 (s, 26H);

MALDI-TOF MS: from 57757.84 to 61540.95

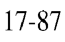

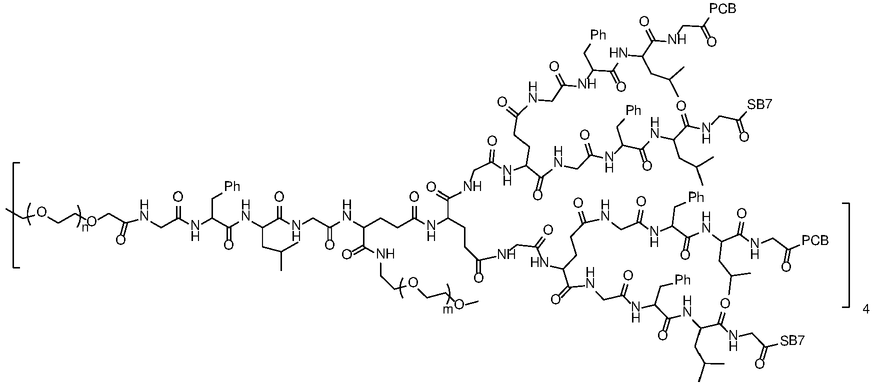

Product 13-217 (2.7000 g,0.0453 mmol) was added to a 500 mL flask and then dissolved with DMF (60 mL), and M-NH2-2K.HCl (0.5592 g, 0.2745 mmol, purchased from JenKem), HBTU (0.1041 g, 0.2745 mmol), and HOBT (0.0370 g, 0.2745 mmol) were then added to the obtained solution; the mixed solution was stirred at -5 °C for about 20 min to react, DIEA (0.1665 mL, 1.0076 mmol) was then slowly added dropwise, and the obtained solution further reacted at -5 °C for 1h; and the solution was stirred for a week in the dark at room temperature at a low speed to react. At the end of the reaction, n-hexane (150 mL) was added to layer the solution, the supernatant was discarded, n-hexane (250 mL × 5) was further added to the lower solution to obtain an oily final product; the oily final product was dissolved with a mixed solvent of methanol (20 mL)-chloromethane (80 mL), silica gel powder (15 g) was added to the obtained solution, and the solution was then evaporated to dryness to obtain a powdery solid; the operations of dry sample loading, column chromatography, and gradient elution with a dichloromethane mixed solution containing 1% of ammonia water and 6-8% of methanol were carried out; the elution product was then collected, concentrated, evaporated to dryness to obtain a solid, and dried for 2 h in vacuum, thus obtain a crude product; the crude product was dissolved with absolute ethanol (15 mL), and n-hexane (100 mL) was added to the obtained solution to separate out a solid; the solution was filtered and the filter cake was washed with n-hexane (50 mL×3) and dried with the vacuum oven, thus obtaining 2.34 g of Product 17-87 with a yield of 74%. ¹H-NMR (400 MHz, DMSO-d₆) δ 10.33 (s, 6H), 8.96 (s, 8H), 8.39 - 7.55 (m, 123H), 7.55-7.41 (m, 20H), 7.32 - 6.85 (m, 199H), 5.83 (s, 8H), 4.77 - 4.48 (m, 16H), 4.40-3.95 (m, 23H), 3.88 (m, 106H), 3.52 (s, 4127H), 2.91 (s, 71H), 2.82-2.65 (m, 21H), 2.42 (s, 24H), 2.29 (m, 112H), 1.84 (m, 80H), 1.53 (m, 86H), 1.37 - 1.15 (m, 38H), 1.09 - 0.72 (m, 162H), 0.50 (s, 11H); MALDI-TOF MS: from 66240.77 to 67307.40.

### Example 11: Synthesis of Compound 17-141

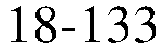

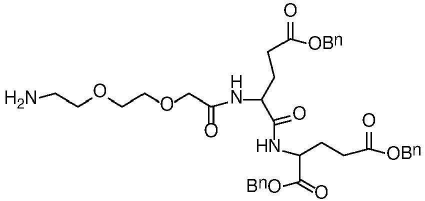

Product 8-125 (5.4407 g, 6.8706 mmol, i.e., Product 10-96) was added in a 250 mL flask and then dissolved with dichloromethane (30 mL), TFA (7.6533 mL, 103.0590 mmol) was then added in the flask, and the mixed solution was stirred overnight at room temperature to react. At the end of the reaction, the reaction solution was evaporated to dryness and concentrated and then transferred to a 2 L separatory funnel and extracted with saturated sodium bicarbonate (120 mL) and ethyl acetate (100 mL); the aqueous phase was washed with ethyl acetate (80 mL×3), and the obtained organic phases were combined and washed with saturated saline solution (80 mL×3); the obtained solution was then concentrated, evaporated to dryness, and dried in a vacuum oven, thus obtaining 79.9 g of Product 18-133 with a yield of 100%.

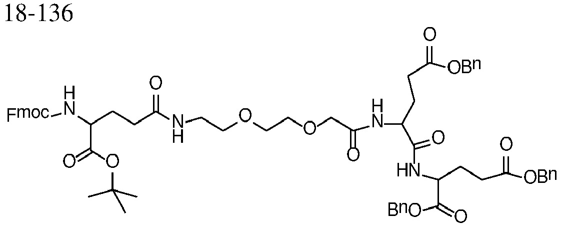

Fmoc-Glu-OtBu (2.6344 g, 6.1067 mmol) was added in a 500 mL flask and dissolved with DMF (50 mL); the obtained solution reacted at -5 °C, and the Product 18-133 (3.8404 g, 5.5516 mmol), HBTU (3.1581 g, 8.3273 mmol) and HOBT (1.1253 g, 8.3273 mmol) were added to the reaction solution; the obtained solution was stirred for 20min, and then DIEA (4.1291 mL, 24.9880 mmol) was slowly added dropwise over 10min; the obtained solution was stirred overnight at -5 °C to react. At the end of the reaction, the reaction solution was transferred to a 1 L separatory funnel and extracted with deionized water (150 mL) and ethyl acetate (100 mL) to obtain the organic phase; the aqueous phase was washed with ethyl acetate (100 mL×3), and the obtained organic phases were combined, washed with saturated saline solution (80 mL×3), then concentrated and evaporated to dryness, and then dissolved with dichloromethane (100 mL), silica gel powder (15 g) was added to the obtained solution, and the solution was then evaporated to dryness to obtain a powdery solid; the operations of dry sample loading, column chromatography, and gradient elution with a petroleum ether mixed solution containing 20%-50% ethyl acetate were carried out; the elution product was then collected, concentrated, evaporated to obtain a solid, and the solid was dried in a vacuum oven, thus obtaining 4.4926 g of Product 18-136 with a yield of 74%.

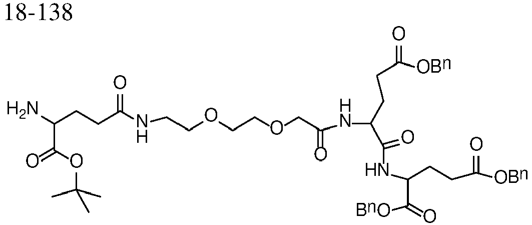

Product 18-136 (4.4926 g, 4.0908 mmol) was added in a 250 mL flask and then dissolved with DMF (20 mL) and morpholine (5.3458 mL, 61.3614 mmol) was then added, and the mixed solution was stirred for 1 h at room temperature to react. At the end of the reaction, the reaction solution was transferred to a 1 L separatory funnel and extracted with deionized water (120 mL) and ethyl acetate (100 mL) to obtain the organic phase; the aqueous phase was washed with ethyl acetate (100 mL×2), and the obtained organic phases were combined and washed with saturated saline solution (80 mL×3); the obtained solution was then concentrated, evaporated to dryness, and dried in a vacuum oven, thus obtaining 3.2 g of Product 18-138 with a yield of 73 %.

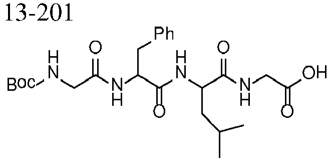

Boc-GFLG-OBn (6.2881 g, 10.7916 mmol) was added in a hydrogenation reactor, 10% Pd/C (0.2000 g) was then added, and the obtained mixture was dissolved with DMF (30 mL); the air in the reactor was then pumped out to reach a vacuum state by a water pump; hydrogen was introduced to a pressure of 0.14 MPa in the reactor, hydrogen was then discharged, the reactor was pumped to reach a vacuum state by the water pump, hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth, the filter cake was washed three times with DMF (20 mL×3), and the filtrate was put into a 500 mL round-bottomed flask, thus obtaining the DMF solution of Product 13-201.

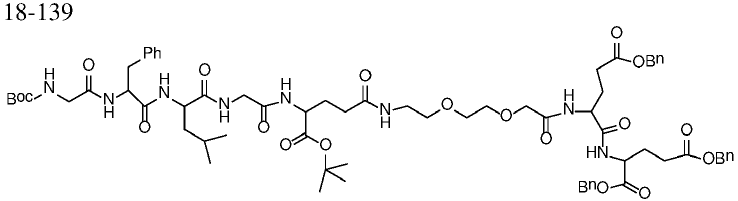

Product 18-138 (3.2000 g, 3.6488), HBTU (2.0757 g, 5.4733 mmol), and HOBT (0.7396 g, 5.4733 mmol) were added to a DMF (100 mL) solution containing Product 13-201 (2.3365 g, 4.7435 mmol); the obtained solution was stirred to react at -5 °C for 20min, and then DIEA (2.7139 mL, 16.4198 mmol) was slowly added dropwise over 5min; the obtained solution was further stirred at -5 °C for 2h, and then stirred overnight at room temperature to react. At the end of the reaction, the reaction solution was transferred to a 1 L separatory funnel and extracted with saturated sodium bicarbonate solution (120 mL) and ethyl acetate (100 mL) to obtain the organic phase; the aqueous phase was washed with ethyl acetate (100 mL×3), and the obtained organic phases were combined, washed with saturated saline solution (80 mL × 3), then concentrated and then dissolved with dichloromethane (100 mL), silica gel powder (10 g) was added to the obtained solution, and the solution was then evaporated to dryness to obtain a powdery solid; the operations of dry sample loading, column chromatography, and gradient elution with a petroleum ether mixed solution containing 1% ammonia water and 3%-6% of methanol were carried out; the elution product was then collected, concentrated, evaporated to obtain a solid, and the solid was dried in a vacuum oven, thus obtaining 3.8 g of Product 18-139 with a yield of 78%.

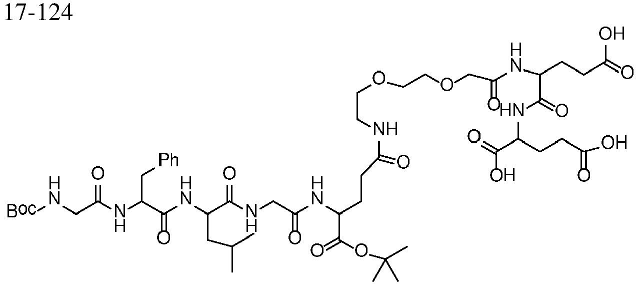

Product 18-139 (0.6152 g, 0.4555 mmol) and Pd/C (0.1000 g) were added in a reactor and then dissolved with DMF (30 mL); the air in the reactor was then pumped out to reach a vacuum state by a water pump; hydrogen was introduced to a pressure of 0.14 MPa in the reactor, hydrogen was then discharged, the reactor was pumped to reach a vacuum state by the water pump, hydrogen was then introduced again, and such operations were repeated three times; finally, hydrogen was introduced again into the reactor, and the mixed solution then was stirred overnight at room temperature to react. At the end of the reaction, the reaction solution was filtered with diatomaceous earth, the filter cake was washed with DMF (20 mL×3), and the filtrate was put into a 500 mL round-bottomed flask as the raw material for the next step.

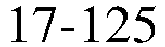

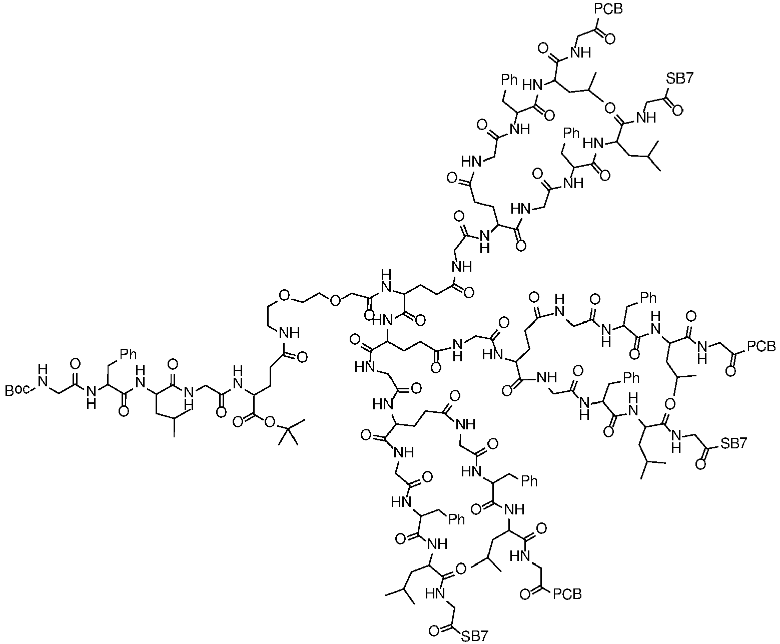

Product 17-102 (3.000 g, 1.5944 mmol), HBTU (0.7774 g, 2.0498 mmol), and HOBT (0.2770 g, 2.0498 mmol) were added to a DMF (90 mL) solution containing Product 17-124 (0.4925 g,0.4555 mmol); the obtained solution was stirred to react at -5 °C for 20min, and then DIEA (1.0160 mL, 6.1493 mmol) was slowly added dropwise over 5min; the obtained solution was further stirred at -5 °C for 1h, and then stirred overnight at room temperature to react. At the end of the reaction, n-hexane (150 mL) was added to layer the reaction solution, the supernatant was discarded, and n-hexane (150 mL × 3) was added to the lower liquid to obtain an oily final product; methyl tert-butyl ether (200 mL) was added to the oily product to separate out a solid; suction filtering was carried out, the resulting filter cake was washed with ethyl tert-butyl ether (100 mL × 3) and then dissolved with a methanol (5 mL)-dichloromethane (20 mL) solution, and ethyl acetate (150 mL) was then added to separate out a solid; then, filtering was carried out and the resulting filter cake was dried in a vacuum oven, thus obtaining 3.039 g of Product 17-125 with a yield of 100%.

MALDI-TOF MS: [M+H⁺] 6667.23

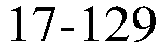

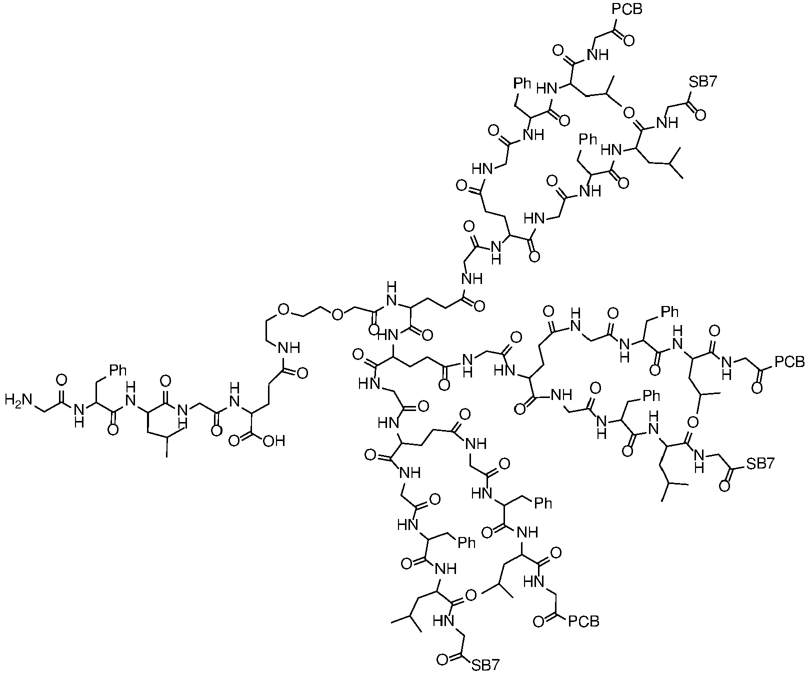

Product 17-125 (3.0390 g, 0.4555 mmol) was added in a 500 mL flask, dichloromethane (15 mL) and TFA (0.6765 mL, 9.1100 mmol) were then added in sequence, and the mixed solution was stirred overnight at room temperature to react. At the end of the reaction, the reaction solution was concentrated and precipitated with methyl tert-butyl ether (200 mL) to obtain a solid; suction filtering was then carried out, the filter cake was washed with methyl tert-butyl ether (50 mL×2) and then dissolved with a mixed solvent of methanol (10 mL)-dichloromethane (40 mL), silica gel powder (15 g) was added to the obtained solution, and the solution was then evaporated to dryness to obtain a powdery solid; the operations of dry sample loading, column chromatography, and gradient elution with a dichloromethane mixed solution containing 1% of ammonia water and 7%-10% of methanol were carried out; the elution product was then collected, concentrated, evaporated to dryness, and dried in a vacuum oven, thus obtaining 1.2341 g of Product 17-129 with a yield of 42%. ¹H- NMR (400 MHz, DMSO-d₆) δ 10.13 (s, 3H), 8.95 (s, 2H), 8.50 - 7.80 (m, 47H), 7.62 - 7.37 (m, 10H), 7.18 (ddd, *J* = 13.0, 9.2, 5.1 Hz, 65H), 5.94 - 5.74 (m, 3H), 4.71-4.50 (m, 4H), 4.42 ― 4.08 (m, 12H), 3.97 ― 3.86(m, 9H), 3.79 ― 3.56 (m, 33H), 3.25 ― 3.02 (m, 27H), 2.98 ― 2.81 (m, 12H), 2.79 - 2.63 (m, 14H), 2.42 (s, 12H), 2.35 ― 2.05 (m, 45H), 1.91 ― 1.74 (m, 28H), 1.63 ― 1.41 (m, 30H), 1.33 ― 1.12 (m, 11H), 0.91 - 0.80 (m, 47H), 0.49 (s, 10H).

MALDI-TOF MS: [M+Na⁺] 6531.83

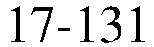

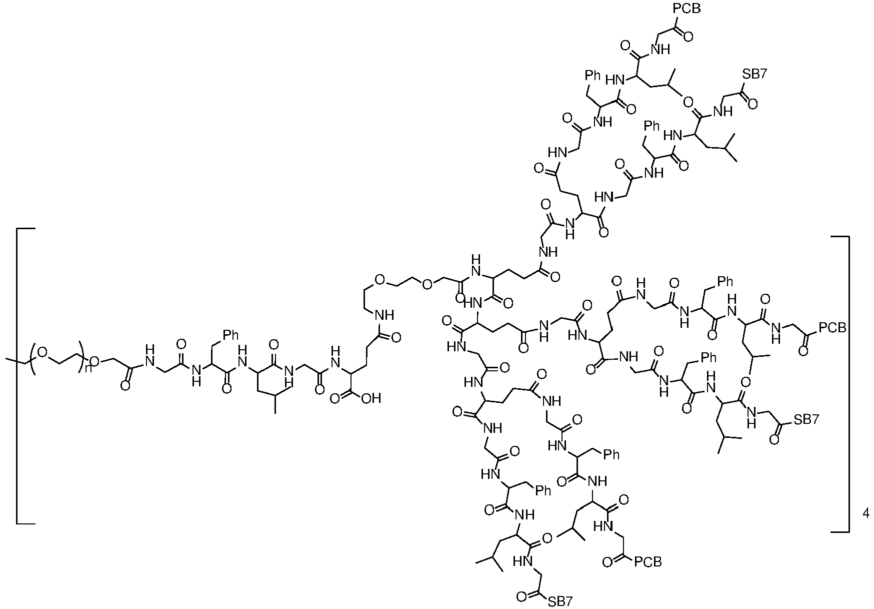

Product 17-129 (1.2341 g, 0.1894 mmol) was added to a 500 mL flask and then dissolved with DMF (50 mL); the mixed solution reacted at -5 °C, DIEA (0.1304 mL,0.7892 mmol) was slowly added, and the obtained solution was stirred for 30min; 4ARM-SCM-40K (1.6556 g, 0.0395 mmol, purchased from JenKem) was then added and dissolved at -5 °C through stirring at a low speed, and the obtained solution was then stirred for a week in the dark at room temperature at a low speed to react. At the end of the reaction, n-hexane (150 mLx 3) was added to precipitate the reaction solution, and methyl tert-butyl ether (200 mL) was added to the lower oily solution to separate out a solid, the solution was then filtered, and the resulting filter cake was dissolved with a mixed solvent of methanol (10 mL)-dichloromethane (40 mL), silica gel powder (15 g) was added to the obtained solution, and the solution was then evaporated to dryness to obtain a powdery solid; the operations of dry sample loading, column chromatography, and gradient elution with a dichloromethane mixed solution containing 1% of ammonia water and 4%-6% of methanol were carried out; the elution product was then collected, concentrated, evaporated to dryness, and dried in a vacuum oven, thus obtaining 1.2812 g of Product 17-131 with a yield of 48%.

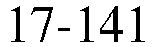

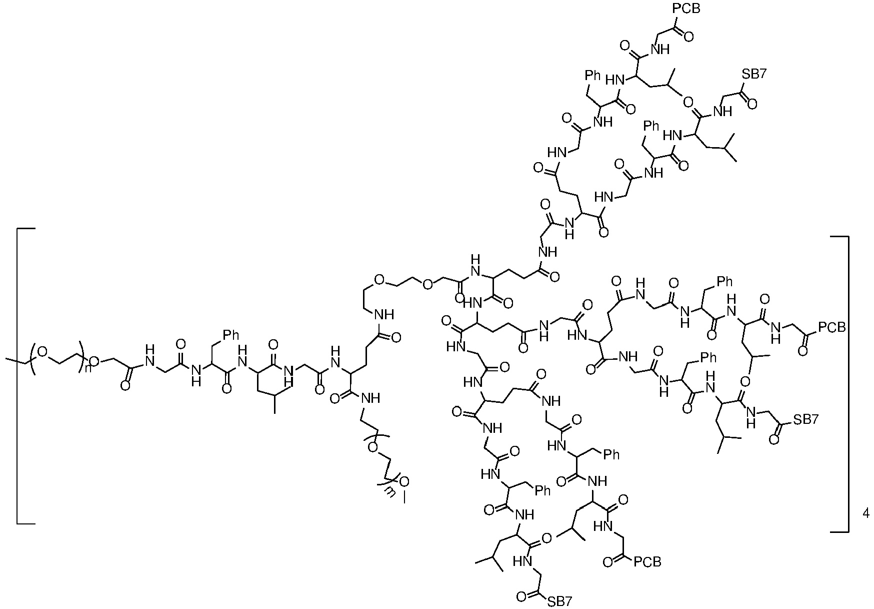

Product 17-131 (1.2812 g, 0.0190 mmol) was added to a 500 mL flask and then dissolved with DMF (15 mL), and M-NH₂-3K.HCl (0.3537 g, 0.1138 mmol), HBTU (0.0863 g, 0.2276 mmol), and HOBT (0.0308 g, 0.2276 mmol) were then added to the obtained solution; the mixed solution was stirred at -5 °C for about 20 min to react, DIEA (0.0689 mL, 0.4171 mmol) was then slowly added dropwise, and the obtained solution further reacted at -5 °C for 0.5h; and the solution was stirred for 6 days in the dark at room temperature at a low speed to react. At the end of the reaction, n-hexane (150 mL) was added to layer the solution, the supernatant was discarded, n-hexane (150 mL × 5) was further added to the lower solution to obtain an oily final product; the oily final product was dissolved with a mixed solvent of methanol (10 mL)-dichloromethane (40 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 4%-6% of methanol were carried out; the elution product was then collected, concentrated, evaporated to dryness to obtain a solid, and dried for 2 h in a vacuum oven; the crude product was dissolved with absolute ethanol (25 mL) and dichloromethane (5 mL) , and n-hexane (200 mL) was added to the obtained solution to separate out a solid; the solution was filtered and the filter cake was washed with n-hexane (50 mL×3) and dried with the vacuum oven, thus obtaining 1.04 g of Product 17-141 with a yield of 69%. MALDI-TOF MS: from 79562.81 to 79931.28.

### Example 12: Synthesis of Compound 19-42

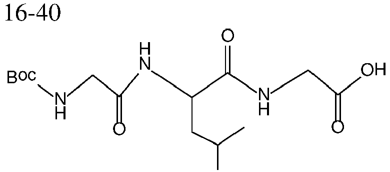

The reactant Boc-GLG-OBn (home-made, 10 g, 22.9616 mmol) and 10% Pd/C (250mg) were added in the hydrogenation reactor and then dissolved with DMF (30 mL), the reactor was sealed and vacuumized, hydrogen (18psi) was then introduced in the reactor, and the solution was stirred overnight to react. At the end of the reaction, the reaction solution was filtered by suction with diatomaceous earth (20 g) as a filter cake, and then the filter cake was washed with DMF (30 mL×3), and the filtrate was collected for the next step.

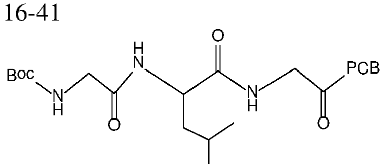

PCB (PaLbocicLib (PD-0332991)) (5.1380 g, 11.4808 mmol), HOBT (2.3271 g, 17.2212 mmol), and HBTU (6.5310 g, 17.2212 mmol) were added to the DMF solution of Product 16-40 (14.9250 mmol), the obtained solution was placed in a -5 °C low-temperature constant temperature bath and stirred to react for 30min, and then DIEA (8.5390 mL, 51.6636 mmol) was added dropwise. Two hours later, the obtained solution reacted overnight at room temperature. At the end of the reaction, deionized water (1 L) was added to the reaction solution, suction filtering was carried out, and the filter cake was washed three times (200 mL×3) with methyl tert-butyl ether. The filter cake was collected and dried in a vacuum oven, thus obtaining 10 g of the product with a yield of 100%.

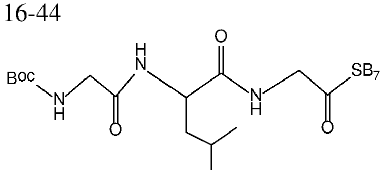

SB7 (SB-743921) (2.4659 g, 4.7689 mmol), HOBT (0.9666 g, 7.1534 mmol), and HBTU (2.7129 g, 7.1534 mmol) were added to the DMF solution of Product 16-40 (6.1996 mmol), the obtained solution was placed in a -5 °C low-temperature constant temperature bath and stirred to react for 30min, and then DIEA (3.5470 mL, 21.4061 mmol) was added dropwise. Two hours later, the obtained solution was stirred overnight at room temperature to react. At the end of the reaction, saturated sodium bicarbonate solution (300 mL) and ethyl acetate (200 mL) were added to the reaction solution, the obtained solution was shaken repeatedly and then stood still to be layered, the organic phase was separated, and the aqueous phase was extracted three times with ethyl acetate (200 mL ×3); the obtained organic phases were combined, and then washed three times with saturated sodium chloride solution (200 mL×3), then dried with anhydrous sodium sulfate (100 g); 10 min later, suction filtering was carried out, and the filter cake was washed with ethyl acetate (20 mL×3), the filtrate was evaporated to dryness, and the obtained solid was dried in a vacuum oven, thus obtaining 5 g of the product with a yield of 100%.

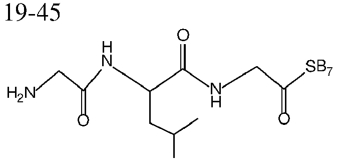

The reactant Product 16-44 (4.7689 mmol) was added to a 250 mL reaction flask, dichloromethane (20 mL) and TFA (7.0840 mL, 95.3780 mmol) were added in sequence to dissolve the reactant, and the obtained solution was stirred and reacted overnight. At the end of the reaction, saturated sodium bicarbonate solution (300 mL) and ethyl acetate (200 mL) were added to the reaction solution, the obtained solution was shaken repeatedly and then stood still to be layered, the organic phase was separated, and the aqueous phase was extracted three times with ethyl acetate (200 mL ×3); the obtained organic phases were combined, and then washed three times with saturated sodium chloride solution (200 mL×3), then dried with anhydrous sodium sulfate (100 g); 10 min later, suction filtering was carried out, and the filter cake was washed with ethyl acetate (20 mL×3), the filtrate was evaporated to dryness, and the obtained solid was dissolved with a mixed solvent (methanol 20%/dichloromethane, 200 mL) and then silica gel powder (20 g) was added to the obtained solution, and the solution was then evaporated to dryness; the operations of dry sample loading and column chromatography were carried out. Gradient elution with 1% ammonia water: 4%-6% methanol/dichloromethane was carried out, and product points were collected, concentrated and evaporated to dryness and dried in a vacuum oven, thus obtaining 3.5496 g of the product with a yield of 80%.

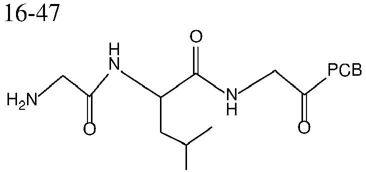

The reactant Product 16-41 (11.4808 mmol) was added to a 500 mL reaction flask, dichloromethane (20 mL) and TFA (17.0548 mL, 229.616 mmol) were added in sequence, and the obtained solution was stirred and reacted overnight. At the end of the reaction, the reaction solution was first evaporated to dryness, n-hexane (150 mL) was added to the solution, the obtained solution stood still for 10min, the supernatant was discarded, ethyl acetate (15 mL) was added to carry out ultrasonic treatment to obtain a homogeneous phase, then the homogeneous phase was precipitated with n-hexane, and such operations were repeated until a solid came out; suction filtering was carried out, the solid was dissolved in a mixed solvent (methanol 20%/dichloromethane, 200 mL), silica gel powder (20 g) was then added, the solution was evaporated to dryness, and the operations of dry sample loading and column chromatography were carried out. Gradient elution with 1% ammonia water: 3%-5% methanol/dichloromethane was carried out, and product points were collected, concentrated and evaporated to dryness and dried in a vacuum oven, thus obtaining 6.4 g of the product with a yield of 83%.

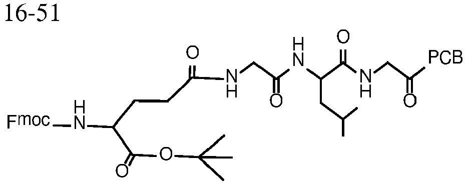

Product 16-47 (2.5 g, 1.6338 mmol), Fmoc-GLu-OtBu (0.9732 g, 2.2873 mmol), and PyAOP (1.1926 g, 2.2873 mmol) were added to a 250 mL reaction flask and dissolved with DMF (20 mL), and the obtained solution was placed in a 0 °C low-temperature constant temperature bath and stirred to react for 20min; TMP (0.2152 mL, 1.6338 mmol) was slowly added dropwise, and then the mixed solution reacted overnight. At the end of the reaction, methyl tert-butyl ether (300 mL) was added to precipitate the reaction solution, suction filtering was carried out, the filter cake was dissolved with a mixed solvent (methanol 20%/dichloromethane, 200 mL) and then silica gel powder (15 g) was added; the obtained solution was then evaporated to dryness, and the operations of dry sample loading and column chromatography were carried out. Gradient elution with an eluent (5%-10% methanol/dichloromethane) was then carried out, the product points were collected, concentrated, evaporated to dryness, and dried in a vacuum oven, thus obtaining 2.1 g of the product with a yield of 67.64%.

¹H-NMR (400 MHz, DMSO-*d*6)δ 10.17 (s, 1H), 8.96 (s, 1H), 8.14- 7.99 (m, 4H), 7.89 (s, 3H), 7.73 (s, 3H), 7.51 (s, 1H), 7.42 (s, 2H), 7.33 (s, 2H), 5.84 (s, 1H), 4.49- 4.19 (m, 4H), 4.05 -3.83 (m, 3H), 3.79 -3.54 (m, 6H), 3.16 (s, 5H), 2.42 (s, 3H), 2.31 (s, 3H), 2.27- 2.20 (m, 3H), 1.93 (s, 4H), 1.77 (s, 2H), 1.66 -1.47 (m, 5H), 1.39 (s, 9H), 0.86 (s, 6H);

MALDI-TOF MS: [M+H⁺] 1082.60

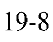

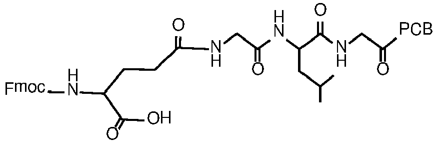

Product 16-51 (5.0386 mmol) was dissolved with dichloromethane (20 mL) and TFA (7.4849 mL, 100.772 mmol), and the obtained solution was stirred to react overnight. At the end of the reaction, the reaction solution was evaporated to dryness and dissolved with ethyl acetate (10 mL), and methyl tert-butyl ether was added for precipitation (300 mL). After that, suction filtering was carried out, the filter cake was dissolved with a mixed solvent (methanol 20%/dichloromethane, 200 mL) and then silica gel powder (20 g) was added; the obtained solution was then evaporated to dryness, and the operations of dry sample loading and column chromatography were carried out. Gradient elution with an eluent (8%-10% methanol/dichloromethane) was then carried out, and the product points were collected, concentrated, evaporated to dryness, and dried in a vacuum oven, thus obtaining 5 g of the product with a yield of 96.71%.

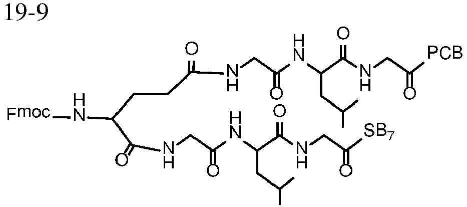

The reactant Product 19-8 (5 g, 4.8276 mmol) was weighed and then dissolved with DMF (30 mL); the obtained solution was placed in a 0 °C low-temperature constant temperature bath and stirred; Product 16-45 (3.45 g, 4.6401 mmol) and PyAOP (3.3870 g, 6.4961 mmol) were added in sequence, the obtained solution was stirred to react for 30 min and then TMP (0.6118 mL, 4.6401 mmol) was slowly added dropwise. At the end of the reaction, n-hexane (150 mL) was added to the reaction solution, the obtained solution stood still for 10min, the supernatant was discarded, ethyl acetate (15 mL) was added to carry out ultrasonic treatment to obtain a homogeneous phase, then the homogeneous phase was precipitated with n-hexane, and such operations were repeated until a solid came out; suction filtering was carried out, and the filter cake was collected and dried in a vacuum oven, thus obtaining 10.8 g of the product with a yield of 100%.

¹H-NMR (400 MHz, DMSO-d₆) δ 10.16 (s, 1H), 8.96 (s, 1H), 8.24 (s, 1H), 8.17 ― 7.98 (m, 7H), 7.89 (m, 4H), 7.79 ― 7.64 (m, 3H), 7.61 ― 7.10 (m, 16H), 5.94 - 5.68 (m, 2H), 4.46 ― 4.12 (m, 6H), 4.00-2.82 (m, 3H), 3.77 ― 3.56 (m, 9H), 3.24 ― 3.13 (m, 4H), 3.01-2.51 (m, 7H), 2.42 (s, 3H), 2.36 ― 2.21 (m, 10H), 2.18-1.88 (m, 3H), 1.83 ― 1.72 (m, 7H), 1.67 ― 1.43 (m, 8H), 1.3-0.95 (m, 1H),0.90-0.61 (m, 13H), 0.52 (s, 2H);

MALDI-TOF MS: [M+H⁺] 1751.90

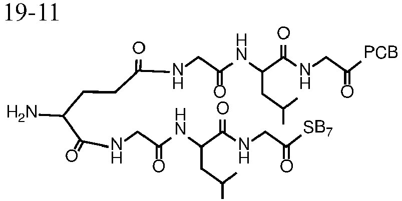

Product 19-9 (4.4601 mmol) was dissolved with DMF (20 mL), morpholine (12.13 mL, 139.203 mmol) was then added to the obtained solution, and the solution was stirred to react; 1 h later, morpholine (9 mL) was added again, and the reaction continued for 3h. At the end of the reaction, n-hexane (150 mL) was added to the reaction solution, the obtained solution stood still for 10min, the supernatant was discarded, ethyl acetate (15 mL) was added to carry out ultrasonic treatment to obtain a homogeneous phase, then the homogeneous phase was precipitated with n-hexane, and such operations were repeated until a solid came out; suction filtering was carried out, the solid was dissolved in a mixed solvent (methanol 20%/dichloromethane, 200 mL), silica gel powder (30 g) was then added, the solution was evaporated to dryness, and the operations of dry sample loading and column chromatography were carried out. Gradient elution with 1% ammonia water: 4%-8% methanol/dichloromethane was carried out, and product points were collected, concentrated and evaporated to dryness and dried in a vacuum oven, thus obtaining 4.2 g of the product with a yield of 59.23%.

¹H-NMR (400 MHz, DMSO-d₆) δ 10.14 (s, 1H), 8.96 (s, 1H), 8.09 ― 8.03 (m, 9H), 7.95-7.63 (m, 1H),7.60- 7.52 (m, 1H), 7.50 (s, 2H), 7.25 (s, 6H), 7.14 (s, 2H), 5.91 ― 5.69 (m, 3H), 5.55-4.32 (m, 1H), 4.29 (s, 1H),4.20 -3.86 (m, 3H), 3.79 ― 3.67 (m, 6H), 3.59 (s, 10H), 2.89 (s, 1H), 2.81-2.31 (m, 11H), 2.28-1.99 (m, 7H), 1.94 ― 1.71 (m, 9H), 1.62-0.91 (m, 4H), 0.87-0.62(m, 18H), 0.52 (s, 3H).

MALDI-TOF MS: [M+H⁺] 1529.37, [M+Na⁺] 1551.33

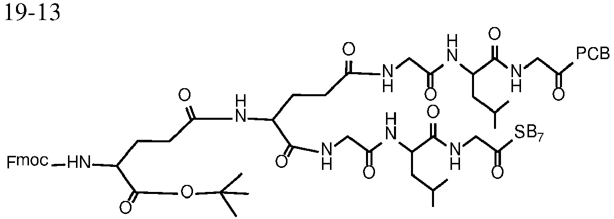

The reactant Product 19-11 (4.2 g, 2.7447 mmol) was weighed and then dissolved with DMF (30 mL), the obtained solution was placed in a low-temperature constant temperature bath and stirred; and then reactants Fmoc-GLu-OtBu (1.6350 g, 3.8426 mmol) and PyAOP (2.0035 g, 3.8426 mmol) were added in sequence and the obtained solution was stirred to react for 30min; TMP (0.3615 mL, 2.7447 mmol) was then added dropwise, and the obtained solution reacted overnight. At the end of the reaction, n-hexane (150 mL) was added to the reaction solution, the obtained solution stood still for 10min, the supernatant was discarded, ethyl acetate (15 mL) was added to carry out ultrasonic treatment to obtain a homogeneous phase, then the homogeneous phase was precipitated with n-hexane, and such operations were repeated until a solid came out; suction filtering was carried out, and the filter cake was collected and dried in a vacuum oven, thus obtaining 5.6 g of the product with a yield of 100%.

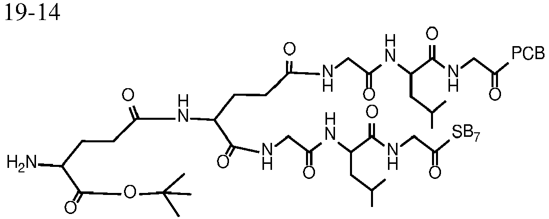

Product 19-13 (2.7447 mmol) was dissolved with DMF (20 mL), morpholine (7.1735 mL, 82.341) was then added to the obtained solution, and the obtained solution was stirred to react for 3h. At the end of the reaction, n-hexane (150 mL) was added to the reaction solution, the obtained solution stood still for 10min, the supernatant was discarded, ethyl acetate (15 mL) was added to carry out ultrasonic treatment to obtain a homogeneous phase, then the homogeneous phase was precipitated with n-hexane, and such operations were repeated until a solid came out; suction filtering was carried out, the solid was dissolved in a mixed solvent (20% methanol/dichloromethane, 200 mL), silica gel powder (20 g) was then added, the solution was evaporated to dryness, and the operations of dry sample loading and column chromatography were carried out. Gradient elution with 1% ammonia water: 7% methanol/dichloromethane was carried out, and product points were collected, concentrated and evaporated to dryness and dried in a vacuum oven, thus obtaining 4.3 g of the product with a yield of 91.49%.

MALDI-TOF MS: [M+H⁺] 1714.93, [M+Na⁺] 1736.57

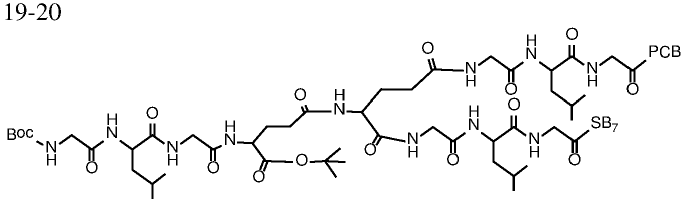

The reactants, Product 19-14 (1.6 g, 0.9327 mmol), Boc-GLG-OH (home-made, 1.2125 mmol), and HOBT (0.2 g, 1.3991 mmol), HBTU (0.53 g, 1.3991 mmol) were dissolved with DMF (50 mL), the obtained solution was placed in a -5 °C low-temperature constant temperature bath and stirred for 30min; DIEA (0.69 mL, 4.1922 mmol) was slowly added dropwise, and the obtained solution first reacted for 2 h and then further reacted overnight at room temperature. At the end of the reaction, n-hexane (150 mL) was added to the reaction solution, the obtained solution stood still for 10min, the supernatant was discarded, ethyl acetate (15 mL) was added to carry out ultrasonic treatment to obtain a homogeneous phase, then the homogeneous phase was precipitated with n-hexane, and such operations were repeated until a solid came out; suction filtering was carried out, the solid was dissolved in a mixed solvent (methanol 20%/dichloromethane, 200 mL), silica gel powder (15 g) was then added, the solution was evaporated to dryness, and the operations of dry sample loading and column chromatography were carried out. Gradient elution with 1% ammonia water: 4% methanol/dichloromethane was carried out, and product points were collected, concentrated and evaporated to dryness and dried in a vacuum oven, thus obtaining 1.5 g of the product with a yield of 52.53%.

MALDI-TOF MS: [M+H⁺] 2042.13, [M+Na⁺] 2064.40

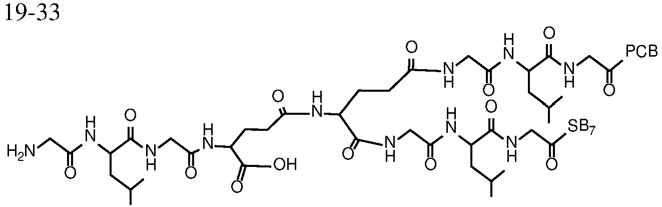

The reactant Product 19-20 (1 g, 0.4896 mmol) was weighed and dissolved with dichloromethane (20 mL) and TFA (1.454 mL, 19.5808 mmol), and the obtained solution was stirred to react overnight. At the end of the reaction, n-hexane (150 mL) was added to the reaction solution, the obtained solution stood still for 10min, the supernatant was discarded, ethyl acetate (15 mL) was added to carry out ultrasonic treatment to obtain a homogeneous phase, then the homogeneous phase was precipitated with n-hexane, and such operations were repeated until a solid came out; suction filtering was carried out, and the filter cake was collected and dried in a vacuum oven, thus obtaining 1.2 g of the product with a yield of 100%.

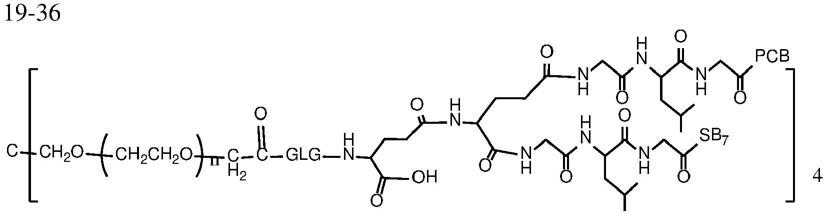

Product 19-33 (0.4896 mmol) was weighed and then dissolved with DMF (50 mL), DIEA (0.869 mL, 5.268 mmol) was added; 4ARM-SCM-40K (4.4492 g, 0.102 mmol, purchased from JenKem) was dissolved with dichloromethane (20 mL) and then added to Product 19-33; the mixed solution was stirred to react. At the end of the reaction, methyl tert-butyl ether (300 mL) was added to precipitate the reaction solution, suction filtering was carried out, the filter cake was dissolved with a mixed solvent (methanol 20%/dichloromethane, 200 mL) and then silica gel powder (20 g) was added; and the operations of dry sample loading and column chromatography were carried out. Gradient elution with an eluent (10%-15% methanol/dichloromethane) was then carried out, the product points were collected, concentrated, evaporated to dryness, and dried in a vacuum oven, thus obtaining 1.4 g of the product with a yield of 27%.

¹H-NMR (400 MHz, DMSO-d₆) δ 10.12 (s, 3H), 8.96 (s, 9H), 8.77-8.39 (m, 16H), 8.27-8.12 (m, 6H), 8.09-7.91 (m, 15H), 7.85-7.70 (m, 7H), 7.66 ― 7.57 (m, 2H), 7.57 ― 7.46 (m, 2H), 7.41 ― 7.29 (m, 23H), 7.24 (s, 16H), 7.16-7.10 (m, 6H), 6.54-6.50 (m, 17H), 5.85 ― 5.79 (m, 1H), 5.11 (s, 7H), 4.53-3.90 (m, 8H), 3.70 ― 3.65 (m, 41H), 3.50-2.99 (m, 4001H), 2.90-2.88 (m, 10H), 2.74-2.72 (m, 9H), 2.70-2.66 (m, 20H), 2.60-2.58 (m, 12H), 2.44-2.38 (m, 13H), 2.35 ― 2.29 (m, 27H), 1.84-1.82 (m, 6H), 1.46-1.142 (m, 10H), 1.41-1.27 (m, 50H), 1.24-1.20 (m, 24H), 1.20-1.16 (m, 46H), 1.13-0 92(m, 15H), 0.87- 0.83 (m, 96H).

MALDI-TOF MS: from 50260.57 to 51539.80

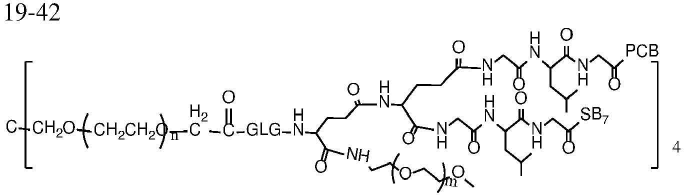

Product 19-36 (1.4 g, 0.0276 mmol), M-NH₂-2K·HCl (0.4418 g, 0.2209 mmol), HOBT (0.0224 g, 0.1657 mmol) and HBTU (0.0628 g, 0.1657 mmol) were dissolved with DMF (50 mL), the obtained solution was placed in a -5 °C low-temperature constant temperature bath and stirred to react for 30min, and then DIEA (0.1787 mL, 0.7179 mmol) was added dropwise; the mixed solution was stirred to react and 2 h later, the reaction solution further reacted at room temperature. At the end of the reaction, methyl tert-butyl ether (300 mL) was added to precipitate the reaction solution, suction filtering was carried out, the filter cake was dissolved with a mixed solvent (methanol 20%/dichloromethane, 200 mL) and then silica gel powder (8 g) was added; the obtained solution was then evaporated to dryness, and the operation of dry sample loading was carried out. Gradient elution with 1% ammonia water: 2%-5% methanol/dichloromethane was carried out, and product points were collected, concentrated and evaporated to dryness and dried in a vacuum oven, thus obtaining 0.1 g of the product with a yield of 6.1%.

¹H-NMR (400 MHz, DMSO-d₆) δ 8.98-8.94 (m, 12H), 8.27 ― 8.18 (m, 8H), 8.08-8.06 (m, 48H), 7.92 ― 7.83 (m, 7H), 7.65-7.30 (m, 9H), 7.22-6.26 (m, 78H), 3.45-3.38 (m, 6355H), 2.36-2.30 (m, 161H), 2.11-1.72 (m, 59H), 1.58-1.56 (m, 35H), 1.50-1.46 (m, 48H), 1.34-0.96 (m, 18H), 0.88-0.63 (m, 96H), 0.59- 0.32 (m, 9H).

Example 13: Synthesis of Compound 19-72

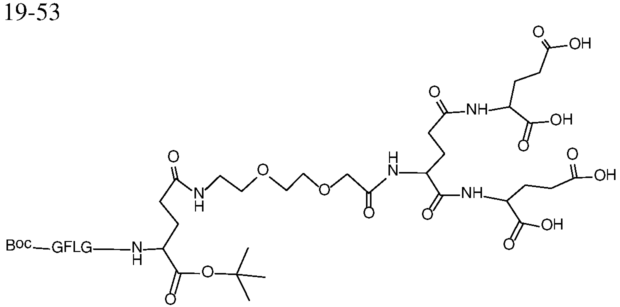

Product 9-103 (0.4 g, 0.2547 mmol) and 10% Pd/C (300 mg) were added in the hydrogenation reactor and then dissolved with DMF (30 mL), the reactor was sealed and vacuumized, hydrogen (18 psi) was then introduced in the reactor, and the solution was stirred overnight. At the end of the reaction, the reaction solution was filtered by suction with diatomaceous earth (20 g) as a filter cake, and then the filter cake was washed with DMF (30 mL×3), and the filtrate was collected for the next step.

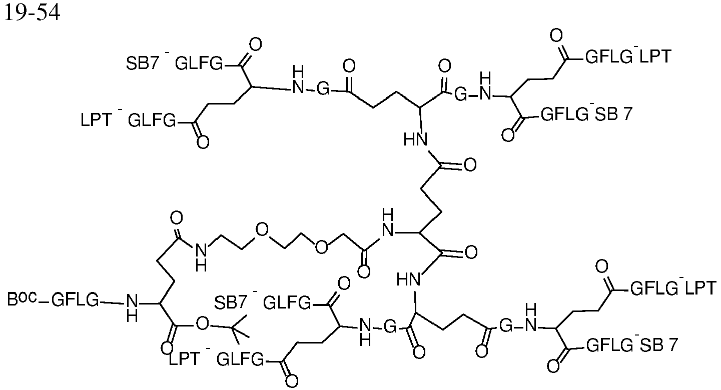

Product 3-161 (2.5484 g, 1.2226 mmol, for its structure and synthesis process, see the synthesis of Compound L-10 in Example M-10 of PCT International Patent Application PCT/CN2018/073662, it was prepared by replacing Boc-GLG-OBn with Boc-GFLG-OBn and replacing PCB with LPT), HOBT (0.2065 g, 1.5282 mmol), and HBTU (0.5796 g, 1.5282 mmol) were added to Product 19-53 (0.2547 mmol), the obtained solution was then placed in a -5 °C low-temperature constant temperature bath and stirred to react; 0.5 h later, DIEA (0.7577 mL, 4.5846 mmol) was added dropwise and the mixed solution reacted overnight. At the end of the reaction, saturated sodium bicarbonate solution (300 mL) and ethyl acetate (200 mL) were added to the reaction solution, the obtained solution was shaken repeatedly and then stood still to be layered, the organic phase was separated, and the aqueous phase was extracted three times with ethyl acetate (200 mL ×3); the obtained organic phases were combined, and then washed three times with saturated sodium chloride solution (200 mL×3), then dried with anhydrous sodium sulfate (100 g); 10 min later, suction filtering was carried out, and the filter cake was washed with ethyl acetate (20 mL×3), and then silica gel powder (10 g) was added; the obtained solution was then evaporated to dryness, the operations of dry sample loading and elution with 1% ammonia water: 6% methanol/dichloromethane were carried out, and product points were collected, concentrated and evaporated to dryness and dried in a vacuum oven, thus obtaining 1.6 g of the product with a yield of 69%.

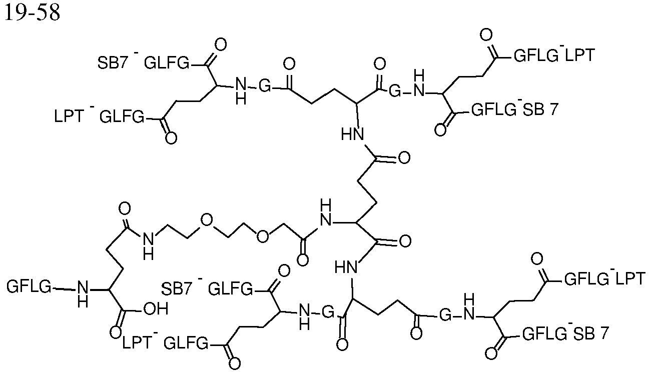

Product 19-54 (1.6 g) was dissolved with TFA (0.26 mL, 3.5055 mmol) and dichloromethane (30 mL) and the obtained solution was stirred overnight. At the end of the reaction, the reaction solution was first evaporated to dryness, n-hexane (150 mL) was added to the solution, the obtained solution stood still for 10min, the supernatant was discarded, ethyl acetate (15 mL) was added to carry out ultrasonic treatment to obtain a homogeneous phase, then the homogeneous phase was precipitated with n-hexane, and such operations were repeated until a solid came out; suction filtering was carried out, the filter cake was dissolved in a mixed solvent (methanol 20%/dichloromethane, 200 mL), silica gel powder (10 g) was then added, the solution was evaporated to dryness, and the operations of dry sample loading and column chromatography were carried out. Gradient elution with an eluent (1% ammonia water: 7%-8% methanol/dichloromethane) was then carried out, and the product points were collected, concentrated, evaporated to dryness, and dried in a vacuum oven, thus obtaining 1.2 g of the product with a yield of 75.8%.

¹H-NMR (400 MHz, DMSO-d₆) δ 9.87-9.53 (m,5H), 9.03-8.76 (m, 5H), 8.56 (s, 5H), 8.36-8.23 (m,10H), 8.16 ― 7.99 (m, 37H), 7.87-7.83 (m, 8H), 7.75 ― 7.67 (m, 9H), 7.58-7.43 (m, 18H), 7.36 ― 7.13 (m,84H), 6.90 (s, 2 H ) , 3.76-6.68 (m, 5H), 6.55-6.03 (m, 4H), 5.26 (s,11H), 4.86-4.72 (m,12 H), 4.63-4.45 (m, 17H), 4.37-4.29 (m, 10H), 4.25-3.95 (m, 12H), 3.92 -3.86 (m, 20H), 3.78 ― 3.64 (m, 20H), 3.65 ― 3.47 (m, 29H), 3.27 -3.12(m, 8H), 3.05 (s, 7H), 3.03 (s, 10H), 2.93 (s, 15H), 2.89 (s,7H), 2.83 ― 2.69 (m, 36H),2.31 (s,11H),2.13- 1.58 (m, 6H), 1.48-1.38 (m, 19H), 1.36 ― 1.32 (m, 8H), 1.24 ― 1.20 (m, 13H), 0.95 ― 0.88 (m, 16H), 0.88 ― 0.83 (m, 24H), 0.82 ― 0.78 (m, 19H), 0.54 ― 0.46 (m, 11H).

MALDI-TOF MS: [M+Na⁺] 8985.60

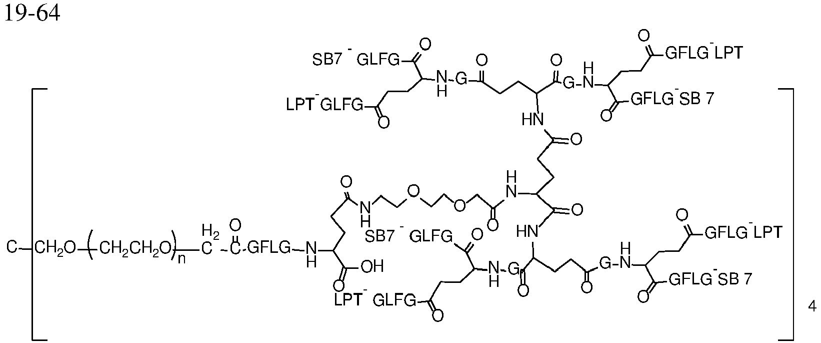

Product 19-58 (1.2 g, 0.1331 mmol) was dissolved with DMF (20 mL), 4AMR-SCM-40K (1.1631 g, 0.02772 mmol, purchased from JenKem) was dissolved with dichloromethane (20 mL), and the two solutions were separately added to a 250 mL flask; then, DIEA (2 mL) was added dropwise, and the obtained solution was stirred to react. At the end of the reaction, methyl tert-butyl ether (300 mL) was added to precipitate the reaction solution, suction filtering was carried out, the filter cake was dissolved with a mixed solvent (methanol 20%/dichloromethane, 200 mL) and then silica gel powder (10 g) was added; the obtained solution was then evaporated to dryness, and the operations of dry sample loading and column chromatography were carried out. Elution with an eluent (8% methanol/dichloromethane) was then carried out, and the product points were collected, concentrated, evaporated to dryness, and dried in a vacuum oven, thus obtaining 1.1 g of the product with a yield of 50.2%.

¹H-NMR (400 MHz, DMSO-d₆) δ 9.97 ― 9.75 (m, 92H), 8.54-8.52 (m, 165H), 8.31 ― 8.15 (m, 60H), 7.94-7.90 (m, 68H), 7.92 ― 7.58 (m, 141H), 7.57 ― 7.42 (m, 46H), 7.23 (m, 77H), 6.52-6.48 (m, 72H), 5.29-5.24 (m, 51H), 3.69 ― 3.66 (m, 659H), 3.46-3.38 (m, 3764H), 2.95-2.86 (m, 16H), 2.72-2.70 (m, 52H), 2.35-2.32 (m, 115H), 2.00-1.96 (m, 24H), 1.48-1.46 (m, 25H), 1.36-1.32 (m, 30H), 1.24-1.20 (m, 207H), 1.18-1.16 (m, 28H), 0.86-0.84 (m, 216H).

MALDI-TOF MS: from 77323.56 to 77347.98

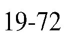

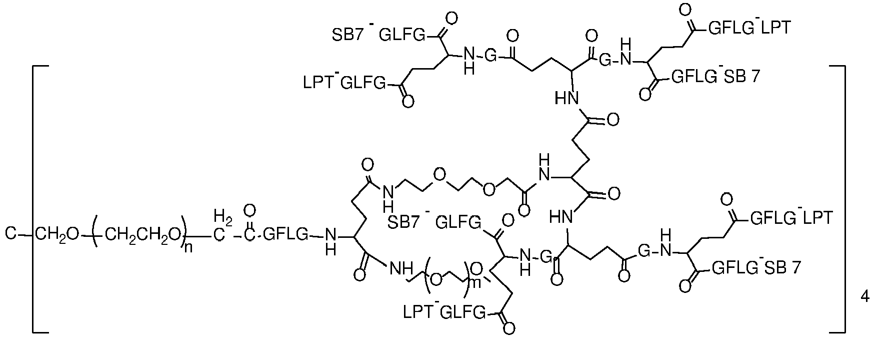

Product 19-64 (1.1 g, 0.0142 mmol) was dissolved with DMF (30 mL) and then placed in a -5 °C low-temperature constant temperature bath; M-NH2-2K·HCl (0.2311 g, 0.1134 mmol), HOBT (0.0115 g, 0.0851 mmol), and HBTU (0.0323 g, 0.0851 mmol) were then added in sequence; 0.5 h later, DIEA (0.06 mL, 0.3687 mmol) was added dropwise, and 2 h later, the solution reacted at room temperature. At the end of the reaction, methyl tert-butyl ether was added to precipitate the reaction solution, suction filtering was carried out, the filter cake was dissolved with a mixed solvent (methanol 20%/dichloromethane, 200 mL) and then silica gel powder (5 g) was added; the obtained solution was then evaporated to dryness, and the operations of dry sample loading, column chromatography, elution with an eluent (6% methanol/dichloromethane) were carried out, thus obtaining 0.6 g of the product with a yield of 50%.

¹H-NMR (400 MHz, DMSO-d₆) δ8.10 ― 8.04 (m, 95H), 7.97 ― 7.91 (m, 84H), 7.89 ― 7.82 (m, 110H), 7.49-7.42(m, 116H), 7.24-7.18 (m, 118H), 7.19-7.13 (m, 84H), 7.08-7.03 (m, 51H), 6.99-6.93 (m, 67H), 5.99-5.86 (m, 131H), 5.58-5.53 (m, 67H), 3.24-3.1 (m, 4490H), 2.89-2.82 (m, 269H), 2.76-2.71 (m, 104H), 2.69-2.65 (m, 141H), 2.38-2.33 (m, 140H), 1.27-1.25 (m, 124H), 1.17-1.14 (m, 136H), 1.06 (m, 96H), 0.88-0.82 (m, 216H). MALDI-TOF MS: from 85257.07 to 85387.78.

### Example 14: Synthesis of Compound 19-80

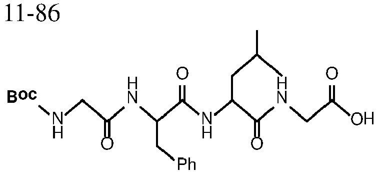

The reactant Boc-GFLG-OBn (home-made, 20 g) and 10% Pd/C (250mg) were added in the hydrogenation reactor and then dissolved with DMF (30 mL), the reactor was sealed and vacuumized, hydrogen (18psi) was then introduced in the reactor, and the solution was stirred overnight to react. At the end of the reaction, the reaction solution was filtered by suction with diatomaceous earth (20 g) as a filter cake, and then the filter cake was washed three times with DMF (30 mL × 3) and the filtrate was collected for the next step.

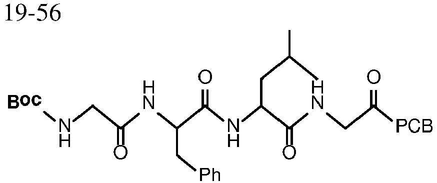

PCB (PaLbocicLib (PD-0332991)) (4.7264 g, 10.5611 mmol), product 11-86 (13.7294 mmol), HOBT (2.1407 g, 15.8417 mmol), and HBTU (6.0080 g, 15.8417 mmol) were added in a 500 mL flask and dissolved with DMF (30 mL), and the obtained solution was placed in a -5 °C low-temperature constant temperature bath; 0.5 h later, DIEA (0.7508 mL, 4.5428 mmol) was dropwise added, and 2 h later, the mixed solution was placed at room temperature to react overnight. At the end of the reaction, deionized water (500 mL) was added to the reaction solution, the obtained solution was filtered by suction, the filter cake was washed three times with methyl tert-butyl ether (300 mL×3), and then dissolved with a mixed solvent (20% methanol/dichloromethane, 200 mL), silica gel powder (50 g) was added, and the operation of dry sample loading was carried out. Gradient elution with an eluent (3%-5% methanol/dichloromethane) was then carried out, and the product points were collected, concentrated, evaporated to dryness, and dried in a vacuum oven, thus obtaining 10.3 g of the product with a yield of 100%.

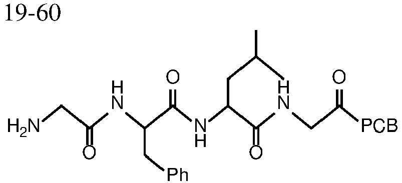

TFA (19.6107 mL, 264.0275 mmol) and dichloromethane (20 mL) were added to Product 19-56 (10.5611 mmol) and the obtained solution was stirred to react. At the end of the reaction, n-hexane was added to precipitate the reaction solution and the solution stood still for 10min, the supernatant was discarded, a small amount of ethyl acetate was then added for ultrasonic treatment, and n-hexane was added again; such operations were repeated until the solid came out. After that, suction filtering was carried out, the filter cake was dissolved with a mixed solvent (methanol 20%/dichloromethane, 200 mL) and then silica gel powder (50 g) was added; the obtained solution was then evaporated to dryness, and the operations of dry sample loading and column chromatography were carried out. Elution with an eluent (3% methanol/dichloromethane) was then carried out, and the product points were collected, concentrated, evaporated to dryness, and dried in a vacuum oven, thus obtaining 9 g of the product with a yield of 80%.

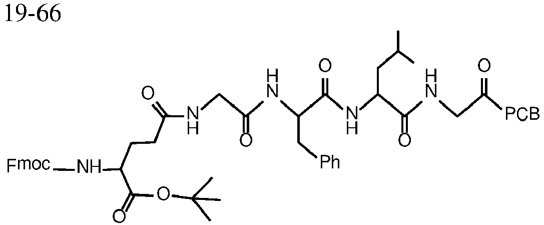

Fmoc-Glu-OtBu (6.23 g, 14.65 mmol), GFLG-PCB (8.6 g, 10.46 mmol, i.e., Product 19-60), and PyAOP (8.2 g, 15.7 mmol) were added in a 500 mL flask and then dissolved with DMF (40 mL); the obtained solution was placed in a -5 °C low-temperature constant temperature bath; 0.5 h later, TMP (1.38 mL, 10.46 mmol) was added dropwise, and the mixed solution was stirred to react. At the end of the reaction, methyl tert-butyl ether (150 mL) and n-hexane (100 mL) were added to precipitate the reaction solution, the precipitation process was repeated three times to obtain a powder; suction filtering was carried out, the filter cake was dissolved with a mixed solvent (methanol 20%/dichloromethane, 200 mL) and then silica gel powder (25 g) was added; the obtained solution was then evaporated to dryness, and the operations of dry sample loading and column chromatography were carried out. Elution with an eluent (2% methanol/dichloromethane) was then carried out, and the product points were collected, concentrated, evaporated to dryness, and dried in a vacuum oven, thus obtaining 5.9 g of the product.

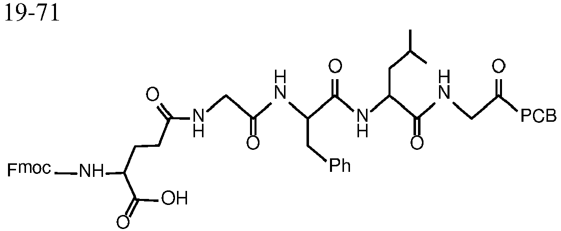

Product 19-66 (7.3 g, 5.93 mmol) was dissolved with dichloromethane (30 mL), TFA (6.61 mL, 89 mmol) was then added, and the obtained solution was stirred to react. At the end of the reaction, methyl tert-butyl ether (150 mL) and n-hexane (100 mL) were added to precipitate the reaction solution, the precipitation process was repeated three times to obtain a powder; suction filtering was carried out, the filter cake was dissolved with a mixed solvent (methanol 20%/dichloromethane, 200 mL) and then silica gel powder (25 g) was added; the obtained solution was then evaporated to dryness, and the operations of dry sample loading and column chromatography were carried out. Gradient elution with an eluent (2%-5% methanol/dichloromethane) was then carried out, and the product points were collected, concentrated, evaporated to dryness, and dried in a vacuum oven, thus obtaining 6.1 g of the product.

MALDI-TOF MS: [M+H⁺] 1173.65, [M+Na⁺] 1195.59

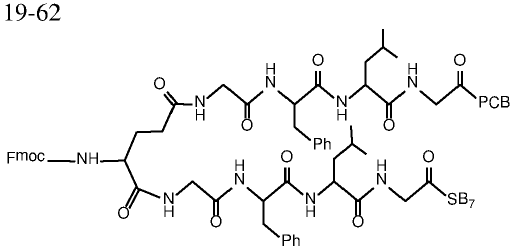

Product 19-71 (1.3 g, 1.1105 mmol) was added in a 500 mL flask and then dissolved with DMF (20 mL); GFLG-SB7 (0.9 g, 1.0095 mmol), HOBT (0.2046 g, 1.5143 mmol), and HBTU (0.5743, 1.5143 mmol) were added to the obtained solution in sequence and the solution was then placed in a -5 °C low-temperature constant temperature bath; 0.5 h later, DIEA (0.7508 mL, 4.5428 mmol) was added dropwise; 2 h later, the obtained solution reacted at room temperature; next day, HOBT (0.1023 g), HBTU (0.2871 g), and DIEA (0.38 mL) were added again to the reaction solution. At the end of the reaction, methyl tert-butyl ether (300 mL) was added to precipitate the reaction solution; suction filtering was carried out, and the filter cake was collected and dried in a vacuum oven, thus obtaining 2.6 g of the product with a yield of 100%.

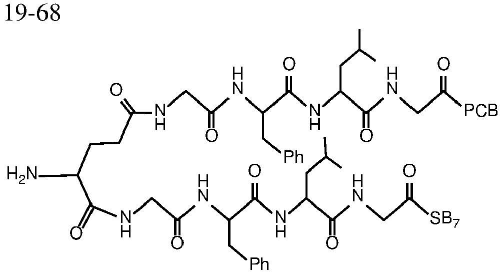

Product 19-62 (1.2295 mmol) was dissolved with DMF (20 mL), morpholine (2.638 mL, 20.285 mmol) was then added, and the obtained solution was stirred to react. At the end of the reaction, methyl tert-butyl ether (300 mL×3) was added to the reaction solution. After the precipitation, suction filtering was carried out, the filter cake was dissolved with a mixed solvent (methanol 20%/dichloromethane, 200 mL) and then silica gel powder (10 g) was added; the obtained solution was then evaporated to dryness, and the operations of dry sample loading, column chromatography, and elution with an eluent (1% ammonia water: 3% methanol/dichloromethane) was then carried out, the product points were collected, concentrated, evaporated to dryness, and dried in a vacuum oven, thus obtaining 2.1 g of the product with a yield of 100%.

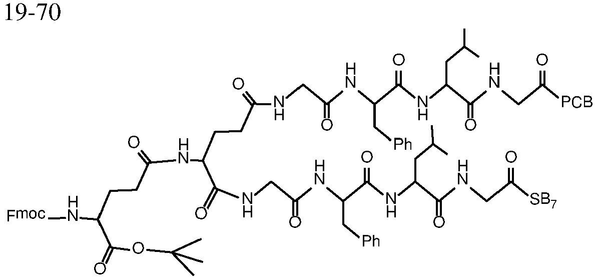

DMF (20 mL) was added to Product 19-68 (1.0095 mmol) and the obtained solution was then placed in a -5 °C low-temperature constant temperature bath; Fmoc-Glu-OtBu (0.6013 g, 1.4133 mmol), HOBT (0.2046 g, 1.5143 mmol), and HBTU (0.5743 g, 15143 mmol) were added in sequence; 0.5 h later, DIEA (0.7508 mL, 4.5428 mmol) was added dropwise; and 2 h later, the obtained solution reacted at room temperature. At the end of the reaction, methyl tert-butyl ether was added to the reaction solution to separate out a solid, suction filtering was carried out, the filter cake was dissolved with a mixed solvent (methanol 20%/dichloromethane, 200 mL) and then silica gel powder (10 g) was added; the obtained solution was evaporated to dryness, and the operations of dry sample loading and column chromatography were carried out. Elution with an eluent (1% ammonia water: 3% methanol/dichloromethane) was then carried out, and the product points were collected, concentrated, evaporated to dryness, and dried in a vacuum oven, thus obtaining 1.9 g of the product with a yield of 83.3%.

¹H-NMR (400 MHz, DMSO-d₆) δ 0.12 (s, 2H), 8.95 (s, 2H), 8.10 ― 8.03 (m, 7H), 7.91-7.80 (m, 5H), 7.69-7.55 (m, 4H), 7.41 (s, 4H), 7.35 ― 7.28 (m, 4H), 7.22 (s, 11H), 4.06 ― 3.97 (m, 6H), 3.88 (s, 3H), 3.66-3.39 (m,11H), 3.16 (m, 9H), 3.06 ― 2.99 (m,4H), 2.42 (s, 4H), 2.31 (s,7H), 2.28-2.19 (m, 5H), 2.15 ― 2.09 (m, 3H), 1.99 (s, 5H), 1.63 ― 1.55 (m,5H), 1.50 -1.41(m, 7H), 1.35 (s, 9H), 1.19 (s, 1H), 1.17 (s, 2H), 1.15 (s, 1H), 1.07-0.91 (m, 4H), 0.90 ― 0.77 (m, 17H).

MALDI-TOF MS: [M+H⁺] 2259.78, [M+Na⁺] 2281.78.

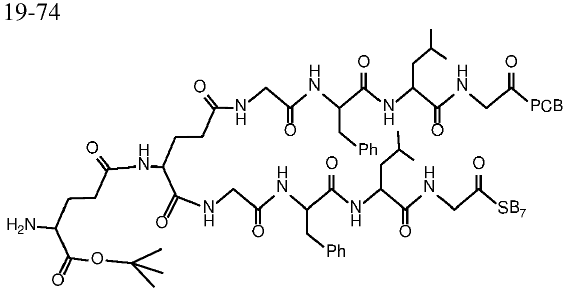

Product 19-70 (1.9 g, 0.8407 mmol) was dissolved with DMF (20 mL), morpholine (2.1970 mL, 25.2205 mmol) was then added, and the obtained solution was stirred to react. At the end of the reaction, methyl tert-butyl ether was added to the reaction solution to separate out a solid; suction filtering was carried out, and the filter cake was collected, concentrated, evaporated to dryness, and dried in a vacuum oven, thus obtaining the product with a yield of 88.2%.

¹H-NMR (400 MHz, DMSO-d₆) δ 10.13 (s, 2H), 8.96 (s,2H), 8.22 ― 7.76 (m, 18H), 7.52 -728.(m, 4H), 7.19 -559(m, 14H), 5.03-4.53 (m, 3H), 4.35 (s, 2H), 4.26 ― 4.19 (m, 2H), 4.16 ― 4.08 (m,2H), 4.00 (s, 2H), 3.82-.29 (m, 10H), 3.17 -2.91(m ,7H), 2.89 (s,4H), 2.71-2.59 (m, 7H), 2.42 (s, 3H), 2.31 (s,5H), 2.13 (s, 2H), 1.90 ― 1.74 (m, 8H), 1.62 ― 1.46 (m, 10H), 1.38 (s, 9H), 0.93 ― 0.80 (m, 15H), 0.51 (s, 3H).

MALDI-TOF MS: [M+H⁺] 2037.81, [M+Na⁺] 2059.87.

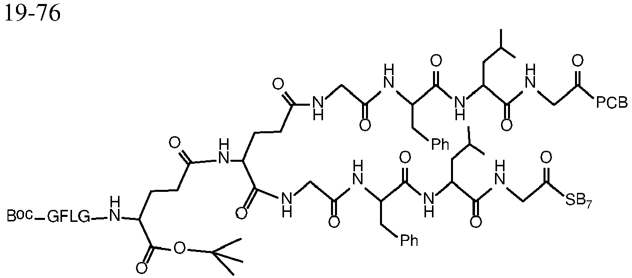

DMF (20 mL) was added to Product 19-74 (1.5 g, 0.7361 mmol) and the obtained solution was then placed in a -5 °C low-temperature constant temperature bath; Boc-GFLG-OH (home-made, 1.0305 mmol), HOBT (0.1492 g, 1.1042 mmol) and HBTU (0.5658 g, 1.1042 mmol) were added in sequence; 0.5 h later, DIEA (0.55 mL, 3.3145 mmol) was added dropwise; and 2 h later, the obtained solution reacted at room temperature. At the end of the reaction, methyl tert-butyl ether was added to the reaction solution to separate out a solid; suction filtering was carried out, and the filter cake was collected, concentrated, evaporated to dryness, and dried in a vacuum oven, thus obtaining 1.5 g of the product with a yield of 83%.

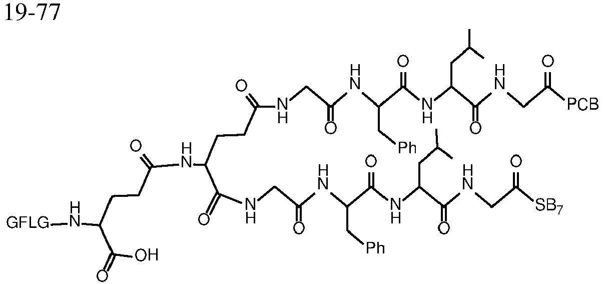

Product 19-76 (1.5 g, 0.5937 mmol) was dissolved with dichloromethane (20 mL), TFA (1.7639 mL, 23.7491 mmol) was then added, and the obtained solution was stirred to react. At the end of the reaction, n-hexane was added to precipitate the reaction solution and the solution stood still for 10min, the supernatant was discarded, a small amount of ethyl acetate was then added for ultrasonic treatment, and n-hexane was added for precipitation; such precipitation process was repeated until the solid came out. At the precipitation, suction filtering was carried out, and the filter cake was collected, concentrated, evaporated to dryness, and dried in a vacuum oven, thus obtaining 1.7 g of the product with a yield of 100%.

¹H-NMR (400 MHz, DMSO-d₆) δ 8.98 (s, 2H), 8.32-8.16 (m,2H), 8.13 -7.92 (m, 10H), 7.89 (s,6H), 7.54-7.36 (m, 2H), 7.29 ― 7.12 (m, 28H), 3.9 9-2.23 (m, 49H), 2.43 (s, 4H), 2.32 (s, 6H), 1.63 ― 1.54 (m, 6H),1.49-1.30 (m, 8H), 1.25 -1.03(m, 3H), 0.93 ― 0.77 (m, 27H).

MALDI-TOF MS: [M+H⁺] 2312.26, [M+Na⁺] 2334.75.

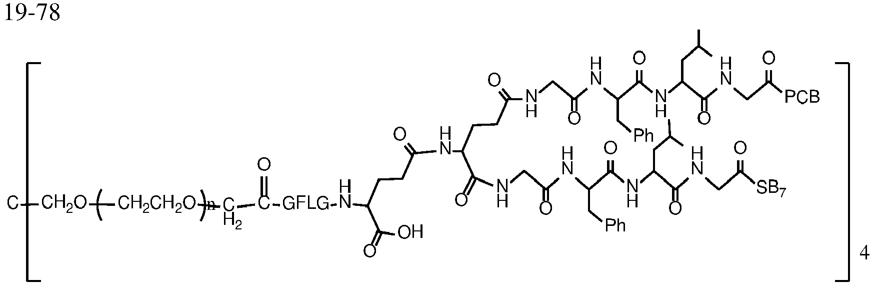

Product 19-77 (1 g, 0.421 mmol) was dissolved with DMF (20 mL), and DIEA (0.3627 mL, 2.1944 mmol) was then added dropwise; 4AMR-SCM-40K (3.5405 g, 0.0844 mmol) was dissolved with dichloromethane (20 mL); the two solutions were separately added to a 250 mL flask and the obtained mixed solution was stirred to react. At the end of the reaction, methyl tert-butyl ether was added to the reaction solution to separate out a solid, suction filtering was carried out, the filter cake was dissolved with a mixed solvent (methanol 20%/dichloromethane, 200 mL) and then silica gel powder (10 g) was added; the operation of column chromatography was carried out. Gradient elution with an eluent (8%-20% methanol/dichloromethane) was then carried out, and the product points were collected, concentrated, evaporated to dryness, and dried in a vacuum oven, thus obtaining 2.6 g of the product with a yield of 60%.

¹H-NMR (400 MHz, DMSO-d₆) δ 10.53 (s, 7H), 10.15 (s, 10H), 8.96 (s, 11H), 8.16 (s, 18H), 8.12-8.01 (m, 29H), 7.97 -7.93(m, 19H), 7.89-7.82 (m, 11H), 7.80 ― 7.72 (m, 14H), 7.53-7.30 (m, 26H), 7.25 ― 7.18 (m, 49H) , 6.98-6.96(m, 9H), 6.67-4.83 (m, 4H), 4.57-4.54 (m, 13H), 4.32-4.16 (m, 12H), 4.10-3.99 (m, 16H), 3.889-3.86(m, 14H), 3.78 ― 3.74 (m, 28H), 3.71 - 3.39 (m, 2839H), 3.16 ― 3.09 (m, 16H), 3.06-3.01 (m, 4H), 3.00 (s, 3H), 2.93-2.86 (m, 25H), 2.76-2.71 (m, 27H), 2.67-2.64 (s, 5H), 2.59-2.54 (m, 9H), 2.44-2.41 (m, 8H), 2.39-2.26 (m, 17H), 2.24-2.21 (s, 6H), 2.18-2.16 (s, 5H), 2.00 -1.69(m, 12H), 1.63-1.51 (m, 17H), 1.47-1.43 (m, 17H), 1.35 (s, 2H), 1.34-1.32 (m, 9H), 1.30-1.28 (m, 3H), 1.27- 1.26 (s, 16H), 1.25 ― 1.20 (m, 41H), 0.96-0.82 (m, 72H).

MALDI-TOF MS: from 50565.51 to 51768.44

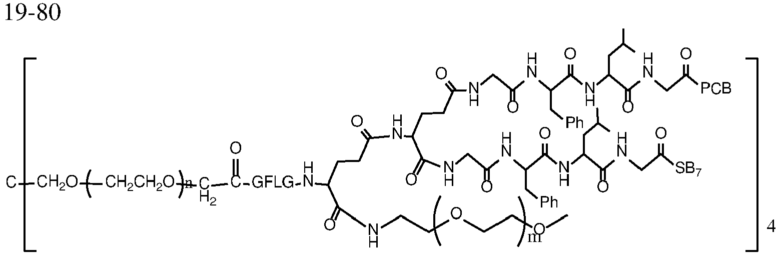

Product 19-78 (2.6 g, 0.051 mmol) was dissolved with DMF (30 mL) and then placed in a -5 °C low-temperature constant temperature bath; PEG-2K·HCl (0.8314 g, 0.4080 mmol, purchased from JenKem), HOBT (0.0413 g, 0.3060 mmol), and HBTU (0.1161 g, 0.3060 mmol) were then added in sequence; 0.5 h later, DIEA (0.219 mL, 1.326 mmol) was added dropwise, and 2 h later, the solution reacted at room temperature. At the end of the reaction, methyl tert-butyl ether was added to precipitate the reaction solution, suction filtering was carried out, the filter cake was dissolved with a mixed solvent (methanol 20%/dichloromethane, 200 mL) and then silica gel powder (10 g) was added; the operation of column chromatography was carried out. Elution with an eluent (8% methanol/dichloromethane) was then carried out, and the product points were collected, concentrated, evaporated to dryness, and dried in a vacuum oven, thus obtaining 1.9 g of the product with a yield of 63.3%.

¹H-NMR (400 MHz, DMSO-*d₆*) δ 8.99-8.96(m, 22H), 8.11-8.09 (m, 22H), 7.89-7.85 (m, 25H), 7.50-7.48 (m, 43H), 7.35-7.30 (m, 27H), 7.21-7.13 (m, 66H), 3.50-3.42 (m, 4513H), 2.91-2.88 (m, 105H), 2.75 - 2.64 (m, 43H), 2.35-2.34 (m, 32H), 2.19-2.15 (m, 35H), 1.66-1.25 (m, 118H), 0.90-0.83 (m, 72H). MALDI-TOF MS: from 55413.56 to 59721.74.

### Example 15: Synthesis of Compound 20-101

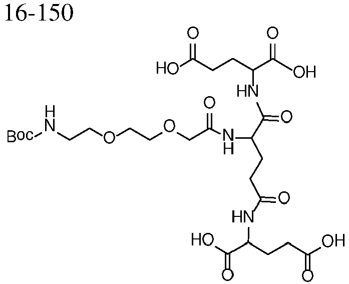

The reactant Boc-LC-E[E(OBn)₂]₂ (home-made 1.0251 g, 1.0138 mmol) and 10% Pd/C (150mg) were added in the micro-reactor and then dissolved with DMF; the reactor was vacuumized, H₂ was introduced in the reactor, and such operations were repeated three times. The obtained solution was stirred to react. At the end of the reaction, the reaction solution was filtered by suction with diatomaceous earth as a filter cake to remove Pd/C; the diatomaceous earth was washed 3-4 times with DMF to obtain the DMF solution of the product for the next reaction.

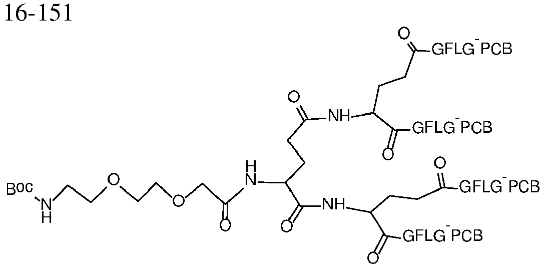

GFLG-PCB (4 g, 4.8664 mmol), HBTU (2.3068 g, 6.0828 mmol), and HOBT (0.8219 g, 6.0828 mmol) were weighed and added in a 500 mL reaction flask and then dissolved with the DMF solution of product 16-150, and the ultrasonic treatment was carried out to completely dissolve the reactants; the obtained solution was stirred for 30 min at -5 °C, DIEA (3 mL, 18.2484 mmol) was slowly added dropwise, and the obtained solution was first stirred at a low temperature for 2 h, and then reacted at room temperature to the end. Next day, a part of product has been precipitated from the reaction solution; deionized water (1 L) was added to the reaction solution and the solution was filtered by suction. Since the product could not be dissolved in the mixed solvent of dichloromethane and methanol, ultrasonic treatment with ethyl acetate (30 mL) and precipitation with n-hexane settled (100 mL) were carried out many times to remove some impurities, thus obtaining 4.5 g of the product, 0.6 g being extra-quota product, for direct use in the next reaction.

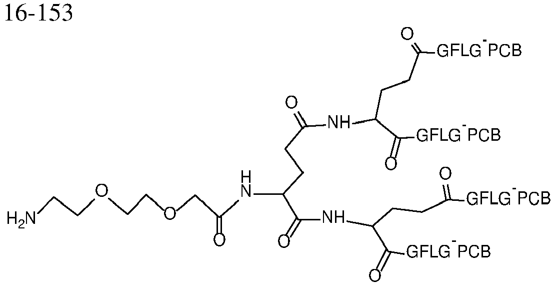

Product 16-151 (3.9 g, 1.0138 mmol) was weighed and added in a 500 mL reaction flask. Dichloromethane and TFA (2.6 mL, 30.141 mmol) were added, and the product was dissolved by ultrasonic. A ground glass stopper was used, and the obtained solution was stirred to react. At the end of the reaction, the reaction solution was first evaporated to dryness, ethyl acetate (20 mL) was added for ultrasonic treatment, n-hexane was added for precipitation (200 mL), the supernatant was discarded, and the dissolution and precipitation process was repeated three times. The suction filtering was carried out to obtain a powder product. Only ethyl acetate (100 mL) was added to carry out ultrasonic treatment to obtain a homogeneous phase, the homogeneous phase was filtered by suction, and there were impurities in the filtrate. 5.8 g of the product was obtained, 2 g being extra-quota product.

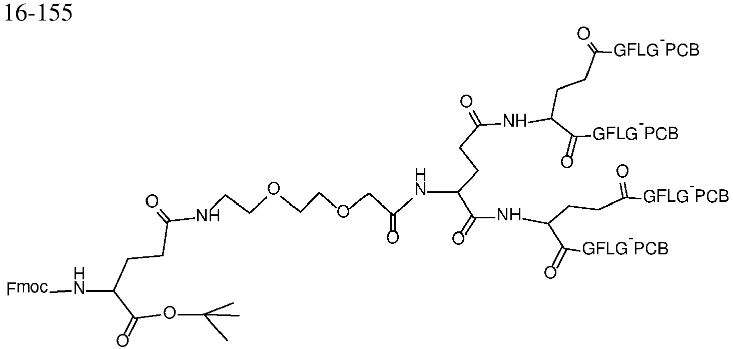

Fmoc-GLu-(OtBu) (0.6039 g, 1.4193 mmol), Product 16-153 (3.8 g, 1.0138 mmol), HBTU (0.5767 g, 1.5207 mmol), and HOBT (0.2053 g, 1.5207 mmol) were added in a 500 mL reaction flask and then dissolved with DMF, the obtained solution was treated by ultrasonic to finally obtain a homogeneous phase, and the homogeneous phase was placed at 0 °C; 30 min later, DIEA (1.2 mL, 6.8432 mmol) was slowly added dropwise, and the obtained solution reacted at a low temperature to the end. TLC was carried out next day, which indicated that the reaction was not completed; Fmoc-GLu-(OtBu), HOBT, and HBTU were added again each in an amount 20% of the feed, and then DMSO was added until the reactants were completely dissolved. On the third day, the reaction ended, deionized water (1 L) was added to the reaction solution, the product was separated out by precipitation, and suction filtering was carried out. Ethyl acetate (200 mL) was added to the product, and ultrasonic treatment was carried out to obtain a homogeneous phase; n-hexane (500 mL) was then added, and suction filtering was carried out, thus obtaining 4.3 g of the final product, 0.07 g being extra-quota product.

¹H-NMR (400 MHz, DMSO-*d₆*) δ 10.13 (s, 3H), 8.95 (s,3H), 8.29 - 7.78 (m, 27H), 7.71 (s, 4H), 7.45 (m, 6H), 7.18 (m, 20H), 4.57 (s, 2H), 4.21 (s, 7H), 3.95 (m, 8H), 3.61 (s, 24H), 3.10 (m, 29H), 2.89 (s, 5H), 2.73 (s, 6H), 2.54 (s, 47H), 2.41 (s, 13H), 2.30 (s, 13H), 1.82 (m, 21H), 1.58 (s, 18H), 1.38 (m, 7H), 0.85 (m, 24H).

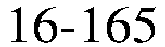

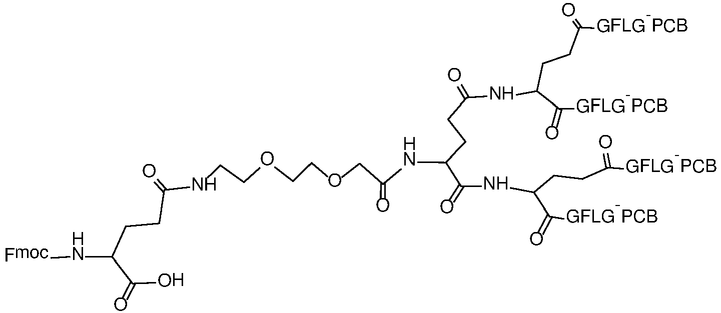

Product 16-155 (4.3 g, 1.0302 mmol) was added in a 500 mL reaction flask and dissolved with dichloromethane (10 mL), TFA (2.2956 mL, 30.9072 mmol) was then added, a ground glass stopper was used, and the obtained solution was stirred to react. At the end of the reaction, the reaction solution was evaporated to dryness to remove dichloromethane, and the product was further dissolved with ethyl acetate (20 mL), n-hexane was added to separate out a solid product, and suction filtering was carried out; ethyl acetate (80 mL) was added for ultrasonic treatment to obtain a homogeneous phase; suction filtering was carried out, thus obtaining 4 g of the product with a yield of 95%.

¹H-NMR (400 MHz, DMSO-*d₆*) δ10.73 (s,4H), 8.97 (s, 4H), 8.48 - 7.57 (m, 38H), 7.40 (s, 3H), 7.33 - 6.98 (m, 21H), 4.57 (s, 19H), 4.14 (m56.4 Hz, 5H), 4.05 (m22.2 Hz, 14H), 3.61 (s, 24H), 3.39 (s,1H), 3.19 (m15.2 Hz, 17H), 3.05 - 2.69 (m, 10H), 2.27 (m43.1 Hz, 34H), 1.99 (s, 4H), 1.91 (s, 7H), 1.78 (s, 11H), 1.54 (m34.4 Hz, 19H), 1.16 (m6.6 Hz, 3H), 0.84 (m18.2 Hz, 24H).

MALDI-TOF MS: [M+H⁺]4116.24, [M+Na⁺]4138.52

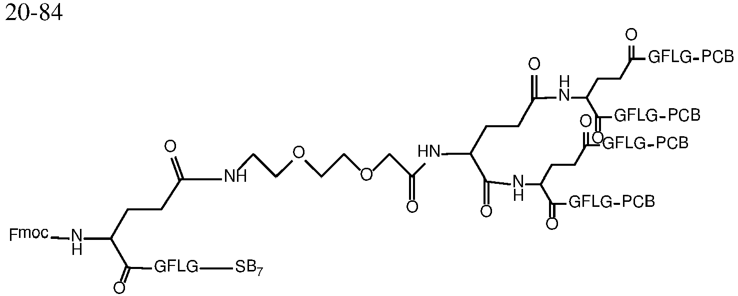

Product 16-165 (4 g, 0.9714 mmol), GFLG-SB7 (0.9525 g, 1.0685 mmol), HOBT (0.1968 g, 1.4571 mmol), and HBTU (0.5525 g, 4.3714 mmol) were weighed and then dissolved with DMSO solution (100 mL), and the obtained solution was placed in a low-temperature constant temperature bath (-5 °C) and stirred for 30 min; DIEA (0.7225 mL, 47.992 mmol) was added dropwise; the solution reacted for 2 h at a low temperature and then was stirred at room temperature to react. At the end of the reaction, the reaction solution was poured into deionized water (400 mL) to separate out the product. Suction filtering was carried out. The filter cake was washed once with methyl tert-butyl ether (70 mL). Then ethyl acetate (30 mL) was added.Ultrasonic treatment was carried out to obtain a homogeneous phase, and then n-hexane (150 mL) was added. A powder was obtained after precipitation. Suction filtering was performed again, and the solid was dried in a vacuum oven, thus obtaining 4.9 g of the product.

¹H-NMR (400 MHz, DMSO-*d₆*) δ10.13 (s, 4H), 8.90 (s, 4H), 8.43 - 8.25 (m, 0H), 8.08 (s, 23H), 7.83 (s, 12H), 7.62 (s, 3H), 7.47 (s, 7H), 7.37 - 7.30 (m, 2H), 7.24 (s, 3H), 7.17 (s, 25H), 7.10 (s, 8H), 5.84 - 5.74 (m, 4H), 4.53 (s, 4H), 4.32 (s, 5H), 4.17 (s, 5H), 3.97 (s, 9H), 3.85 (s, 2H), 3.57 (s, 31H), 3.47 (s, 3H), 2.72 (s, 3H), 2.69 (s, 2H), 2.67 (s, 1H), 2.65 (s,1H), 2.37 (s, 15H), 2.33 (s, 2H), 2.26 (s, 17H), 2.19 (s,11H), 2.09 (s, 9H), 1.78 (s, 30H), 1.50 (s, 28H), 1.24 - 1.18 (m, 1H), 1.06 (s, 9H), 0.87 (s, 4H), 0.80 (s, 30H), 0.46 (s,2H)

MALDI-TOF MS: [M+H⁺]4988.58,[M+Na⁺]5010.27

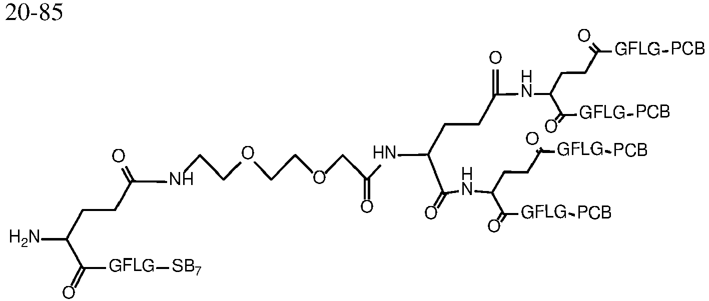

Product 20-84 (4.9 g) was added in a 500 mL flask and then dissolved with the DMSO solution (80 mL), morpholine (2.35 mL, 29.142 mmol) was added in the flask, the mixed solution was stirred at room temperature to react, and TLC was carried out every 0.5 h. Two hours later, the reaction ended. The reaction solution was precipitated with methyl tert-butyl ether; the lower solution was oily liquid, and the upper solution was turbid liquid. The oily liquid and the turbid liquid obtained by precipitation were poured into 400 mL of deionized water, and a yellow solid was separated out by precipitation. Suction filtering was carried out, and the filter cake was washed once with methyl tert-butyl ether (70 mL). Then ethyl acetate (30 mL) was added to the filter cake, and ultrasonic treatment was carried out to obtain a homogeneous phase, and then n-hexane (150 mL) was added. A powder was obtained after precipitation. Suction filtering was performed again, and the solid was dried in a vacuum oven, thus obtaining 3.6 g of the product with a yield of 78%.

¹H-NMR (400 MHz, DMSO-*d₆*) δ10.25 (s, 3H), 8.95 (s, 3H), 8.56 (s, 0H), 8.12 (s, 25H), 7.85 (s, 8H), 7.55 (s, 6H), 7.18 (s, 34H), 5.81 (s, 4H), 4.57 (s, 5H), 3.61 (s, 38H), 3.24 - 3.10 (m, 27H), 3.02 (s, 8H), 2.89 (s, 4H), 2.70 (s, 17H), 2.42 (s, 16H), 2.31 (s, 35H), 1.83 (s, 27H), 1.54 (s, 24H), 1.23 (s, 2H), 0.85 (m, 30H), 0.51 (s, 2H).MALDI-TOF MS: [M+H⁺]4766.74, [M+Na⁺]4788.82

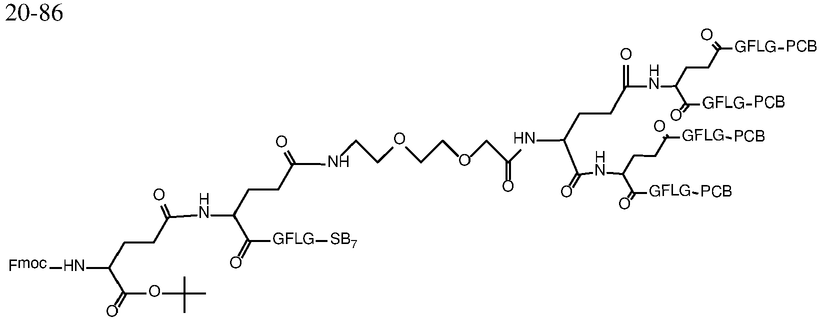

Fmoc-Glu-OtBu (0.38 g, 0.9032 mmol), Product 20-85 (3.6 g, 0.7526 mmol), HOBT (0.1525 g, 1.1289 mmol), and HBTU (0.4281 g, 1.1289 mmol) were weighed and dissolved with the DMSO solution (100 mL); DIEA (0.55 mL, 3.3867 mmol) was then added dropwise, and the obtained solution was stirred at room temperature to react. At the end of the reaction, the reaction solution was poured into deionized water (400 mL) to separate out the product. Suction filtering was carried out, and the filter cake was washed once with methyl tert-butyl ether (70 mL). Then ethyl acetate (30 mL) was added to the filter cake, and ultrasonic treatment was carried out to obtain a homogeneous phase, and then n-hexane (150 mL) was added. A powder was obtained after precipitation. Suction filtering was carried out again, the filtrate was applied to the TLC plate and a large amount of impurities and a very small amount of product were obtained; the solid was dried in a vacuum oven and put into the next step.

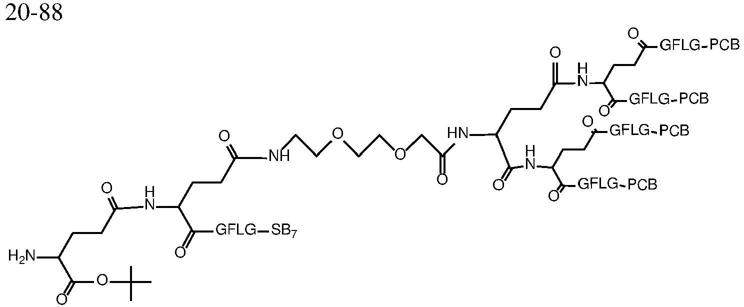

Product 20-86 (0.7526 mmol) was weighed and added in a 500 mL flask and then dissolved with the DMSO solution (100 mL), morpholine (1.96 mL, 22.578 mmol) was added in the flask, the mixed solution was stirred at room temperature to react, and TLC was carried out every 0.5 h. 1.5 hours later, the reaction ended. The reaction solution was poured into deionized water (400 mL), and a yellow solid was separated out by precipitation. Suction filtering was carried out, and the filter cake was washed once with methyl tert-butyl ether (70 mL). Then ethyl acetate (30 mL) was added to the filter cake, and ultrasonic treatment was carried out to obtain a homogeneous phase, and then n-hexane (150 mL) was added. A powder was obtained after precipitation. Suction filtering was performed again, and the solid was dried in a vacuum oven, thus obtaining 3.8 g of the product, 0.1 g being extra-quota product.

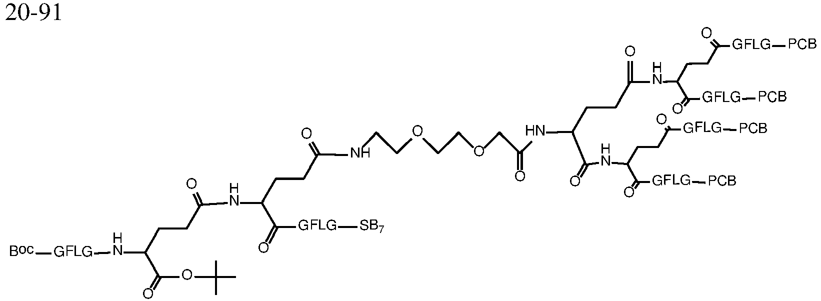

Boc-GFLG-OH (0.9783 mmol), Product 20-88 (0.7526 mmol), HOBT (0.1525 g, 1.1289 mmol), and HBTU (0.4281 g, 1.1289 mmol) were dissolved with the DMSO solution (100 mL), DIEA (0.55 mL, 3.3867 mmol) was then added dropwise, and the obtained solution was stirred at room temperature to react. At the end of the reaction, the reaction solution was poured into deionized water (400 mL) to separate out the product. Suction filtering was carried out, and the filter cake was washed once with methyl tert-butyl ether (70 mL). Then ethyl acetate (30 mL) was added to the filter cake, and ultrasonic treatment was carried out to obtain a homogeneous phase, and then n-hexane (150 mL) was added. A powder was obtained after precipitation; suction filtering was performed again, and the solid was dried in a vacuum oven, thus obtaining 4 g of the product with a yield of 100%.

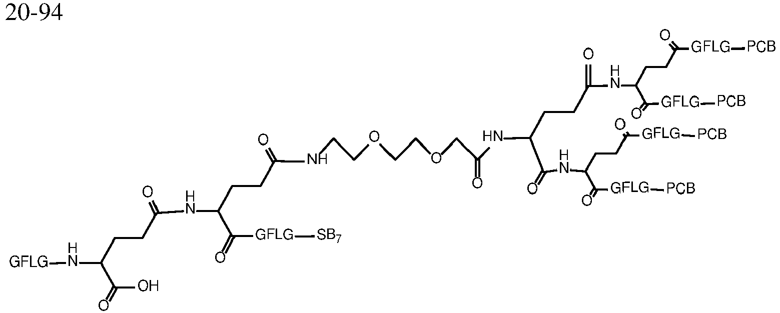

Product 20-91 (4 g, 0.7526 mmol) was weighed and added in a 250 mL flask and then dissolved with dichloromethane (10 mL) and TFA (3 mL, 44.578 mmol) by ultrasonic, and the obtained solution was stirred at room temperature to react. At the end of the reaction, the reaction solution was concentrated by evaporating to dryness. When a little concentrated product was obtained, methyl tert-butyl ether (100 mL) was added to the concentrated solution and ultrasonic treatment was then carried out; n-hexane (200 mL) was added to precipitate the solution to obtain a powder; suction filtering was carried out, the filter cake was dissolved with methanol and dichloromethane, and silica gel powder (15 g) was then added. The operations of dry sample loading and column chromatography were carried out. With the column height of 9cm, elution with 1% ammonia water: 6% methanol/dichloromethane was carried out, thus obtaining 1.5 g of the pure product with a yield of 38%

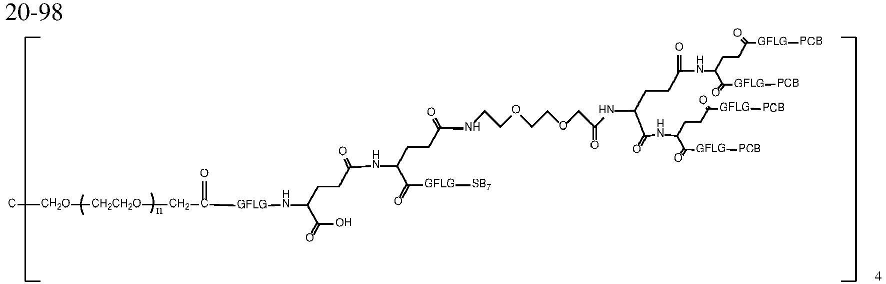

Product 20-94 (1.5 g) was weighed and added in a 250 mL flask and then dissolved with DMF (90 mL), and then DIEA (0.2 mL) was added dropwise. 4ARM-SCM-40K (1.65 g, 0.0395 mmol) was added and dissolved with dichloromethane (5 mL); the obtained solution was placed at room temperature and stirred at a low speed to react in the dark. At the end of the reaction, methyl tert-butyl ether (150 mL) was added in a conical flask, the reaction solution was poured into the conical flask, and then n-hexane (200 mL) was added to separate out the product. Suction filtering and Column chromatography were carried out. With the column height of 5cm, elution with an eluent (1% ammonia water: 6% methanol/dichloromethane) was carried out, thus obtaining 1.3 g of the product with a yield of 36%. ¹H-NMR (400 MHz, DMSO-d₆) δ10.39-10.20 (m, 6H), 8.94 -8.90(m, 2H), 8.19-8.10 (m, 35H), 8.06-8.01 (m, 45H), 7.92 - 7.79 (m, 19H), 7.65 - 7.47 (m, 7H), 7.27-7.25 (m, 178H), 7.14-7.12 (m, 27H), 7.08-7.04 (m, 36H), 6.95-6.90 (m, 6H), 5.94 - 5.78 (m, -6H), 4.56-4.52 (m, 28H), 4.36 -4.32(m, 53H), 4.16 -4.12(m, 47H), 3.98 -3.90(m, 125H), 3.31 -3.28(m, 127H), 3.22 - 3.08 (m, 42H), 3.00-2.96 (m, 2H), 2.76-2.72 (m, 9H), 2.63-2.60 (m, 24H), 2.42-2.40 (m, 44H), 2.28 -2.24(m, 53H), 2.11 -2.06(m, 16H), 2.02 - 1.73 (m, 39H), 1.58 -1.54(m, 28H), 1.49-1.44 (m, 28H), 1.33-1.32(m, 9H), 1.30-1.28 (m, 8H), 1.27-1.26 (m, 23H), 1.25-1.24 (m, 57H), 1.23-1.20(m, 110H), 0.88-0.84 (m, 120H). MALDI-TOF MS: from 62548.84 to 62780.18

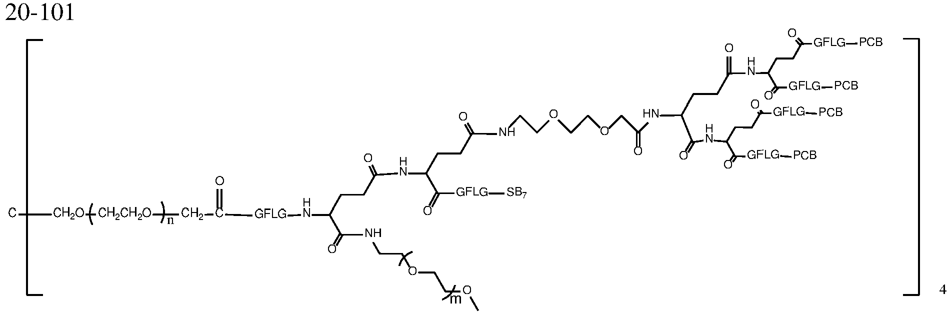

PEG-3K (0.38 g, 0.1246 mmol), Product 20-98 (1.3 g, 0.0207 mmol), HOBT (0.016 g, 0.1246 mmol), and HBTU (0.047 g, 0.1246 mmol) were weighed and added in a 500 mL flask and then dissolved with the DMF solution (100 mL); the obtained solution was placed in a low-temperature constant temperature bath, and 30 min later, DIEA (2 mL) was added dropwise, and one hour later, the solution was taken out and stirred in the dark at room temperature at a low speed to react. At the end of the reaction, TLC was carried out and no obvious point was observed. Methyl tert-butyl ether (150 mL) was added in a conical flask, the reaction solution was poured into the conical flask, and then n-hexane (200 mL) was added to generate precipitate, and then the solution was filtered by suction. The operations of dry sample loading and column chromatography were carried out on the solid. With the column height of 5cm, elution with 1% ammonia water: 4%-10% methanol/dichloromethane was carried out, the elution product was evaporated to dryness and then dissolved with absolute ethanol (10 mL); the obtained solution was treated by ultrasonic to obtain homogeneous phase; n-hexane (100 mL) was added and then suction filtering was carried out. The process of dissolution and precipitation was repeated three times, thus obtaining 1 g of the product with a yield of 66.66%.

¹H-NMR (400 MHz, DMSO-*d₆*) δ10.14-10.10 (m, 10H), 8.98-8.91 (m, 12H), 8.20-8.16 (m, 35H), 8.07-8.00 (m, 59H), 7.98-7.91 (m, 10H), 7.88-7.82 (m, 25H), 7.69-7.60 (m, 20H), 7.55 - 7.34 (m, 17H), 7.19-7.11 (m, 170H), 5.82 -5.77(m, 6H), 3.67 -3.60(m, 258H), 3.32-3.29 (m, 2587H), 3.24 -3.20(m, 30H), 3.15-3.13 (m, 39H), 2.89-2.81 (m, 58H), 2.73-2.20 (m, 28H), 2.69-2.67 (m, 20H), 2.66 -2.62(m, 5H), 2.41-2.39 (m, 81H), 2.33-2.30 (m, 17H), 2.30 -2.26(m, 66H), 2.23 -2.20(m, 34H), 2.12 -2.10(m, 15H), 1.81-1.78 (m, 66H), 1.53 -1.50(m, 82H), 0.88 - 0.81 (m, 120H). MALDI-TOF MS: from 74522.05 to 74732.71.

### Example 16: Synthesis of Compound 23-143

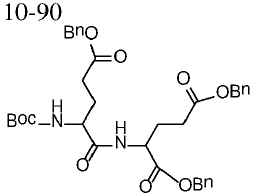

Glu(OBn)2.TsOH (10 g, 20.02 mmol, purchased), Boc-Glu(OH)-OBn (7.43 g, 22.02 mmol, purchased), HBTU (11.39 g, 30.03 mmol) and HOBT (4.06 g, 30.03 mmol) were added in a 500 mL round-bottomed flask and then dissolved with DMF (150 mL), and the mixed solution was stirred at -5 °C for 30min. Then DIEA (14.89 mL, 90.09 mmol) was slowly added dropwise, and then the mixed solution reacted at a low temperature for 2.5h; after that, the reaction device was placed at room temperature and the reaction solution was stirred overnight to react. At the end of the reaction, saturated sodium bicarbonate (200 mL) was added to wash DMF, the product in the aqueous phase was extracted three times with ethyl acetate (150 mL×3), the organic phases were combined and dried with anhydrous sodium sulfate, and then filtered by suction, concentrated, evaporated to dryness, dried in a vacuum oven, thus obtaining 14.7 g of the product which was put into the next deprotection reaction.

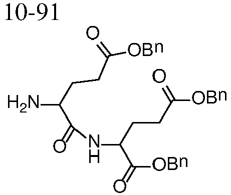

Product 10-90 (12.95 g, 20.02 mmol) was added in a 500 mL round-bottomed flask, and then dissolved with dichloromethane (100 mL), TFA (22.03 mL, 300.3 mmol) was then added, the obtained solution was stirred overnight at room temperature; at the end of the reaction, the reaction solution was concentrated under reduced pressure, saturated sodium bicarbonate (200 mL) was added to neutralize the TFA, the product in the aqueous phase was extracted three times with ethyl acetate (150 mL×3); the organic phases were combined and then dried with anhydrous sodium sulfate, and then filtered by suction, concentrated, evaporated to dryness and dried in a vacuum oven, thus obtaining 11.8 g of the product.

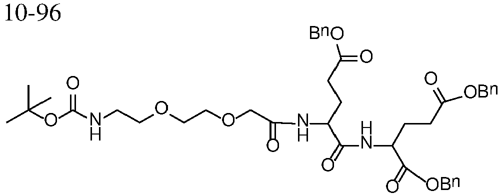

Product 10-91 (10.94 g, 20.02 mmol), Product 21-130 (4.8 g, 18.2 mmol, i.e., Product 15-49), HBTU (20.7 g, 54.6 mmol) and HOBT (7.4 g, 54.6 mmol) were added in a 1000 mL round-bottomed flask and then dissolved with DMF (150 mL), and the mixed solution was stirred at -5 °C for 30min. Then DIEA (13.5 mL, 81.9 mmol) was slowly added dropwise, and then the mixed solution reacted at a low temperature for 2.5h; after that, the reaction device was placed at room temperature and the reaction solution was stirred overnight to react. At the end of the reaction, saturated sodium bicarbonate (200 mL) was added to wash DMF, the product in the aqueous phase was extracted three times with ethyl acetate (150 mL×3), the organic phases were combined and dried with anhydrous sodium sulfate, and then filtered by suction and concentrated; the operations of dry sample loading, column chromatography, and elution with 50% ethyl acetate/50% petroleum ether were carried out; the elution product was collected, concentrated, evaporated to dryness and dried in a vacuum oven, thus obtaining 10.8 g of the product with a yield of 75.0%.

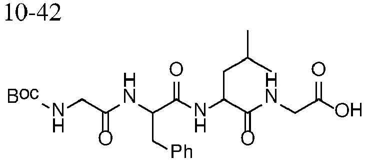

The raw material Boc-GFLG-OBn (13.54 g, 23.244 mmol) and 10% Pd/C catalyst (200 mg) were added into a hydrogenation reactor and then dissolved with DMF (45 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (90 mL) until the reaction device did not contain any product, and then the reaction product 10-42 was obtained.

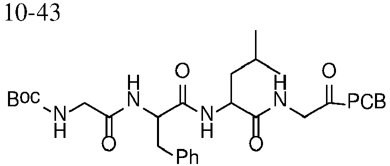

Product 10-42 Boc-GFLG-OH (23.24 mmol), Palbociclib (8 g, 17.88 mmol, referred to as PCB), HBTU (10.17 g, 26.82 mmol) and HOBT (3.6 g, 26.82 mmol) were added in a 500 mL round-bottomed flask and then dissolved with DMF (90 mL), and the mixed solution was stirred at -5 °C for 30 minutes. Then DIEA (13.3 mL, 80.46 mmol) was slowly added dropwise, and then the mixed solution reacted at a low temperature for 2h; after that, the reaction device was placed at room temperature and the reaction solution was stirred overnight to react. At the end of the reaction, the reaction solution was transferred to a 1000 mL separatory funnel, saturated sodium bicarbonate solution (200 mL) was then added, the obtained solution was extracted three times with ethyl acetate (200 mL×3), and the obtained organic phases were combined and washed with saturated sodium bicarbonate (100 mL) once, and saturated sodium chloride (100 mL) was added to remove water; the organic phase was dried with anhydrous sodium sulfate and filtered by suction. The filtrate was concentrated and evaporated to dryness and then dried in a vacuum oven, thus obtaining 16.48 g of the product for the next reaction.

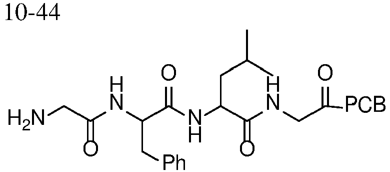

Product 10-43 (16.48 g, 17.88 mmol) was added in a 500 mL round-bottomed flask and then dissolved with dichloromethane (100 mL), and then TFA (18.67 mL, 251.37 mmol) was added and the obtained solution was stirred at room temperature to react overnight. At the end of the reaction, the reaction solution was concentrated and evaporated to dryness under reduced pressure, and the obtained dry product was then dissolved with an appropriate amount of ethyl acetate and transferred to a 500 mL separatory funnel; saturated sodium bicarbonate (100 mL) was added to neutralize the remaining TFA, the organic phase was then separated, the product in the aqueous phase was extracted three times with ethyl acetate (150 mL×3), and the obtained organic phases were combined, then dried with anhydrous sodium sulfate, filtered by suction, and concentrated; the operations of dry sample loading, column chromatography, and elution with 4% methanol/1% ammonia water/dichloromethane were carried out, and then the elution product was then collected, concentrated, evaporated to dryness, and dried in a vacuum oven, thus obtaining 15.4 g of the product.

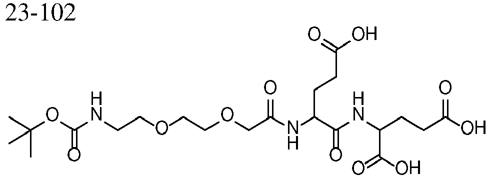

The raw material Product 10-96 (1.46 g, 1.84 mmol) and 10% Pd/C catalyst (100 mg) were added into a hydrogenation reactor and then dissolved with DMF (50 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (10 mL×3) until the reaction device did not contain any product, and then the reaction product was obtained and used for next reaction.

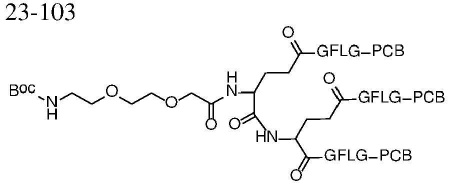

Product 23-102 (1.84 mmol), Product 10-44 (5.0 g, 6.08 mmol), HBTU (3.15 g, 8.30 mmol) and HOBT (1.12 g, 8.30 mmol) were added in a 500 mL round-bottomed flask and then dissolved with DMF (150 mL), and the mixed solution was stirred at -5 °C for 30min. Then DIEA (4.12 mL, 24.90 mmol) was slowly added dropwise, and then the mixed solution reacted at a low temperature for 2.5h; after that, the reaction device was placed at room temperature and the reaction solution was stirred overnight to react. At the end of the reaction, n-hexane (100 mL) was first added to precipitate the reaction solution, and the supernatant was then discarded; methyl tert-butyl ether (300 mL) was then added for precipitation, and the supernatant was discarded; suction operations were repeated twice, and the solid product was obtained by filtering; the solid product was washed twice with n-hexane (100 mL), and the filter cake was dried in a vacuum oven, thus obtaining 5.4 g of the product with a yield of 100%.

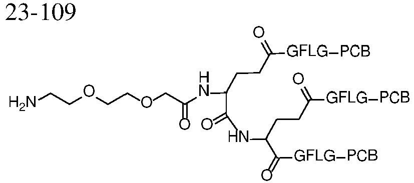

Product 23-103 (5.4 g, 1.84 mmol) was added in a 500 mL round-bottomed flask and dissolved with dichloromethane (50 mL), TFA (2.06 mL, 27.6 mmol) was then added, and the obtained solution was stirred at room temperature to react overnight; at the end of the reaction, the reaction solution was concentrated under reduced pressure and then precipitated three times with n-hexane (150 mL×3), and then precipitated with methyl tert-butyl ether (250 mL×3); the precipitate was dried in vacuum, and the operations of dry sample loading, column chromatography and elution with 6% methanol/1% ammonia water/dichloromethane were carried out; the elution product was then collected, concentrated, evaporated to dryness, and dried in a vacuum oven, thus obtaining 3.20 g of the product.

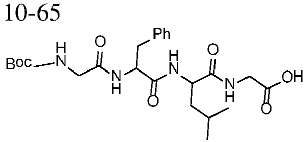

The raw material Boc-GFLG-OBn (6.30 g, 10.80 mmol) and 10% Pd/C catalyst (100 mg) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (20 mL×3) three times until the reaction device did not contain any product, and then the reaction product 10-65 was obtained.

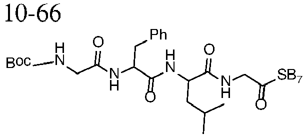

Product 10-65 (5.33 g, 10.80 mmol), SB-743921 (4.00 g, 7.74 mmol, referred to as SB7), HOBT (1.70 g, 12.58 mmol) and HBTU (4.66 g, 12.29 mmol) were added in a 500 mL round-bottomed flask and then dissolved with DMF (170 mL), and the mixed solution was stirred at -5 °C for 30min. Then DIEA (5.80 mL, 35.09 mmol) was slowly added dropwise, and then the mixed solution reacted at a low temperature for 2h; after that, the reaction device was placed at room temperature and the reaction solution was stirred overnight to react. At the end of the reaction, saturated sodium chloride (300 mL) was added to the reaction solution to wash DMF, and extraction with ethyl acetate (200 mL) was carried out three times; the organic phases were combined and concentrated by rotary evaporation to 100 mL, and the water was removed by anhydrous sodium sulfate; the suction filtering was carried out, and the filtrate was evaporated to dryness and then dried in a vacuum oven, thus obtaining 7.68 g of the product.

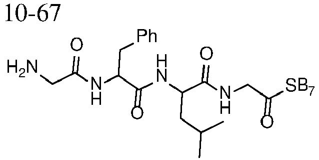

Product 10-66 (7.68 g, 7.74 mmol) was added in a 500 mL round-bottomed flask and then dissolved with dichloromethane (40 mL), and the mixed solution was stirred at room temperature. TFA (9.00 mL, 67.32 mmol) was then added dropwise, and the mixed solution reacted at room temperature for 2h. At the end of the reaction, the reaction solution was evaporated to dryness, the solid was dissolved with ethyl acetate (200 mL), saturated sodium chloride (200 mL) was then added to the obtained solution to neutralize the remaining TFA, and extraction with ethyl acetate (200 mL×3 mL) was carried out three times; the organic phases were combined and concentrated to 100 mL, and the water was removed by anhydrous sodium sulfate; the suction filtering was carried out, and the filtrate was evaporated to dryness; the operations of dry sample loading, column chromatography, elution with 3% methanol/1% ammonia water/dichloromethane were carried out; the elution product was collected and concentrated, evaporated to dryness and dried in a vacuum oven, thus obtaining 3.20 g of the product.

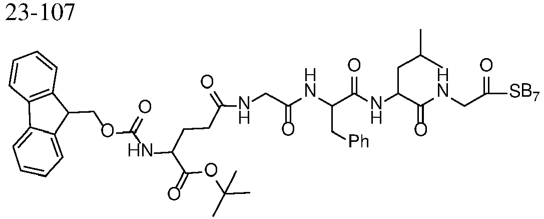

Product Fmoc-Glu-OtBu (0.8 g, 1.89 mmol), Product 10-67 (1.2 g, 1.35 mmol), HBTU (0.77 g, 2.02 mmol), and HOBT (0.28 g, 2.02 mmol) were added in a 500 mL round-bottomed flask and then dissolved with DMF (100 mL), and the mixed solution was stirred at -5 °C for 30min. Then DIEA (1.00 mL, 6.06 mmol) was slowly added dropwise, and then the mixed solution reacted at a low temperature for 2h; after that, the reaction device was placed at room temperature and the reaction solution was stirred overnight to react. At the end of the reaction, saturated sodium chloride (100 mL) was added to wash DMF, the product in the aqueous phase was extracted three times with ethyl acetate (150 mL×3), the organic phases were combined and dried with anhydrous sodium sulfate, and then filtered by suction, concentrated, evaporated to dryness, dried in a vacuum oven, thus obtaining 1.75 g of the product with a yield of 100%.

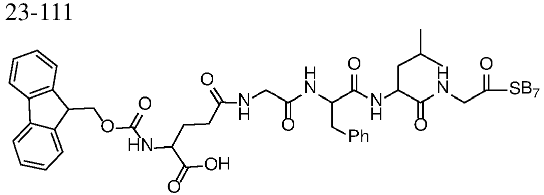

Product 23-107 (1.75 g, 1.35 mmol) was added in a 500 mL round-bottomed flask and dissolved with dichloromethane (50 mL), TFA (1.5 mL, 20.25 mmol) was then added, and the obtained solution was stirred at room temperature to react overnight; at the end of the reaction, the reaction solution was concentrated under reduced pressure and then precipitated three times with n-hexane (150 mL×3), and the supernatant was discarded; the obtained solution was then precipitated three times with methyl tert-butyl ether (250 mL×3), and the supernatant was discarded; and the operations of dry sample loading, column chromatography and elution with 6% methanol/dichloromethane were carried out; the elution product was then collected, concentrated, evaporated to dryness, and dried in a vacuum oven, thus obtaining 1.3 g of the product with a yield of 77.8%.

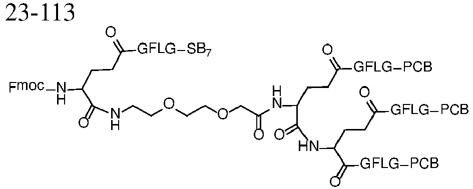

Product 23-111 (1.1 g, 0.89 mmol), Product 23-109 (2.51 g, 0.89 mmol), HBTU (0.5 g, 1.33 mmol) and HOBT (0.18 g, 1.33 mmol) were added in a 500 mL round-bottomed flask and then dissolved with DMF (100 mL), and the mixed solution was stirred at -5 °C for 30min. Then DIEA (0.66 mL, 3.98 mmol) was slowly added dropwise, and then the mixed solution reacted at a low temperature for 2h; after that, the reaction device was placed at room temperature and the reaction solution was stirred overnight to react. At the end of the reaction, n-hexane (100 mL) was first added to precipitate the reaction solution, and the supernatant was then discarded; methyl tert-butyl ether (200 mL) was then added for precipitation, and the supernatant was discarded; suction operations were repeated three times, and then filtering was carried out; the filter cake was washed with n-hexane (100 mL) and then dried in a vacuum oven, thus obtaining 3.61 g of the solid product with a yield of 100%.

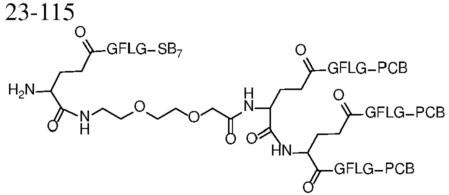

Product 23-113 (3.7 g, 0.89 mmol) was added in a 500 mL round-bottomed flask and dissolved with DMF (80 mL), morpholine (2.33 mL, 2.67 mmol) was then added and the obtained solution was stirred at room temperature to react for 2h; at the end of the reaction, methyl tert-butyl ether (300 mL) was added to precipitate the reaction solution, the filtering was carried out, washing with n-hexane (150 mL) was carried out twice, and then the product was dissolved with methanol (8 mL) and dichloromethane (50 mL); silica gel powder (10 g) was added to the obtained solution; and the operations of dry sample loading, column chromatography and elution with 6% methanol/1% ammonia water/dichloromethane were carried out; the elution product was then collected, concentrated, evaporated to dryness, and dried in a vacuum oven, thus obtaining 2.7 g of the product with a yield of 79.4%.

¹H-NMR (400 MHz, DMSO-d₆) δ 10.22 (s, 3H), 8.95 (s, 3H), 8.51 (s, 1H), 8.27-8.07 (m, 17H), 7.98-7.85 (m, 10H), 7.55-7.52 (m, 5H), 7.23-7.10 (m, 28H), 5.82 (t, *J*=16.0Hz, 3H), 4.59-4.54(m, 4H), 4.36 (d, *J*=8.0Hz, 1H), 4.06-3.97 (m, 5H), 3.90 (s, 3H), 3.75-3.55 (m, 35H), 3.19-3.13 (m, 13H), 3.05-3.00 (m, 5H), 2.89 (s, 3H), 2.80-2.67 (m, 8H), 2.42 (s, 11H), 2.31-2.23 (m, 12H), 2.12 (s, 4H), 1.89 (s, 10H), 1.83-1.77 (m, 12H), 1.62-1.49 (m, 22H), 0.92-0.81 (m, 30H).

MALDI-TOF MS: [M+H⁺] 3836.21, [M+Na⁺] 3856.06

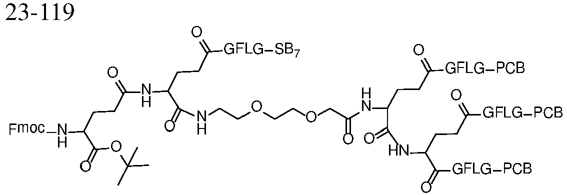

Fmoc-Glu-OtBu (0.42 g, 0.98 mmol), Product 23-115 (2.5 g, 0.652 mmol), HBTU (0.38 g, 0.98 mmol), and HOBT (0.13 g, 0.98 mmol) were added in a 500 mL round-bottomed flask and then dissolved with DMF (100 mL), and the mixed solution was stirred at -5 °C for 30min. Then DIEA (0.49 mL, 2.93 mmol) was slowly added dropwise, and then the mixed solution reacted at a low temperature for 2h; after that, the reaction device was placed at room temperature and the reaction solution was stirred overnight to react. At the end of the reaction, methyl tert-butyl ether (300 mL) was then added for precipitation and suction operation was repeated three times, and then filtering was carried out; the filter cake was washed with n-hexane (100 mL), then collected and dried in a vacuum oven, thus obtaining 3.0 g of the product with a yield of 100%.

¹H-NMR (400 MHz, DMSO-d₆) δ 10.13 (s, 3H), 8.95 (s, 3H), 8.35-8.17 (m, 5H), 8.09-8.05 (m, 10H), 7.98-7.95 (m, 6H), 7.89-7.87 (m, 8H), 7.70 (t, *J*=20.0Hz, 4H), 7.55-7.48 (m, 5H), 7.40 (t, *J*=16.0Hz, 2H), 7.33-7.15 (m, 30H), 4.59-4.51 (m, 3H), 4.37-4.35 (m, 5H), 4.29-4.20 (m, 5H), 4.01-3.90 (m, 10H), 3.61-3.53 (m, 25H), 3.40-3.38 (m, 2H), 3.15 (d, *J*=20.0Hz, 12H), 3.08-3.02 (m, 6H), 2.89 (s, 5H), 2.80-2.67 (m, 8H), 2.42 (s, 10H), 2.30 (s, 10H), 2.23 (s, 6H), 2.12-2.10 (m, 4H), 1.88-1.76 (m, 21H), 1.61-1.49 (m, 22H), 1.37 (s, 9H), 1.26-1.24 (m, 2H), 1.11 (m, 3H), 0.88-0.79 (m, 30H).

MALDI-TOF MS: [M+H⁺] 4240.59, [M+Na⁺] 4264.41

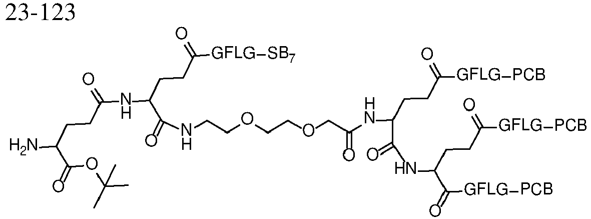

Product 23-119 (2.98 g, 0.70 mmol) was added in a 500 mL round-bottomed flask and dissolved with DMF (90 mL), morpholine (1.84 mL, 21.1 mmol) was then added and the obtained solution was stirred at room temperature to react for 2h; at the end of the reaction, methyl tert-butyl ether (300 mL) was added to precipitate the reaction solution to separate out a solid product, and the precipitation was carried out three times; the filtering was carried out, and the filter cake was washed with n-hexane (100 mL) and then collected and dried in a vacuum oven, thus obtaining 2.5 g of the product with a yield of 100%.

¹H-NMR (400 MHz, DMSO-d₆) δ 10.13 (s, 3H), 8.95 (s, 3H), 8.35-8.18 (m, 5H), 8.09-8.07 (m, 8H), 8.03-7.94 (m, 9H), 7.89-7.82 (m, 7H), 7.55-7.48 (m, 5H), 7.22-7.16 (m, 30H), 4.59-4.51 (m, 3H), 4.37-4.35 (m, 4H), 4.29-4.20 (m, 3H), 4.01-3.90 (m, 6H), 3.61-3.53 (m, 20H), 3.18-3.13 (m, 14H), 3.08-3.02 (m, 5H), 2.89 (s, 14H), 2.73-2.66 (m, 13H), 2.42 (s, 9H), 2.30-2.23 (m, 13H), 2.12 (s, 5H), 1.88-1.77 (m, 23H), 1.58-1.50 (m, 23H), 1.39 (s, 9H), 1.26-1.24 (m, 1H), 0.88-0.82 (m, 30H).

MALDI-TOF MS: [M+H⁺] 4023.32, [M+Na⁺] 4040.41

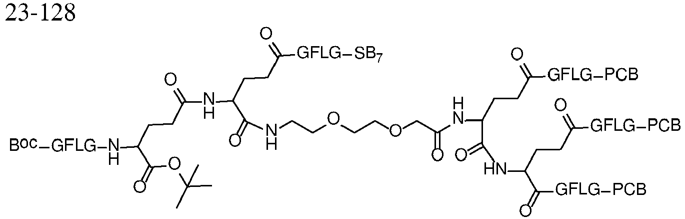

Product Boc-GFLG-OH (0.87 mmol), Product 23-123 (2.5 g, 0.62 mmol), HBTU (0.35 g, 0.93 mmol) and HOBT (0.13 g, 0.93 mmol) were added in a 500 mL round-bottomed flask and then dissolved with DMF (100 mL), and the mixed solution was stirred at -5 °C for 30min. Then DIEA (0.46 mL, 2.80 mmol) was slowly added dropwise, and then the mixed solution reacted at a low temperature for 2h; after that, the reaction device was placed at room temperature and the reaction solution was stirred overnight to react. At the end of the reaction, methyl tert-butyl ether (400 mL) was then added for precipitation and then filtering was carried out; the filter cake was washed three times with methyl tert-butyl ether (100 mL×3 mL), then collected and dried in a vacuum oven, thus obtaining 2.6 g of the product with a yield of 93.3%.

¹H-NMR (400 MHz, DMSO-d₆) δ 10.12 (s, 3H), 8.95 (s, 3H), 8.24-8.07 (m, 18H), 7.99-7.95 (m, 7H), 7.88 (d, *J*=8.0Hz, 8H), 7.55-7.48 (m, 5H), 7.22-7.15 (m, 34H), 4.58-4.51 (m, 4H), 4.37-4.32 (m, 4H), 4.22-4.18 (m, 2H), 4.06-4.00 (m, 8H), 3.90 (s, 2H), 3.74-3.52 (m, 24H), 3.39 (s, 2H), 3.18-3.13 (m, 8H), 3.08-2.99 (m, 7H), 2.89 (s, 7H), 2.80-2.73 (m, 12H), 2.42 (s, 10H), 2.30-2.24 (m, 20H), 2.12 (s, 6H), 1.88-1.76 (m, 23H), 1.58-1.49 (m, 22H), 1.37-1.35 (m, 18H), 1.11 (s, 5H), 0.86-0.82 (m, 36H).

MALDI-TOF MS: [M+H⁺] 4493.29, [M+Na⁺] 4516.30

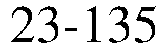

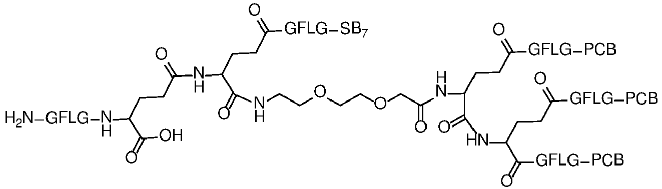

Product 23-128 (2.6 g, 0.58 mmol) was added in a 500 mL round-bottomed flask and dissolved with dichloromethane (50 mL), TFA (1.72 mL, 11.57 mmol) was then added, and the obtained solution was stirred at room temperature to react overnight; at the end of the reaction, the reaction solution was concentrated under reduced pressure and then precipitated three times with n-hexane (100 mL×3), and the supernatant was discarded; the obtained solution was then precipitated three times with methyl tert-butyl ether (300 mL×3), the filtering was carried out, and the filter cake was washed with n-hexane (100 mL), then collected and dissolved with methanol (10 mL) and dichloromethane (60 mL); silica gel power (6 g) was then added, and the operations of dry sample loading, column chromatography and elution with 5% methanol/dichloromethane were carried out; the elution product was then collected, concentrated, evaporated to dryness, and dried in a vacuum oven, thus obtaining 1.20 g of the product with a yield of 48%.

¹H-NMR (400 MHz, DMSO-d₆) δ 10.09 (s, 3H), 8.91 (s, 3H), 8.53 (d, *J*=8.0Hz, 1H), 8.31 (d, *J*=8.0Hz, 1H), 8.21-8.14 (m, 5H), 8.08-8.02 (m, 11H), 7.96-7.92 (m, 6H), 7.86 (d, *J*=12.0Hz, 7H), 7.52-7.44 (m, 5H), 7.21-7.17 (m, 28H), 7.14-6.96 (m, 7H), 4.63-4.50 (m, 4H), 4.31-4.27 (m, 5H), 4.21-4.14 (m, 3H), 3.99-3.93 (m, 3H), 3.86 (s, 3H), 3.73-3.72 (m, 3H), 3.58-3.50 (m, 22H), 3.44-3.35 (m, 8H), 3.14-3.09 (m, 17H), 3.02-2.96 (m, 4H), 2.86 (s, 1H), 2.77-2.70 (m, 6H), 2.52 (s, 3H), 2.38 (s, 8H), 2.27 (s, 12H), 2.20-2.18 (m, 7H), 2.09-2.05 (m, 6H), 1.84-1.81 (m, 10H), 1.74-1.73 (m, 12H), 1.58-1.53 (m, 13H), 1.46-1.42 (m, 10H), 1.33-1.30 (m, 1H), 1.23-1.19 (m, 4H), 0.89-0.76 (m, 36H).

MALDI-TOF MS: [M+H⁺] 4338.95, [M+Na⁺] 4360.27

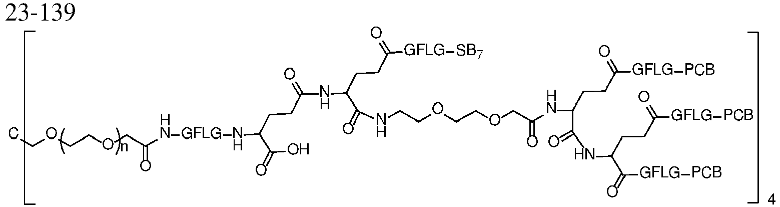

Product 23-135 (1.15 g, 0.265 mmol) was added in a 250 mL round-bottomed flask, and dissolved with dichloromethane (10 mL) and DMF (100 mL), then DIEA (0.20 mL, 1.21 mmol) was added, and finally high molecular 4ARM-SCM-40K (2.50 g, 0.06 mmol, purchased from JenKem) was added; the obtained solution was stirred for 4 days in the dark at room temperature at the lowest speed to react. At the end of the reaction, the reaction solution was concentrated under reduced pressure, and then n-hexane (300 mL) and methyl tert-butyl ether (400 mL) were added to precipitate the solution; the filtering was then carried out, and the filter cake was washed three times with n-hexane (200 mL), and then collected and dissolved with dichloromethane (50 mL) and methanol (20 mL); silica gel powder (5 g) was then added, and the operations of dry sample loading, column chromatography, and elution with 5% methanol/dichloromethane were carried out; the elution product was collected and concentrated, evaporated to dryness, and dried in a vacuum oven, thus obtaining 3.2 g of the product with a yield of 91.2%.

¹H-NMR (400 MHz, DMSO-d₆) δ 10.28 (m, 11H), 8.96 (s, 10H), 8.25-8.17 (m, 16H), 8.08-8.06 (m, 48H), 7.98-7.96 (m, 40H), 7.87-7.85 (m, 27H), 7.79-7.77 (m, 2H), 7.69-7.67 (m, 1H), 7.55-7.53 (m, 9H), 7.22-7.10 (m, 146H), 6.98 (s, 4H), 5.84-5.79 (m, 4H), 4.62-4.52 (m, 4H), 4.37-4.35 (m, 10H), 4.23-4.18 (m, 14H), 4.04-4.00 (m, 31H), 4.90-4.88 (m, 24H), 3.74-3.51 (m, 3693H), 3.20-3.17 (m, 56H), 3.14-3.12 (m, 30H), 3.05-3.03 (m, 32H), 2.90 (s, 82H), 2.74 (s, 94H), 2.60-2.57 (m, 22H), 2.42 (s, 44H), 2.31 (s, 47H), 2.25-2.21 (m, 30H), 2.15-2.10 (m, 23H), 1.91-1.78 (m, 88H), 1.60-1.50 (m, 92H), 1.23-1.11 (m, 20H), 0.92-0.82 (m, 144H).

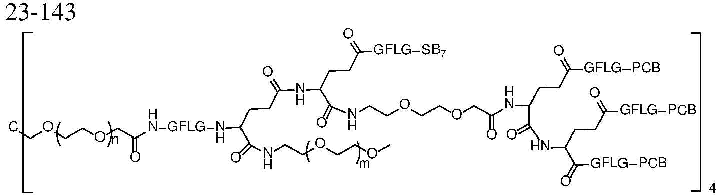

23-139 (3.1 g, 0.053 mmol), M-NH₂-2K. HCl (Lot: ZZ255p053) (0.64 g, 0.32 mmol, purchased from JenKem), HBTU (0.12 g, 0.32 mmol) and HOBT (0.043 g, 0.32 mmol) were added in a 500 mL round-bottomed flask and then dissolved with DMF (60 mL), and the mixed solution was stirred at -5 °C for 30min. Then DIEA (0.19 mL, 1.16 mmol) was slowly added dropwise, and then the mixed solution reacted at a low temperature for 2 h; after that, the reaction device was placed at room temperature and the reaction solution was stirred overnight to react. At the end of the reaction, the reaction solution was precipitated with n-hexane (100 mL) and methyl tert-butyl ether (400 mL) to separate out a solid, the filtering was then carried out, and the filter cake was washed three times with n-hexane (100 mL) and then collected and dissolved with dichloromethane (100 mL) and methanol (20 mL); silica gel powder (10 g) was then added, and the operations of dry sample loading, column chromatography, and elution with 5% methanol/dichloromethane were carried out; the elution product was collected and concentrated, evaporated to dryness, and dried in a vacuum oven, thus obtaining 2.2 g of the product.

¹H-NMR (400 MHz, DMSO-d₆) δ 10.12 (s, 12H), 8.95 (s, 10H), 8.28-8.24 (m, 2H), 8.18-8.16 (m, 18H), 8.12-8.06 (m, 46H), 7.98-7.95 (m, 36H), 7.89-7.87 (m, 25H), 7.78-7.75 (m, 2H), 7.68-7.66 (m, 2H), 7.55-7.48 (m, 16H), 7.21-7.15 (m, 146H), 5.84-5.79 (m, 6H), 4.60-4.50 (m, 13H), 4.35-4.33 (m, 11H), 4.20-4.18 (m, 14H), 4.06-4.00 (m, 24H), 3.92-3.86 (m, 30H), 3.78-3.43 (m, 4447H), 3.24-3.13 (m, 83H), 3.08-3.02 (m, 61H), 2.89 (s, 42H), 2.76-2.67 (m, 75H), 2.42 (s, 47H), 2.30-2.24 (m, 82H), 2.14-2.08 (m, 31H), 1.88-1.76 (m, 88H), 1.58-1.49 (m, 92H), 1.16-1.11 (m, 20H), 0.88-0.81 (m, 144H).

### Example 17: Synthesis of Compound 23-161

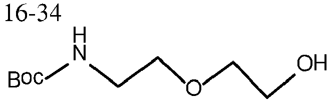

2-(-2-aminoethoxy) ethanol (18.8680 g, 190.2226 mmol) was weighed and poured into a 500 mL round-bottomed flask and then diluted with dichloromethane (100 mL), triethylamine (38.4972 mL, 380.4452 mmol) was then added, and (Boc)₂O (49.8261 g, 228.2671 mmol) was then added slowly with stirring, and the obtained solution was stirred to react at room temperature. At the end of the reaction, the reaction solution was evaporated to dryness, then sodium bicarbonate powder was added, the obtained mixture was diluted with dichloromethane, silica gel powder (30 g) was added, and the operations of evaporating to dryness, dry sample loading, and column chromatography were carried out. Elution with 50% ethyl acetate/petroleum ether was carried out, and the elution product was collected, concentrated, evaporated to dryness and dried in a vacuum oven, thus obtaining 27.33 g of the product with a yield of 70%.

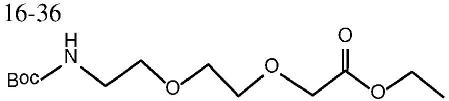

Product 16-34 (27.3 g, 132.8144 mmol) was added in a 500 mL reaction flask, nitrogen was introduced in the flask for protective purpose, the THF solution of potassium tert-butoxide was added, the mixed solution was placed at 0 °C to react, ethyl bromoacetate (17.6265 mL, 159.3773 mmol) was then added, and the obtained solution was first stirred for 3h, and then moved to room temperature for reaction. At the end of the reaction, the reaction solution was first evaporated to dryness, then deionized water and ethyl acetate were added to separate the organic phase, the aqueous phase was extracted with ethyl acetate until there was no product, the organic phases are combined, dried with anhydrous sodium sulfate powder, and filtered by suction. The filtrate was subjected to dry sample loading and column chromatography. Gradient elution with 30%-100% ethyl acetate/petroleum ether was carried out, and the elution product was collected, concentrated, evaporated to dryness and dried in a vacuum oven, thus obtaining 20 g of the product with a yield of 52%.

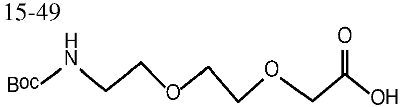

Product 16-36 (8.8 g, 30.2053 mmol) was added in a 250 mL reaction flask, 1,4-dioxane was added, and lithium hydroxide (1.5922 g, 66.4516 mmol) was further added with stirring, and 30 min later, deionized water was added until the solution became clear. At the end of the reaction, the reaction solution was extracted three times (100 mL×3) with a mixed solvent of methyl tert-butyl ether and n-hexane (1:1). The aqueous phase was adjusted to pH=1 with concentrated hydrochloric acid, and then extracted three times with ethyl acetate (300 mL×3), the ethyl acetate phases were combined, the dissolution and washing with saturated sodium chloride was carried out three times (100 mL×3), the obtained solution was concentrated, and the operations of dry sample loading, column chromatography, and eluted with 40% ethyl acetate/petroleum ether were carried out, the elution product was concentrated, evaporated to dryness, and dried in an vacuum oven, thus obtaining 6.0 g of the product with a yield of 75%.

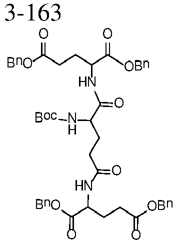

Boc-Glu-(OH)₂ (6 g, 24.2669 mmol), Glu(OBn)2 (25.4588 g, 50.9605 mmol), HBUT (27.6089 g, 72.8007 mmol), and HOBT (9.8368 g, 72.8007 mmol) were weighed and added in a 1 L reaction flask and dissolved with DMF (100 mL); the obtained solution was stirred for 30 min at -5 °C, DIEA (40 mL, 242.669 mmol) was added dropwise, and the mixed solution was first stirred at a low temperature for 2 h and then reacted at room temperature. At the end of the reaction, the reaction solution was poured into a 1 L separatory funnel, a saturated sodium bicarbonate solution (400 mL) and ethyl acetate (300 mL) were then added to separate the organic phase, the aqueous phase was extracted three times with ethyl acetate (150 mL×3), the obtained organic phases were combined, and washing with deionized water was carried out three times (200 mL×3); the obtained solution was then concentrated and the operations of dry sample loading and column chromatography were carried out. Gradient elution with 60%-70% ethyl acetate/petroleum ether was carried out, and the elution product was collected, concentrated, evaporated to dryness and dried in a vacuum oven, thus obtaining 25.0 g of the product, 4.0 g being extra-quota product.

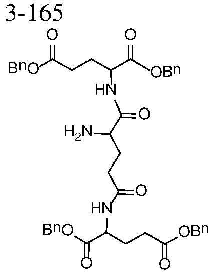

Product 3-163 (47.4 g, 54.7369 mmol) was weighed and dissolved by ultrasonic with 50 mL of dichloromethane and TFA (60 mL, 821.0541 mmol), a ground glass stopper was used, and the obtained solution was stirred to react at room temperature. At the end of the reaction, the reaction solution was poured into a 2 L separatory funnel, a saturated sodium bicarbonate solution (600 mL) and ethyl acetate (500 mL) were then added to separate the organic phase, the aqueous phase was extracted three times with ethyl acetate (150 mL×3), the obtained organic phases were combined, and washing with deionized water was carried out three times (300 mL); the obtained solution was then concentrated and evaporated to dryness, and the operations of dry sample loading and column chromatography were carried out. Gradient elution with 5% methanol/1% ammonia water/dichloromethane was carried out, the elution product was concentrated, evaporated to dryness and dried in a vacuum oven, thus obtaining 34 g of the product with a yield of 83%.

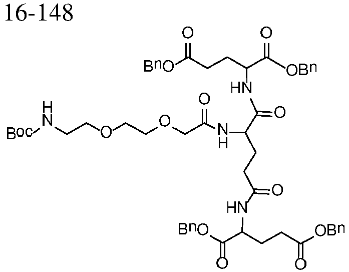

Product 15-49 (9 g, 24.7736 mmol), Product 3-165 (17.2481 g, 22.5215 mmol), HBUT (11.9575 g, 31.5301 mmol), and HOBT (4.2603 g, 31.5301 mmol) were weighed and added in a 500 mL reaction flask and dissolved with DMF (200 mL); the obtained solution was stirred for 30 min at -5 °C, DIEA (17 mL, 101.3468 mmol) was added dropwise, and the mixed solution was first stirred at a low temperature for 2 h and then reacted at room temperature. At the end of the reaction, the reaction solution was poured into a 2 L separatory funnel, a saturated sodium bicarbonate solution (600 mL) and ethyl acetate (500 mL) were then added to separate the organic phase, the aqueous phase was extracted three times with ethyl acetate (150 mL × 3), the obtained organic phases were combined, and washing with deionized water was carried out once (300 mL); the obtained solution was then concentrated and evaporated to dryness, and the operations of dry sample loading and column chromatography were carried out. Elution with 60% ethyl acetate/petroleum ether was carried out, and the elution product was concentrated, evaporated to dryness and dried in a vacuum oven, thus obtaining 12.5 g of the product with a yield of 56%.

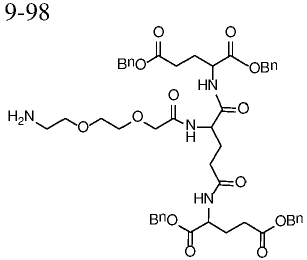

Product 16-148 (2 g, 1.978 mmol) was weighed and partially dissolved with dichloromethane (10 mL), and then TFA (4.4075 mL, 59.340 mmol) was added, the obtained solution was treated by ultrasonic until the Product 16-148 was dissolved completely; a ground glass stopper was used, and the mixed solution was stirred at room temperature. After TLC detection and color development with phosphomolybdic acid, the reaction was stopped; the reaction solution was evaporated to dryness, the dichloromethane was removed, and then the obtained solid was dissolved with ethyl acetate (200 mL), and a saturated sodium bicarbonate solution was added until the aqueous phase became alkaline; then, the organic phase was separated, and the aqueous phase was extracted with ethyl acetate until there was no product in the aqueous phase; the organic phases were combined, and washing with saturated saline solution was carried out three times (100 mL×3); the obtained solution was concentrated and evaporated to dryness, and then subjected to column chromatography. With the column height of 5cm, gradient elution with 1% ammonia water/3% methanol/dichloromethane was carried out, and the elution product was concentrated, evaporated to dryness and dried in a vacuum oven, thus obtaining 1.8 g of the product with a yield of 99.9%.

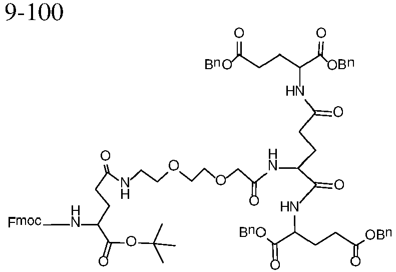

The reactant Fmoc-Glu-OtBu (1.1769 g, 2.7682 mmol) was added in a 250 mL reaction flask and then dissolved with DMF (20 mL), and the obtained solution was then placed at 0 °C; then, Product 9-98 (1.8 g, 1.9758 mmol) and PyAOP (1.4433 g, 2.7682 mmol) were added to the solution and the obtained solution was stirred for 0.5h; TMP (0.2602 mL, 1.9758 mmol) was slowly added dropwise and the obtained solution was stirred at a low temperature until the reaction ended. At the end of the reaction, a saturated sodium bicarbonate solution and ethyl acetate were then added to separate the organic phase, the aqueous phase was extracted three times with ethyl acetate (150 mL×3) until there was no product in the aqueous phase, the obtained organic phases were combined, and washing with a saturated saline solution was carried out three times (200 mL×3); the obtained solution was then concentrated, and the operations of dry sample loading and column chromatography were carried out. Gradient elution with 2% methanol/dichloromethane was carried out, the elution product was concentrated, evaporated to dryness and dried in a vacuum oven, thus obtaining 2.8 g of the product, 0.2 g being extra-quota product.

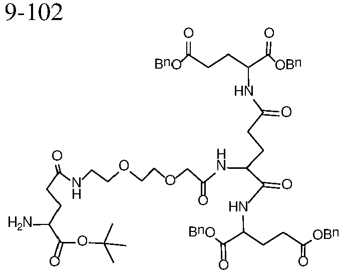

The reactant Product 9-100 (theoretical value, 1.9758 mmol) was added in a 250 mL round-bottomed flask and dissolved with a proper amount of DMF, morpholine (5.1639 mL, 59.274 mmol) was then added, and the obtained solution was stirred at room temperature and applied to the TLC plate every 0.5h. One hour later, the reaction ended. A saturated sodium bicarbonate solution (200 mL) and ethyl acetate (300 mL) were then added to separate the organic phase, the aqueous phase was extracted with ethyl acetate until there was no product in the aqueous phase, the obtained organic phases were combined, and washing with a saturated saline solution was carried out; the obtained solution was then concentrated and evaporated to dryness, and the operations of dry sample loading, column chromatography, and gradient elution with 1%-5% methanol/dichloromethane were carried out; the elution product was concentrated, evaporated to dryness and dried in a vacuum oven, thus obtaining 1.8 g of the product with a yield of 83.106%.

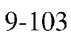

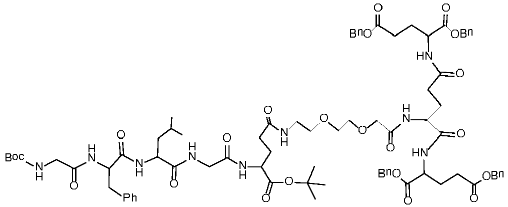

Product 9-102 (1.8 g, 1.6420 mmol), Product 3-30 (theoretical value, 2.2988 mmol), HBTU (84.3030 g, 222.2946 mmol), and HOBT (30.0387 g, 222.2946 mmol) were added in a round-bottomed flask and dissolved with DMF (50 mL); the obtained solution was stirred at -5 °C for 0.5 h and DIEA (132.2681 mL, 800.2606 mmol) was then slowly added dropwise; the obtained solution first reacted for 2 h and then stirred at room temperature overnight to react. At the end of the reaction, a saturated sodium bicarbonate solution (200 mL) and ethyl acetate (300 mL) were then added to separate the organic phase, the aqueous phase was extracted with ethyl acetate until there was no product in the organic phase, the obtained organic phases were combined, and washing with a saturated saline solution was carried out; the obtained solution was then concentrated and evaporated to dryness, and the operations of dry sample loading, column chromatography, and elution with 3% methanol/dichloromethane were carried out, thus obtaining 1.8 g of the product with a yield of 72 %.

MALDI-TOF MS: [M+H⁺] 1570.36, [M+Na⁺] 1592.52.

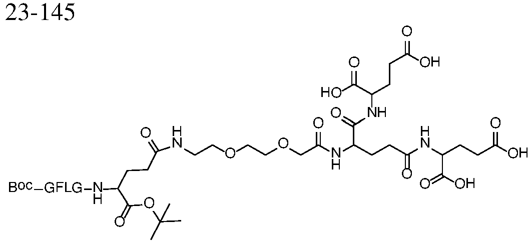

The raw material Product 9-102 (0.43 g, 0.28 mmol) and 10% Pd/C catalyst (100 mg) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum water pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (10 mL×3) until the reaction device did not contain any product, and then the reaction product was obtained and used for next reaction.

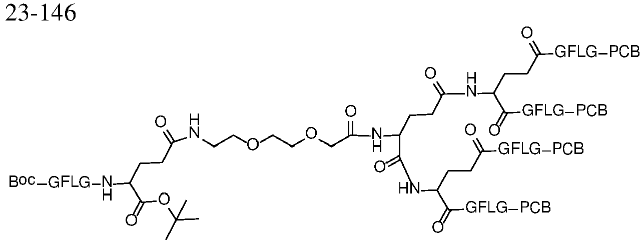

Product 23-145 (0.28 mmol), GFLG-PCB (1 g, 1.22 mmol), HBTU (0.63 g, 1.65 mmol) and HOBT (0.23 g, 1.65 mmol) were added in a 500 mL round-bottomed flask and then dissolved with DMF (100 mL), and the mixed solution was stirred at -5 °C for 30min. Then DIEA (0.82 mL, 4.98 mmol) was slowly added dropwise, and then the mixed solution reacted at a low temperature for 2h; after that, the reaction device was placed at room temperature and the reaction solution was stirred overnight to react. At the end of the reaction, n-hexane (100 mL) and methyl tert-butyl ether (400 mL) were then added for precipitation to separate out a solid, and then filtering was carried out; the filter cake was washed with n-hexane (100 mL), then collected and dried in a vacuum oven, thus obtaining 1.5 g of the product, 0.28 g being extra-quota product.

¹H-NMR (400 MHz, DMSO-d₆) δ 10.14 (s, 3H), 8.96-8.95 (m, 3H), 8.45 (d, *J*=8.0Hz, 1H), 8.35 (d, *J*=8.0Hz, 1H), 8.18-8.17 (m, 4H), 8.13-8.03 (m, 12H), 7.99-7.95 (m, 6H), 7.89-7.83 (m, 9H), 7.74-7.66 (m, 6H), 7.55-7.49 (m, 4H), 7.44-7.40 (m, 1H), 7.29-7.28 (m, 3H), 7.21-7.14 (m, 17H), 4.62-4.57 (m, 3H), 4.36-4.29 (m, 3H), 4.07-4.00 (m, 5H), 3.89 (s, 1H), 3.72-3.71 (m, 3H), 3.62-3.51 (m, 20H), 3.41 (s, 20H), 3.18-3.13 (m, 12H), 3.03 (s, 20H), 2.94-2.89 (m, 5H), 2.75-2.73 (m, 2H), 2.69-2.64 (m, 7H), 2.44-2.42 (m, 9H), 2.31-2.30 (m, 7H), 2.26-2.23 (m, 3H), 2.14-2.13 (m, 2H), 1.88-1.86 (m, 24H), 1.62-1.50 (m, 24H), 1.37-1.22 (m, 25H), 0.91-0.82 (m, 30H).

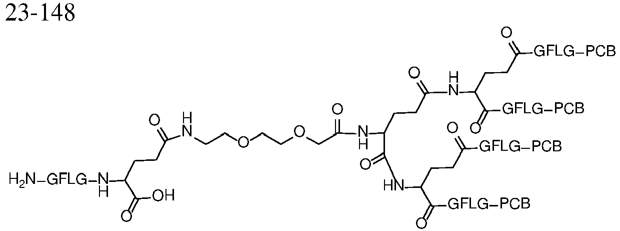

Product 23-146 (1.22 g, 0.28 mmol) was added in a 500 mL round-bottomed flask and dissolved with dichloromethane (30 mL), TFA (0.31 mL, 4.20 mmol) was then added, and the obtained solution was stirred at room temperature to react overnight; at the end of the reaction, the reaction solution was concentrated under reduced pressure; the obtained solution was then precipitated three times with methyl tert-butyl ether (300 mL×3), the filtering was carried out, and the filter cake was washed with n-hexane (100 mL), then collected and dissolved with methanol (10 mL) and dichloromethane (60 mL); silica gel power (5 g) was then added, and the operations of dry sample loading, column chromatography and elution with 6% methanol/dichloromethane were carried out; the elution product was then collected, concentrated, evaporated to dryness, and dried in a vacuum oven, thus obtaining 0.9 g of the product.

¹H-NMR ( 400MHz, DMSO-d₆) δ 10.56 (s, 3H), 8.97 (s, 3H), 8.56 (d, *J*=8.0Hz, 1H), 8.36 (d, *J*=8.0Hz, 1H), 8.31-8.17 (m, 7H), 8.11-8.03 (m, 11H), 7.97-7.93 (m, 4H), 7.92-7.84 (m, 8H), 7.83-7.81 (m, 4H), 7.74-7.69 (m, 5H), 7.44-7.40 (m, 1H), 7.34-7.14 (m, 25H), 4.68-4.64 (m, 1H), 4.62-4.56 (m, 3H), 4.37-4.33 (m, 2H), 4.17-4.16 (m, 2H), 4.06-3.97 (m, 6H), 3.90-3.87 (m, 3H), 3.74-3.63 (m, 17H), 3.60-3.51 (m, 31H), 3.44-3.38 (m, 4H), 3.20-3.15 (m, 8H), 3.06-3.03 (m, 4H), 2.89 (s, 4H), 2.75-2.73 (m, 6H), 2.43 (s, 10H), 2.32 (s, 9H), 2.23-2.13 (m, 10H), 1.91-1.78 (m, 24H), 1.59-1.50 (m, 24H), 1.37-1.34 (m, 2H), 1.23-1.18 (m, 5H), 0.90-0.82 (m, 30H).

MALDI-TOF MS: [M+H⁺] 4268.27, [M+Na⁺] 4289.57.

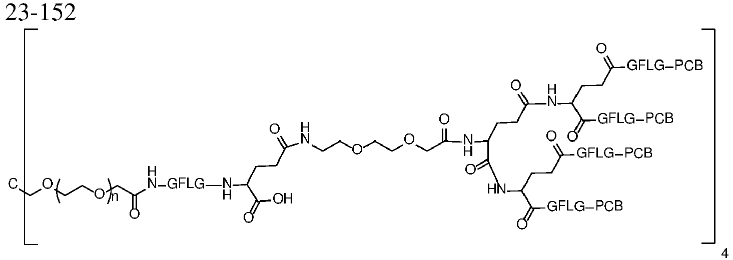

Product 23-148 (0.9 g, 0.21 mmol) was added in a 500 mL round-bottomed flask and then dissolved with dichloromethane (20 mL) and DMF (100 mL), and then DIEA (0.16 mL, 0.95 mmol) was added. High-molecular 4ARM-SCM-40K (Lot: C231-N171101) (2.01 g, 0.048 mmol, purchased from JenKem) was added last in turn, and the mixed solution was stirred in the dark at the lowest speed at room temperature for 4 days. At the end of the reaction, the reaction solution was concentrated under reduced pressure, and then n-hexane (150 mL) and methyl tert-butyl ether (300 mL) were added to precipitate the solution, and such precipitation was carried out three times; the filtering was then carried out to obtain a solid product, and the obtained solid product was dissolved with dichloromethane (50 mL) and methanol (10 mL); silica gel powder (5 g) was then added, and the operations of dry sample loading, column chromatography, and elution with 6% methanol/dichloromethane were carried out; the elution product was collected and concentrated, evaporated to dryness, and dried in a vacuum oven, thus obtaining 1.5 g of the product.

¹H-NMR (400 MHz, DMSO-d₆) δ 10.23 (s, 9H), 9.05-8.90 (m, 17H), 8.80-8.76 (m, 9H), 8.18-8.06 (m, 82H), 7.87-7.85 (m, 26H), 7.76-7.65 (m, 6H), 7.55-7.53 (m, 13H), 7.38-7.33 (m, 20H), 7.21-7.14 (m, 83H), 7.03-7.69 (m, 3H), 6.72-6.52 (m, 16H), 6.15-5.98 (m, 44H), 5.20-5.10 (m, 13H), 4.60-4.58 (m, 59H), 4.40-4.35 (m, 73H), 4.13-3.51 (m, 3776H), 3.17-3.11 (m, 115H), 3.93-2.73 (m, 41H), 2.67-2.59 (m, 38H), 2.42 (s, 31H), 2.31-2.14 (m, 66H), 1.90-1.76 (m, 56H), 1.58-1.50 (m, 72H), 1.35-1.32 (m, 21H), 1.23-1.22 (m, 70H), 0.87-0.82 (m, 120H).

MALDI-TOF MS: from 58533.26 to 58665.97.

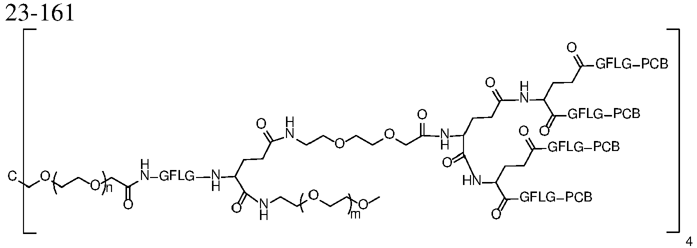

23-152 (1.5 g, 0.025 mmol), M-NH₂-2K.HCl (Lot: ZZ255p053) (0.64 g, 0.32 mmol, purchased from JenKem), HBTU (0.06 g, 0.15 mmol) and HOBT (0.02 g, 0.15 mmol) were added in a 500 mL round-bottomed flask and then dissolved with DMF (80 mL), and the mixed solution was stirred at -5 °C for 30min. Then DIEA (0.10 mL, 0.6 mmol) was slowly added dropwise, and then the mixed solution reacted at a low temperature for 2h; after that, the reaction device was placed at room temperature and the reaction solution was stirred overnight to react. At the end of the reaction, n-hexane (100 mL) and methyl tert-butyl ether (400 mL) were added to precipitate the solution, and such precipitation was carried out three times; the filtering was then carried out to obtain a solid product, and the obtained solid product was dissolved with dichloromethane (50 mL) and methanol (10 mL); silica gel powder (8 g) was then added, and the operations of dry sample loading, column chromatography, and elution with 5% methanol/dichloromethane were carried out; the elution product was collected and concentrated, evaporated to dryness, and dried in a vacuum oven, thus obtaining 0.63 g of the product.

¹H-NMR (400 MHz, DMSO-d₆) δ 10.13 (s, 8H), 8.95 (s, 8H), 8.25-8.15 (m, 67H), 8.10-8.06 (m, 51H), 8.03-7.95 (m, 13H), 7.89-7.77 (m, 26H), 7.70-7.63 (m, 4H), 7.50-7.48 (m, 11H), 7.36-7.34 (m, 3H), 7.21-7.15 (m, 93H), 4.58-4.35 (m, 48H), 4.30-4.18 (m, 70H), 4.14-4.35 (m, 4462H), 3.24-3.13 (m, 109H), 3.06-3.02 (m, 41H), 2.91-2.89 (m, 45H), 2.80-2.67 (m, 71H), 2.42 (s, 53H), 2.33-2.12 (m, 90H), 1.88-1.78 (m, 95H), 1.58-1.50 (m, 95H), 1.37-1.35 (m, 3H), 1.30-1.22 (m, 21H), 1.15-1.12 (m, 6H), 0.87-0.82 (m, 120H). MALDI-TOF MS: from 66445.77 to 66551.28.

### Example 18: Synthesis of Compound 24-12

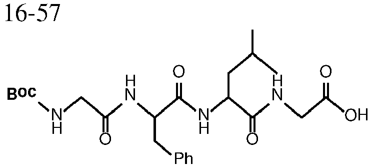

Boc-GFLG-OBn (home-made 20 g, 34.3231 mmol) and 10% Pd/C (0.4 g) were added in a micro-reactor, DMF (40 mL) was then added to the micro-reactor, the device was set ready, the air was pumped out to reach a vacuum state, H2 (16 psi) was introduced, and such operations were repeated three times. The solution was stirred overnight at room temperature. At the end of the reaction, diatomaceous earth was used as a filter cake to carry out suction filtering, and then washing with DMF was carried out three times (20 mL×3), and the solution was then transferred to a 500 mL flask as the raw material for Product 16-158.

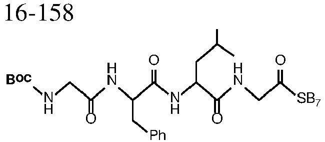

SB7 (SB-743921) (13.6519 g, 26.4024 mmol), HBTU (15.0193 g, 39.6036 mmol) and HOBT (5.3512 g, 39.6036 mmol) were weighed and added to the flask with Product 16-157, and the reaction solution was placed in a low-temperature constant temperature reaction bath (-5 °C) and stirred for 20min, DIEA (19.7 mL, 118.8108 mmol) was added dropwise, and the mixed solution first reacted for 2 h and then taken out and placed at room temperature overnight on a magnetic stirrer. At the end of the reaction, the reaction solution was transferred to a 2 L separatory funnel, saturated sodium bicarbonate solution (300 mL) and ethyl acetate (400 mL) were added to the separatory funnel, the obtained solution was shaken thoroughly to separate the organic phase, and the aqueous phase was extracted twice with ethyl acetate (150 mL × 2); the obtained organic phases were combined, washing with a saturated sodium bicarbonate solution (200 mL) was carried out once, and washing with a saturated sodium chloride solution was carried out twice (200 mL × 2); the obtained organic phase was poured into a 2 L conical flask, dried with anhydrous magnesium sulfate, and then filtered by suction, concentrated and dried in vacuum, thus obtaining 28 g of the product, 2 g being extra-quota product.

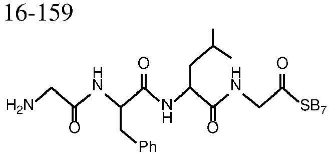

Product 16-158 (26 g, 26.2197 mmol) was added in a 500 mL flask and dissolved with dichloromethane (15 mL), TFA (4.2 mL, 55.8910 mmol) was then added to the solution, and the obtained solution was stirred overnight at room temperature. At the end of the reaction, the reaction solution was evaporated to remove the dichloromethane solvent; the obtained product was dissolved with ethyl acetate and the obtained solution was then transferred to a 2 L separatory funnel; saturated sodium bicarbonate solution (300 mL, being in aqueous phase and alkaline) and ethyl acetate (400 mL) were added to the separatory funnel, the obtained solution was shaken thoroughly to separate the organic phase, and the aqueous phase was extracted three times with ethyl acetate (150 mL× 3); the obtained organic phases were combined and washed twice with saturated sodium chloride solution (200 mL×2); the obtained organic phase was poured into a 2 L conical flask, dried with anhydrous magnesium sulfate, and then filtered and concentrated to 100 mL; silica gel powder was added to the filtrate and the obtained solution was subjected to rotary evaporation to obtain a solid solution; the operations of dry sample loading, column chromatography, and elution with 1% ammonia water: 2%methanol-5% methanol/dichloromethane were carried out, the elution product was then collected, rotary evaporated, and dried in vacuum, thus obtaining 18.8 g of the product with a yield of 80%.

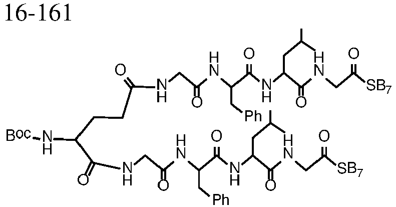

Reactants, i.e., Product 16-159 (7 g, 7.8518 mmol), Boc-Glu-OH (0.9245 g, 3.7390 mmol), HBTU (4.2526 g, 11.2170 mmol), and HOBT (1.5156 g 11.2170 mmol) were added in a 500 mL reaction flask and dissolved with DMF (100 mL), and then the obtained solution was placed in a low-temperature constant temperature bath (-5 °C) and stirred for 30min. DIEA (5.6 mL, 33.6510 mmol) was then added dropwise, and the mixed solution first reacted at a low temperature for 2 h and then reacted at room temperature. At the end of the reaction, the reaction solution was transferred to a 2 L separatory funnel, saturated sodium bicarbonate solution (300 mL) and ethyl acetate (400 mL) were added to the separatory funnel, the obtained solution was shaken thoroughly to separate the organic phase, and the aqueous phase was extracted three times with ethyl acetate (150 mL× 2); the obtained organic phases were combined, and washing with a saturated sodium chloride solution was carried out twice (200 mL × 2); the obtained organic phase was poured into a 2 L conical flask, dried with anhydrous magnesium sulfate, and then filtered, evaporated to dryness and dried in vacuum, thus obtaining 8.5 g of the product, 1.1 g being extra-quota product.

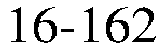

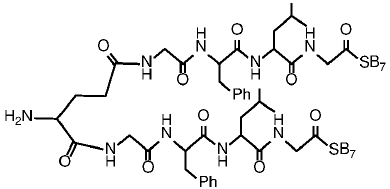

Product 16-161 (7.4 g, 3.7390 mmol) was added in a 500 mL flask and dissolved with dichloromethane (20 mL), TFA (8.5 mL, 112.17 mmol) was then added to the solution, and the obtained solution was stirred overnight at room temperature. At the end of the reaction, the reaction solution was evaporated to remove the dichloromethane solvent; the obtained product was dissolved with ethyl acetate (35 mL) and the obtained solution was then transferred to a 2 L separatory funnel; saturated sodium bicarbonate solution (300 mL, being in aqueous phase and alkaline) and ethyl acetate (400 mL) were added to the separatory funnel, the obtained solution was shaken thoroughly to separate the organic phase, and the aqueous phase was extracted three times with ethyl acetate (150 mL× 3); the obtained organic phases were combined and washed twice with saturated sodium chloride solution (200 mL×2); the obtained organic phase was poured into a 2 L conical flask, dried with anhydrous magnesium sulfate, and then filtered by suction and concentrated to 100 mL; silica gel powder was added and the obtained solution was subjected to rotary evaporation to obtain a solid solution; the operations of dry sample loading, column chromatography, and elution with 8% methanol-1% ammonia water: 9% methanol/dichloromethane were carried out, the elution product was then collected, rotary evaporated, and dried in vacuum, thus obtaining 4 g of the pure product and 1.8 g of the impure product, with the total yield of 82.86%.

¹H -NMR (400 MHz, DMSO-*d*₆) δ 8.19 - 8.04 (m, 11H), 7.81 (s, 2H), 7.57 ― 7.46 (m, 5H), 7.27 - 7.11 (m, 32H), 4.53 (s, 3H), 4.29 - 4.12 (m, 5H), 3.89 (s, 2H), 3.72 - 3.48 (m, 9H), 3.17 (s, 2H), 3.04 - 2.98 (m, 2H), 2.82 - 2.72 (m, 4H), 2.58 (s, 5H), 2.31 (s, 7H), 2.15 (s, 2H), 1.84 - 1.74 (m, 1H), 1.67 - 1.46 (m, 9H), 1.24 (s, 5H), 0.92 (s, 6H), 0.86 (s, 6H), 0.81 (s, 6H), 0.51 (s, 6H). MALDI-TOF MS: [M+H⁺]1892.12, [M+Na⁺] 1914.61.

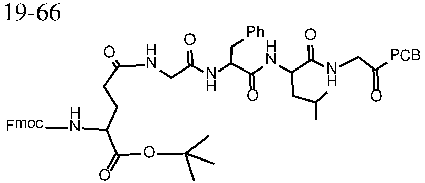

Fmoc-GLu-OtBu (2.174 g, 5.1097 mmol) was weighed and dissolved with DMF (20 mL), the obtained solution was placed in a low-temperature constant temperature reaction bath (0 °C), and GFLG-PCB (3 g, 3.6498 mmol) and PyAop (2.6641 g, 5.1097 mmol) were then added; the obtained solution was stirred for 30min, TMP (0.4807 mL, 3.6498 mmol) was dropwise added to the solution, and the obtained solution was stirred overnight at a low temperature. At the end of the reaction, methyl tert-butyl ether was added to the reaction solution until a solid came out, suction filtering was carried out, the solid was dissolved with a mixed solvent (20% methanol: 80% dichloromethane, 30 mL) and then silica gel powder (15 g) was added; the obtained solution was then evaporated to dryness, and the operations of dry sample loading, column chromatography, and gradient elution with an eluent (3% methanol-5% methanol/dichloromethane) were carried out, thus obtaining 3.6 g of the product with a yield of 80%.

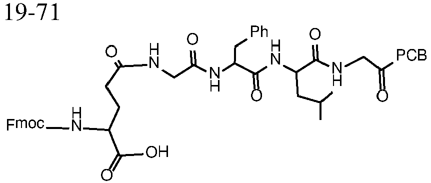

Product 19-66 (3.6 g, 2.9280 mmol) was added in a 500 mL flask and dissolved with dichloromethane (20 mL), TFA (4.4 mL, 58.5637 mmol) was then added to the solution, and the obtained solution was stirred overnight at room temperature. At the end of the reaction, the reaction solution was evaporated to remove dichloromethane; ethyl acetate (15 mL) was added for ultrasonic treatment to obtain a homogeneous phase; n-hexane (100 mL) was added for precipitation, the obtained solution was plated in a refrigerator for 20min, and the supernatant was discarded; ethyl acetate (15 mL) and n-hexane (100 mL) were further added for repeating the dissolution and precipitation until a solid product was obtained; the solid product was dissolved with a mixed solvent (20% methanol: 80% dichloromethane 50 mL); silica gel powder was then added. The operations of evaporation, sample loading, column chromatography, and gradient elution with 5%-8% methanol/dichloromethane were carried out, thus obtaining 3.4 g of the product with a yield of 100%.

¹H-NMR (400 MHz, DMSO-*d*₆) δ 10.36 (s, 1H), 8.15 (s, 1H), 8.07 - 8.01 (m, 3H), 7.86 (s, 4H), 7.76 - 7.56 (m, 4H), 7.41 (s, 2H), 7.32 (s, 2H), 7.24 (s, 4H), 7.22 - 7.13 (m, 1H), 4.54 (s, 1H), 4.29 - 4.18 (m, 3H), 4.05 - 3.92 (m, 3H), 3.78 - 3.56 (m, 18H), 3.05 (s, 1H), 2.54 (s, 1H), 2.43 (s, 3H), 2.32 (s, 3H), 2.22 (s, 3H), 1.90 (s, 2H), 1.78 (s, 3H), 1.63 - 1.50 (m, 4H), 0.87 (s, 6H).

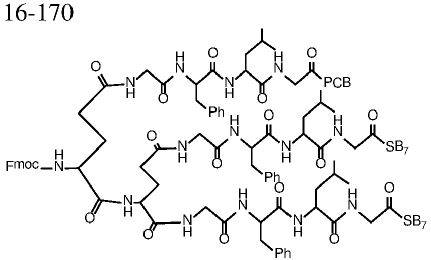

Reactants, i.e., Product 16-162 (4.7817 g, 2.5246 mmol), Product 19-71 (3 g, 2.5246 mmol), HBTU (1.4248 g, 3.7869 mmol), and HOBT (0.5117 g 3.7869 mmol) were added in a 500 mL reaction flask and dissolved with DMF (150 mL), and then the obtained solution was placed in a low-temperature constant temperature bath (-5 °C) and stirred for 30min. DIEA (1.9 mL, 11.3607 mmol) was then added dropwise, and the mixed solution first reacted at a low temperature for 2 h and then reacted at room temperature. At the end of the reaction, deionized water (300 mL) was directly added to the reaction solution, the solid product was separated out by precipitation, suction filtering was carried out, and ethyl acetate (300 mL) was added to the filter cake, and ultrasonic treatment was carried out to obtain a homogeneous phase; n-hexane (200 mL) was then added for precipitation, and suction filtering and vacuum drying were carried out, thus obtaining 8 g of the final product, 0.3 g being extra-quota product.

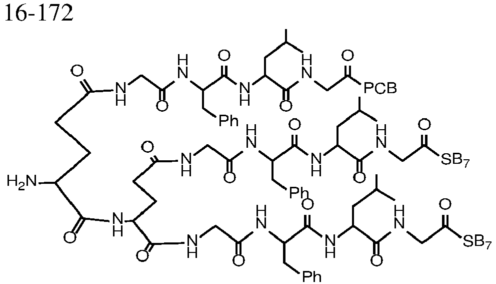

Reactant Product 16-170 (7.7 g, 2.5246 mmol) was added in a 500 mL reaction flask and dissolved with DMF (50 mL), morpholine (6.6 mL, 75.738 mmol) was then added, the obtained solution was stirred to react and TLC was carried out every 0.5h. One hour later, the reaction ended, methyl tert-butyl ether (100 mL) was added to the reaction solution until a solid came out, suction filtering was carried out, and ethyl acetate (100 mL) was added to the filter cake, and ultrasonic treatment was carried out to obtain a homogeneous phase; n-hexane (50 mL) was then added, and suction filtering and drying were carried out, thus obtaining 6.4 g of the final product with a yield of 88%.

¹H-NMR (400 MHz, DMSO-*d*₆) δ 10.12 (s, 1H), 8.96 (s, 1H), 8.25 (s, 1H), 8.15 (s, 4H), 8.10 - 8.01 (m, 10H), 7.95 (s, 2H), 7.92 - 7.86 (m, 3H), 7.80 (s, 1H), 7.57 - 7.47 (m, 5H), 7.28 - 7.12 (m, 31H), 4.54 (s, 4H), 4.29 - 4.11 (m, 6H), 3.89 (s, 3H), 3.63 (s, 8H), 3.21 - 3.13 (m, 6H), 3.09 - 2.97 (m, 5H), 2.89 (s, 8H), 2.73 (s, 8H), 2.42 (s, 4H), 2.31 (s, 9H), 2.19 - 2.09 (m, 4H), 1.99 (s, 3H), 1.85 (s, 10H), 1.54 (s, 15H), 1.33 (s, 2H), 1.17 (s, 4H), 0.94 - 0.78 (m, 25H), 0.51 (s, 5H). MALDI-TOF MS: [M+Na⁺] 2862.85.

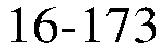

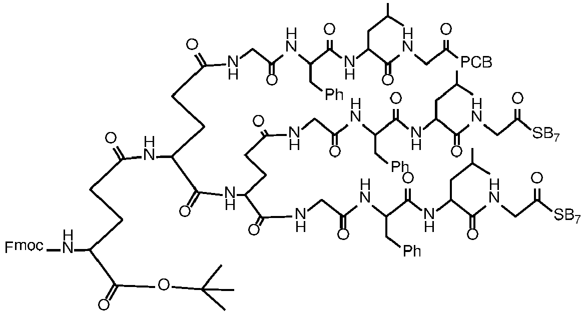

Reactants, i.e., Product 16-172 (2.4 g, 0.8444 mmol), Fmoc-GLu-OtBu (0.4311 g, 1.0133 mmol), HBTU (0.1712 g, 1.2666 mmol), and HOBT (0.4803 g 1.2666 mmol) were added in a 250 mL reaction flask and then dissolved with DMF (40 mL), and then the obtained solution was placed in a low-temperature constant temperature bath (-5 °C) and stirred for 30min. DIEA (0.63 mL, 3.7998 mmol) was then added dropwise, and the mixed solution first reacted at a low temperature for 2 h and then reacted at room temperature. Two hours later, the reaction was not completed which was indicated by TLC; Fmoc-GLu-OtBu, HBTU, and HOBT were added again each in an amount which was 20% of the feed; 3 h later, TLC was carried out, no significant change was observed, and methyl tert-butyl ether (100 mL) was then added until a solid came out; suction filtering was carried out, ethyl acetate (50 mL) and n-hexane (100 mL) were added to dissolve the solid, and the obtained solution was filtered by suction and dried, thus obtaining 2.8 g of the product, 0.1 g being extra-quota product.

¹H-NMR (400 MHz, DMSO-*d*₆) δ 10.20 (s, 1H), 9.03 (s, 1H), 8.26 - 8.12 (m, 18H), 8.08 - 8.02 (m, 6H), 7.93 (s, 8H), 7.79 (s, 5H), 7.63 - 7.55 (m, 5H), 7.47 (s, 3H), 7.34 - 7.25 (m, 26H), 4.62 (s, 4H), 4.35 - 4.23 (m, 9H), 4.09 (s, 3H), 3.96 (s, 4H), 3.70 (s, 15H), 3.28 - 3.19 (m, 5H), 3.11 (s, 8H), 2.96 (s, 5H), 2.80 (s, 6H), 2.49 (s, 3H), 2.38 (s, 9H), 2.19 (s, 4H), 1.89 (s, 11H), 1.67 - 1.53 (m, 12H), 1.45 (s, 9H), 1.33 (s, 4H), 1.25 (s, 2H), 0.93 (s, 25H), 0.58 (s, 5H).

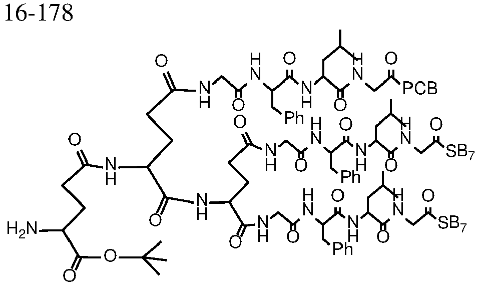

Reactant Product 16-173 (2.7 g, 0.8444 mmol) was added in a 250 mL reaction flask and dissolved with DMF (20 mL), morpholine (2.2 mL, 25.332 mmol) was then added, the obtained solution was stirred to react and TLC was carried out every 0.5h. One hour later, the reaction ended, methyl tert-butyl ether (150 mL) and n-hexane (100 mL) were successively added to the reaction solution, ultrasonic treatment was carried out to obtain a homogeneous phase, and suction filtering was carried out; ethyl acetate (100 mL) and n-hexane (100 mL) were then added in sequence, and suction filtering and drying were carried out, thus obtaining 2.7 g of the final product, 0.15 g being extra-quota product.

¹H-NMR (400 MHz, DMSO-*d*₆) δ 10.13 (s, 1H), 8.96 (s, 1H), 8.22 - 7.95 (m, 16H), 7.89 (s, 2H), 7.83 - 7.75 (m, 3H), 7.57 - 7.45 (m, 5H), 7.29 ― 7.07 (m, 36H), 4.55 (s, 2H), 4.26 - 4.12 (m, 4H), 4.01 (s, 2H), 3.89 (s, 1H), 3.74 - 3.60 (m, 17H), 3.01 (s, 7H), 2.90 (s, 18H), 2.74 (s, 6H), 2.43 (s, 5H), 2.31 (s, 8H), 2.12 (s, 4H), 1.93 - 1.73 (m, 10H), 1.53 (m, 14H), 1.40 (s, 9H), 0.86 (s, 25H), 0.58 - 0.40 (m, 5H).

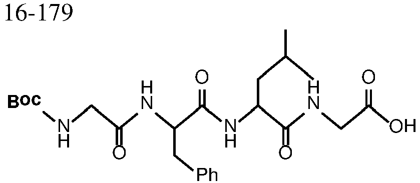

Boc-GFLG-OBn (home-made 2.5854 g, 4.4370 mmol) and 10% Pd/C (0.5 g) were added in a micro-reactor, DMF (30 mL) was then added to the micro-reactor, the air was pumped out to reach a vacuum state, H2 (16psi) was introduced, and such operations were repeated three times. The obtained solution was placed overnight at room temperature. At the end of the reaction, diatomaceous earth was used as a filter cake to carry out suction filtering, and then the reactor and the funnel were washed with DMF (200 mL×3) three times, and the solution was then transferred to a 250 mL flask as the raw material for Product 16-182.

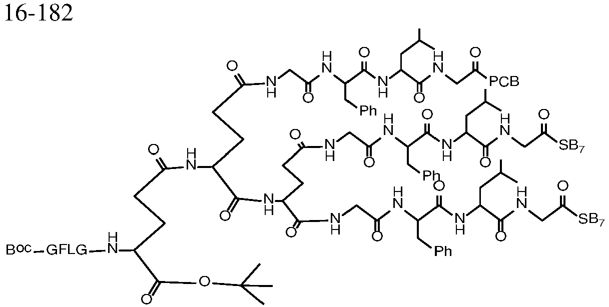

Product 16-178 (2.5 g, 0.8444 mmol), HBTU (0.4803 g, 1.2666 mmol) and HOBT (0.1711 g, 1.2666 mmol) were weighed and added to the flask with Product 16-179, and the reaction solution was placed in a low-temperature constant temperature reaction bath (-5 °C) and stirred for 20min, DIEA (0.6280 mL, 3.7998 mmol) was slowly added dropwise, and the mixed solution first reacted for 2 h and then taken out and placed at room temperature overnight on a magnetic stirrer. At the end of the reaction, methyl tert-butyl ether (100 mL) and n-hexane (150 mL) were added to the reaction solution, suction filtering was carried out, and the obtained solid was dissolved with methanol and dichloromethane; the silica gel powder was then added and the operations of evaporation, dry sample loading, column chromatography, and elution with an elutent (0.5% ammonia water: 5% methanol/dichloromethane) were carried out, thus obtaining 1.5 g of the pure product and 0.38 g of the impure product.

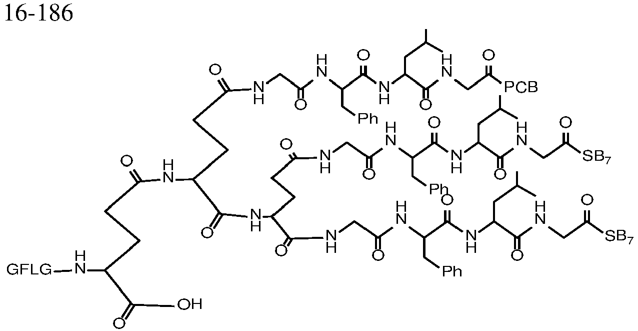

Product 16-182 (1.5 g, 0.4283 mmol) was added in a 250 mL flask and dissolved with dichloromethane (10 mL), TFA (0.96 mL, 12.8490 mmol) was then added to the solution, and the obtained solution was stirred overnight at room temperature. At the end of the reaction, the reaction solution was evaporated to dryness to remove the dichloromethane solvent, n-hexane (100 mL) was poured into the reaction solution, the obtained solution was cooled in the refrigerator for 20 min, and the supernatant was discarded; ethyl acetate (20 mL) was added to the lower solution and treated by ultrasonic, n-hexane (100 mL) was added for precipitation, and the product attached to the wall of the flask was oily; when the supernatant became clear and then was discarded; ethyl acetate (15 mL) and a large amount of n-hexane were added again in sequence to separate out a solid product; suction filtering was carried out; the solid was dissolved with a mixed solvent (20 % Methanol: 80% dichloromethane (50 mL)); silica gel powder was added; the operations of evaporation, dry loading, column chromatography, and elution with 1% ammonia water: 8% methanol/dichloromethane were carried out, and the elution was collected and subjected to rotary evaporation, thus obtaining 0.7 g of the product with a yield of 50%.

¹H-NMR (400 MHz, DMSO-*d*₆) δ 10.06 (s, 1H), 8.89 (s, 1H), 8.49 (m, 1H), 8.28 (s, 1H), 8.12 - 7.98 (m, 17H), 7.91 (s, 4H), 7.81 (s, 5H), 7.73 (s, 1H), 7.45 (s, 5H), 7.19 ― 7.04 (m, 37H), 4.61 (s, 1H), 4.49 (s, 4H), 4.28 (m, 3H), 4.22 - 4.07 (m, 9H), 3.94 (s, 3H), 3.82 (s, 3H), 3.70 (s, 3H), 3.57 (s, 19H), 3.13 - 3.03 (m, 2H), 2.96 (s, 3H), 2.81 (s, 2H), 2.68 (s, 5H), 2.35 (s, 3H), 2.24 (s, 9H), 2.11 (s, 6H), 1.76 (s, 9H), 1.46 (s, 16H), 1.29 - 1.14 (m, 6H), 0.89 ― 0.70 (m, 35H), 0.44 (s, 5H).

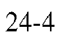

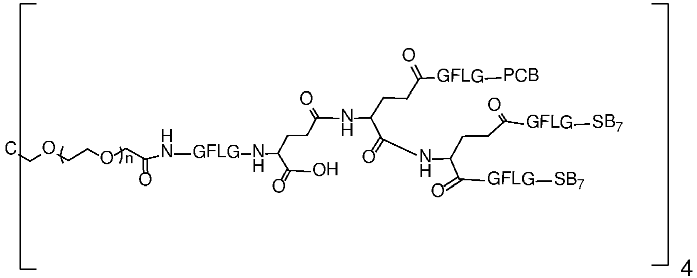

Product 16-186 (0.7 g, 0.2092 mmol) was weighed and then dissolved with DMF (40 mL), DIEA (0.6915 mL, 4.184 mmol) and 4ARM-SCM-40K (1.8285 g, 0.04358 mmol) were added in sequence, dichloromethane (30 mL) was added again, and the obtained solution reacted at a low speed stirring in the dark. 11 days later, the reaction ended, methyl tert-butyl ether (200 mL) and n-hexane (200 mL) were added to the reaction solution, the mixed solution was filtered by suction, and the filter cake was dissolved with a mixed solvent (20% methanol: 80% dichloromethane 30 mL); silica gel powder (9 g) was added, and then the operations of evaporation to dryness, dry sample loading and column chromatography were carried out. Gradient elution with 6% methanol-7% methanol/dichloromethane was carried out, thus obtaining 1.7 g of the product with a yield of 74%.

¹H-NMR (400 MHz, DMSO-*d*₆) δ 10.13 (s, 1H), 8.90-8.88 (m, 5H), 8.18 - 8.01 (m, 54H), 7.94 - 7.68 (m, 20H), 7.55 - 7.48 (m, 16H), 7.38-7.34 (m, 11H), 7.24 - 7.20 (m, 74H), 7.15-7.11 (m, 29H), 4.58-7.50 (m, 26H), 4.37 - 4.10 (m, 103H), 4.01 - 3.87 (m, 100H), 3.32 -3.28(m, 1479H), 2.89-2.84 (m, 15H), 2.79 - 2.63 (m, 33H), 2.42-2.41 (m, 11H), 2.02 - 1.95 (m, 19H), 1.526-1.50(m, 58H), 1.36-1.30 (m, 134H), 0.85 ― 0.75 (m, 127H), 0.50 (s, 20H).

MALDI-TOF MS: from 54744.54 to 54889.20

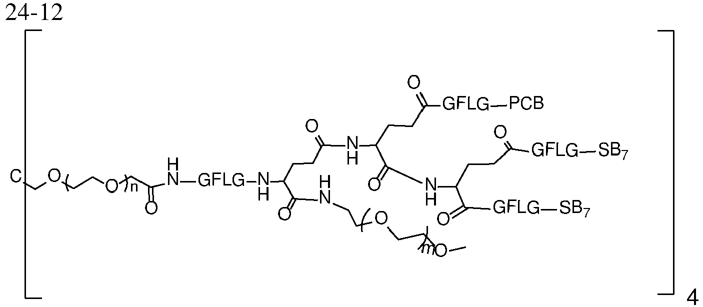

The reactants, Product 24-4 (1.7 g, 0.03098 mmol), M-NH₂-2K.HCl (0.5 g, 0.2478 mmol, purchased from JenKem), HBTU (0.0705 g, 0.18588 mmol), and HOBT (0.0251 g, 0.18588 mmol), were weighed and added in a 250 mL reaction flask and dissolved with DMF (40 mL); the obtained solution was stirred at -5 °C for 30min; DIEA (0.1331 mL, 0.80548 mmol) was slowly added dropwise and the obtained solution was stirred for 1 h and cooled to room temperature to react in the dark at a low speed stirring. (The reaction solution became turbid at a low temperature, and it became clear and transparent when treated by ultrasonic at room temperature.) At the end of the reaction, methyl tert-butyl ether (200 mL) and n-hexane (300 mL) were added to the reaction solution and ultrasnic treatment was carried out to obtain a homogeneous phase; the suction filtering was then carried out, and the product was dissolved with a mixed solvent (20% methanol: 80% dichloromethane 40 mL); silica gel powder was added, and then the operations of evaporation, dry sample loading and column chromatography were carried out. Gradient elution with 6%-7% methanol/dichloromethane was carried out, the pure product was collected and evaporated to dryness, absolute ethanol (10 mL) and dichloromethane (10 mL) were added for ultrasonic treatment, methyl tert-butyl ether was added to the obtained solution until a solid came out; suction filtering was carried out, and the obtained solid was further dissolved with absolute ethanol (10 mL) and dichloromethane (10 mL); the obtained solution was precipitated with methyl tert-butyl ether (100 mL); the process of dissolution and precipitation was repeated three times, thus obtaining 1 g of the product with a yield of 52%.

MALDI-TOF MS: from 62266.66 to 63113.03

¹H- NMR (400 MHz, DMSO-*d*₆) δ 10.13 (s, 4H), 8.96-8.94 (m, 5H), 8.29 - 7.73 (m, 98H), 7.59 - 7.40 (m, 26H), 7.13-7.10 (m, 159H), 4.56-4.52 (m, 24H), 4.17-4.08 (m, 101H), 3.30-3.35 (m, 1281H), 3.06 - 2.88 (m, 76H), 2.72-2.74 (m, 55H), 2.32-2.30 (m, 59H), 2.14-2.10 (m, 27H), 1.86-1.80 (m, 40H), 1.54-1.50 (m, 57H), 1.24-1.16 (m, 21H), 0.88-0.86 (m, 101H), 0.50 (s, 20H).

### Example 19: Synthesis of Compound 26-18

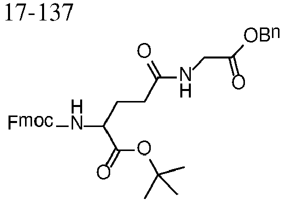

Fmoc-Glu-OtBu (9.3412 g, 21.9550 mmol), H-Gly-OBn.TsOH (8.0 g, 23.7114 mmol), HBTU (12,4893 g, 32.9325 mmol), and HOBT (4.4502 g, 32.9325 mmol) were added in a 500 mL flask and then dissolved with an appropriate amount of DMF (200 mL), the obtained solution was stirred at -5 °C for 30min, and then DIEA (19.9581 mL, 120.7525 mmol) was slowly added dropwise over 40min, and the obtained solution was further stirred at -5 °C for 1 h and then stirred at room temperature to react overnight. At the end of the reaction, the reaction solution was transferred to a 2 L separatory funnel and extracted with saturated sodium bicarbonate solution (300 mL) and ethyl acetate (200 mL) to obtain the organic phase; the aqueous phase was extracted with ethyl acetate (200 mL×3), and the obtained organic phases were combined and washed with saturated sodium chloride solution (150 mL×3); the obtained solution was then concentrated, evaporated to dryness, and dried in a vacuum oven, thus obtaining 12.5 g of Product 17-137 with a yield of 100%.

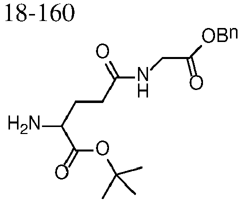

Product 17-137 (12.5 g, 21.9550 mmol) was added in a 250 mL flask and then dissolved with DMF (50 mL), morpholine (19.127 mL, 219.550 mmol) was then added to the obtained solution, and the mixed solution was stirred at room temperature to react for 1h. At the end of the reaction, the reaction solution was transferred to a 2 L separatory funnel and extracted with saturated sodium bicarbonate solution (300 mL) and ethyl acetate (200 mL) to obtain the organic phase; the aqueous phase was extracted with ethyl acetate (200 mL×3), and the obtained organic phases were combined, washed with saturated sodium chloride solution (150mLx 3), then concentrated and dried, the solid was dissolved with a methanol/dichloromethane (1:4) mixed solvent (200 mL), silica gel powder (30 g) was added to the obtained solution, and the solution was then evaporated to dryness to obtain a powdery solid; the operations of dry sample loading, column chromatography, and gradient elution with a dichloromethane mixed solution containing 1% of ammonia water and 2%-5% of methanol were carried out; the elution product was then collected, concentrated and evaporated to obtain a solid, and the solid was dried in a vacuum oven, thus obtaining 7.693 g of Product 18-160 with a yield of 91%.

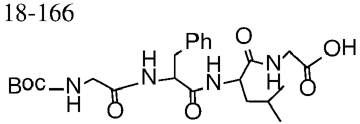

Boc-GFLG-OBn (8.7 g, 14.4840 mmol) and 10% Pd/C (0.2 g) were added into a hydrogenation reactor and then dissolved with DMF (30 mL); the hydrogenation reaction device was then sealed to perform the "three pumping and three charging" operation (i.e., pumping the air from the reaction system with a vacuum pump for about 3 minutes—charging hydrogen—pumping hydrogen—charging hydrogen—pumping hydrogen—charging hydrogen) so that the pressure on the hydrogenation reaction device was read as 0.18 MPa, and then the obtained solution reacted overnight at room temperature. At the end of the reaction, the reaction solution was filtered with diatomaceous earth, the filter cake was washed with DMF (20 mL×3), thus obtaining Product 18-166 with a yield of 100%.

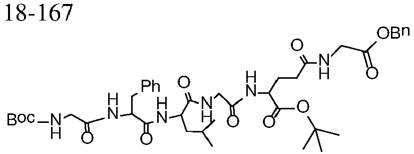

Product 18-160 (4.0 g, 11.4152 mmol), HBTU (6.4937 g, 17.1228 mmol) and HOBT (2.3138 g, 17.1228 mmol) were added in a 500 mL flask with Product 18-166 (7.1344 g,14.4840 mmol) and then dissolved with a proper amount of DMF (100 mL), and the mixed solution was stirred at -5 °C for 30min. DIEA (8.49 mL, 51.3684 mmol) was slowly added dropwise over 20min; the reaction solution was further stirred at -5 °C for 1h, and then stirred at room temperature to react overnight. At the end of the reaction, the reaction solution was transferred to a 2 L separatory funnel and extracted with saturated sodium bicarbonate solution (300 mL) and ethyl acetate (200 mL) to obtain the organic phase; the aqueous phase was extracted with ethyl acetate (200 mL×3), and the obtained organic phases were combined, washed with saturated sodium chloride solution (150 mLx 3), then concentrated and dried, the solid was dissolved with a methanol/dichloromethane (1:4) mixed solvent (100 mL), silica gel powder (30 g) was added to the obtained solution, and the solution was then evaporated to dryness to obtain a powdery solid; the operations of dry sample loading, column chromatography, and gradient elution with a dichloromethane mixed solution containing 2%-3% of methanol and 98%-97% of dichloromethane were carried out; the elution product was then collected, concentrated and evaporated to obtain a solid, and the solid was dried in a vacuum oven, thus obtaining 7.8 g of Product 18-167 with a yield of 83%.

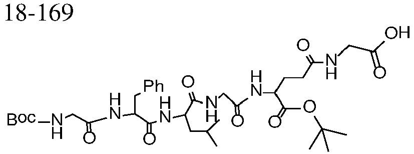

Product 18-167 (0.4559 g, 0.4677 mmol) and 10% Pd/C (0.1 g) were added into a hydrogenation reactor and then dissolved with DMF (30 mL); the hydrogenation reaction device was then sealed to perform the "three pumping and three charging" operation (i.e., pumping the air from the reaction system with a vacuum pump for about 3 minutes—charging hydrogen—pumping hydrogen—charging hydrogen—pumping hydrogen—charging hydrogen) so that the pressure on the hydrogenation reaction device was read as 0.18 MPa, and then the obtained solution reacted overnight at room temperature. At the end of the reaction, the reaction solution was filtered with diatomaceous earth, the filter cake was washed with DMF (20 mL×3), thus obtaining Product 18-169 with a yield of 100%.

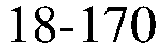

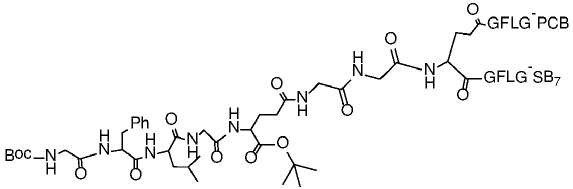

Product 17-102 (0.8 g, 0.4252 mmol), HBTU (0.2419 g, 0.6378 mmol) and HOBT (0.0862 g, 0.6378 mmol) were added in a 250 mL flask with Product 18-169 (0.3437 g, 0.4677 mmol) and then dissolved with a proper amount of DMF (100 mL), and the mixed solution was stirred at -5 °C for 30min. DIEA (0.3162 mL, 1.9133 mmol) was then slowly dropwise over 10min. The reaction solution was first stirred at -5 °C to react for 1h, and then stirred at room temperature to further react overnight. At the end of the reaction, n-hexane (100 mL) was added, the reaction solution was then shaken, and the supernatant was discarded; the above operations were repeated three times. Methyl tert-butyl ether (80 mL) and a small amount of n-hexane (10 mL) were then added, the obtained solution was then shaken, and the supernatant was discarded; the above operations were repeated three times, and the powdery solid in the reaction solution was separated out by precipitation; suction filtering was carried out, the filter cake was washed with methyl tert-butyl ether (40 mL × 3) and then collected, dried in a vacuum oven, thus obtaining 1.08 g of the product 18-170, with a yield of 100%.

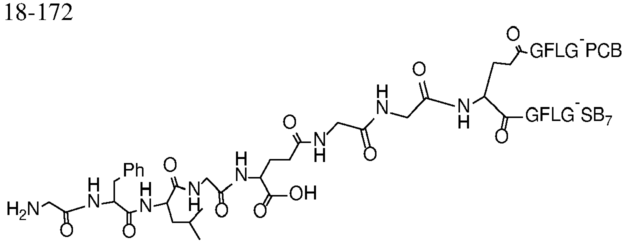

Product 18-170 (1.0805 g, 0.4252 mmol) was added in a 500 mL flask and then dissolved with dichloromethane (10 mL) and TFA (0.4736 mL, 6.3780 mmol), and the mixed solution was stirred at room temperature to react overnight. At the end of the reaction, the reaction solution was transferred to a 1 L separatory funnel, saturated sodium bicarbonate (300 mL) was added to adjust the pH to alkaline, and the obtained solution was then extracted with ethyl acetate (200 mL) to obtain the organic phase; the aqueous phase was extracted with ethyl acetate (200 mL×3), and the obtained organic phases were combined, washed with saturated sodium chloride solution (150 mLx 3), then concentrated and dried, the solid was dissolved with a methanol/dichloromethane (1:4) mixed solvent (100 mL), silica gel powder (10 g) was added to the obtained solution, and the solution was then evaporated to dryness; the operations of dry sample loading, column chromatography, and gradient elution with a dichloromethane mixed solution containing 1% of ammonia water and 3%-10% of methanol were carried out; the elution product was then collected, concentrated and dried in a vacuum oven, thus obtaining 1.014 g of Product 18-172 with a yield of 87%.

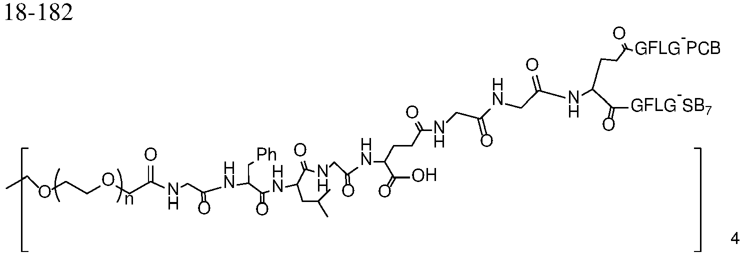

Product 18-172 (0.5457 g, 0.2288 mmol) was added in a 250 mL flask and dissolved with DMF (20 mL), the reaction solution was stirred at -5 °C for 30min, and DIEA (0.1733 mL, 1.0487 mmol) was then slowly added dropwise over 5min. The obtained solution was further stirred for 30min, 4ARM-SCM-40K (2.0 g, 0.0476 mmol, purchased from JenKem) was added and then dissolved with dichloromethane (10 mL), and the reaction solution was further stirred for 20 min at -5 °C; and then the reaction solution was stirred in the dark at a low speed at room temperature to react for one week. At the end of the reaction, the dichloromethane in the reaction solution was evaporated out, and methyl tert-butyl ether (20 mL) was then added to separate a solid out; suction filtering was then carried out, the filter cake was washed with methyl tert-butyl ether (40 mL×3) and then dissolved with a methanol/dichloromethane (1:4) mixed solvent (100 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 8%-9% of methanol were carried out; the elution product was then collected, concentrated, evaporated to obtain a solid, and the solid was dried in a vacuum oven, thus obtaining 1.6184 g of Product 18-182 with a yield of 77%.

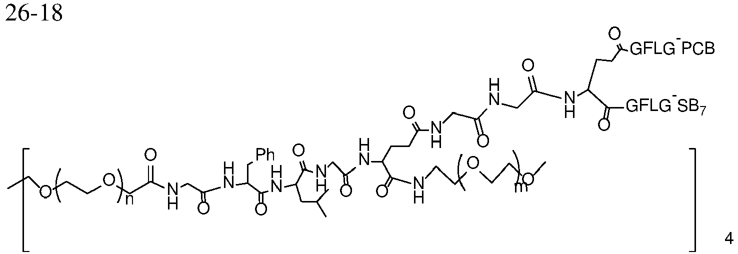

Product 18-182 (0.9 g, 0.0176 mmol), M-NH₂-2K.HCl (0.2151 g, 0.1056 mmol, purchased from JenKem), HBTU (0.0467 g, 0.1056 mmol) and HOBT (0.0166 g, 0.1056 mmol) were added in a 250 mL flask and then dissolved with DMF (50 mL), and the mixed solution was stirred at -5 °C for 30min. DIEA (0.08 mL, 0.4229 mmol) was then slowly dropwise over 5min. The reaction solution was first stirred at -5 °C to react for 1h, and then stirred at room temperature to further react overnight. At the end of the reaction, n-hexane (100 mL) and methyl tert-butyl ether (30 mL) were added, the reaction solution was then shaken, and the supernatant was discarded; the above operations were repeated three times. Methyl tert-butyl ether (100 mL) and a small amount of n-hexane (10 mL) were then added, the obtained solution was then shaken to separate out a solid; the obtained solution was filtered by suction, the resulting filter cake was washed with methyl tert-butyl ether (40 mLx3) and dissolved with a methanol/dichloromethane (1: 4) mixed solvent (100 mL), silica gel powder (10 g) was added to the obtained solution, and the solution was then evaporated to dryness to obtain a powdery solid; the operations of dry sample loading, column chromatography, and gradient elution with a dichloromethane mixed solution containing 1% of ammonia water and 5% of methanol were carried out; the elution product was then collected, concentrated, and evaporated into a solid, and the solid was dried in a vacuum oven, thus obtaining a crude product; the crude product was dissolved with absolute ethanol (15 mL) and dichloromethane (5 mL), and methyl tert-butyl ether (100 mLx3) was added to the obtained solution to separate out a powdery solid; the suction filtering was carried out and the filter cake was washed with methyl tert-butyl ether (40 mL×3) and dried in the vacuum oven, thus obtaining 0.5559 g of Product 26-18 with a yield of 57%.

### Example 20: Synthesis of Compound 22-207

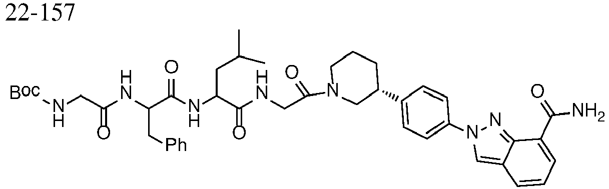

Boc-GFLG-OH (9.99 g, 20.2878 mmol), Niraparib (5 g, 15.606 mmol, referred to as NPB), HBTU (8.88 g, 23.409 mmol), and HOBT (3.16 g, 23.409 mmol) were added in a 500 mL flask and dissolved with DMF (20 mL); the obtained solution was stirred at -5 °C to react for 30min, and DIEA (11.61 mL, 70.2269 mmol) was then slowly added dropwise; the obtained solution further reacted at -5 °C for 1h, and then stirred at room temperature to react overnight. At the end of the reaction, the reaction solution was transferred to a 1 L separatory funnel, saturated NaHCO₃ (300 mL) and ethyl acetate (200 mL) were then added, and the obtained solution was shaken and allowed to stand still. The upper organic phase was collected, the lower aqueous phase was extracted with ethyl acetate (150 mL×3), and the organic phases were collected and combined, washed with saturated sodium chloride solution (100 mL×2), concentrated and evaporated to dryness, and dried in a vacuum oven, thus obtaining 17.6 g of Product 22-157 with a yield of 100%.

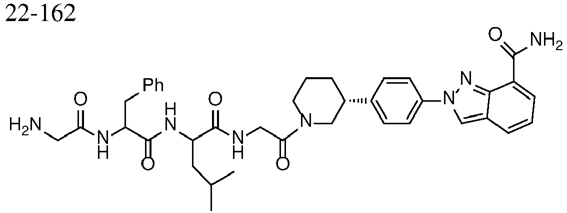

Product 22-157 (12.41 g, 15.606 mmol) was added in a 500 mL flask and then dissolved with dichloromethane (20 mL), TFA (17.4 mL, 234.09 mmol) was then added with stirring, and the mixed solution was stirred at room temperature to react overnight. At the end of the reaction, the dichloromethane in the reaction solution was evaporated in vacuum, and methyl tert-butyl ether (200 mL) was then added to separate out a solid; suction filtering was then carried out, the filter cake was washed with methyl tert-butyl ether (40 mL×3) and then dissolved with a methanol/dichloromethane (1:4) mixed solvent (100 mL), silica gel powder (50 g) was added to the obtained solution, and the operations of dry sample loading, column chromatography, and gradient elution with an eluent (0-1% ammonia water: 6%-8% methanol: 94%-91% dichloromethane) were carried out; the elution solution was then collected and concentrated, thus obtaining 10.8 g of Product 22-162 with a yield of 99%.

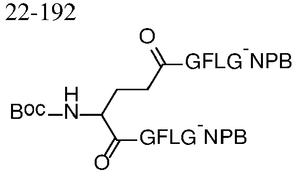

Product 22-162 (2.6 g, 3.742 mmol), Boc-Glu-OH (0.42 g, 1.7009 mmol), HBTU (1.93 g, 5.1027 mmol), and HOBT (0.7 g, 5.1027 mmol) were added in a 500 mL flask and dissolved with DMF (20 mL); the obtained solution was stirred at -5 °C to react for 30min, and DIEA (2.5 mL, 15.3081 mmol) was then slowly added dropwise; the obtained solution further reacted at -5 °C for 1h, and then stirred at room temperature to react overnight. At the end of the reaction, n-hexane (100 mL) was added to the reaction solution, the obtained solution was then shaken, the supernatant was discarded, and the above operation was repeated three times. After dissolution with dichloromethane (8 mL), methyl tert-butyl ether (100 mL) was then added to separate out a solid, suction filtering was carried out, the filter cake was washed with methyl tert-butyl ether (40 mL x3), the solid was collected and dried in a vacuum oven, thus obtaining 3.2106 g of Product 22-192 with a yield of 100 %.

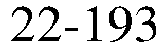

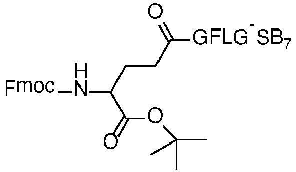

Fmoc-Glu-(OtBu)(OH) (1.8 g, 4.3074 mmol), GFLF-SB7 (3.2 g, 3.5895 mmol), HBTU (2.1 g, 5.3842 mmol), and HOBT (0.7 g, 5.3842 mmol) were added in a 500 mL flask and dissolved with DMF (20 mL); the obtained solution was stirred at -5 °C to react for 30min, and DIEA (2.7 mL, 16.1647 mmol) was then slowly added dropwise; the obtained solution further reacted at -5 °C for 1h, and was then stirred at room temperature to react overnight. At the end of the reaction, n-hexane (100 mL) was added to the reaction solution, the obtained solution was then shaken, the supernatant was discarded, and the above operation was repeated three times. After dissolution with dichloromethane (6 mL), methyl tert-butyl ether (100 mL) was then added to separate out a solid, suction filtering was carried out, the filter cake was washed with methyl tert-butyl ether (40 mL x3), the solid was collected and dried in a vacuum oven, thus obtaining 5.5 g of Product 22-193 with a yield of 100 %.

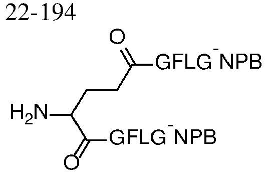

Product 22-192 (2.73 g, 1.7009 mmol) was added in a 250 mL flask and then dissolved with dichloromethane (20 mL), TFA (1.89 mL, 25.5135 mmol) was then added with stirring, and the mixed solution was stirred at room temperature to react overnight. At the end of the reaction, the dichloromethane in the reaction solution was evaporated in vacuum, and methyl tert-butyl ether (200 mL) was then added to separate out a solid; suction filtering was then carried out, the filter cake was washed with methyl tert-butyl ether (40 mL×3) and then dissolved with a methanol/dichloromethane (1:4) mixed solvent (100 mL), silica gel powder (10 g) was added to the obtained solution, and the operations of dry sample loading, column chromatography, and gradient elution with an eluent (1% ammonia water: 4%-6% methanol: 95%-93% dichloromethane) were carried out; the elution solution was then collected and concentrated, thus obtaining 2.246 g of Product 22-194 with a yield of 88%.

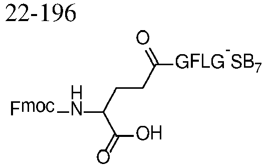

Product 22-193 (4.66 g, 3.5895 mmol) was added in a 250 mL flask and then dissolved with dichloromethane (20 mL), TFA (4 mL, 53.8425 mmol) was then added with stirring, and the mixed solution was stirred at room temperature to react overnight. At the end of the reaction, the dichloromethane in the reaction solution was evaporated in vacuum, and methyl tert-butyl ether (200 mL) was then added to separate out a solid; suction filtering was then carried out, the filter cake was washed with methyl tert-butyl ether (40 mL×3) and then dissolved with a methanol/dichloromethane (1:4) mixed solvent (100 mL), silica gel powder (15 g) was added to the obtained solution, and the operations of dry sample loading, column chromatography, and gradient elution with an eluent (1% ammonia water: 5%-8% methanol: 94%-91% dichloromethane) were carried out; the elution solution was then collected and concentrated, thus obtaining 4 g of Product 22-196 with a yield of 89.7%.

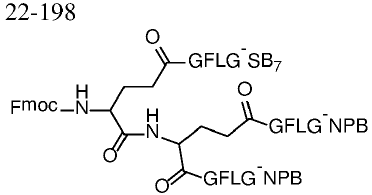

Product 22-194 (0.35 g, 0.2322 mmol), Product 22-196 (0.35 g, 0.2774 mmol), HBTU (0.13 g, 0.3467 mmol), and HOBT (0.05 g, 0.3467 mmol) were added in a 250 mL flask and dissolved with DMF (15 mL); the obtained solution was stirred at -5 °C to react for 30min, and DIEA (0.17 mL, 1.0402 mmol) was then slowly added dropwise; the obtained solution further reacted at -5 °C for 1h, and then stirred at room temperature to react overnight. At the end of the reaction, n-hexane (100 mL) was added to the reaction solution, the obtained solution was then shaken, the supernatant was discarded, and the above operation was repeated three times. After dissolution with dichloromethane (6 mL), methyl tert-butyl ether (100 mL) was then added to separate out a solid, suction filtering was carried out, the filter cake was washed with methyl tert-butyl ether (40 mL x3), and the solid was collected and dried in a vacuum oven, thus obtaining 1.3 g of Product 22-198 with a yield of 100 %.

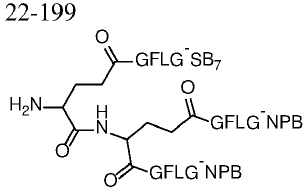

Product 22-198 (0.63 g, 0.2322 mmol) was added in a 250 mL flask and then dissolved with DMF (10 mL), morpholine (0.4 g, 4.6432 mmol) was then added to the obtained solution, and the mixed solution was stirred at room temperature to react for 1h. At the end of the reaction, n-hexane (100 mL) was added, the reaction solution was then shaken, and the supernatant was discarded; the above operations were repeated three times. Methyl tert-butyl ether (100 mL) was then added to separate out a solid; suction filtering was then carried out, the filter cake was washed with methyl tert-butyl ether (40 mL×3) and then dissolved with a methanol/dichloromethane (1:4) mixed solvent (100 mL), silica gel powder (15 g) was added to the obtained solution, and the operations of dry sample loading, column chromatography, and gradient elution with an eluent (1% ammonia water: 6%-8% methanol: 93%-91% dichloromethane) were carried out; the elution solution was then collected and concentrated, thus obtaining 0.49 g of Product 22-199 with a yield of 85%.

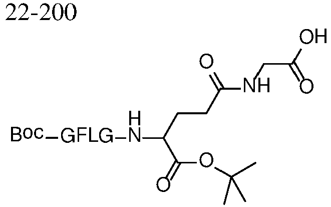

Product 22-177 (0.19 g, 0.2253 mmol, i.e., Product 18-167) and 10% Pd/C (0.1 g) were added into a hydrogenation reactor and then dissolved with DMF (30 mL) where the level of the solvent was above a stirrer; the hydrogenation reaction device was then sealed to perform the "three pumping and three charging" operation (i.e., pumping the air from the reaction system with a vacuum pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth, and suction filtering was carried out. The reaction device was washed with DMF (20 mL×3) until the reaction device did not contain any product, and then the product 22-200 was obtained.

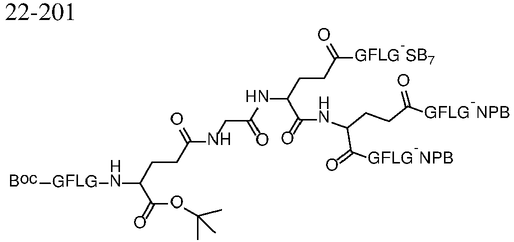

Product 22-199 (0.47 g, 0.1878 mmol), Product 22-200 (0.17 g, 0.2253 mmol), HBTU (0.11 g, 0.2817 mmol), and HOBT (0.04 g, 0.2817 mmol) were added in a 250 mL flask and dissolved with DMF (60 mL); the obtained solution was stirred at -5 °C to react for 30min, and DIEA (0.14 mL, 0.8451 mmol) was then slowly added dropwise; the obtained solution further reacted at -5 °C for 1h, and then stirred at room temperature to react overnight. At the end of the reaction, n-hexane (100 mL) was added to the reaction solution, the obtained solution was then shaken, the supernatant was discarded, and the above operation was repeated three times. After dissolution with dichloromethane (6 mL), methyl tert-butyl ether (100 mL) was then added to separate out a solid, suction filtering was carried out, the filter cake was washed with methyl tert-butyl ether (40 mL x3), the solid was collected and dried in a vacuum oven, thus obtaining 1 g of Product 22-201 with a yield of 100 %.

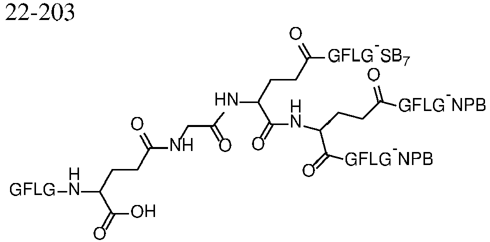

Product 22-201 (0.6 g, 0.1878 mmol) was added in a 250 mL flask and then dissolved with dichloromethane (10 mL), TFA (0.42 mL, 5.634 mmol) was then added to the obtained solution, and the mixed solution was stirred at room temperature to react overnight. At the end of the reaction, the dichloromethane in the reaction solution was evaporated in vacuum, and methyl tert-butyl ether (100 mL) was then added for precipitation and a solid was separated out; the filter cake was dissolved with a methanol/dichloromethane (1:4) mixed solvent, silica gel powder (10 g) was added to the obtained solution, and the solution was then evaporated to dryness; the operations of dry sample loading, column chromatography, and gradient elution with an eluent (1% ammonia water: 3%-9% methanol: 96%-90% dichloromethane) were carried out; the filtrate was then collected, concentrated and evaporated to dryness, thus obtaining 0.437 g of Product 22-203 with a yield of 76%.

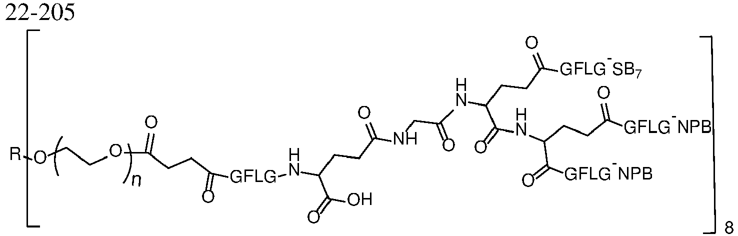

Product 22-203 (0.417 g, 0.1361 mmol) was added to a 250 mL flask and then dissolved with DMF (20 mL), the obtained solution was stirred at -5 °C to react 30min, and DIEA (0.02 mL, 0.1237 mmol) was then slowly added dropwise; then, 8ARM-SS-40K (0.68 g) was added and dissolved with dichloromethane (10 mL), and the reaction solution was stirred in the dark at room temperature at a low speed to react for one week. At the end of the reaction, n-hexane (100 mL) was added, the reaction solution was then shaken, and the supernatant was discarded; the above operations were repeated three times. Methyl tert-butyl ether (100 mL) was then added to separate out a solid; suction filtering was then carried out, the filter cake was washed with methyl tert-butyl ether (40 mL×3) and then dissolved with a methanol/dichloromethane (1:4) mixed solvent (100 mL), silica gel powder (25 g) was added to the obtained solution, and the operations of dry sample loading, column chromatography, and gradient elution with an eluent (1% ammonia water: 3%-12% methanol: 96%-87% dichloromethane) were carried out; the filtrate was then collected, concentrated and evaporated to dryness, thus obtaining 0.918 g of Product 22-205 with a yield of 88%.

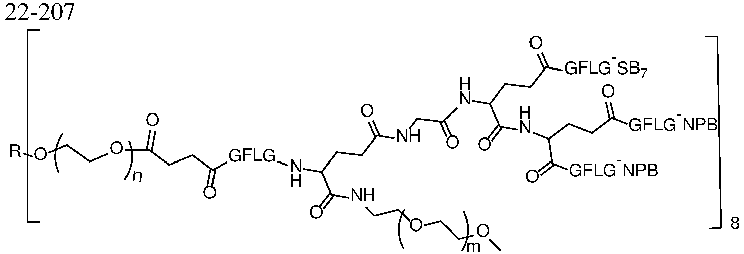

Product 22-205 (0.898 g, 0.01331 mmol), M-NH₂.HCl-2K (0.27 g, 0.1331 mmol), HBTU (0.06 g, 0.1597 mmol), and HOBT (0.022 g, 0.1597 mmol) were added in a 250 mL flask and dissolved with DMF (20 mL); the obtained solution was stirred at -5 °C to react for 30min, and DIEA (0.097 mL, 0.5855 mmol) was then slowly added dropwise; the obtained solution further reacted at -5 °C for 1h, and then stirred at room temperature to react overnight. At the end of the reaction, n-hexane (100 mL) was added, the reaction solution was then shaken, and the supernatant was discarded; the above operations were repeated three times. Methyl tert-butyl ether (100 mL) was then added to separate out a solid; suction filtering was then carried out, the filter cake was washed with methyl tert-butyl ether (40 mL×3) and then dissolved with a methanol/dichloromethane (1:4) mixed solvent (100 mL), silica gel powder (25 g) was added to the obtained solution, and the operations of dry sample loading, column chromatography, and gradient elution with an eluent (1% ammonia water: 2%-10% methanol: 97%-89% dichloromethane) were carried out; the filtrate was then collected, concentrated and evaporated to dryness, thus obtaining 1 g of Product 22-207 with a yield of 90%.

### Example 21: Synthesis of control Compound 13-141

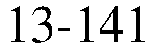

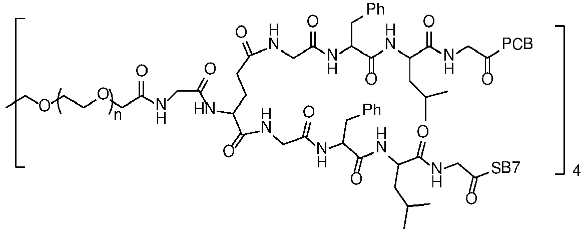

Product 13-140 (0.5953 g, 0.3164 mmol, for its structure and synthesis process, see the synthesis of Compound L-10 in Example M-10 of PCT International Patent Application PCT/CN2018/073662, it was prepared by replacing Boc-GLG-OBn with Boc-GFLG-OBn) was added in a 250 mL flask and then dissolved with dichloromethane (20 mL) and DMF (10 mL); 4ARM-SCM-40K (3.0 g, 0.0678 mmol, purchased from JenKem) was then added, and the obtained solution was then stirred in the dark at room temperature at a low speed to react for one week. At the end of the reaction, the reaction solution was concentrated, methyl tert-butyl ether (150 mL) was added to the solution to separate out a solid, and the filtering was carried out to obtain a crude product; the crude product was dissolved with dichloromethane (80 mL) and methanol (20 mL); silica gel powder was added, and the solution was then evaporated to dryness to obtain a powdery solid; the operations of dry sample loading, column chromatography, and elution with a dichloromethane mixed solution containing 1% of ammonia water and 8% of methanol were carried out; the product was then collected, concentrated, and evaporated to dryness, thus obtaining a crude product; the crude product was dried in a vacuum oven for 2 h and then dissolved with absolute ethanol (10 mL) and dichloromethane (5 mL), and n-hexane (150 mL) was added to separate out a solid; then, suction filtering was carried out, the filter cake was washed with n-hexane (100 mL×4) and dried in a vacuum oven, thus obtaining 2.8 g of Product 13-141 with a yield of 80%. ¹H-NMR (400 MHz, DMSO-d₆) δ 10.13 (s, 3H), 8.96 (s, 2H), 8.34 - 7.77 (m, 50H), 7.65 - 7.41 (m, 8H), 7.27-7.15 (m, 79H), 5.87 - 5.56 (m, 3H), 4.75 - 3.91 (m, 31H), 3.51 (s, 3526H), 3.14 (s, 22H), 3.01 (d, *J* = 11.7 Hz, 18H), 2.83 - 2.66 (m, 19H), 2.42 (s, 15H), 2.12 (s, 15H), 1.98-1.39 (m, 61H), 1.23 (s, 5H), 0.98 - 0.84 (m, 46H), 0.51 (s, 7H). MALDI-TOF MS: from 50407.96 to 50723.18.

### Example 22: Synthesis of control Compound 24-6

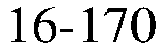

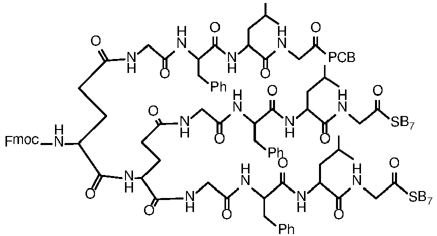

Reactants, i.e., Product 16-162 (4.7817 g, 2.5246 mmol), Product 19-71 (3 g, 2.5246 mmol), HBTU (1.4248 g, 3.7869 mmol), and HOBT (0.5117 g, 3.7869 mmol) were added in a 500 mL reaction flask and dissolved with DMF (150 mL), and then the obtained solution was placed in a low-temperature constant temperature bath (-5 °C) and stirred for 30min. DIEA (1.9 mL, 11.3607 mmol) was then added dropwise, and the mixed solution first reacted at a low temperature for 2 h and then reacted at room temperature. At the end of the reaction, deionized water (300 mL) was directly added to the reaction solution, the solid product was separated out by precipitation, suction filtering was carried out, and ethyl acetate (300 mL) was added to the solid, and ultrasonic treatment was carried out to obtain a homogeneous phase; n-hexane (200 mL) was then added for precipitation, and suction filtering and drying were carried out, thus obtaining 8 g of the product, 0.3 g being extra-quota product.

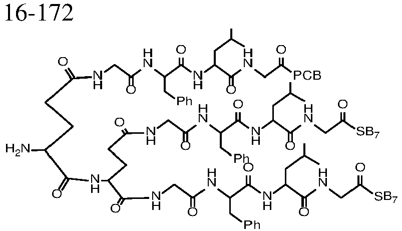

Reactant Product 16-170 (7.7 g, 2.5246 mmol) was added in a 500 mL reaction flask and dissolved with DMF (50 mL), morpholine (6.6 mL, 75.738 mmol) was then added, the obtained solution was stirred to react and TLC was carried out every 0.5h. One hour later, the reaction ended, methyl tert-butyl ether (100 mL) was added to the reaction solution and a solid was separated out by precipitation, suction filtering was carried out, and ethyl acetate (100 mL) was added to the solid and ultrasonic treatment was carried out to obtain a homogeneous phase; n-hexane (50 mL) was then added, and suction filtering and drying were carried out, thus obtaining 6.4 g of the final product with a yield of 88%.

¹H-NMR (400 MHz, DMSO-*d*₆) δ 10.12 (s, 1H), 8.96 (s, 1H), 8.25 (s, 1H), 8.15 (dd, *J* = 15.9, 7.8 Hz, 4H), 8.10 - 8.01 (m, 10H), 7.95 (s, 2H), 7.92 - 7.86 (m, 3H), 7.80 (s, 1H), 7.57 - 7.47 (m, 5H), 7.28 - 7.12 (m, 31H), 4.54 (ddd, J = 14.5, 10.3, 6.0 Hz, 4H), 4.29 - 4.11 (m, 6H), 3.89 (d, *J* = 14.7 Hz, 3H), 3.63 (t, *J* = 9.0 Hz, 8H), 3.21 - 3.13 (m, 6H), 3.09 - 2.97 (m, 5H), 2.89 (s, 8H), 2.73 (s, 8H), 2.42 (s, 4H), 2.31 (s, 9H), 2.19 - 2.09 (m, 4H), 1.99 (s, 3H), 1.85 (dd, *J* = 35.7, 19.3 Hz, 10H), 1.54 (ddt, *J* = 47.0, 14.1, 6.7 Hz, 15H), 1.33 (s, 2H), 1.17 (d, *J* = 7.1 Hz, 4H), 0.94 - 0.78 (m, 25H), 0.51 (s, 5H).

MALDI-TOF MS: [M]2839.75, [M+Na⁺]2862.85.

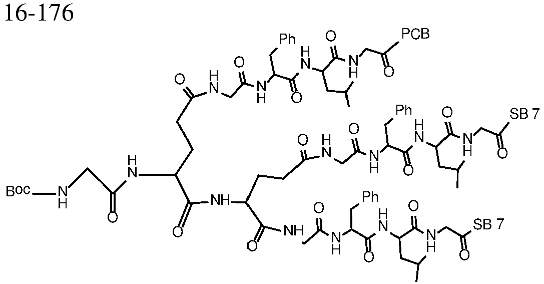

Reactants, i.e., Product 16-172 (4 g, 1.4074 mmol), Boc-Gly-OH (0.2959 g, 1.6889 mmol), HBTU (0.8006 g, 2.1111 mmol), and HOBT (0.2853 g, 2.1111 mmol) were added in a 500 mL reaction flask and dissolved with DMF (50 mL), and then the obtained solution was placed in a low-temperature constant temperature bath (-5 °C) and stirred for 30min; DIEA (1.1 mL, 6.3333 mmol) was added dropwise; the obtained solution first reacted at a low temperature reaction for 2h, and then reacted at room temperature. At the end of the reaction, methyl tert-butyl ether (200 mL) was added to the reaction solution, the obtained solution was allowed to stand still for 1 h in the refrigerator, the supernatant was discarded, and a small amount of ethyl acetate was added for ultrasonic treatment; n-hexane (200 mL) was then added, the obtained solution was allowed to stand still for 30min, and the supernatant was discarded; then, ethyl acetate (20 mL) and n-hexane (200 mL) were added again; suction filtering and drying were carried out, thus obtaining 6.6 g of the product, 1.7 g being extra-quota product.

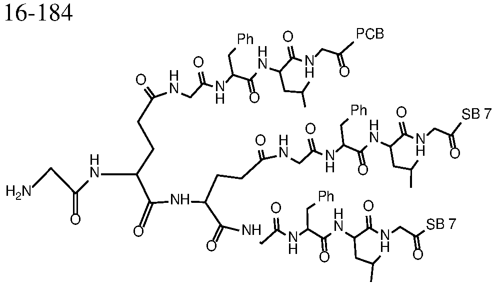

Product 16-176 (theoretically, 4.9 g, 1.6537 mmol) was added in a 500 mL flask and dissolved with dichloromethane (20 mL), TFA (3.6849 mL, 49.611 mmol) was then added to the solution, and the obtained solution was stirred overnight at room temperature. At the end of the reaction, n-hexane (200 mL) was added to the reaction solution and allowed to stand still in the refrigerator for 20min, and the supernatant was discarded; a small amount of ethyl acetate was added to exactly dissolve the oily product; n-hexane (200 mL) was added and the supernatant was discarded, and at this time, the oily product was obviously decreased; ethyl acetate (20 mL) was added again, n-hexane (300 mL) was added to separate out a solid product; suction filtering was carried out, a mixed solvent of methanol and dichloromethane was added to dissolve the solid product; silica gel powder was added, and the operations of evaporation, dry sample loading, and column chromatography were carried out. Elution with 1% ammonia water/5% methanol/dichloromethane was carried out, thus obtaining 2.1 g of the pure product and 3.2 g of product with some impurities.

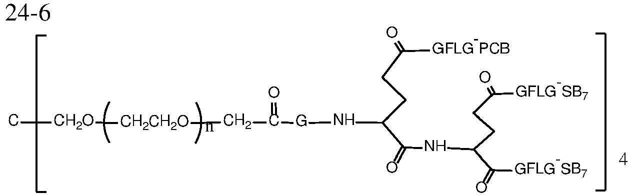

Reactant Product 16-184 (1.05 g, 0.3622 mmol) was added in the reaction flask and dissolved with DMF (50 mL), 4ARM-SCM-40k (3.1662 g, 0.07546 mmol) was added and then dissolved with dichloromethane (70 mL), a ground glass stopper was used, and the obtained solution reacted at a low speed stirring in the dark. At the end of the reaction, the dichloromethane was first removed with a rotary evaporator, methyl tert-butyl ether (400 mL) was then added, and the solution became turbid; n-hexane (300 mL) was then added for suction filtering; the obtained solid was dissolved with a mixed solvent of methanol and dichloromethane, and silica gel powder was added; the operations of evaporation, sample loading, and column chromatography were carried out. Gradient elution with 4%-7% methanol/chloromethane was carried out, the pure product was collected and evaporated to dryness, 20 mL of a mixed solvent of absolute ethanol and dichloromethane (1:1) was added for ultrasonic treatment, methyl tert-butyl ether was added to the obtained solution and a solid was separated out by precipitation; suction filtering was carried out, and the obtained solid was further dissolved with absolute ethanol and dichloromethane; the obtained solution was precipitated with methyl tert-butyl ether; the process of dissolution and precipitation was repeated three times, thus obtaining 3.3 g of the product with a yield of 82%.

### Example 23: Synthesis of control Compound 18-158

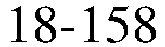

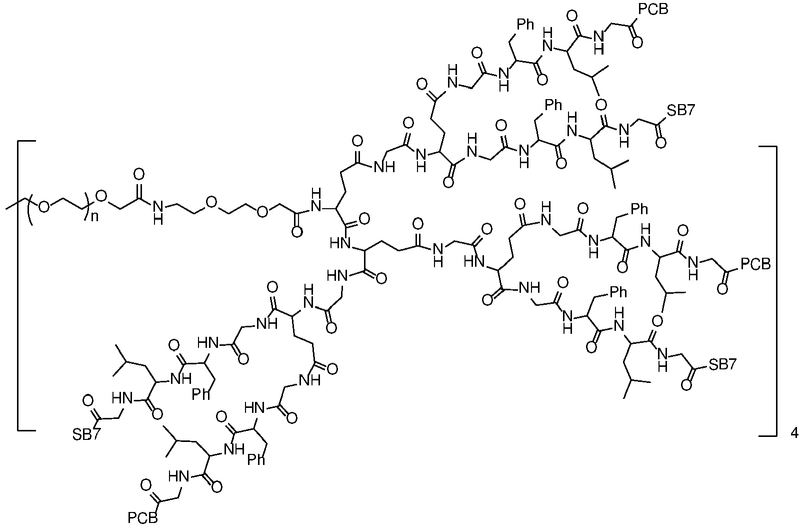

Product 18-153 (0.6753 g, 0.1123 mmol) was added in a 250 mL flask and then dissolved with dichloromethane (5 mL) and DMF (30 mL), M-SCM-40K (0.9818 g, 0.0234 mmol) was then added, and the mixed solution was stirred in the dark at room temperature to react. At the end of the reaction, dichloromethane in the reaction solution was evaporated up, about 30 mL of methyl tert-butyl ether was added to the solution to separate out a solid, and the filtering was carried out to obtain a crude product; the crude product was dissolved with dichloromethane and methanol; silica gel powder was added; the operations of evaporation, dry sample loading, column chromatography, and elution with an eluent (1% ammonia water: 12 % methanol: 87% dichloromethane) were carried out; the product was then collected, concentrated, and evaporated to dryness, thus obtaining Product 18-158; the product was dissolved with absolute ethanol (20 mL) and dichloromethane (5 mL), and methyl tert-butyl ether (50 mL) was added to separate out a solid product; then, suction filtering was carried out, and the filter cake was washed with methyl tert-butyl ether (50 mL×3) and dried, thus obtaining 1.0 g of Product 18-158 with a yield of 65%.

### Example 24: Synthesis of Compound 11-180

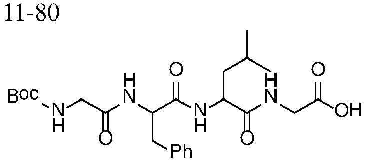

The raw material Boc-GFLG-OBn (5.9147 g, 10.1508 mmol) and 10% Pd/C catalyst (150mg) were added into a hydrogenation reactor and then dissolved with DMF (45 mL) where the level of the solvent was above a stirrer; the hydrogenation reaction device was then sealed to perform the "three pumping and three charging" operation (i.e., pumping the air from the reaction system with a vacuum pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (30 mL×3) until the reaction device did not contain any product, and then the reaction product 11-80 was obtained.

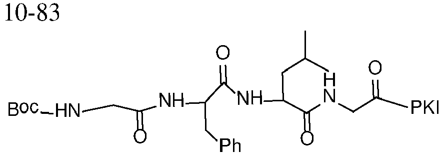

Product 11-80 (5 g, 10.1508 mmol), PKI (4.4792, 7.8083 mmol, i.e., allosteric PKI-587), HBTU (1.5827 g, 11.7125 mmol) and HOBT (4.4418 g, 11.7125 mmol) were added in a 500 mL round-bottomed flask and then dissolved with DMF (90 mL), and the mixed solution was stirred at -5 °C for 30min. Then DIEA (5.8075 mL, 35.1373 mmol) was slowly added dropwise, and then the mixed solution reacted at a low temperature for 2h; after that, the reaction device was placed at room temperature and the reaction solution was stirred overnight to react. At the end of the reaction, the reaction solution was transferred to a 1 L separatory funnel, saturated sodium bicarbonate solution (200 mL) was then added, the obtained solution was extracted three times with ethyl acetate (200 mL×3), and the obtained organic phases were combined and washed with saturated sodium bicarbonate (100 mL) once, and the saturated sodium chloride solution (100 mL) was added to remove water; the organic phase was dried with anhydrous sodium sulfate and filtered by suction. The filtrate was concentrated and dried in a vacuum oven, thus obtaining 9.6 g of Product 11-83 with a yield of 100% for the next reaction.

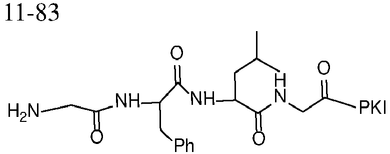

Product 11-81 (9.6 g, 7.8083 mmol) was added in a 500 mL round-bottomed flask and then dissolved with dichloromethane (100 mL), and then TFA (17 mL, 234.249 mmol) was added and the obtained solution was stirred at room temperature to react overnight. At the end of the reaction, the reaction solution was concentrated and evaporated to dryness under reduced pressure, and the obtained dry product was then dissolved with an appropriate amount of ethyl acetate and transferred to a 500 mL separatory funnel; the saturated sodium bicarbonate solution (100 mL) was added to neutralize the remaining TFA, the organic phase was then separated, the product in the aqueous phase was extracted three times with ethyl acetate (150 mL×3), and the obtained organic phases were combined, then dried with anhydrous sodium sulfate, filtered by suction, and concentrated; the operations of dry sample loading, column chromatography, and gradient elution with 6% methanol-10% methanol/1% ammonia water/dichloromethane were carried out, and then the elution product was then collected, concentrated, and dried in a vacuum oven, thus obtaining 5.7 g of the product 11-83 with a yield of 77%.

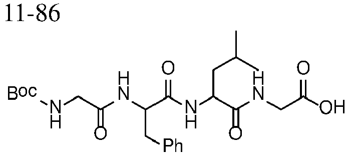

The raw material Boc-GFLG-OBn (12 g, 20.5941 mmol) and 10% Pd/C catalyst (200 mg) were added into a hydrogenation reactor and then dissolved with DMF (100 mL) where the level of the solvent was above a stirrer; the hydrogenation reaction device was then sealed to perform the "three pumping and three charging" operation (i.e., pumping the air from the reaction system with a vacuum pump for about 3 minutes—charging hydrogen—pumping hydrogen—charging hydrogen—pumping hydrogen—charging hydrogen) so that the pressure on the hydrogenation reaction device was read as 18 Psi, and then the obtained solution reacted overnight at room temperature. On the second day, after the reaction was found to be completed from the monitoring of the TLC (thin-layer chromatography), workup procedures were performed. The reaction solution was taken out and evenly added dropwise to a suction funnel filled with compacted diatomaceous earth. The reaction device was washed with DMF (30 mL×3) until the reaction device did not contain any product, and then the reaction product 11-86 was obtained.

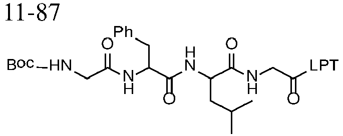

Product 11-86 (20.5941 mmol), Lapatinib (referred as to LPT, 9.5730 g, 16.4752 mmol), HBTU (9.3721 g, 24.7129 mmol) and HOBT (3.3394 g, 24.7129 mmol) were added in a 500 mL round-bottomed flask and then dissolved with DMF (100 mL), and the mixed solution was stirred at -5 °C for 30min. Then DIEA (12.25 mL, 74.1384 mmol) was slowly added dropwise, and then the mixed solution reacted at a low temperature for 2.5h; after that, the reaction device was placed at room temperature and the reaction solution was stirred overnight to react. At the end of the reaction, n-hexane (100 mL) was first added to precipitate the reaction solution, the supernatant was discarded, methyl tert-butyl ether (300 mL) was then added for precipitation, and such operations were repeated three times; the filter cake was dried in a vacuum oven to obtain 17.4 g of the crude product 11-87 with a yield of 100% for the next reaction.

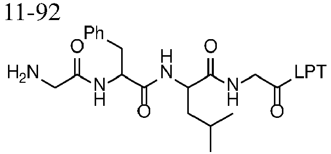

Product 11-87 (17.4 g, 16.4752 mmol) was added in a 500 mL round-bottomed flask and dissolved with dichloromethane (80 mL), TFA (36.7 mL, 494.256 mmol) was then added, and the obtained solution was stirred at room temperature to react overnight; at the end of the reaction, the reaction solution was concentrated under reduced pressure and then precipitated three times with n-hexane (150 mL×3), and the supernatant was discarded; the obtained solution was then precipitated three times with methyl tert-butyl ether (250 mL×3), and the suction filtering was carried out; and the operations of dry sample loading, column chromatography and gradient elution with 2%-10% methanol/1% ammonia water/dichloromethane were carried out; the elution product was then collected, concentrated, and dried in a vacuum oven, thus obtaining 11.2 g of Product 11-92 with a yield of 71%.

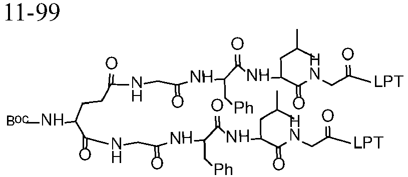

Product 11-92 (6.8225 g, 7.1403 mmol), Boc-Glu-OH (0.8406 g, 3.4001 mmol), HBTU (3.8683 g, 10.2004 mmol) and HOBT (1.783 g, 10.2004 mmol) were added in a 500 mL round-bottomed flask and then dissolved with DMF (50 mL), and the mixed solution was stirred at -5 °C for 30min. Then DIEA (5.1 mL, 30.6009 mmol) was slowly added dropwise, and then the mixed solution reacted at a low temperature for 2.5h; after that, the reaction device was placed at room temperature and the reaction solution was stirred overnight to react. At the end of the reaction, n-hexane (100 mL) was first added to precipitate the reaction solution, the supernatant was discarded, methyl tert-butyl ether (300 mL×3) was then added for precipitation; the filter cake was dried in a vacuum oven to obtain 5.8 g of the crude product 11-99 with a yield of 80% for the next deprotection reaction.

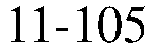

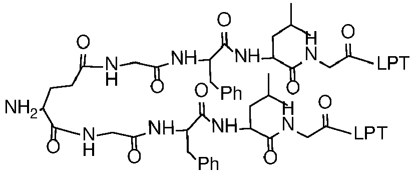

Product 11-99 (5.8 g, 2.733 mmol) was added in a 500 mL round-bottomed flask and dissolved with dichloromethane (80 mL), TFA (6.1 mL, 81.99 mmol) was then added, and the obtained solution was stirred at room temperature to react overnight; at the end of the reaction, the reaction solution was concentrated under reduced pressure and then precipitated three times with n-hexane (150 mL×3), and the supernatant was discarded; the obtained solution was then precipitated three times with methyl tert-butyl ether (250 mL×3), and the suction filtering was carried out; and the operations of dry sample loading, column chromatography and gradient elution with 5%-8% methanol/dichloromethane were carried out; the elution product was then collected, concentrated, and dried in a vacuum oven, thus obtaining 5.3 g of Product 11-105 with a yield of 96%.

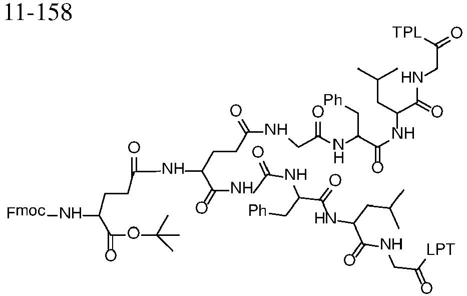

Product 11-105 (2.5 g, 1.2363 mmol), Fmoc-Glu-OtBu (0.7364 g, 1.7308 mmol), HBTU (0.7032 g, 1.8544 mmol) and HOBT (0.2505 g, 1.8544 mmol) were added in a 500 mL round-bottomed flask and then dissolved with DMF (100 mL), and the mixed solution was stirred at -5 °C for 30min. Then DIEA (0.9 mL, 5.5633 mmol) was slowly added dropwise, and then the mixed solution reacted at a low temperature for 2h; after that, the reaction device was placed at room temperature and the reaction solution was stirred overnight to react. At the end of the reaction, n-hexane (100 mL) was first added to precipitate the reaction solution, the supernatant was discarded, methyl tert-butyl ether (200 mL×3) was then added for precipitation; the filter cake was dried in a vacuum oven to obtain 3.7364 g of a solid product 11-158 with a yield of 100% for the next deprotection reaction.

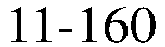

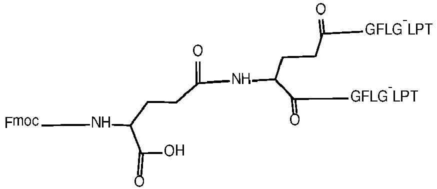

Product 11-158 (3.7364 g, 1.2363 mmol) was added in a 500 mL round-bottomed flask and dissolved with dichloromethane (50 mL), TFA (2.8 mL, 37.0439 mmol) was then added, and the obtained solution was stirred at room temperature to react overnight; at the end of the reaction, the reaction solution was concentrated under reduced pressure and then precipitated three times with n-hexane (150 mL×3), and the supernatant was discarded; the obtained solution was then precipitated with methyl tert-butyl ether (250 mL×3), and the suction filtering was carried out; and the filter cake was then dried in a vacuum oven, thus obtaining 3.2 g of the crude product 11-160 with a yield of 100%.

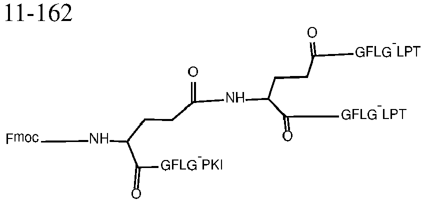

Product 11-160 (3.2693 g, 1.2425 mmol), Product 11-83 (1.2958 g, 1.3668 mmol), HBTU (0.7067 g, 1.8637 mmol) and HOBT (0.2518 g, 1.8637 mmol) were added in a 500 mL round-bottomed flask and then dissolved with DMF (50 mL), and the mixed solution was stirred at -5 °C for 30min. Then DIEA (0.9 mL, 5.5912 mmol) was slowly added dropwise, and then the mixed solution reacted at a low temperature for 2h; after that, the reaction device was placed at room temperature and the reaction solution was stirred overnight to react. At the end of the reaction, n-hexane (100 mL) was first added to precipitate the reaction solution, the supernatant was discarded, methyl tert-butyl ether (200 mL×3) was then added for precipitation, and suction filtering was carried out to obtain a solid crude product; the filter cake was dried in a vacuum oven to obtain 4.7447 g of Product 11-162 with a yield of 100% for the next deprotection reaction.

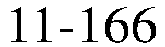

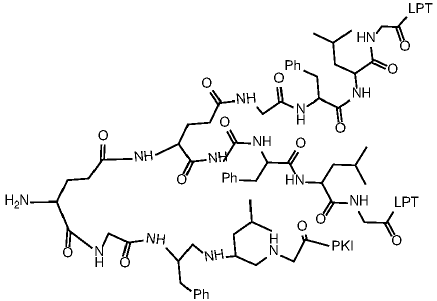

Product 11-162 (4.7447 g, 1.2411 mmol) was added in a 500 mL round-bottomed flask and then dissolved with DMF (40 mL), morpholine (3.3 mL, 37.2332 mmol) was then added, and the obtained solution was stirred at room temperature for 1.5h. At the end of the reaction, n-hexane (100 mL) was first added to precipitate the reaction solution, methyl tert-butyl ether (200 mL) was then added for precipitation, and such operations were repeated three times; the filter cake was taken and the operations of dry sample loading, column chromatography, and gradient elution with 5%-10% methanol/dichloromethane were carried out; the elution product was dried in a vacuum oven, thus obtaining 2.2 g of Product 11-166 with a yield of 57%.

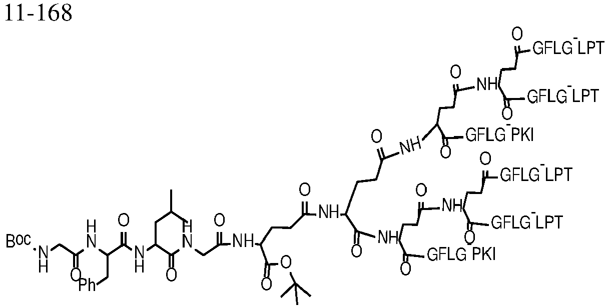

Product 11-166 (2.2 g, 0.714 mmol), Product 24-40 (0.238 g, 0.295 mmol), HBTU (0.3356 g, 0.885 mmol) and HOBT (0.1058 g, 0.885 mmol) were added in a 250 mL round-bottomed flask and then dissolved with DMF (50 mL), and the mixed solution was stirred at -5 °C for 30min. Then DIEA (0.5 mL, 2.655 mmol) was slowly added dropwise, and then the mixed solution reacted at a low temperature for 2h; after that, the reaction device was placed at room temperature and the reaction solution was stirred overnight to react. At the end of the reaction, n-hexane (100 mL) was first added to precipitate the reaction solution, the supernatant was discarded, methyl tert-butyl ether (200 mL×3) was then added for precipitation, and a solid was separated out; and suction filtering was carried out; the filter cake was washed with n-hexane (50 mL×2) and then dried in a vacuum oven, thus obtaining 2.0 g of Product 11-168 with a yield of 83% for the next deprotection reaction.

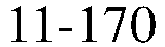

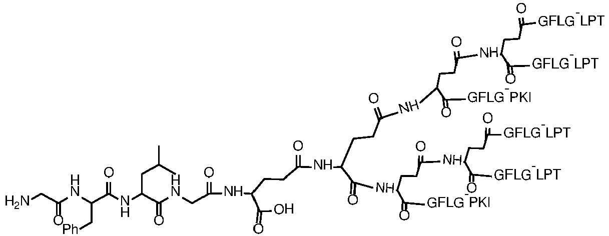

Product 11-168 (2.0 g, 0.2884 mmol) was added in a 250 mL round-bottomed flask and dissolved with dichloromethane (35 mL), TFA (0.7 mL, 8.6537 mmol) was then added, and the obtained solution was stirred at room temperature to react overnight; at the end of the reaction, the reaction solution was concentrated under reduced pressure and then precipitated three times with n-hexane (150 mL×3); the obtained solution was then precipitated three times with methyl tert-butyl ether (250 mL×3), and the suction filtering was carried out; and the operations of dry sample loading, column chromatography and gradient elution with 6%-8% methanol/dichloromethane were carried out; the elution product was then collected, concentrated, and dried in a vacuum oven, thus obtaining 0.7 g of Product 11-170 with a yield of 36%.

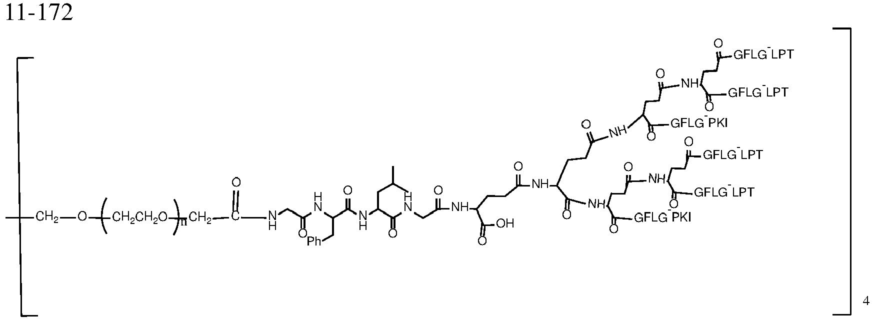

Product 11-170 (0.7 g, 0.1032 mmol) was added in a 250 mL round-bottomed flask and then dissolved with DMF (50 mL), DIEA (0.1 mL, 0.43 mmol) was then added for neutralization; then, 4ARM-SCM-40K (0.9028 g, 0.0215 mmol, purchased from JenKem, Lot Number: C231-N171101) was added to the obtained solution and the solution reacted in the dark for one week at a low speed stirring at room temperature. At the end of the reaction, methyl tert-butyl ether (200 mL×3) was added for precipitation to separate out a solid the suction filtering was carried out, the filter cake was washed repeatedly with methyl tert-butyl ether (20 mL×3) and the operations of dry sample loading, column chromatography, and gradient elution with 5%-8% methanol/dichloromethane were carried out; the elution product was collected, concentrated, and dried in a vacuum oven, thus obtaining 1.1 g of Product 11-172 with a yield of 78%.

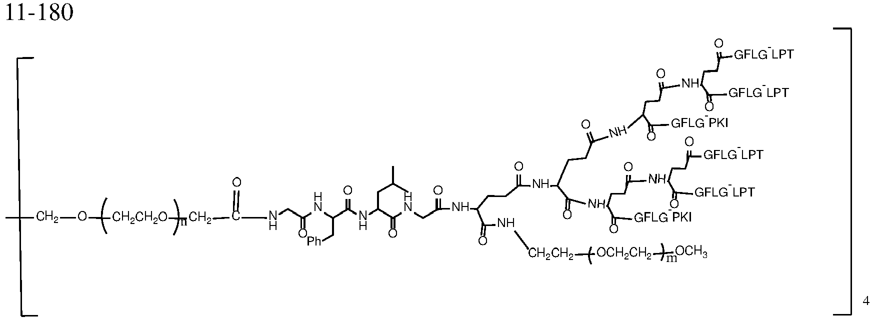

Product 11-172 (1.1 g, 0.016 mmol), HBTU (0.036 g, 0.0961 mmol) and HOBT (0.055 g, 0.0961 mmol) were added in a 250 mL round-bottomed flask and then dissolved with DMF (50 mL); M-NH₂-3K.HCl (0.29 g, 0.0961 mmol, purchased from JenKem, Lot Number: zz313p022) was then added to the obtained solution, and the solution reacted in the dark for 2 days at a low speed stirring at room temperature. At the end of the reaction, methyl tert-butyl ether (200 mL×3) was added for precipitation to separate out a solid, and the precipitation process was repeated three times; the suction filtering was carried out, the filter cake was washed repeatedly with methyl tert-butyl ether (20 mL×3), and the operations of dry sample loading, column chromatography, and gradient elution with 5%-10% methanol/dichloromethane were carried out; the elution product was collected, concentrated, and dried in a vacuum oven, thus obtaining 0.9 g of Product 11-180 with a yield of 70%.

### Example 25 Solubility test of Compound 24-12 and control Compound 24-6 in normal saline

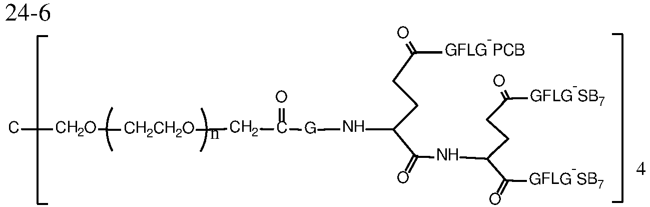

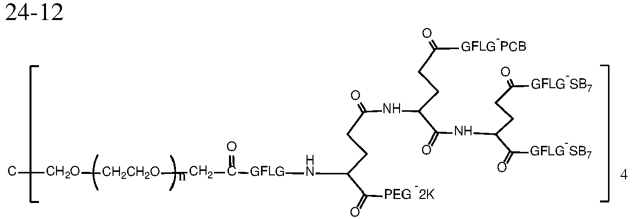

Two 2 mL sample bottles were prepared and marked as Bottle A and Bottle B, respectively.

| | Normal saline | Total drug load PCB (4 equivalents) + SB7 (8 equivalents) | Required dose of Compound 24-6 or 24-12 | |
|---|---|---|---|---|
| Bottle A | 0.2 mL | 0.5mg | 24-6 | 4.5mg |
| Bottle B | 0.2 mL | 0.5mg | 24-12 | 5.3mg |

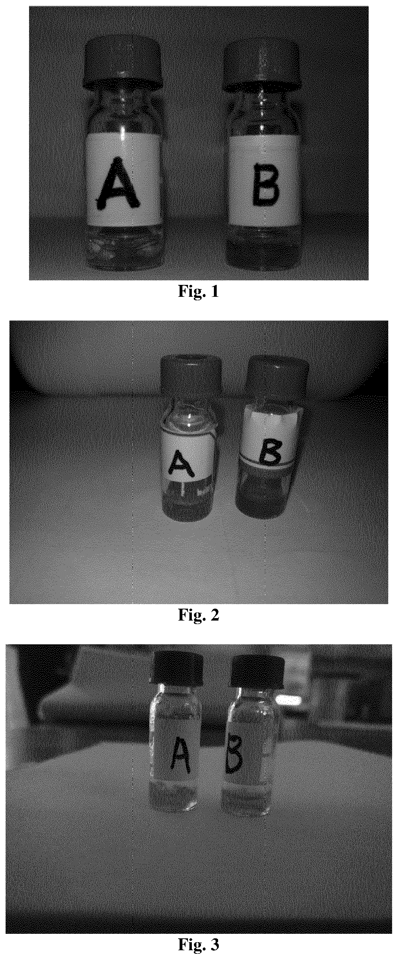

Compound 24-6 (4.5mg) was added in Bottle A, Compound 24-12 (5.3mg) was added in Bottle B, 0.2 mL of normal saline was added to the two bottles respectively, and shaken and treated by ultrasonic at the same time; 20 min later, it was observed that Compound 24-6 in Bottle A was still yellow lumps while Compound 24-12 in Bottle B was completely dissolved and became a yellow transparent solution. The test results are shown in Fig. 1.

Example 26 Solubility test of Compound 17-141 and control Compound 18-158

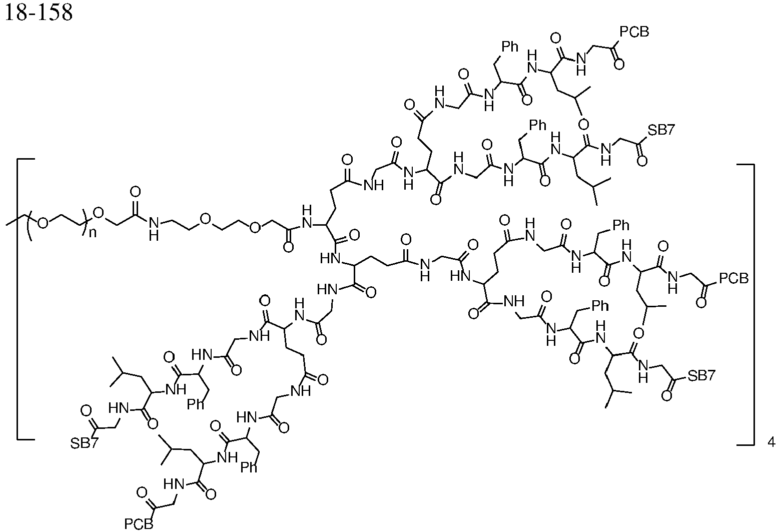

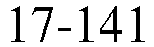

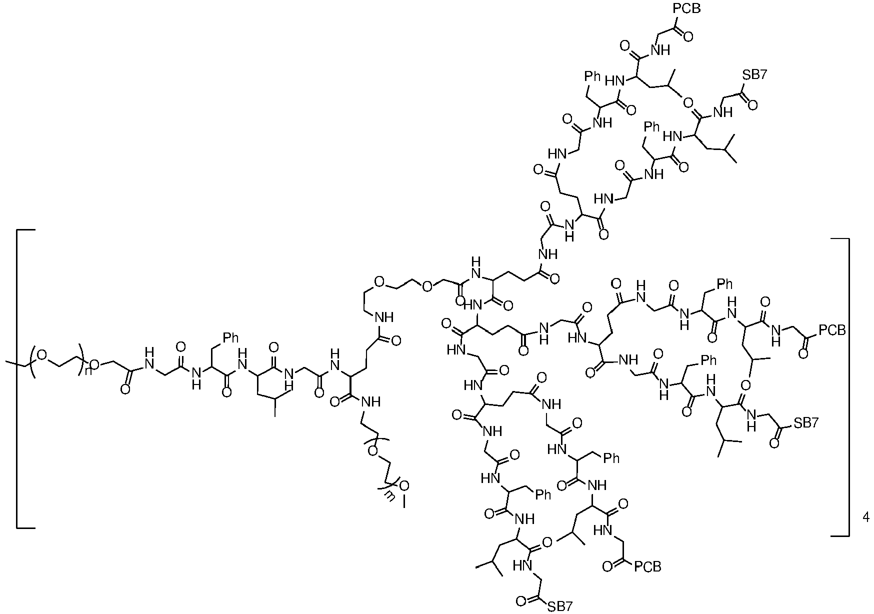

Two 2 mL sample bottles were prepared and marked as Bottle A and Bottle B, respectively.

| | Normal saline | Total drug load PCB (12 equivalents) + SB7 (12 equivalents) | Required dose of Compound 18-158 or 17-141 | |
|---|---|---|---|---|
| Bottle A | 0.2 mL | 0.5mg | 18-158 | 2.8mg |
| Bottle B | 0.2 mL | 0.5mg | 17-141 | 3.4mg |

Compound 18-158 (2.8mg) was added in Bottle A, Compound 17-141 (3.4mg) was added in Bottle B, 0.2 mL of normal saline was added to the two bottles respectively, and shaken and treated by ultrasonic at the same time; 30 min later, it was observed that Compound 18-158 in Bottle A was still yellow lumps while Compound 17-141 in Bottle B was completely dissolved and became a slightly viscous yellow transparent solution. The test results are shown in Fig. 2.

### Example 27 Solubility test of Compound 26-18 and control Compound 13-141

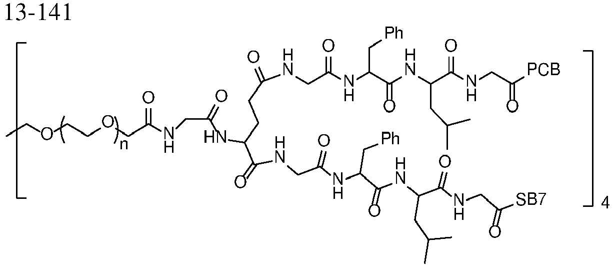

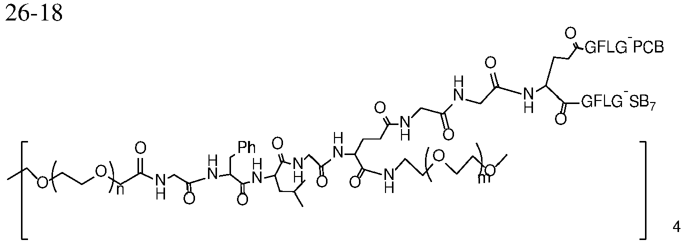

Two 2 mL sample bottles were prepared and marked as Bottle A and Bottle B, respectively.

| | Normal saline | Total drug load PCB (4 equivalents) + SB7 (4 equivalents) | Required dose of Compound 13-141 or 26-18 | |
|---|---|---|---|---|
| Bottle A | 0.2 mL | 0.5mg | 13-141 | 6.6mg |
| Bottle B | 0.2 mL | 0.5mg | 26-18 | 7.6mg |

Compound 13-141 (6.6mg) was added in Bottle A, Compound 26-18 (7.6mg) was added in Bottle B, 0.2 mL of normal saline was added to the two bottles respectively, and shaken and treated by ultrasonic at the same time; 20 min later, it was observed that Compound 13-141 in Bottle A was still yellow lumps while Compound 26-18 in Bottle B was completely dissolved and became a yellow transparent solution. The test results are shown in Fig. 3.

Experimental Example 1 Study on the anti-tumor efficacy of Compound 19-80 on subcutaneously transplanted tumor of human colon cancer cell colo205

### I. Experimental purpose

To study the anti-tumor efficacy of the test substance injected intravenously (or other drugs injected intraperitoneally or administrated orally) on subcutaneously transplanted tumor of human colon cancer cell colo205.

### II. Experimental materials

Cell: human colon cancer cell colo205;
Drugs: compound drugs and their corresponding small molecular drugs as shown in the table below.

| Compound number | Molecular weight and drug loading form of PEG-DRUG | Small molecular drug, and its molecular weight and drug loading | Compoun d weight |
|---|---|---|---|
| 19-80 | 58910 | SB7:517.07, 3.51% | 1.7 |
| | 4^{∗}SB7+4^{∗}PCB | PCB:447.54, 3.04% | |

Animal: Balb/c mice, female

### III. Anti-tumor experiment of drug

1. Tumor strain and transplantation method of solid tumor: Human colon cancer cell colo205 was routinely preserved in the cell room of the State Key Laboratory of Molecular Oncology, Institute of Cancer Research, Chinese Academy of Medical Sciences. The tumor cells were taken out of liquid nitrogen, quickly thawed at 37 °C, resuscitated and expanded, and then inoculated subcutaneously in the right axillary of healthy Balb/c mice. When the transplanted tumor grew to about 1.0 cm in diameter, the mice were sacrificed. Under aseptic conditions, the tumors were peeled off for passage by conventional methods.
2. Tumor inoculation and grouping: The tumor-bearing mice were sacrificed, the tumors were peeled off under aseptic condition and washed with normal saline several times, the necrotic tissues were removed, the well-growing tumor tissues were selected and divided into 2×2mm tissue blocks, and the tumor tissue blocks were inoculated subcutaneously in the right axillary with a special metal trocar. After the transplantation of the tumor tissue blocks, administration in groups was carried out on the day of the transplantation.

**Table 1 Determined final dosage regimen**

| Animal type | Drug number | Tumor cell line | Drug dose (mg/kg) | Number of animals in each group | Group | Mode of administration |
|---|---|---|---|---|---|---|
| balb/C nude mice | 19-80 | colo205 | 56.98 | 6 | 2 | Tail vein injection |
| | SB7 + PCB (injection + oral administration for this group) | | 2 (SB7 injected) + 1.73 (PCB orally administrated) | 6 | 14 | Tail vein injection with SB7 Intragastric administration with PCB |
| | Normal saline group | | / | 6 | 15 | Tail vein injection |

3. Administration route: mixed administration of intraperitoneal and oral administration and tail vein injection. The modes of administration during the experiment operation were shown in Table 2.

**Table 2 Actual dosage regimen**

| January 4 | January 7 | January 10 | January 11 | January 13 | January 15 | January 16 | January 19 | January 22 |
|---|---|---|---|---|---|---|---|---|
| Arrival of animals+i | Tumor measurem | Tumor measurem | Administr ation | Tumor measurem | Administr ation | Tumor measurem | Tumor measurem | Tumor measurem |
| noculatio n | ent+admi nistration | ent | | ent | | ent | ent+admi nistration | ent+tumo r taking |

4. Data collection: the mice were weighed, the volume of tumors was measured, the mice were scarified on Day 19, the tumors were weighed, and the tumor inhibition rate (%) was calculated.

### IV. Laboratory animals and environment

1. Animal strain: Balb/c nude mice
2. Animal level: SPF level
3. Animal sex and number: female, totaling 18 mice.
4. Animal age: 6-7 weeks old.
5. Animal weight: 16-18 g
6. Animal source: Beijing Charles River Laboratory Animal Technology Co., Ltd., license number: SCXK (Beijing) 2016-0006, person in charge of quality: Colin Samuel Dunn
7. Rearing condition: all experimental operations were carried out in the animal room of the Beijing Laboratory Animal Research Center (license number SYXK (Beijing) 2015-0046). Laboratory animals were kept in clean-grade animal rooms and reared by qualified personnel. Thermometer and hygrometer were hung in the animal room, the temperature and humidity were measured and recorded once every morning from 8:00 to 10:00, the room temperature was controlled at 22.6±0.4 °C, and the humidity was controlled at 75.8±6.2%, the mice were kept in light for 12 h and in the dark for 12 h. The mice were reared in standard feeding boxes, 5 mice per box; the boxes were kept clean, and experimental operation were carried out in accordance with the regulations of the animal laboratory during the test.
8. Feed: clean rat feed, Laboratory Animal Center of the Academy of Military Medical Sciences, production license number: Jingdong (2000) No. 015.
9. Drinking water: The electrodialysis ultrafiltration purified water was supplied from the drinking water bottles, and the drinking water bottles were rinsed once a day and the drinking water was changed once a day.

### V. Experimental design

Basis of experimental design: "Guiding Principles of Pharmacodynamics of Antitumor Drugs" issued by the State Food and Drug Administration for soliciting opinions and "Guiding Principles of Non-clinical Research of Cytotoxic Anticancer Drugs"

Evaluation indicators:
Average tumor weight in the treatment group
Tumor inhibition rate (%) = (1-average tumor weight of treatment group/average tumor weight of negative control group) × 100%

### VI. Experimental results

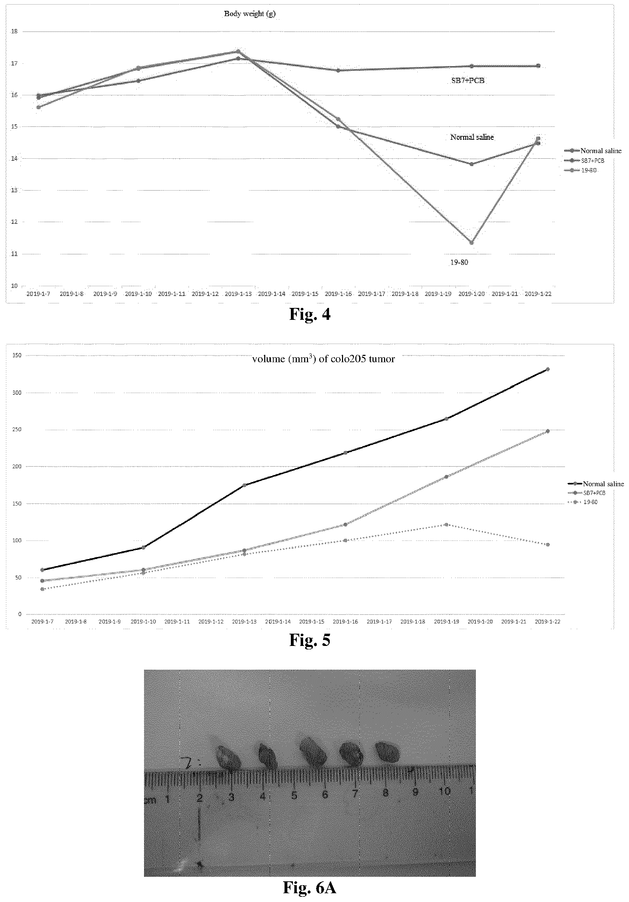

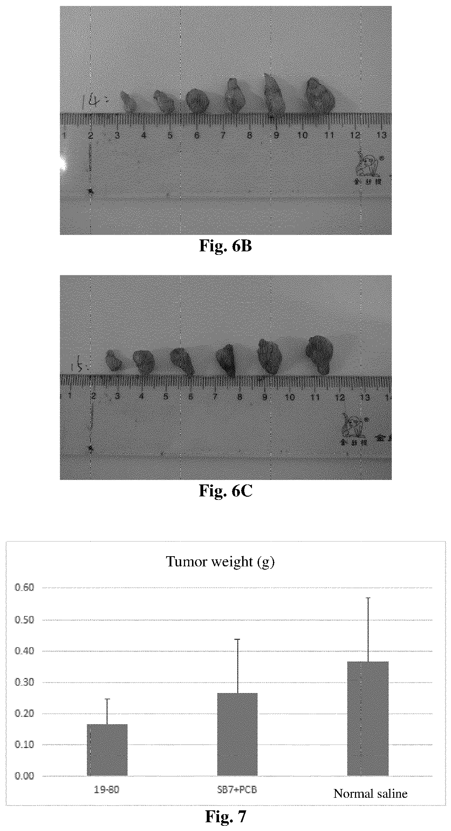

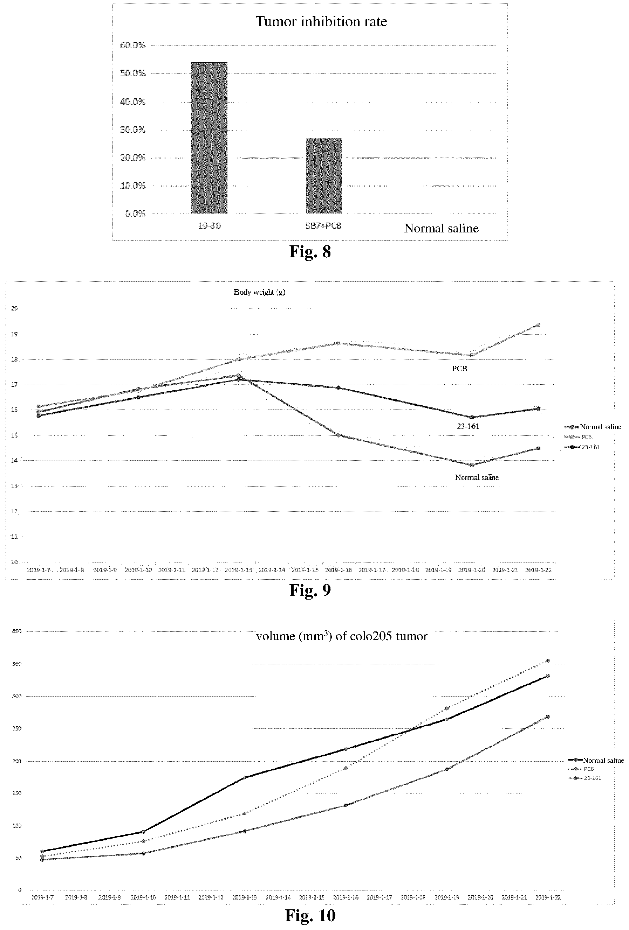

Fig. 4 shows the weight growth curve of the laboratory animals. Fig. 5 shows the tumor growth curve (volume). Figs. 6A-6C show the photos of the tumor tissues of each group, and from top to bottom, they are the 19-80 group, the SB7 + PCB group, and the normal saline group. Fig. 7 is a histogram of tumor weight, and Table 3 shows the average and variance of tumor weight. Fig. 8 and Table 4 show the tumor inhibition rate of the drug.

**Table 3 Tumor tissue weight**

| | Tumor weight (g) | |
|---|---|---|
| Group | Average | Variance |
| 19-80 | 0.17 | 0.08 |
| SB7+PCB | 0.27 | 0.17 |
| Normal saline | 0.37 | 0.20 |

**Table 4 Tumor inhibition rate of the drug**

| Group | Tumor inhibition rate |
|---|---|
| 19-80 | 54.2% |
| SB7+PCB | 27.0% |
| Normal saline | 0.0% |

The results show that the tumor inhibition rate of Compound 19-80 is higher than or equal to 50.0%, which is better than the common combination of SB7+PCB and achieves a significant therapeutic effect.

Experimental Example 2 Study on the anti-tumor efficacy of Compound 23-161 on subcutaneously transplanted tumor of human colon cancer cell colo205

### I. Experimental purpose

1. To study the anti-tumor efficacy of the test substance injected intravenously (or other drugs injected intraperitoneally or administrated orally) on subcutaneously transplanted tumor of human colon cancer cell colo205.

### II. Experimental materials

Cell: human colon cancer cell colo205;
Drugs: compound drugs and their corresponding small molecular drugs as shown in the table below

| Compound number | Molecular weight and drug loading form of PEG-DRUG | Small molecular drug, and its molecular weight and drug loading | Compound weight |
|---|---|---|---|
| 23-161 | 66509 16^{∗}PCB | PCB: 447.54, 10.77% | 0.63 |

Animal: Balb/c mice, female

### III. Anti-tumor experiment of drug

1. Tumor strain and transplantation method of solid tumor: Human colon cancer cell colo205 was routinely preserved in the cell room of the State Key Laboratory of Molecular Oncology, Institute of Cancer Research, Chinese Academy of Medical Sciences. The tumor cells were taken out of liquid nitrogen, quickly thawed at 37 °C, resuscitated and expanded, and then inoculated subcutaneously in the right axillary of healthy Balb/c mice. When the transplanted tumor grew to about 1.0 cm in diameter, the mice were sacrificed. Under aseptic conditions, the tumors were peeled off for passage by conventional methods.
2. Tumor inoculation and grouping: the tumor-bearing mice were sacrificed, the tumors were peeled off under aseptic condition and washed with normal saline several times, the necrotic tissues were removed, the well-growing tumor tissues were selected and divided into 2×2mm tissue blocks, and the tumor tissue blocks were inoculated subcutaneously in the right axillary with a special metal trocar. After the transplantation of the tumor tissue blocks, administration in groups was carried out on the day of the transplantation.

**Table 5 Determined final dosage regimen**

| Animal type | Drug number | Tumor cell line | Drug dose (mg/kg) | Number of animals in each group | Group | Mode of administration |
|---|---|---|---|---|---|---|
| balb/C nude mice | 23-161 | colo205 | 16.06 | 6 | 9 | Tail vein injection |
| | PCB | | 1.73 (Oral administration of PCB) | 6 | 13 | Oral administration |
| | Normal saline group | | / | 6 | 15 | Tail vein injection |

3. Administration route: mixed administration of intraperitoneal and oral administration and tail vein injection. The modes of administration during the experiment operation were shown in Table 6.

**Table 6 Actual dosage regimen**

| Date | | | | | | | | |
|---|---|---|---|---|---|---|---|---|
| January 4 | January 7 | January 10 | January 11 | January 13 | January 15 | January 16 | January 19 | January |
| | | | | | | | | 22 |
| Arrival of animals+ino culation | Tumor measurem ent+admin istration | Tumor measurem ent | Administr ation | Tumor measurem ent | Administr ation | Tumor measurem ent | Tumor measurem ent+admin istration | Tumor measurem ent+tumor taking |

4. Data collection:the mice were weighed, the volume of tumors was measured, the micewere scarified on Day 19, the tumors were weighed, and the tumor inhibition rate (%) was calculated.

### IV. Laboratory animals and environment

1. Animal strain: Balb/c nude mice
2. Animal level: SPF level
3. Animal sex and number: female, totaling 18 mice.
4. Animal age: 6-7 weeks old.
5. Animal weight: 16-18 g
6. Animal source: Beijing Charles River Laboratory Animal Technology Co., Ltd., license number: SCXK (Beijing) 2016-0006, person in charge of quality: Colin Samuel Dunn
7. Rearing condition: all experimental operations were carried out in the animal room of the Beijing Laboratory Animal Research Center (license number SYXK (Beijing) 2015-0046). Laboratory animals were kept in clean-grade animal rooms and reared by qualified personnel. Thermometer and hygrometer were hung in the animal room, the temperature and humidity were measured and recorded once every morning from 8:00 to 10:00, the room temperature was controlled at 22.6±0.4 °C, and the humidity was controlled at 75.8±6.2%, the mice were kept in light for 12 h and in the dark for 12 h. The mice were reared in standard feeding boxes, 5 mice per box; the boxes were kept clean, and experimental operation were carried out in accordance with the regulations of the animal laboratory during the test.
8. Feed: clean rat feed, Laboratory Animal Center of the Academy of Military Medical Sciences, production license number: Jingdong (2000) No. 015.
9. Drinking water: The electrodialysis ultrafiltration purified water was supplied from the drinking water bottles, and the drinking water bottles were rinsed once a day and the drinking water was changed once a day.

### V. Experimental design

Basis of experimental design: "Guiding Principles of Pharmacodynamics of Antitumor Drugs" issued by the State Food and Drug Administration for soliciting opinions and "Guiding Principles of Non-clinical Research of Cytotoxic Anticancer Drugs"

Evaluation indicators:
Tumor inhibition rate (%) = (1-average tumor weight of treatment group/average tumor weight of negative control group) × 100%

### VI. Experimental results

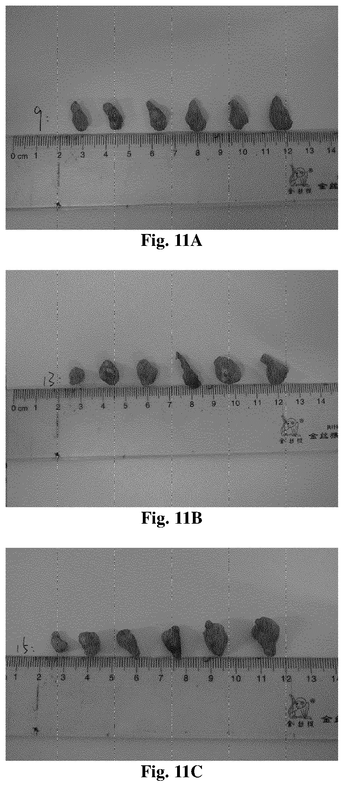

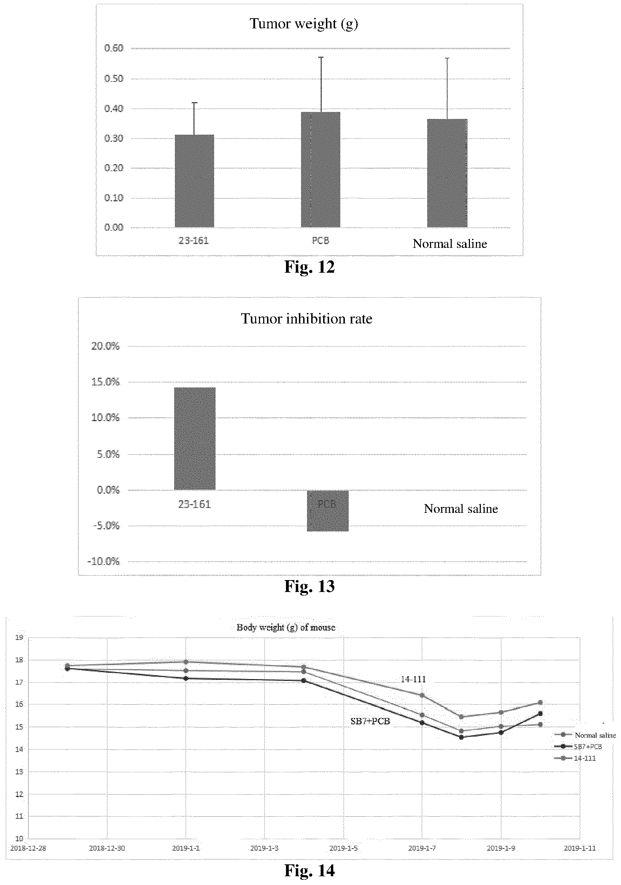

Fig. 9 shows the weight growth curve of the laboratory animals. Fig. 10 shows the tumor growth curve (volume). Figs. 11A-11C show the photos of the tumor tissues of each group, and from top to bottom, they are the 23-161 group, the PCB group, and the normal saline group. Fig. 12 is a histogram of tumor weight, and Table 7 shows the average and variance of tumor weight. Fig. 13 and Table 8 show the tumor inhibition rate of the drug.

**Table 7 Tumor tissue weight**

| | Tumor weight (g) | |
|---|---|---|
| Group | Average | Variance |
| 23-161 | 0.31 | 0.11 |
| PCB | 0.39 | 0.18 |
| Normal saline | 0.37 | 0.20 |

**Table 8 Tumor inhibition rate of the drug**

| Group | Tumor inhibition rate |
|---|---|
| 23-161 | 14.3% |
| PCB | -5.8% |
| Normal saline | 0.0% |

The results show that the tumor inhibition rate of Compound 23-161 is superior to that of PCB.

Experimental Example 3 Study on the anti-tumor efficacy of Compound 14-111 on subcutaneously transplanted tumor of mouse colon cancer cell CT26

### I. Experimental purpose

1. To study the anti-tumor efficacy of the test substance injected intravenously (or other drugs injected intraperitoneally or administrated orally) on subcutaneously transplanted tumor of mouse colon cancer cell CT26.

### II. Experimental materials

Cell: mouse colon cancer cell CT26;
Drugs: compound drugs and their corresponding small molecular drugs;
Animal: Balb/c mice, female.

### III. Anti-tumor experiment of drug

1. Tumor strain and transplantation method of solid tumor: Mouse colon cancer cell CT26 was routinely preserved in the cell room of the State Key Laboratory of Molecular Oncology, Institute of Cancer Research, Chinese Academy of Medical Sciences. The tumor cells were taken out of liquid nitrogen, quickly thawed at 37 °C, resuscitated and expanded, and then inoculated subcutaneously in the right axillary of healthy Balb/c mice. When the transplanted tumor grew to about 1.0 cm in diameter, the mice were sacrificed. Under aseptic conditions, the tumors were peeled off for passage by conventional methods.
2. Tumor inoculation and grouping: the tumor-bearing mice were sacrificed, the tumors were peeled off under aseptic condition and washed with normal saline several times, the necrotic tissues were removed, the well-growing tumor tissues were selected and divided into 2×2mm tissue blocks, and the tumor tissue blocks were inoculated subcutaneously in the right axillary with a special metal trocar. After the transplantation of the tumor tissue blocks, administration in groups was carried out on the day of the transplantation.

**Table 9 Determined final dosage regimen**

| Animal type | Drug number | Tumor cell line | Drug dose (mg/kg) | Number of animals in each group | Group | Mode of administrati on |
|---|---|---|---|---|---|---|
| balb/C mice | 14-111 | CT26 | 60.42 | 6 | 1 | Tail vein injection |
| | SB7 + PCB | | 2 (SB7 injected) + 3.46 (PCB orally administrated) | 6 | 2 | Tail vein injection with SB7 Orally administrati on with PCB |
| | Normal saline | | / | 6 | 3 | Intraperiton eal injection |

3. Administration route: mixed administration of intraperitoneal and oral administration and tail vein injection. Because of the changes in the mode of administration and frequency of administration in the later stage, the modes of administration during the experiment operation were shown in Table 10.

**Table 10 Actual dosage regimen**

| Date | | | | | | | |
|---|---|---|---|---|---|---|---|
| 12/29/2018 | 1/1/2019 | January 4 | January 7 | January 8 | January 9 | January 10 | January 11 |
| Inoculation+ administrati on | Administrati on | Tumor measureme nt+adminis | Tumor measureme nt +adminis | Tumor measureme nt+adminis | Tumor measureme nt+adminis | Tumor measureme nt +adminis | Mouse sacrifice+m easurement |
| | | tration | tration | tration | tration | tration | |

4. Data collection: the mice were weighed, the volume of tumors was measured, the mice were scarified on Day 13, the tumors were weighed, and the tumor inhibition rate (%) was calculated.

### IV. Laboratory animals and environment

1. Animal strain: Balb/c mice
2. Animal level: SPF level
3. Animal sex and number: female, totaling 18 mice.
4. Animal age: 6-7 weeks old.
5. Animal weight: 17-19 g
6. Animal source: Beijing Charles River Laboratory Animal Technology Co., Ltd., license number: SCXK (Beijing) 2016-0006, person in charge of quality: Colin Samuel Dunn
7. Rearing condition: all experimental operations were carried out in the animal room of the Beijing Laboratory Animal Research Center (license number SYXK (Beijing) 2015-0046). Laboratory animals were kept in clean-grade animal rooms and reared by qualified personnel. Thermometer and hygrometer were hung in the animal room, the temperature and humidity were measured and recorded once every morning from 8:00 to 10:00, the room temperature was controlled at 22.6±0.4 °C, and the humidity was controlled at 75.8±6.2%; the mice were kept in light for 12 h and in the dark for 12h. The mice were reared in standard feeding boxes, 5 mice per box; the boxes were kept clean, and experimental operation were carried out in accordance with the regulations of the animal laboratory during the test.
8. Feed: clean rat feed, Laboratory Animal Center of the Academy of Military Medical Sciences, production license number: Jingdong (2000) No. 015.
9. Drinking water: The electrodialysis ultrafiltration purified water was supplied from the drinking water bottles, and the drinking water bottles were rinsed once a day and the drinking water was changed once a day.

### V. Experimental design

Experimental design basis: "Guiding Principles of Pharmacodynamics of Antitumor Drugs" issued by the State Food and Drug Administration for soliciting opinions and "Guiding Principles of Non-clinical Research of Cytotoxic Anticancer Drugs"

Evaluation indicators:
Average tumor weight in the treatment group
Tumor inhibition rate (%) = (1-average tumor weight of treatment group/average tumor weight of negative control group) × 100%

### VI. Experimental results

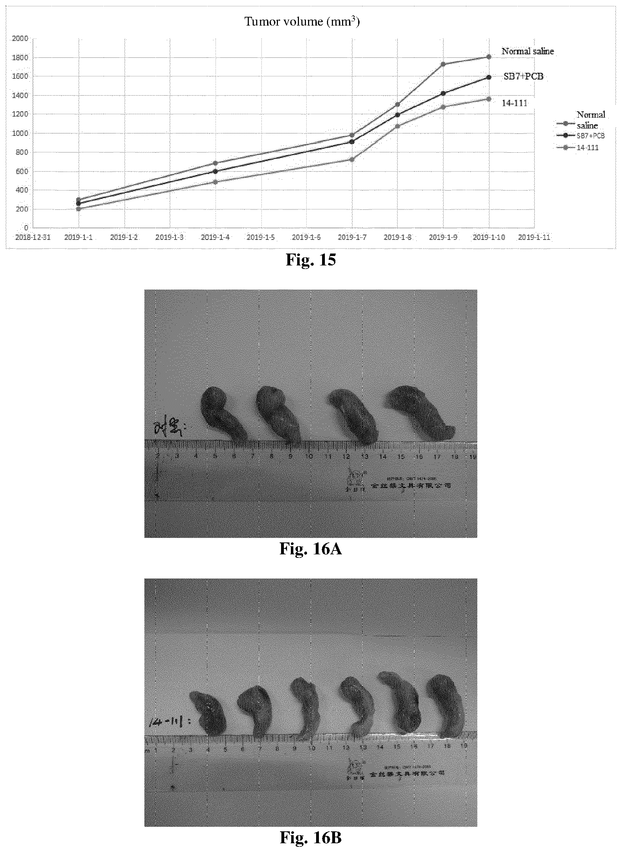

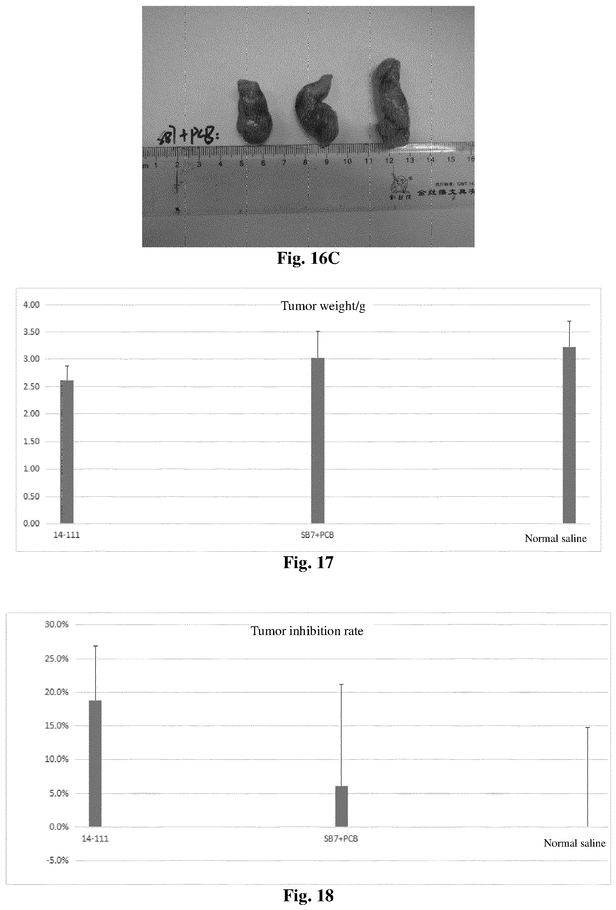

Fig. 14 shows the weight growth curve of the laboratory animals. Fig. 15 shows the tumor growth curve (volume). Figs. 16A-16C show the photos of the tumor tissues of each group, and from top to bottom, they are the normal saline group, the 14-111 group, and the SB7 + PCB group. Fig. 17 is a histogram of tumor weight, and Table 11 shows the average and variance of tumor weight. Fig. 18 and Table 12 show the tumor inhibition rate of the drug.

**Table 11 Tumor tissue weight**

| Tumor weight (g) | | |
|---|---|---|
| Group | Average | Variance |
| 14-111 | 2.619 | 0.263 |
| SB7+PCB | 3.027 | 0.488 |
| Normal saline | 3.221 | 0.475 |

**Table 12 Tumor inhibition rate of the drug**

| Tumor inhibition rate | | |
|---|---|---|
| Group | Average | Variance |
| 14-111 | 19% | 8% |
| SB7+PCB | 6% | 15% |
| Normal saline | 0% | 15% |

The results show that the inhibitory effect of Compound 14-111 on mouse colon cancer CT26 cell tumor model is better than that of SB7+PCB.

Experimental Example 4 Study on the anti-tumor efficacy of Compound 17-161 on colon cancer cells (COLO-205) in nude mice

### I. Experimental purpose

To observe the efficacy of Compound 17-161 on colon cancer model animals.

### II. Experimental materials

Cell: Colon cancer cell (COLO-205); Cell source: Cell Bank of Type Culture Collection, Chinese Academy of Sciences; Culture condition: 37 °C, 5% CO₂; Medium: 90% RPMI-1640+10% FBS.
Drugs: Compound 17-161 and its corresponding small molecular drugs SB7 and PCB.
Animals: SPF-grade Balb/c nude mice, half male and half female, 31-38 days old, with body weight of 14.2-18.1 g; laboratory animal quality certificate number: No.43004700056279; production unit: Hunan SJA Laboratory Animal Co., Ltd, laboratory animal production license number: SCXK (Hunan) 2016-0002.

### III Animal rearing environment, experimental environment and rearing management

### 1. Animal rearing environment

Location and room: The barrier environment animal room of the North District of the Drug Evaluation Center of Guangzhou Boji Pharmaceutical Biotechnology Co., Ltd., Balb/c mice were kept in the quarantine room during the quarantine period and dosing period; Balb/c nude mice were kept in the Mouse Rearing Room 2 and the Mouse Laboratory 1 during the quarantine period and dosing period.

Laboratory animal license number: SYXK (Guangdong) 2017-0134; number of air changes: ≥15 per hour; rearing density: ≤ 10 mice per cage during the quarantine period, ≤ 5 mice per cage during the test period; lighting: light (12 h) and dark (12 h); frequency of cleaning of rearing cages and drinking bottles: twice a week; type of rearing cages: ordinary PP material.

### 2. Animal experiment environment

Administration environment: quarantine room, temperature: 18.0 °C-22.0 °C, humidity: 44.0%-68.0%; mouse rearing room 2: temperature: 19.0 °C-21.0 °C, humidity: 52.0%-63.0%; mouse laboratory 1: temperature: 22.8 °C-26.7 °C, humidity: 49.7%-70.3%.

### 3. Animal rearing management

Feed: growth and reproduction feed for SPF-level rats and mice; Source: Beijing Keao Xieli Feed Co., Ltd.; batch number: 18103313, 19103323.

Drinking water: purified water; source: Purified Water Station of Guangzhou Boji Pharmaceutical Biotechnology Co., Ltd.; main control indicators: quality meets the relevant regulations of purified water in the Chinese Pharmacopoeia (2015 Edition). After being filtered and sterilized, water was drunk by animals freely with the drinking water bottles.

Litter; corn cob, purchased from Guangzhou Hancheng Experimental Equipment Co., Ltd., and autoclaved by a pulse vacuum sterilizer before use; batch number: 20181225.

### IV. Preparation of drug solution

1. Basis for dose setting: SB7=2mg/kg was determined as the effective dose for the anti-tumor activity screening test of Compound 17-161. It was determined that the dose of Compound 17-161 was 23.15 mg/kg, the dose of Compound SB7 was 2.00 mg/kg, and the dose of compound PCB was 1.75 mg/kg.
2. Solution preparation: 1.54 mg of Compound 17-161 was weighed and added in an EP tube and then vortex dissolved with 0.10 mL of absolute ethanol; deionized water was then added to 1 mL (the ethanol concentration was 10%), and then the 17-161 solution with a concentration of 1.54 mg/mL was obtained.

PCB: 0.12 mg of PCB was weighed and added in an EP tube, a small amount of 0.5% sodium carboxymethyl cellulose solution was first added and the obtained solution was vortex mixed well, and 0.5% sodium carboxymethyl cellulose solution was then added to 1 mL, and a PCB suspension with a concentration of 0.12 mg/mL was then obtained.

SB7: 0.13 mg of SB7 was weighed and added in an EP tube and then vortex dissolved with a small amount of sodium chloride injection; sodium chloride injection was then added to 1 mL, and a SB7 solution with a concentration of 0.13 mg/mL was then obtained.

### IV. Experimental process

1. Cell culture and COLO-205 model establishment: the resuscitated COLO-205 cells were cultured in RPMI-1640 medium containing 10% of FBS, and then incubated in a 37 °C thermostatic incubator containing 5% of CO₂, with the culture medium changed every 2-3 days. Cells in the logarithmic growth phase were collected and adjusted to obtain a single cell suspension with a density of 2×10⁷ cells/mL. The skin of the nude mouse at the injection site was disinfected, and 0.2 mL of the cell suspension was taken with a 1 mL syringe and inoculated subcutaneously in the right axillary back of the nude mouse.
2. Animal grouping and administration: Nude mice with tumors larger than 100 mm³ were divided into 3 groups, 6 mice in each group, namely the model control group, the 17-161 group and the SB7+PCB group. Except PCB for intragastric administration, the rest were administered by tail vein injection with a volume of 0.15 mL/10 g. The model control group was administered 5 times, with an interval of 3 days each time, i.e., on Day 1, Day 4, Day 7, Day 10, and Day 13. 17-161 group and SB7+PCB group were administered totally 3 times, i.e., on Day 1, Day 5, and Day 12. Dosage regimen is shown in Table 13.

**Table 13 Dosage regimen**

| Group | Number of animals | Name of drug | Administr ation route | Administra tion concentrati on (mg/mL) | Administra tion dose (mg/kg) | Administ ration volume (mL/kg) | Admi nistra tion frequ encv |
|---|---|---|---|---|---|---|---|
| Model control group (Group A) | 6 | Normal saline | iv | / | / | 15 | 5 |
| 17-161 group (Group H) | 6 | 17-161 | iv | 1.54 | 23.15 | 15 | 3 |
| SB7+PCB group (Group I) | 6 | SB7, PCB | SB7: iv PCB: ig | SB7: 0.13 PCB: 0.12 | SB7: 2.00 PCB: 1.75 | 15 | 3 |

| | | | | | | | |
|---|---|---|---|---|---|---|---|
| Note: The SB7+PCB group was administrated with SB7 first, and then PCB. | | | | | | | |

3. Indicator detection method:
From the start of administration, general indicators such as tumor growth and animal status after administration were observed. Before each administration and at the time of sacrifice, the animals were weighed, the tumor volume was measured, and the relative tumor proliferation rate was calculated.

Relative tumor proliferation rate (%): the tumor volume was calculated before each administration and at the time of sacrifice. Calculation formula of tumor volume: V=1/2×a×b², where "a" refers to the long diameter of the tumor and "b" refers to the short diameter of the tumor. Calculation formula of relative tumor proliferation rate T/C (%): T/C (%) =TRTV/CRTV×100%, where "TRTV" refers to the RTV of the treatment group and "CRTV" refers to the RTV of the model control group. Calculation formula of relative tumor volume RTV: RTV=Vt/V0, where "Vt" refers to the tumor volume at a certain time, and "V0" refers to the tumor volume before administration.

Efficacy evaluation criteria: ineffective when T/C% >40%; effective when T/C% ≤40% and P<0.05 in statistics.

Tumor weight: On Day 17 of administration, the animals were sacrificed by dislocation of cervical vertebra, and the tumors were taken out and weighed, and the tumor inhibition rate was calculated. Calculation formula of tumor inhibition rate: tumor inhibition rate (%) = (tumor weight of model control group - tumor weight of treatment group) / tumor weight of model control group × 100%.

### IV. Test results

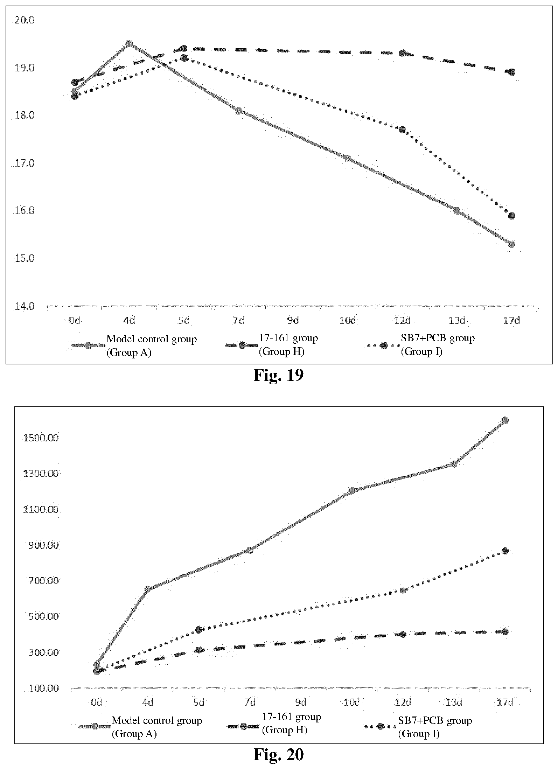

1. General indicator: the nude mice of the model control group and the SB7+PCB group showed weight loss on Day 10 after the administration, the nude mice of the 17-161 group showed weight loss after Day 12 after the administration, and the nude mice of the 17-161 group did not show obvious toxic side effects after Day 17 after the administration.
2. Body weight: The body weight of the model control group increased continuously in Day 1 - Day 4 after administration, and decreased continuously in Day 5 - Day 17 after administration; the weight of the 17-161 group was basically steady; the body weight of the SB7+PCB group increased continuously in Day 1 - Day 5 after administration and decreased continuously in Day 6 - Day 17 after administration. Fig. 19 shows the weight growth curve of the laboratory animals.
3. Tumor volume and relative tumor proliferation rate: The tumor volume of nude mice in each group increased continuously after administration. Compared with the model control group, the treatment group showed slowly increased tumor volume of nude mice (see Fig. 20). On Day 17 after administration, compared with the model control group, the 17-161 group showed significantly reduced tumor volume and relative tumor volume (*p*<0.001), and the T/C value was 29.1%; the SB7+PCB group showed significantly reduced tumor volume and relative tumor volume (*p*<0.01), and the T/C value was 62.9% (see Table 14). The 17-161 group showed significant inhibitory effect on tumor growth.

**Table 14 Relative tumor volume and proliferation rate ( x̅ ± s ) of nude mice in each group on Day 17 after the administration**

| Group | RTV | T/C(%) |
|---|---|---|
| Model control group (group A) | 6.97±0.94 | / |
| 17-161 group (group H) | 2.03±1.09^{▲▲▲} | 29.1 |
| SB7+PCB group (group I) | 4.39±1.14^{▲▲} | 62.9 |

| | | |
|---|---|---|
| Note: In comparison with the model control group, ^{▲▲}*p*<0.01, ^{▲▲▲}*p*<0.001. | | |

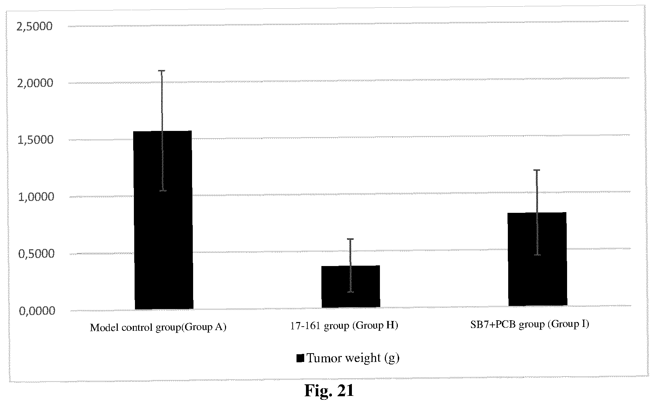

4. Tumor weight: Fig. 21 shows the tumor weight of each group on Day 17 after administration. Compared with the model control group, the 17-161 group and the SB7+PCB group were extremely significantly reduced in tumor weight (p<0.001), and the tumor inhibition rate of the 17-161 group was 76.3%, and the tumor inhibition rate of the SB7+PCB group was 47.5%. Tumor weight and inhibition rate of nude mice in each group were shown in Table 15.

**Table 15 Comparison of tumor weight ( x̅ ± s ) and tumor inhibition rate of nude mice in each group on Day 17 after administration**

| Group | Tumor weight (g) | Tumor inhibition rate (100%) |
|---|---|---|
| Model control group (group A) | 1.5716±0.5281 | / |
| 17-161 group (group H) | 0.3730±0.2905^{▲▲▲} | 76.3 |
| SB7+PCB group (group I) | 0.8252±0.2483^{▲▲▲} | 47.5 |

| | | |
|---|---|---|
| Note: In comparison with the model control group, ^{▲▲}*p*<0.01, ^{▲▲▲}*p*<0.001. | | |

### Conclusion

Compound 17-161 showed significant anti-tumor effect, on COLO-205 transplanted tumor model in nude mice by administration with the dosage of 23.15 mg/kg by tail vein injection for 3 times, i.e., on Day 1, Day 5 and Day 12, and the effectiveness was better than that of SB7 +PCB group.

Although the specific embodiments of the disclosure have been described in detail, those skilled in the art will understand that various modifications and changes can be made to the details according to all the teachings that have been disclosed, and these changes are within the protection scope of the disclosure. The full scope of the disclosure is given by the appended claims and any equivalents thereof.

## Claims

1. A polyethylene glycol conjugated drug of formula I or a pharmaceutically acceptable salt thereof; 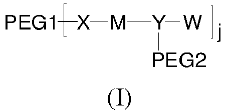 where PEG1 is a single-arm or multi-arm polyethylene glycol segment;
j represents the number of arms of PEG1;
X represents 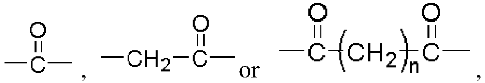 wherein n is 2 or 3; when j is greater than 1 (such as, 4 or 8), there are multiple (such as, 4 or 8) Xs at the same time, and in this case, the Xs are the same or different;
M represents an amino acid residue or polypeptide which is G, GG, GLG, GFA, GLA, or GFLG; when j is greater than 1 (such as, 4 or 8), there are multiple (such as, 4 or 8) Ms at the same time, and in this case, the Ms are the same or different; PEG2 represents a single-arm polyethylene glycol segment, which is linked to Y through an amide bond;
Y represents 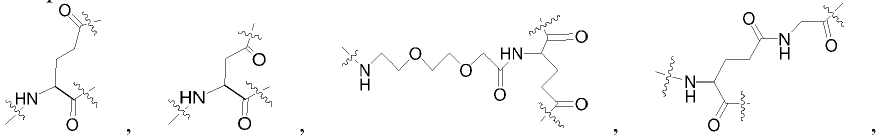 or 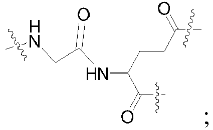
when j is greater than 1 (such as, 4 or 8), there are multiple (such as, 4 or 8) Ys at the same time, and in this case, the Ys are the same or different;
W is selected from Q, 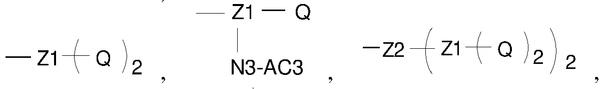 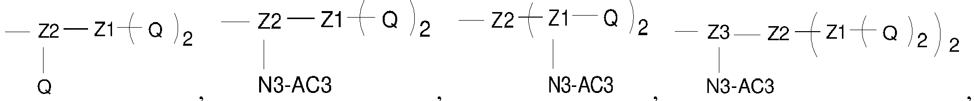 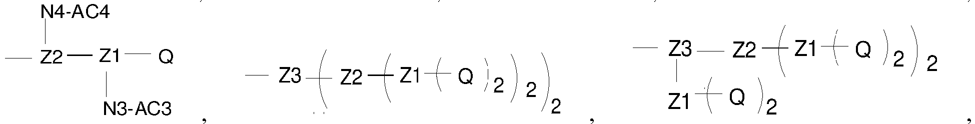 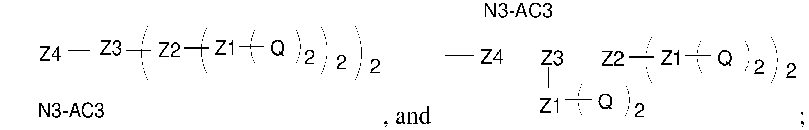
when j is greater than 1 (such as, 4 or 8), there are multiple (such as, 4 or 8) Ws at the same time, and in this case, the Ws are the same or different;
Q represents 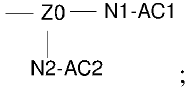 each of Z0, Z1, Z2, Z3and Z4 is independently selected from 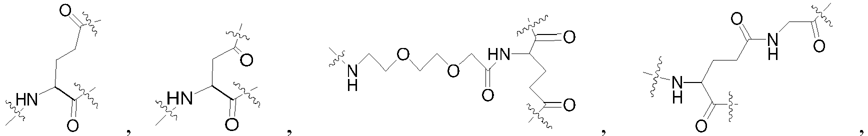 and 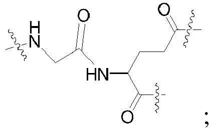
Z0, Z1, Z2, Z3, and Z4 are the same or different, and when there are multiple Z0s, multiple Z1s, multiple Z2s, multiple Z3s, or multiple Z4s at the same time, the Z0s are the same or different, the Z1s are the same or different, the Z2s are the same or different, the Z3s are the same or different or the Z4s are the same or different;
each of N1, N2, N3 and N4 independently is G, GG, GLG, GFA, GLA, or GFLG; N1, N2, N3, and N4 are the same or different, and when there are multiple N1s, multiple N2s, multiple N3s, or multiple N4s at the same time, the N1s are the same or different, the N2s are the same or different, the N3s are the same or different or the N4s are the same or different;
AC1, AC2, AC3, and AC4 are drug molecules (for example, drug molecules with anti-tumor activity); AC1, AC2, AC3 and AC4 are the same or different, and when there are multiple AC1s, multiple AC2s, multiple AC3s, or multiple AC4s at the same time, the AC1s are the same or different, the AC2s are the same or different, the AC3s are the same or different or the AC4s are the same or different.

2. The polyethylene glycol conjugated drug or a pharmaceutically acceptable salt thereof according to claim 1, **characterized in** one or more of the following:
(1) PEG1 is a single-arm, four-arm or eight-arm polyethylene glycol segment;
(2) PEG1 has a number-average molecular weight of 5k-40k, such as 5k-10k or 10k-40k;
(3) X represents 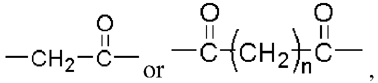 where n is 2 or 3;
(4) M represents G, GLG, or GFLG;
(5) PEG2 has a number-average molecular weight of 2k-3k, 3k-5k, 5k-10k, or 10k-40k;
(6) Y represents 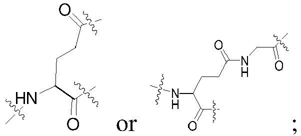
(7) Q represents 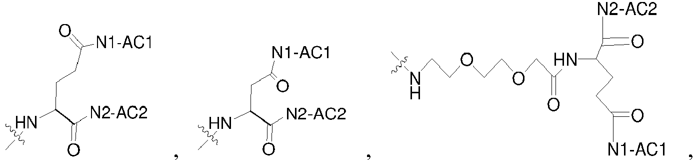 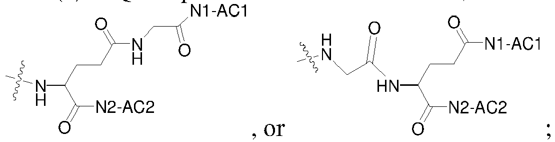 and
(8) each of AC1, AC2, AC3 and AC4 is independently selected from MK2, LPT, PCB, SB7, PKI, and NPB.

3. The polyethylene glycol conjugated drug or a pharmaceutically acceptable salt thereof according to any one of claims 1-2, wherein PEG1 is a single-arm polyethylene glycol segment with a number-average molecular weight of 5k-10k, or a four-arm polyethylene glycol segment with a number-average molecular weight of 10k-40k; X represents 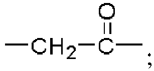 M represents G, GLG, or GFLG; PEG2 has a number-average molecular weight of 2k-3k or 3k-5k; Y represents 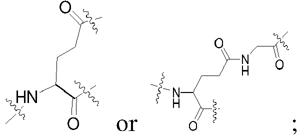
W is Q which is 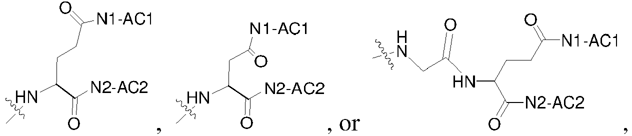 wherein N1 and N2 are both GFLG or GLG, and AC1 and AC2 are selected from:
(1) a combination in which AC1 is MK2 and AC2 is LPT,
(2) a combination in which AC1 is LPT and AC2 is SB7,
(3) a combination in which AC1 is PCB and AC2 is SB7, and
(4) a combination in which AC2 is SB7 and AC1 is PCB;
preferably, the polyethylene glycol conjugated drug is: 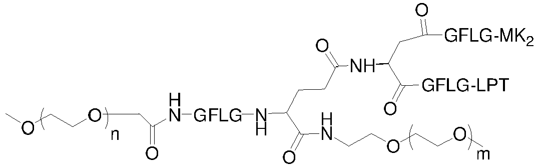
preferably, 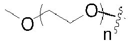 has a number-average molecular weight of 5k; preferably, 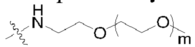 has a number-average molecular weight of 2k; 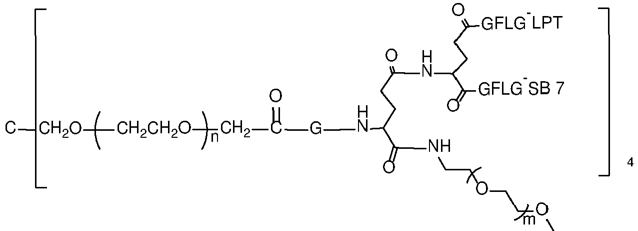
preferably, 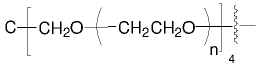 has a number-average molecular weight of 40k; preferably, 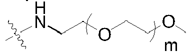 has a number-average molecular weight of 2k. 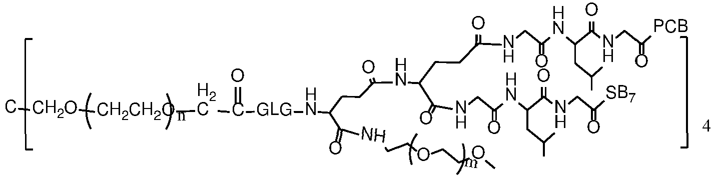
preferably, 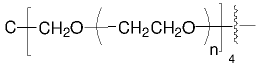 has a number-average molecular weight of 40k; preferably, 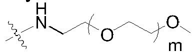 has a number-average molecular weight of 2k; 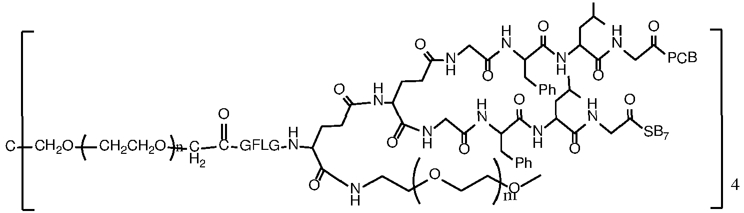
preferably, 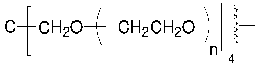 has a number-average molecular weight of 40k; preferably, 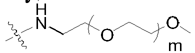 has a number-average molecular weight of 2k; or 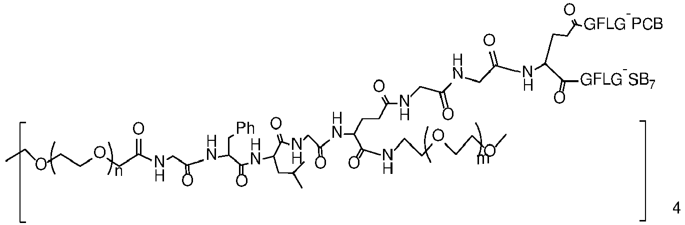
preferably, 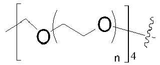 has a number-average molecular weight of 40k; preferably, 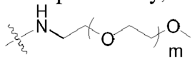 has a number-average molecular weight of 2k.

4. The polyethylene glycol conjugated drug or a pharmaceutically acceptable salt thereof according to any one of claims 1-2, wherein PEG1 is a four-arm polyethylene glycol segment with a number-average molecular weight of 10k-40k; X represents 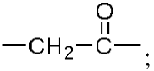 M represents GFLG; PEG2 has a number-average molecular weight of 2k-3k; Y represents 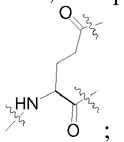
W represents 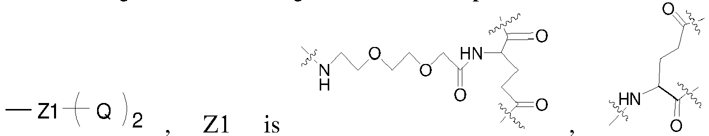 or 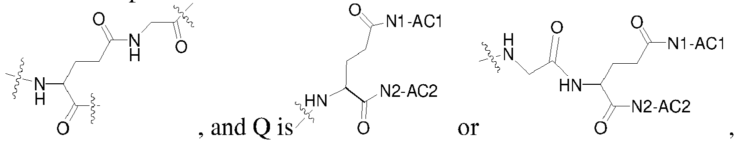 N1 and N2 are both GFLG, and AC1 and AC2 are selected from:
(1) a combination in which AC1 and AC2 are both SB7,
(2) a combination in which AC 1 and AC2 are both LPT,
(3) a combination in which AC1 is PCB and AC2 is SB7, and
(4) a combination in which AC1 and AC2 are both PCB;
preferably, the polyethylene glycol conjugated drug is: 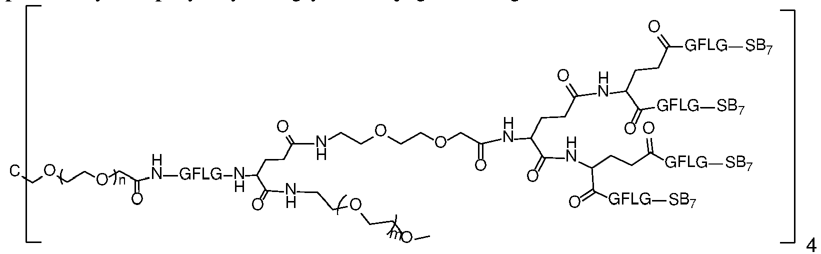
preferably, 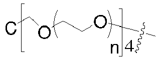 has a number-average molecular weight of 40k; preferably, 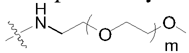 has a number-average molecular weight of 2k; 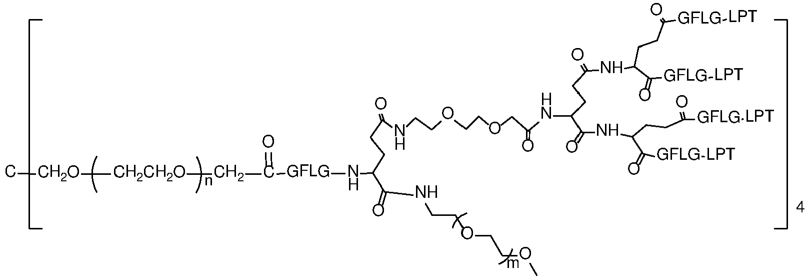
preferably, 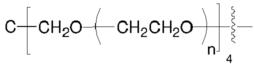 has a number-average molecular weight of 40k; preferably, 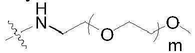 has a number-average molecular weight of 2k; 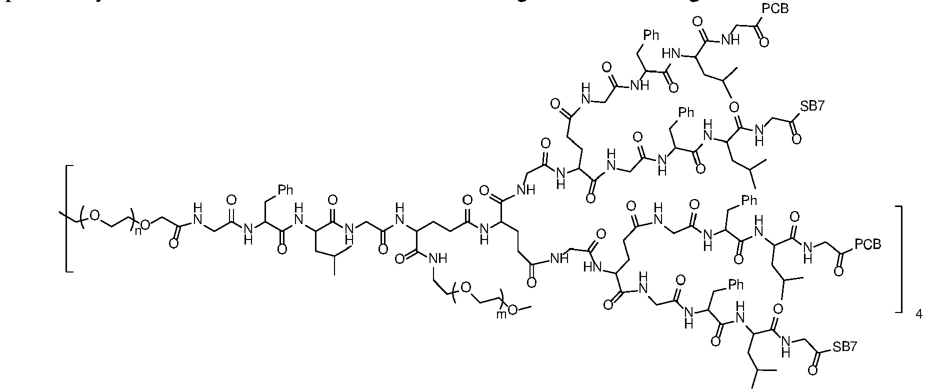
preferably, 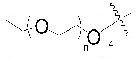 has a number-average molecular weight of 40k; preferably, 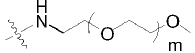 has a number-average molecular weight of 2k; or 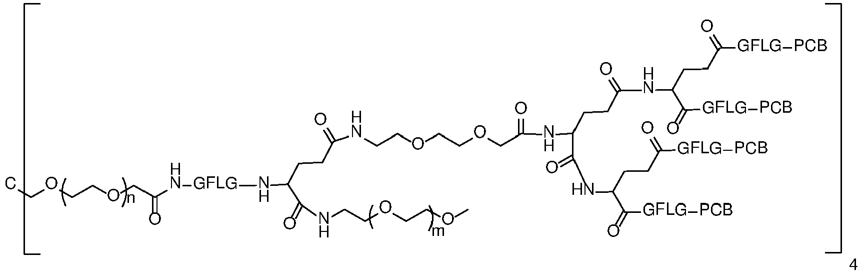
preferably, 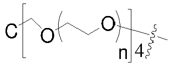 has a number-average molecular weight of 40k; preferably, 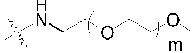 has a number-average molecular weight of 2k.

5. The polyethylene glycol conjugated drug or a pharmaceutically acceptable salt thereof according to any one of claims 1-2, wherein PEG1 is a four-arm polyethylene glycol segment with a number-average molecular weight of 10k-40k; X represents 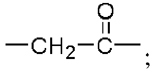 M represents GFLG; PEG2 has a number-average molecular weight of 2k-3k; Y represents 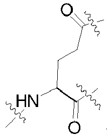 or 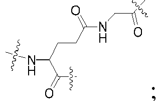
W represents 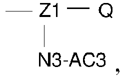 wherein Z1 is 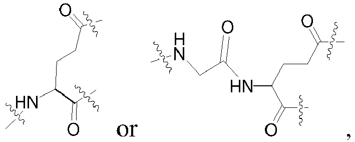 Q represents 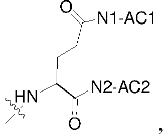 N1, N2, and N3 are all GFLG, and the combination of AC1, AC2, and AC3 is selected from:
(1) a combination in which AC1 is PCB, AC2 is SB7, and AC3 is PKI,
(2) a combination in which AC1 and AC2 are PCB and AC3 is SB7,
(3) a combination in which AC1 and AC2 are NPB and AC3 is SB7,
(4) a combination in which AC1 and AC2 are SB7 and AC3 is PCB,
preferably, the polyethylene glycol conjugated drug is: 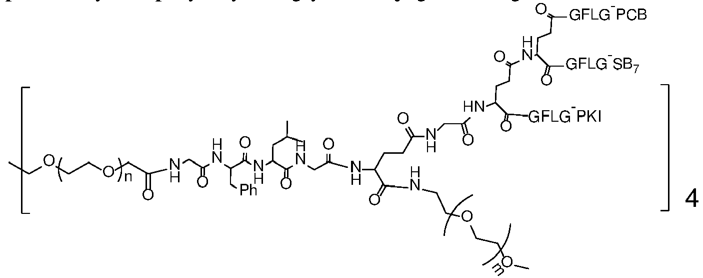
preferably, 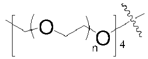 has a number-average molecular weight of 40k; preferably, 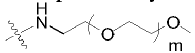 has a number-average molecular weight of 2k; 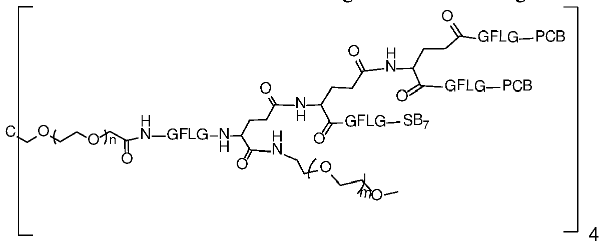
preferably, 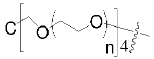 has a number-average molecular weight of 40k; preferably, 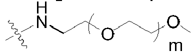 has a number-average molecular weight of 2k; 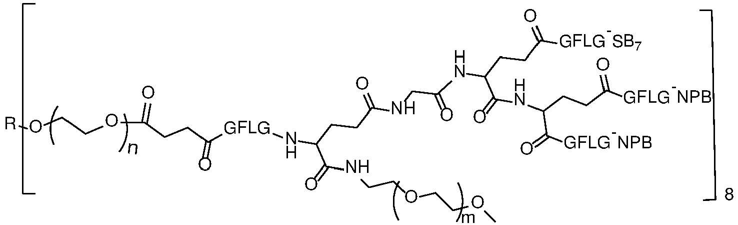
R is a core structure of eight-arm polyethylene glycol; preferably, 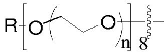 has a number-average molecular weight of 40k; preferably, 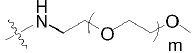 has a number-average molecular weight of 2k; or 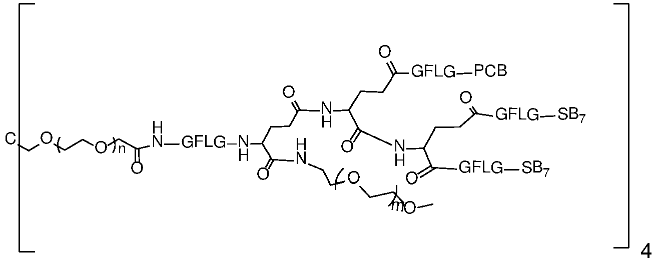
preferably, 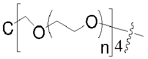 has a number-average molecular weight of 40k; preferably, 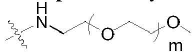 has a number-average molecular weight of 2k.

6. The polyethylene glycol conjugated drug or a pharmaceutically acceptable salt thereof according to any one of claims 1-2, wherein PEG1 is a four-arm polyethylene glycol segment with a number-average molecular weight of 10k-40k; X represents 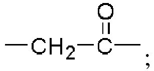 M represents GFLG; PEG2 has a number-average molecular weight of 2k-3k or 3k-5k; Y represents 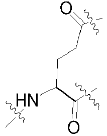
W represents 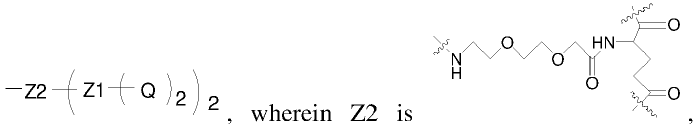 Z1 is 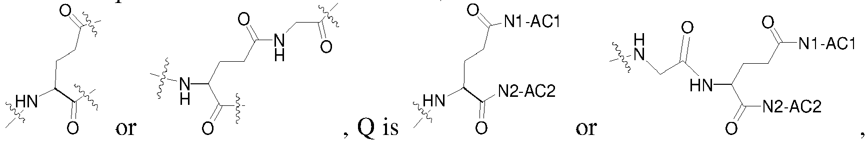 N1 and N2 are both GFLG, and AC1 and AC2 are selected from:
(1) a combination in which AC1 is LPT and AC2 is SB7, and
(2) a combination in which AC1 is PCB and AC2 is SB7:
preferably, the polyethylene glycol conjugated drug is: 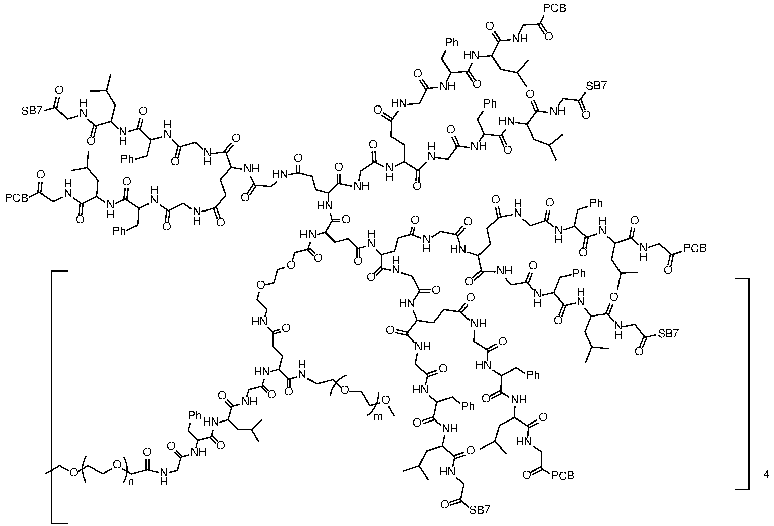
preferably, 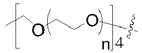 has a number-average molecular weight of 40k; preferably, 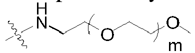 has a number-average molecular weight of 3k or 5k; or 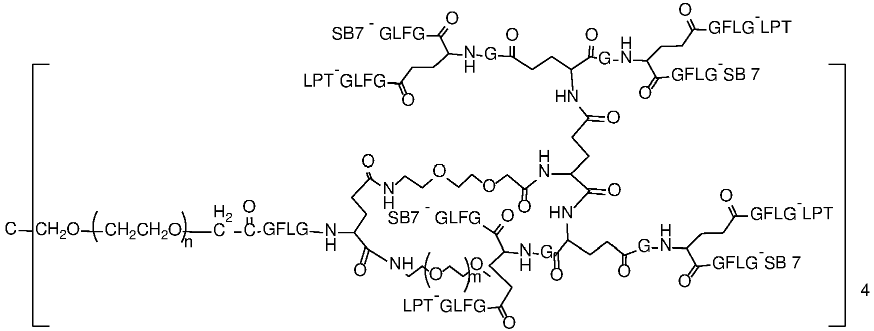
preferably, 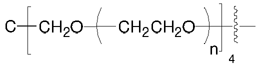 has a number-average molecular weight of 40k; preferably, 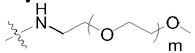 has a number-average molecular weight of 2k.

7. The polyethylene glycol conjugated drug or a pharmaceutically acceptable salt thereof according to any one of claims 1-2, wherein PEG1 is a four-arm polyethylene glycol segment with a number-average molecular weight of 10k-40k; X represents 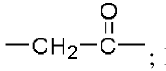 M represents GFLG; PEG2 has a number-average molecular weight of 2k-3k or 3k-5k; Y represents 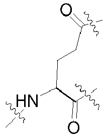
W represents 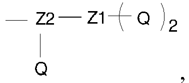 wherein Z2 is 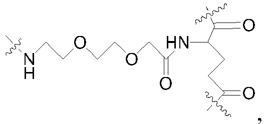 Z1 is 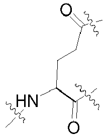 or 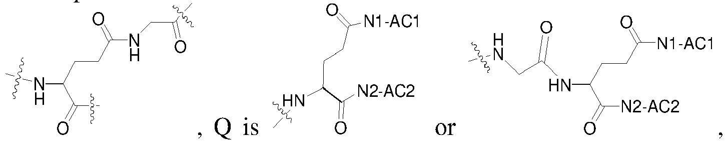 N1 and N2 are both GFLG, AC1 is PCB, and AC2 is SB7:
preferably, the polyethylene glycol conjugated drug is: 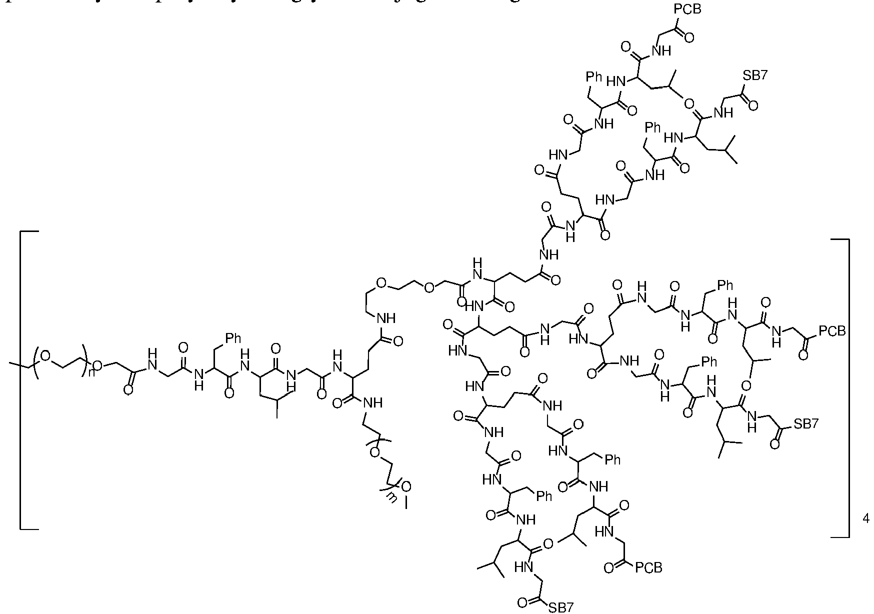
preferably, 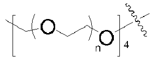 has a number-average molecular weight of 40k; preferably, 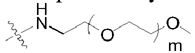 has a number-average molecular weight of 3k.

8. The polyethylene glycol conjugated drug or a pharmaceutically acceptable salt thereof according to any one of claims 1-2, wherein PEG1 is a four-arm polyethylene glycol segment with a number-average molecular weight of 10k-40k; X represents 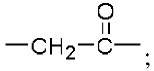 M represents GFLG; PEG2 has a number-average molecular weight of 2k-3k or 3k-5k; Y represents 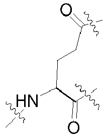
W represents 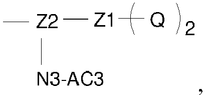 wherein Z2 is 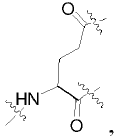 Z1 is 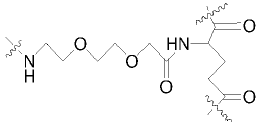 , Q is 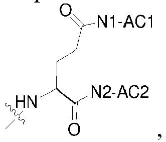 N1, N2 and N3 are all GFLG, AC1 and AC2 are both PCB, and AC3 is SB7:
preferably, the polyethylene glycol conjugated drug is: 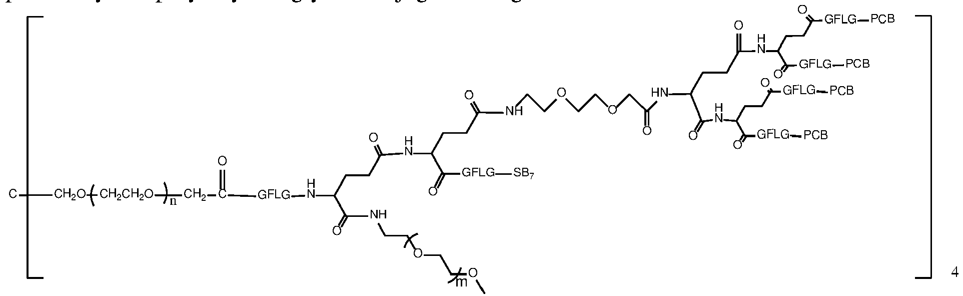
preferably, 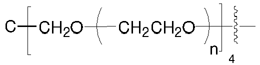 has a number-average molecular weight of 40k; preferably, 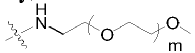 has a number-average molecular weight of 3k.

9. The polyethylene glycol conjugated drug or a pharmaceutically acceptable salt thereof according to any one of claims 1-2, wherein PEG1 is a four-arm polyethylene glycol segment with a number-average molecular weight of 10k-40k; X represents 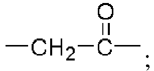 M represents GFLG; PEG2 has a number-average molecular weight of 2k-3k or 3k-5k; Y represents 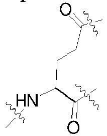
W represents 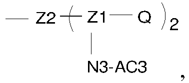 wherein Z2 and Z1 are 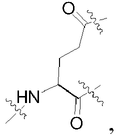 Q is 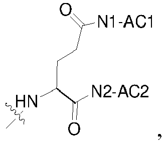 AC1 and AC2 are LPT, and AC3 is PKI;
preferably, the polyethylene glycol conjugated drug is: 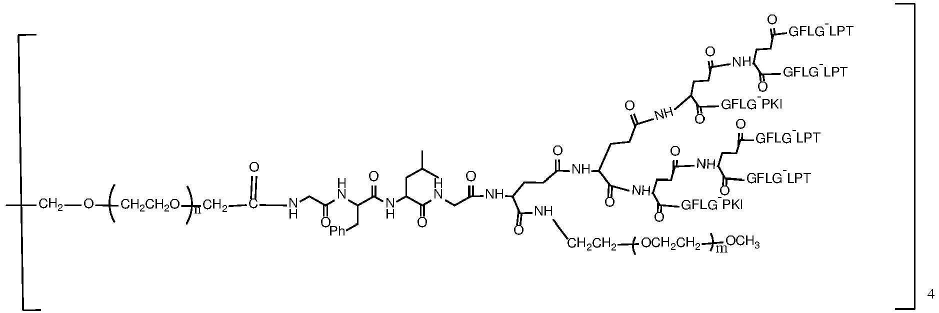
preferably, 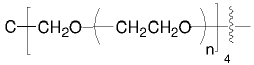 has a number-average molecular weight of 40k; preferably, 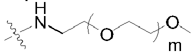 has a number-average molecular weight of 3k.

10. The polyethylene glycol conjugated drug or a pharmaceutically acceptable salt thereof according to any one of claims 1-2, wherein PEG1 is a four-arm polyethylene glycol segment with a number-average molecular weight of 10k-40k; X represents 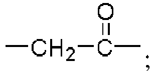 M represents GFLG; PEG2 has a number-average molecular weight of 2k-3k or 3k-5k; Y represents 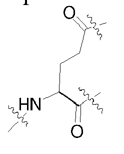 ;
W represents 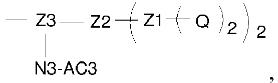 wherein Z3 is 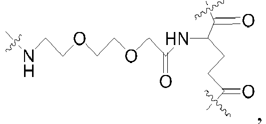 Z2 and Z1 are 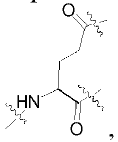 Q is 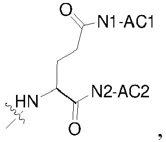 N1, N2 and N3 are GFLG, AC1 and AC2 are LPT, and AC3 is PCB;
preferably, the polyethylene glycol conjugated drug is: 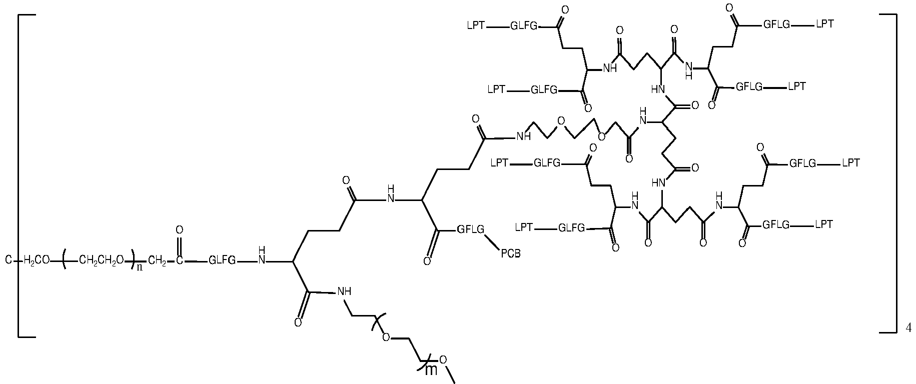
preferably, 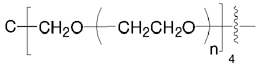 has a number-average molecular weight of 40k; preferably, 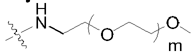 has a number-average molecular weight of 3k.

11. The polyethylene glycol conjugated drug or a pharmaceutically acceptable salt thereof according to any one of claims 1-2, wherein PEG1 is a four-arm polyethylene glycol segment with a number-average molecular weight of 10k-40k; X represents 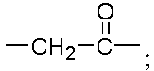 M represents GFLG; PEG2 has a number-average molecular weight of 2k-3k or 3k-5k; Y represents 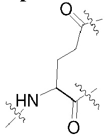
; W represents 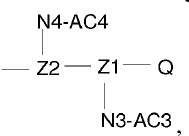 wherein Z2 is 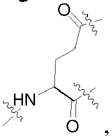 Z1 is 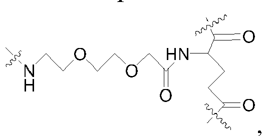 Q is 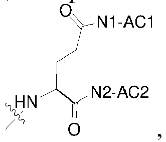 N1, N2, N3 and N4 are GFLG, AC1, AC2 and AC3 are PCB, and AC4 is SB7:
preferably, the polyethylene glycol conjugated drug is: 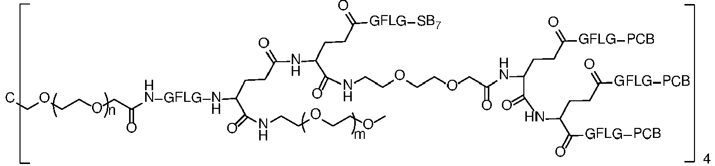
preferably, 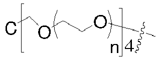 has a number-average molecular weight of 40k; preferably, 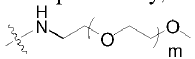 has a number-average molecular weight of 2k.

12. The polyethylene glycol conjugated drug or a pharmaceutically acceptable salt thereof according to any one of claims 1-11, wherein the polyethylene glycol conjugated drug is 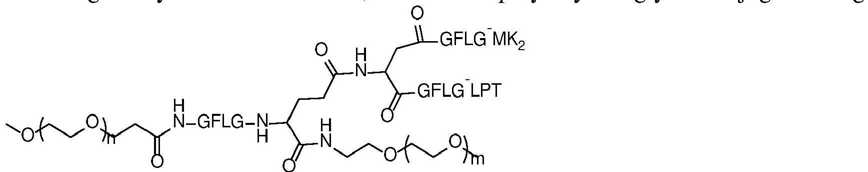
wherein 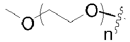 has a number-average molecular weight of 5k, and 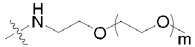 has a number-average molecular weight of 2k; 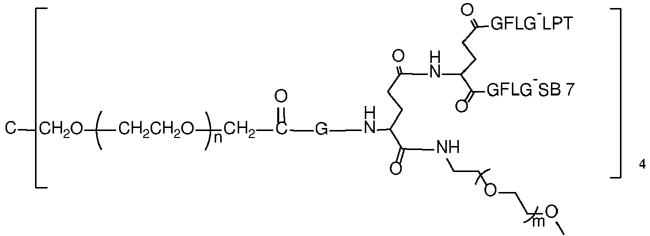
wherein 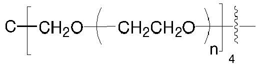 has a number-average molecular weight of 40k, and 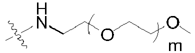 has a number-average molecular weight of 2k; 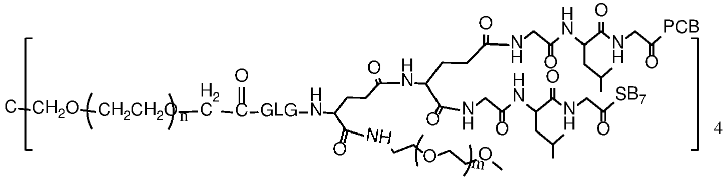
wherein 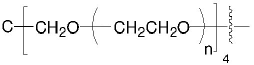 has a number-average molecular weight of 40k, and 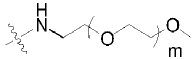 has a number-average molecular weight of 2k; 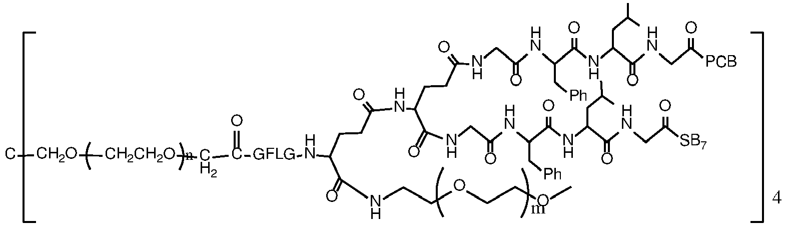
wherein 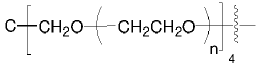 has a number-average molecular weight of 40k, and 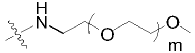 has a number-average molecular weight of 2k; 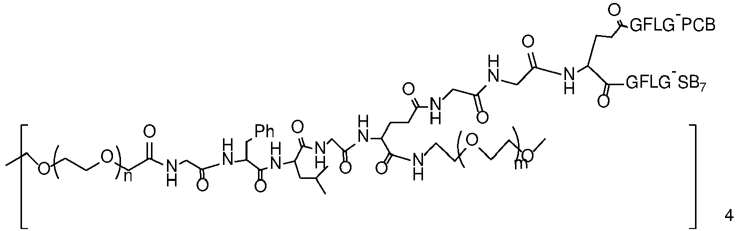
wherein 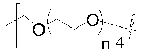 has a number-average molecular weight of 40k, and 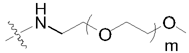 has a number-average molecular weight of 2k; 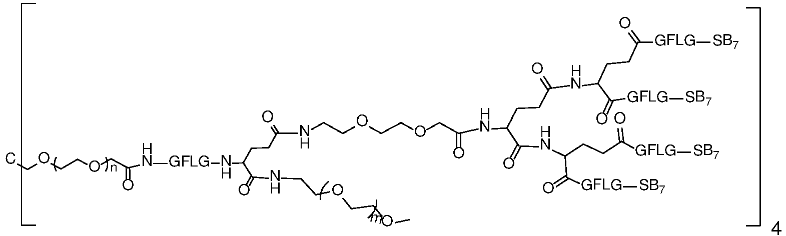
wherein 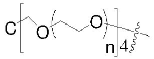 has a number-average molecular weight of 40k, and 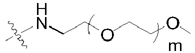 has a number-average molecular weight of 2k; 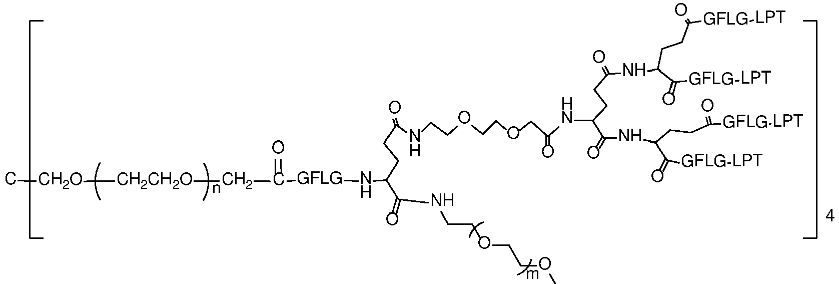
wherein 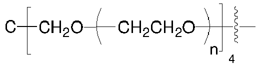 has a number-average molecular weight of 40k, and 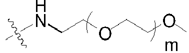 has a number-average molecular weight of 2k; 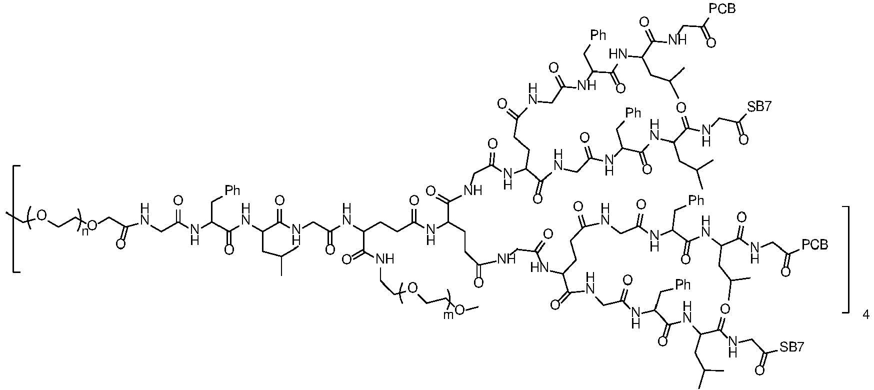
wherein 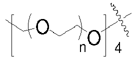 has a number-average molecular weight of 40k, and 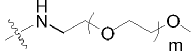 has a number-average molecular weight of 2k; 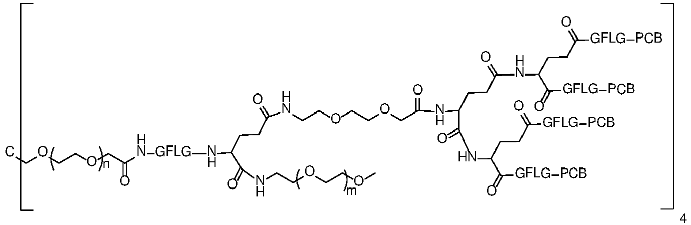
wherein 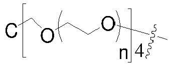 has a number-average molecular weight of 40k, and 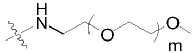 has a number-average molecular weight of 2k; 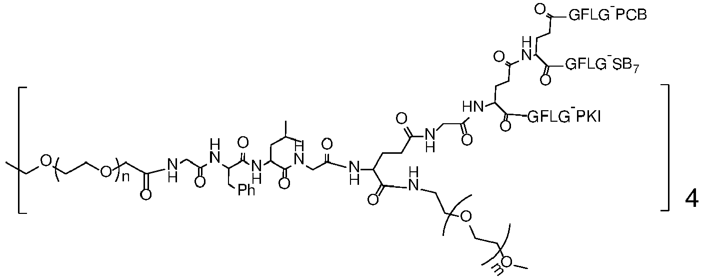
wherein 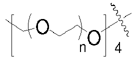 has a number-average molecular weight of 40k, and 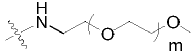 has a number-average molecular weight of 2k; 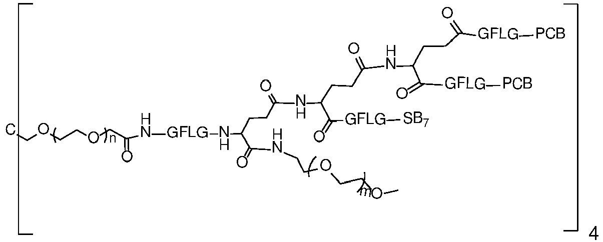
wherein 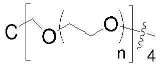 has a number-average molecular weight of 40k, and 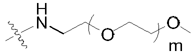 has a number-average molecular weight of 2k; 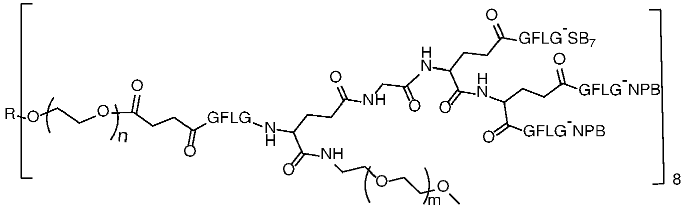
wherein R is a core structure of eight-arm polyethylene glycol; 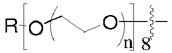 has a number-average molecular weight of 40k, and 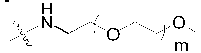 has a number-average molecular weight of 2k; 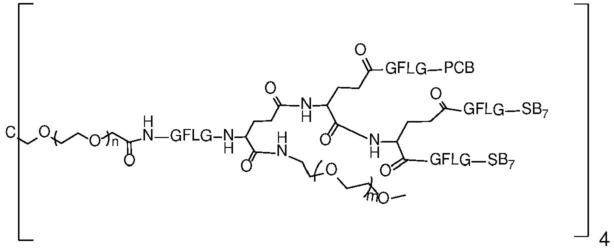
wherein 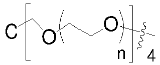 has a number-average molecular weight of 40k, and 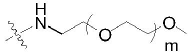 has a number-average molecular weight of 2k; 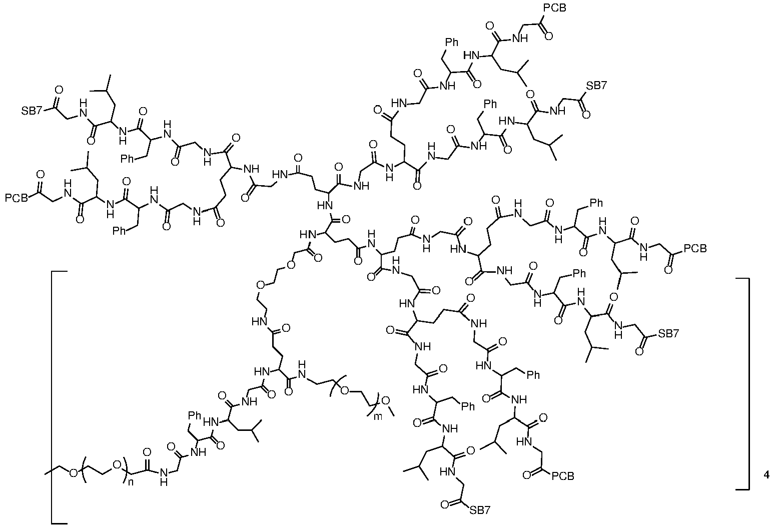
wherein 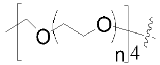 has a number-average molecular weight of 40k, and 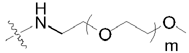 has a number-average molecular weight of 3k; 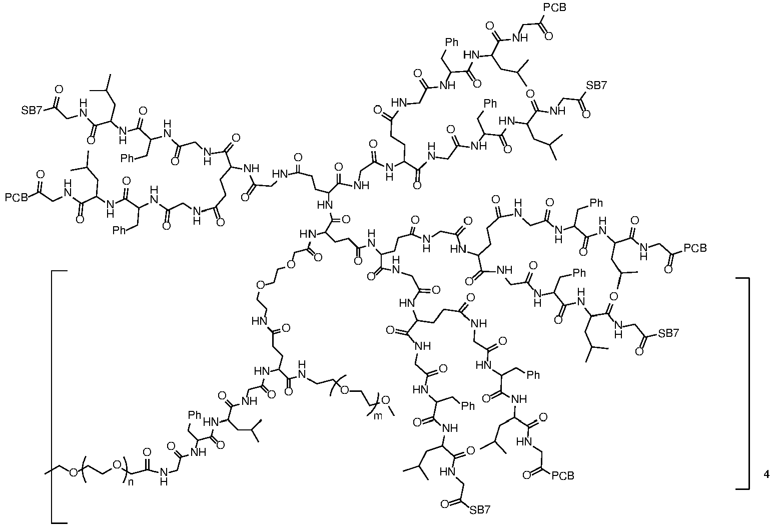
wherein 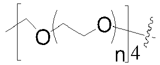 has a number-average molecular weight of 40k, and 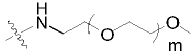 has a number-average molecular weight of 5k; 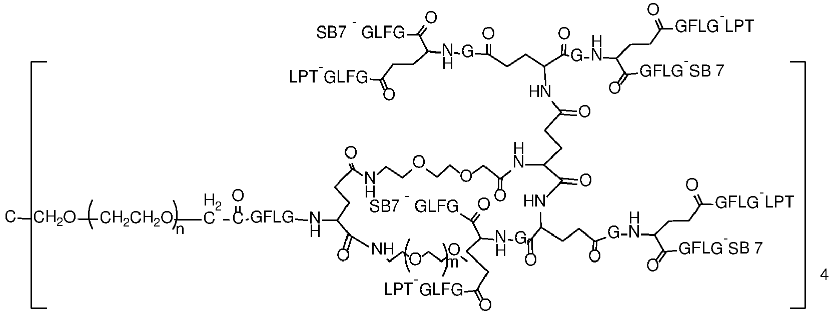
wherein 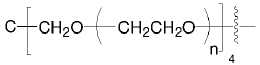 has a number-average molecular weight of 40k, and 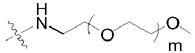 has a number-average molecular weight of 2k; 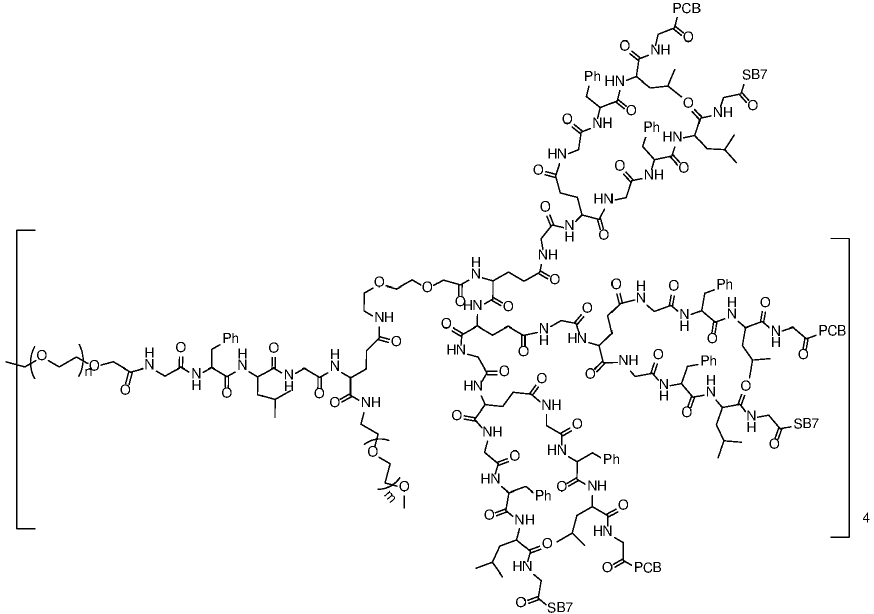
wherein 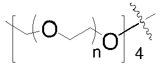 has a number-average molecular weight of 40k, and 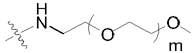 has a number-average molecular weight of 3k; 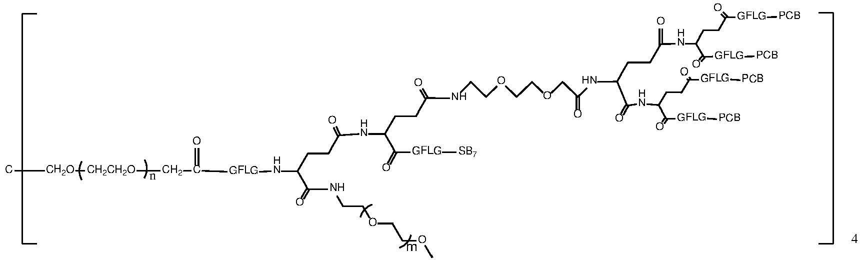
wherein 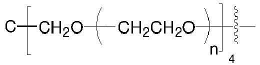 has a number-average molecular weight of 40k, and 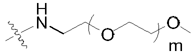 has a number-average molecular weight of 3k; 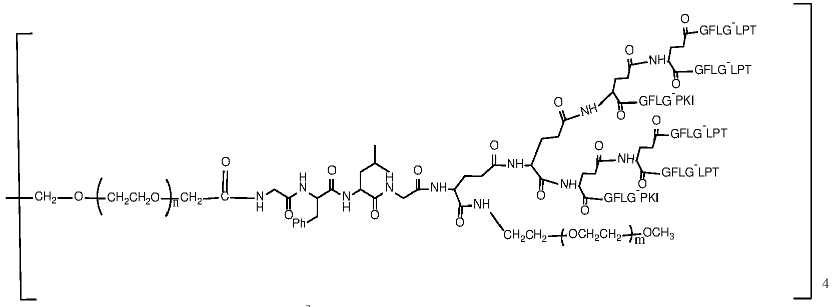
wherein 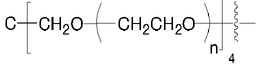 has a number-average molecular weight of 40k, and 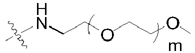 has a number-average molecular weight of 3k; 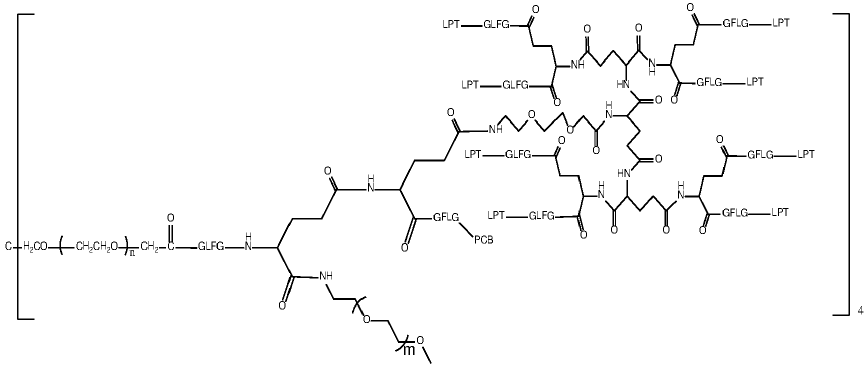
wherein 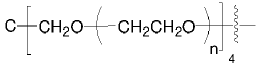 has a number-average molecular weight of 40k, and 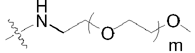 has a number-average molecular weight of 3k; or 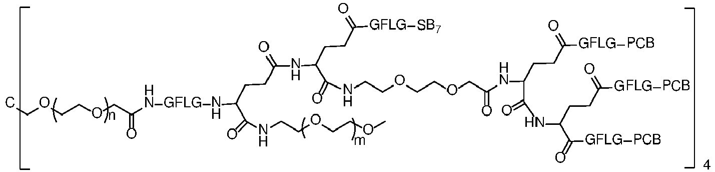
wherein 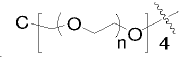 has a number-average molecular weight of 40k, and wherein 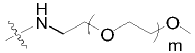 has a number-average molecular weight of 2k.

13. A method for preparing the polyethylene glycol conjugated drug or a pharmaceutically acceptable salt thereof according to any one of claims 1-12, comprising the following steps:
S1: preparing an intermediate with amino group(s) 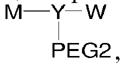 wherein at least one amino group is located on M, and
S2: carrying out an amidation reaction of PEG1 with a carboxyl group or an activated carboxyl group with the intermediate 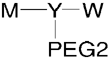 to obtain the polyethylene glycol conjugated drug according to any one of claims 1-12;
preferably, S1 comprises the following steps:
step (1): preparing W;
step (2): carrying out an amidation reaction with raw materials W and a dicarboxylic acid with an amino group to obtain an intermediate W-Y-COOH;
step (3): linking the intermediate W-Y-COOH to PEG2 with an amino group through an amidation reaction to obtain an intermediate W-Y-PEG2; and
step (4): carrying out an amidation reaction with raw materials, i.e., the intermediate W-Y-PEG2, an amino acid or a polypeptide, and a dicarboxylic acid with an amino group, to prepare an intermediate 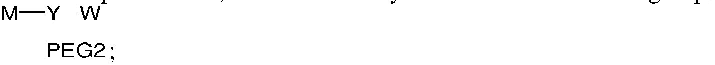
wherein PEG1, PEG2, Y, W, and M are as defined in any one of claims 1-12.

14. A method for preparing the polyethylene glycol conjugated drug or a pharmaceutically acceptable salt thereof according to any one of claims 1-12, comprising the following steps:
S1: preparing an intermediate 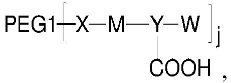 and
S2: linking PEG2 with an amino group to Y through an amidation reaction to obtain the polyethylene glycol conjugated drug according to any one of claims 1-12;
preferably, S1 comprises the following steps:
step (1): preparing W;
step (2): making W to react with a carboxyl group in a dicarboxylic acid with an amino group to obtain an intermediate W-Y-COOH;
step (3): carrying out an amidation reaction with raw materials, i.e., the intermediate W-Y-COOH and an amino acid or a polypeptide to prepare an intermediate with amino group(s) 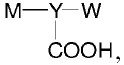 wherein at least one amino group is located on M; and step (4): carrying out an amidation reaction with raw materials, i.e., the intermediate 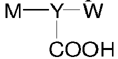 and PEG1 with a carboxyl group or an activated carboxyl group to prepare an intermediate 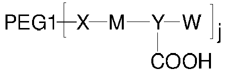
wherein PEG1, PEG2, X, Y, W, M, and j are as defined in any one of claims 1-12.

15. The method according to claim 13 or 14, wherein W in step (1) is prepared by the following method:
(1) when W is Q, the method includes: carrying out an amidation reaction with raw materials, i.e., a drug, an amino acid or a polypeptide, and a dicarboxylic acid with an amino group, to prepare Q;
(2) when W is 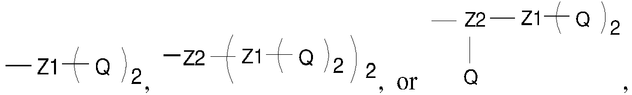 the method comprises: carrying out an amidation reaction with raw materials, i.e., a drug, an amino acid or a polypeptide, and a dicarboxylic acid with an amino group, to prepare Q; carrying out an amidation reaction with raw materials Q and a dicarboxylic acid with an amino group to prepare W;
(3) when W is 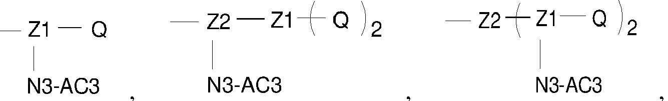 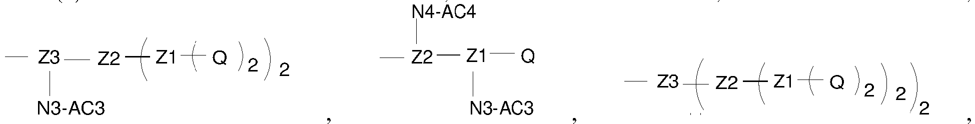 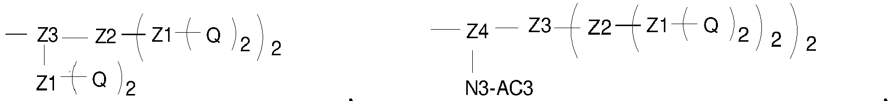 or 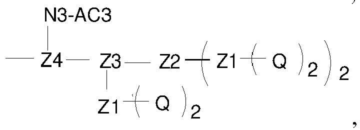 the method comprises: carrying out an amidation reaction with raw materials, i.e., a drug, an amino acid or a polypeptide, and a dicarboxylic acid with an amino group, to prepare Q; preparing N3-AC3 and optionally N4-AC4 by using a drug, and an amino acid or a polypeptide as raw materials; carrying out an amidation reaction with raw materials, i.e., Q, N3-AC3, optionally N4-AC4, and a dicarboxylic acid with an amino group, to prepare W.

16. A pharmaceutical composition, comprising a therapeutically and/or prophylactically effective amount of the polyethylene glycol conjugated drug or a pharmaceutically acceptable salt thereof according to any one of claims 1-12; preferably, the composition further comprises one or more pharmaceutically acceptable excipients;
preferably, the pharmaceutical composition is made into an injection preparation.

17. Use of the polyethylene glycol conjugated drug or a pharmaceutically acceptable salt thereof according to any one of claims 1-12 in preparation of a medicament for treating and/or preventing a disease (such as a cancer), wherein the disease refers to a disease treated by an active ingredient in the polyethylene glycol conjugated drug;
preferably, the cancer is selected from colon cancer, leukemia, lymphoma, bladder cancer, bone cancer, brain tumor, medulloblastoma, glioma, breast cancer, adenoma/carcinoid, adrenal cortical cancer, pancreatic islet cell cancer, cervical cancer, endometrial cancer, ovarian cancer, colorectal cancer, skin cancer, esophageal cancer, eye cancer, gallbladder cancer, stomach cancer, head and neck cancer, liver cancer, melanoma, Kaposi's sarcoma, kidney cancer, oral cancer, lung cancer, nasopharyngeal cancer, neuroblastoma, ovarian cancer, pancreatic cancer, thyroid cancer, parathyroid penile cancer, prostate cancer, urethral cancer, vaginal cancer, vulvar cancer, anal cancer, and sarcoma, as well as metastasis of these cancers.

18. An injection solution, comprising the polyethylene glycol conjugated drug or a pharmaceutically acceptable salt thereof according to any one of claims 1-12, or the pharmaceutical composition according to claim 16; preferably, the injection solution uses normal saline as a carrier.

19. A compound having any one of the following structures 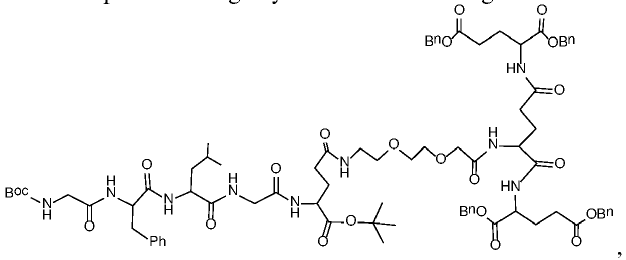 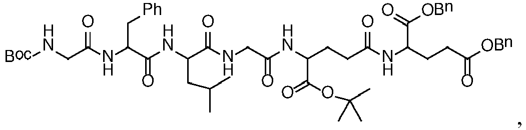 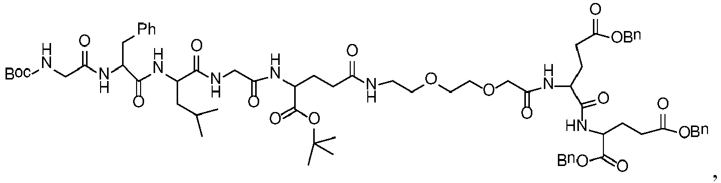 or

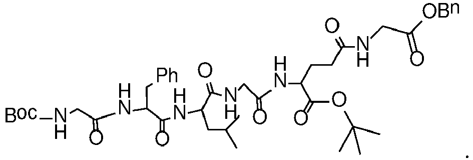

20. Use of the compound according to claim 19 in preparation of the polyethylene glycol conjugated drug or a pharmaceutically acceptable salt thereof according to any one of claims 1-12.
